(19) Europäisches Patentamt
European Patent Office
Office européen des brevets

(11) EP 2 723 865 B1

(12) **EUROPEAN PATENT SPECIFICATION**

(45) Date of publication and mention of the grant of the patent:
**27.03.2019 Bulletin 2019/13**

(21) Application number: **12802770.3**

(22) Date of filing: **21.06.2012**

(51) Int Cl.:
***C12Q 1/6883*** *(2018.01)*

(86) International application number:
**PCT/US2012/043584**

(87) International publication number:
**WO 2012/177906 (27.12.2012 Gazette 2012/52)**

# (54) METHODS FOR DETERMINING ACTIVITY OF RNAi IN A SUBJECT

VERFAHREN ZUR BESTIMMUNG DER AKTIVITÄT VON RNAi IN EINEM PATIENTEN

PROCÉDÉS DE DÉTERMINATION DE L'ACTIVITÉ DU RNAi CHEZ UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**21.06.2011 US 201161499664 P**
**12.07.2011 US 201161507093 P**
**01.09.2011 US 201161530112 P**
**12.09.2011 US 201161533737 P**

(43) Date of publication of application:
**30.04.2014 Bulletin 2014/18**

(73) Proprietor: **Alnylam Pharmaceuticals, Inc.**
**Cambridge, MA 02142 (US)**

(72) Inventors:
- **SAH, Dinah**
  **Cambridge, MA 02142 (US)**
- **BUMCROT, David**
  **Cambridge, MA 02142 (US)**
- **SEHGAL, Alfica**
  **Cambridge, MA 02142 (US)**

(74) Representative: **Vossius & Partner**
**Patentanwälte Rechtsanwälte mbB**
**Siebertstrasse 3**
**81675 München (DE)**

(56) References cited:

| | |
|---|---|
| **WO-A1-2009/015357** | **US-A1- 2005 186 591** |
| **US-A1- 2008 299 097** | **US-A1- 2009 118 370** |
| **US-A1- 2011 027 882** | **US-A1- 2011 053 157** |

- **TAYLOR D D ET AL: "MicroRNA signatures of tumor-derived exosomes as diagnostic biomarkers of ovarian cancer", GYNECOLOGIC ONCOLOGY, ACADEMIC PRESS, LONDON, GB, vol. 110, no. 1, 1 July 2008 (2008-07-01), pages 13-21, XP022795052, ISSN: 0090-8258, DOI: 10.1016/J.YGYNO.2008.04.033 [retrieved on 2008-06-25]**
- **NOBUYOSHI KOSAKA ET AL: "Circulating microRNA in body fluid: a new potential biomarker for cancer diagnosis and prognosis", CANCER SCIENCE, vol. 101, no. 10, 1 October 2010 (2010-10-01), pages 2087-2092, XP055033304, ISSN: 1347-9032, DOI: 10.1111/j.1349-7006.2010.01650.x**
- **ENRICO DE SMAELE ET AL: "MicroRNAs as biomarkers for CNS cancer and other disorders", BRAIN RESEARCH, vol. 1338, 1 June 2010 (2010-06-01), pages 100-111, XP055060322, ISSN: 0006-8993, DOI: 10.1016/j.brainres.2010.03.103**
- **MACIEJ CIESLA ET AL: "MicroRNAs as biomarkers of disease onset", ANALYTICAL AND BIOANALYTICAL CHEMISTRY, SPRINGER, BERLIN, DE, vol. 401, no. 7, 6 May 2011 (2011-05-06), pages 2051-2061, XP019953571, ISSN: 1618-2650, DOI: 10.1007/S00216-011-5001-8**
- **KEIICHI OHSHIMA ET AL: "Let-7 MicroRNA Family Is Selectively Secreted into the Extracellular Environment via Exosomes in a Metastatic Gastric Cancer Cell Line", PLOS ONE, vol. 5, no. 10, 8 October 2010 (2010-10-08), page e13247, XP055099717, ISSN: 1932-6203, DOI: 10.1371/journal.pone.0013247**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MACFARLANE D E ET AL: "ISOLATING RNA FROM CLINICAL SAMPLES WITH CATRIMOX-14 AND LITHIUM CHLORIDE", JOURNAL OF CLINICAL LABORATORY ANALYSIS, NEW YORK, NY, US, vol. 11, no. 3, 1 January 1997 (1997-01-01), pages 132-139, XP001055694, ISSN: 0887-8013, DOI: 10.1002/(SICI)1098-2825(1997)11:3<132::AID-JCLA3>3.0.CO;2-C

## Description

### Field of the Invention

The invention relates to methods for determining activity of an iRNA agent targeting transthyretin (TTR) RNA in a subject as defined by the claims.

### Background of the Invention

Double-stranded RNA molecules (dsRNA) have been shown to block gene expression in a highly conserved regulatory mechanism known as RNA interference (RNAi). dsRNA has also been shown to degrade target RNA in a variety of organisms, including worms (*e.g., C. elegans*), plants, *Drosophila,* and mammals. This natural mechanism has now become the focus for the development of a new class of pharmaceutical agents for treating disorders that are caused by the aberrant or unwanted regulation of a gene. RNAi can also be used to activate expression of a target gene in the treatment of disease. **Summary**

The present invention is defined by the claims. Accordingly the present invention relates to a method for determining the activity of an iRNA agent targeting transthyretin (TTR) RNA in a subject who was administered said iRNA agent in a tissue comprising: (a) detecting the level of the TTR RNA or a cleavage product thereof in a serum sample acquired from the subject, and, (b) comparing the level obtained in (a) to a reference sample; wherein a decreased level of the TTR RNA or an increased level of the cleavage product as compared to the reference sample is indicative that the iRNA agent is active, and an increased or static level of the TTR RNA or a decreased or static level of cleavage product as compared to the reference sample is indicative that the iRNA agent is inactive in the subject. In accordance with a preferred embodiment based on the determination that the iRNA agent is inactive in the subject the dose of iRNA agent to be administered to the subject is increased. Also in accordance with a preferred embodiment based on the determination that the iRNA agent is active in the subject the dose of iRNA agent to be administered to the subject is increased or decreased. In accordance with a yet further preferred embodiment the target RNA or RNA cleavage product is measured by a 5' RACE, hybridization, polymerase chain reaction (PCR), quantitative PCR (qPCR), branched DNA (bDNA) assay, or reverse transcription-PCR (RT-PCR). Also provided herein are methods, kits and compositions for determining activity of a nucleic acid, e.g., a double-stranded ribonucleic acid (dsRNA) in a subject. Such methods are useful for determining efficacy of a therapeutic nucleic acid, such as an RNA effector molecule (*e.g.,* dsRNA) or a DNA effector molecule (*e.g.,* a viral vector encoding a gene product), or monitoring treatment of a subject by detecting the nucleic acid, or a product of nucleic acid activity. The methods provided herein are based, in part, on the discovery that a target mRNA or mRNA cleavage product can be detected in a biological sample that is separate from the site of a dsRNA-mediated gene silencing. For example, a dsRNA can mediate gene silencing of a target mRNA in the liver and the levels of the target mRNA (or cleavage product thereof) can be detected and/or quantified in serum. By detecting mRNA levels in the serum before and after dsRNA administration, the activity of the dsRNA can be assessed or monitored. Also provided are methods for determining the level of expression of one or more exogenous genes (*e.g.*, transgenes, *e.g.*, therapeutic transgenes) delivered using gene therapy delivery methods (such as viral delivery using an adenovirus or retrovirus, naked DNA delivery *etc*.). Such methods comprise measuring the mRNA of the therapeutic or exogenous genes in a biological sample that is separate from the site of exogenous gene expression, *e.g.,* in the serum or urine.

One aspect described herein relates to a method for assaying for activity of a gene in a tissue of a a subject, where the assay includes (i) acquiring a biological sample from a subject, where the biological sample is separate from the tissue, and where the biological sample contains exosomes, and (ii) detecting levels of an RNA in the biological sample, where the RNA is expressed from the gene in the tissue of the subject, where the exosomes are not purified from the biological sample prior to detecting levels of RNA in the biological sample, where a change in the level of the RNA, or an RNA of a downstream target of the target RNA, as compared to a reference sample, is indicative of a change in activity of the gene, and where a change in activity of the gene is indicative of disease progression or recession, an effect of a modulator on the subject, or an increased or decreased risk of disease in the subject.

One aspect featured described herein relates to a method for determining activity of a therapeutic nucleic acid, *e.g.*, an RNA or DNA effector molecule or an iRNA agent, in a subject, comprising: i) acquiring a biological sample from a subject who was administered a therapeutic nucleic acid that targets or expresses a nucleic acid in a tissue that is separate from the biological sample, where the biological sample contains exosomes, and ii) detecting levels of the targeted or expressed nucleic acid in the biological sample of the subject, or a product thereof, *e.g.*, a cleavage product thereof, in the biological sample, wherein the presence of the nucleic acid, or a change in the level of the product of the nucleic acid, as compared to a reference sample is indicative that the therapeutic nucleic acid is active in the subject. In one embodiment, the exosomes are not purified from the biological sample prior to detecting the levels of the targeted or expressed nucleic acid. The therapeutic nucleic acid can be, for example, a dsRNA; an antagomir; a microRNA, *e.g.,* a microRNA mimic; or a gene replacement therapeutic agent.

The method may further comprise increasing the dose of therapeutic nucleic acid administered to the subject, and in another embodiment, the method further comprises increasing or decreasing the dose of therapeutic nucleic acid administered to the subject.

The biological sample may have undergone centrifugation prior to detecting levels of targeted or expressed nucleic acid. The centrifugation can be a high speed centrifugation such as at 100,000 g to 150,000 g, e.g., 110,000 g to 140,000 g.

The therapeutic nucleic acid may be an RNA effector molecule, such as an antagomir or an miRNA mimic, and the target RNA is mR-122, miR-16, miR-192, or miR-194. The RNA effector molecule may be a dsRNA, a microRNA, a promoter-directed RNA (pdRNA), a Piwi-interacting RNA (piRNA), an RNA activating molecule, an expressed interfering RNA (eiRNA), a short hairpin RNA (shRNA), a decoy RNA or an aptamer.

The therapeutic nucleic acid may be an antisense oligonucleotide.

The therapeutic nucleic acid may be a DNA effector molecule that expresses an exogenous DNA molecule, an expressed DNA, a dominant negative mutant, a modified gene product, a mutated gene product, a xenobiotic gene, or a transgene. The therapeutic nucleic acid may be a DNA effector molecule that comprises a viral vector or a plasmid DNA (e.g., naked DNA). The viral vector can be, for example, an adenoviral vector, a retroviral vector, a herpes simplex viral vector, an adeno-associated viral vector, or an Epstein-Barr viral vector. The therapeutic nucleic acid (e.g., naked DNA) may be administered using electroporation, sonoporation, magnetofection, direct injection, or lipid complexes.

The DNA effector molecule can include an inducible or constitutively active promoter. The DNA effector molecule may be inserted into the genome of the subject, such as randomly into the genome of the subject, or targeted to a particular site using homologous recombination.

One aspect described herein features a method for determining activity of an iRNA agent in a subject, the method comprising: i) acquiring a biological sample from a subject who was administered an iRNA agent that targets an RNA in a tissue that is separate from the biological sample, where the sample contains exosomes, and ii) detecting levels of the target RNA or a cleavage product thereof in the biological sample, where the exosomes are not purified from the biological sample prior to detecting levels of the target RNA or cleavage product thereof, where a change in the level of the target RNA or an increased level of the cleavage product as compared to a reference sample is indicative that the iRNA agent is active in the subject.

The method further may comprise increasing the dose of iRNA agent administered to the subject based on the determination that the iRNA agent is inactive, and also the method may further comprise increasing or decreasing the dose of iRNA agent administered to the subject based on the determination that the iRNA agent is active in the subject.

The biological sample may include exosomes and also a fraction of the RNA in the biological sample may be located in exosomes. Exosomes may not be purified from the biological sample prior to detecting the target RNA or cleavage product thereof. In some instances such as for assaying for RNA cleavage products, exosomes may be isolated from the biological sample prior to detecting the cleavage product.

The biological sample may have undergone centrifugation prior to detecting levels of target RNA or cleavage product. The centrifugation can be at 100,000 g to 150,000 g, e.g., 110,000 g to 140,000 g.

The biological sample may include microvesicles and also a fraction of the RNA in the biological sample may be located in microvesicles. Microvesicles may or may not be purified prior to detecting the target RNA or cleavage product thereof.

The biological sample may include extracellular vesicles (e.g., exosomes and microvesicles) and also a fraction of the RNA in the biological sample may be located in extracellular vesicles. Extracellular vesicles may or may not be purified prior to detecting the target RNA or cleavage product thereof.

The subject may be treated with an iRNA agent that targets an RNA in a tissue, such as in liver, kidney, brain, spinal cord, choroid plexus, peripheral neurons or nerve, muscle, endothelial cells, heart, immune cells, skin, eye, lung, stomach, small or large intestines, colon, adrenal gland, tumors, cancer lesions, or spleen.

The iRNA agent may target an RNA in liver. The biological sample obtained from a subject who has been administered the iRNA agent can be from, for example, a fluid such as blood, urine, cerebrospinal fluid (CSF), amniotic fluid, saliva, breast milk, bronchoalveolar lavage fluid, synovial fluid, or malignant ascites. A blood sample can be, for example, a whole blood sample (including red and white blood cells, and cell-free proteins and nucleic acids), or a plasma or serum sample.

The target RNA or RNA cleavage product isolated from a biological sample can be measured by methods known in the art, such as by 5' RACE, hybridization, polymerase chain reaction (PCR), quantitative PCR (qPCR), branched DNA (bDNA), or reverse transcription coupled to PCR (RT-PCR).

The iRNA agent may be administered to the central nervous system (CNS). For example, a subject administered an iRNA to the CNS may have a neurological disease such as, *e.g.*, Huntington's disease, Parkinson's disease, or Alzheimer's disease. For example, the subject, *e.g.*, a patient, may have Huntington's disease and the target RNA is Huntingtin (Htt) RNA. In another embodiment, the subject, *e.g.,* a patient, has Parkinson's disease and the target RNA is alpha-synuclein (SNCA) RNA. The subject *e.g.,* a patient, may have the leptomeningial/central nervous system form

of transthyretin (TTR) amyloidosis and the target RNA is TTR RNA.

A therapeutic nucleic acid, such as an RNA or DNA effector molecule or an iRNA agent, can be administered to the subject by a variety of methods, such as by direct injection, intravenous, intraperitoneal, subcutaneous, intramuscular, inhalation, topical, intracerebral including intraparenchymal, intracerebroventricular, epidural, intrathecal, intraarterial, intravitrial, intradermal, oral, or intracardiac delivery. When the organism to be treated is a mammal such as a human, the composition may be administered by any means known in the art including, but not limited to oral, intraperitoneal, or parenteral routes, including intracranial (e.g., intraventricular, intraparenchymal and intrathecal), epidural, intramuscular, intraperitoneal, intravenous, intramuscular, subcutaneous, transdermal, airway (aerosol), nasal, rectal, vaginal, and topical (including buccal and sublingual) administration.

The therapeutic nucleic acid may be administered by peripheral administration, and also the therapeutic nucleic acid may be administered directly into the CNS. The pharmaceutical compositions described herein may be administered by intravenous, intrathecal or intracranial infusion or injection. The therapeutic nucleic acid may be administered by intravenous delivery, and also the therapeutic nucleic acid may be administered by intracranial delivery.

An iRNA agent used in the methods disclosed herein can target any RNA, e.g., any mRNA or miRNA. For example, the target RNA can be proprotein convertase subtilisin/kexin type 9 (PCSK9) (GenBank Accession No. NM_174936), transthyretin (TTR) (GenBank reference NM_000371.3), vascular endothelial growth factor A (VEGF) (GenBank Accession No. NM_001025366.2), kinesin-like protein (KSP) (GenBank Accession No. NM_004523), Parotid proline-rich salivary protein Pc (PCS) (GenBank Accession No. NM_020872.1), angiopoietin-like 3 (ANGPTL3) (GenBank Accession No. NM_014495.2), apolipoprotein C-III (ApoC3) (GenBank Accession No. NM_000040.1), Hepcidin (SLC11A2) (GenBank Accession No. NM_001174129.1), egl nine homolog (EGLN) (GenBank Accession No. BC111057.1), hepatitis C Virus (HCV) (GenBank Accession No. AK367384.1), Hepatitis B Virus (HBV) (GenBank Accession No. AB602818.1), polo-like kinase 1 (PLK) (GenBank Accession No. NM_005030.3), alpha-antitrypsin (AAT) (GenBank Accession No. NM_000295.4), activated protein C, transmembrane protease, serine 6 (TMPRSS6) (GenBank Accession No. NM_153609.2), Kruppel-like factor 4 (KLF) (GenBank Accession No. DQ658241.1), B-cell CLL/lymphoma 11A (BCL11A) (GenBank Accession No. NM-022893.3), p53 (GenBank Accession No. NM_001126114.1), caspase 2 (GenBank Accession No. NM_001224.4), β-catenin (GenBank Accession No. NM_001904.3), protein kinase N3 (PKN3) (GenBank Accession No. NM_013355.3), huntingtin (HTT) (Genbank Accession No. NM_002111.6) or synuclein, alpha (SNCA) (GenBank Accession No. NM_007308.2).

A biological sample obtained from a subject, *e.g.*, a patient administered a an iRNA agent, can be isolated from the subject after the subject receives treatment with the therapeutic nucleic acid, *e.g.,* 3 hours to 28 days after the subject receives treatment with the therapeutic nucleic acid, *e.g.,* 3 hours or 6, 12, 24, 36, 48, 72, 96, 120 or 168 hours after the subject receives treatment with the iRNA agent.

The target RNA may be expressed in liver, and the target RNA level is normalized to the level of an RNA of another liver protein in the biological sample such as, *e.g.,* factor VII, albumin, or alpha-antitrypsin (AAT). Alternatively, the target RNA level is normalized to 18s RNA, GAPDH RNA or β-actin RNA. The target RNA may be expressed in smooth muscle cells and the target RNA is normalized to the level of an RNA of another smooth muscle cell associated protein such as Acta2. The target RNA may be expressed in the central nervous system and the target RNA is normalized to the level of an RNA of another central nervous system protein.

The target RNA may be an mRNA, or an miRNA. One aspect, described herein features a method for determining activity of an iRNA agent in a subject, where the assay includes (i) acquiring a biological sample from a subject who was administered an iRNA agent that targets an RNA in a tissue separate from the biological sample, and (ii) detecting levels of a cleavage product of the target RNA in the biological sample, where an increased level of the cleavage product as compared to a referenct sample is indicative that the iRNA agent is active, and a decreased or static level of cleavage product as compared to a reference sample is indicative that the iRNA agent is inactive in the subject. The cleavage product can be the result of RNA interference by the iRNA agent.

method may further comprise increasing the dose of iRNA agent administered to the subject based on the determination that the iRNA agent is inactive, and also the method may further comprised increasing or decreasing the dose of iRNA agent administered to the subject based on the determination that the iRNA agent is active in the subject.

The biological sample may have undergone centrifugation prior to detecting levels of target RNA or cleavage product. The centrifugation can be at 100,000 g to 150,000 g, e.g., 110,000 g to 140,000 g.

The biological sample may contain exosomes, microvesicles or a combination thereof, and also a fraction of the target RNA cleavage product in the biological sample may be in the exosomes and/or microvesicles.

The exosomes or microvesicles are purified prior to detecting the cleavage product, and in other embodiments, the exosomes or microvesicles are not purified prior to detecting the cleavage product.

Exosomes derived from the tissue, such as from the liver, may be selected for, such as by a tissue-specific marker, prior to detecting the cleavage product.

The cleavage product may be detected by 5'RACE, hybridization, polymerase chain reaction (PCR), quantitative PCR (qPCR), branched DNA (bDNA) assay, or reverse transcription-PCR (RT-PCR).

The biological sample may be blood or CSF, and also the iRNA agent targets an RNA expressed in liver or CNS.

One described herein features an assay for determining activity of an iRNA agent in a subject, where the assay includes (i) contacting a biological sample from a subject who was administered an iRNA agent that targets an RNA in a tissue separate from the biological sample, with a probe for detecting the amount of the target RNA or cleavage product thereof, where the biological sample contains exosomes, and (ii) detecting the amount of target RNA or cleavage product thereof in the biological sample, where the exosomes are not purified from the biological sample prior to measuring the amount of target RNA or cleavage product thereof, (iii) comparing the amount of target RNA or cleavage product thereof with a reference sample, where a decrease in the amount of target RNA or an increased amount of the cleavage product as compared to the reference sample is indicative of iRNA agent activity in the subject. The cleavage product can be the result of RNA interference by the iRNA agent.

The biological sample may be blood or CSF, and in certain embodiments, the iRNA agent targets an RNA expressed in liver or CNS.

The method may further comprise increasing the dose of iRNA agent administered to the subject based on the determination that the iRNA agent is inactive, and also the method may further comprise increasing or decreasing the dose of iRNA agent administered to the subject based on the determination that the iRNA agent is active in the subject.

The biological sample may have undergone centrifugation prior to detecting levels of target RNA or cleavage product. The centrifugation can be at 100,000 g to 150,000 g, e.g., 110,000 g to 140,000 g.

One aspect, described herein features an assay for determining activity of an iRNA agent in a subject, where the assay includes (i) contacting a biological sample from a subject who was administered an iRNA agent with a probe for detecting the amount of cleavage product of a target RNA, where the biological sample contains exosomes, and (ii) detecting the amount of cleavage product in the biological sample, and (iii) comparing the amount of cleavage product with a reference sample, where a change in the amount of cleavage product as compared to the reference sample is indicative of iRNA agent activity in the subject. The cleavage product can be the result of RNA interference by the iRNA agent. The reference sample can be an RNA that is not targeted by the iRNA agent, e.g., 18s RNA or GADPH RNA.

The biological sample may be blood or CSF, and also the iRNA agent may target an RNA expressed in liver or CNS.

The method may further comprised increasing the dose of iRNA agent administered to the subject based on the determination that the iRNA agent is inactive, and also the method may further comprise increasing or decreasing the dose of iRNA agent administered to the subject based on the determination that the iRNA agent is active in the subject.

The biological sample may have undergone centrifugation prior to detecting levels of target RNA or cleavage product. The centrifugation can be at 100,000 g to 150,000 g, e.g., 110,000 g to 140,000 g.

One aspect described herein features a method for determining activity of a modulator in a subject, where the assay includes (i) acquiring a biological sample from a subject who was administered a modulator that targets a protein or RNA in a tissue that is separate from the biological sample, where the target protein or RNA is selected from the group consisting of proprotein convertase subtilisin/kexin type 9 (PCSK9), transthyretin (TTR), vascular endothelial growth factor (VEGF), kinesin-like protein (KSP), Parotid proline-rich salivary protein Pc (PCS), angiopoetin-like 3 (ANGPTL3), Hepcidin, hepatitis C virus (HCV), egl nine homolog (EGLN), hepatitis B virus (HBV), polo-like kinase 1 (PLK), alpha-antitrypsin (AAT), activated protein C (APC), transmembrane protease, serine 6 (TMPRSS6), Kruppel-like factor 4 (KLF), B-cell CLL/lymphoma 11A (BCL11A), p53, caspase 2, β-catenin, protein kinase N3 (PKN3), huntingtin (HTT), and synuclein, alpha (SNCA), and wherein the biological sample comprises exosomes, and (ii) detecting the level of the target RNA, an RNA downstream of the target RNA or a cleavage product of the target RNA in the biological sample, where the exosomes are not purified from the biological sample prior to detecting the amount of the target RNA, or downstream RNA or a cleavage product of the target RNA, where detecting a change in the level of target RNA, an RNA downstream of the target RNA or a cleavage product of the target RNA as compared to the reference sample is indicative that the modulator is active in the subject.

The biological sample may be blood or CSF, and in certain embodiments, the iRNA agent targets an RNA expressed in liver or CNS.

method further comprise increasing the dose of the modulator administered to the subject based on the determination that the modulator is inactive, and also the method may further comprise increasing or decreasing the dose of the modulator administered to the subject based on the determination that the modulator is active in the subject.

The biological sample may have undergone centrifugation prior to detecting levels of target RNA or cleavage product. The centrifugation can be at 100,000 g to 150,000 g, e.g., 110,000 g to 140,000 g.

One aspect, described herein features a method for determining activity of a modulator in a subject, where the assay includes (i) acquiring a biological sample from a subject who was administered a modulator that targets a protein or RNA in a tissue that is separate from the biological sample, where the target protein or RNA is selected from the group consisting of proprotein convertase subtilisin/kexin type 9 (PCSK9), transthyretin (TTR), vascular endothelial growth factor (VEGF), kinesin-like protein (KSP), Parotid proline-rich salivary protein Pc (PCS), angiopoetin-like 3 (ANGPTL3), Hepcidin, hepatitis C virus (HCV), egl nine homolog (EGLN), hepatitis B virus (HBV), polo-like kinase 1

(PLK), alpha-antitrypsin (AAT), activated protein C (APC), transmembrane protease, serine 6 (TMPRSS6), Kruppel-like factor 4 (KLF), B-cell CLL/lymphoma 11A (BCL11A), p53, caspase 2, β-catenin, protein kinase N3 (PKN3), huntingtin (HTT), and synuclein, alpha (SNCA), and (ii) detecting the amount of cleavage product of the target RNA in the biological sample, where detecting a change in the amount of cleavage product of the target RNA as compared to the reference sample is indicative that the modulator is active in the subject.

The biological sample may be blood or CSF, and also the iRNA agent may be target an RNA expressed in liver or CNS.

The method further comprised increasing the dose of the modulator administered to the subject based on the determination that the modulator is inactive, and also the method may further comprise increasing or decreasing the dose of the modulator administered to the subject based on the determination that the modulator is active in the subject.

The biological sample may have undergone centrifugation prior to detecting the amount of cleavage product. The centrifugation can be at 100,000 g to 150,000 g, e.g., 110,000 g to 140,000 g.

In one aspect, an assay is disclosed for determining activity of a therapeutic nucleic acid in a subject. One such assay includes, for example: i) contacting a biological sample obtained from a subject who was administered a therapeutic nucleic acid with a probe for detecting activity of the nucleic acid; ii) measuring the activity level; and iii) comparing the activity level with the activity level observed in a reference sample. The measure of activity level is indicative of the activity of the nucleic acid in the subject. The probe can be, for example, a primer or set of primers that hybridizes to and, optionally, amplifies a target RNA, or the probe can be, for example, an antibody that measure a protein product of a target RNA or a gene therapy.

The therapeutic nucleic acid may be an iRNA agent, and the activity level is assayed by measuring the amount of target RNA in the biological sample, or the amount of target RNA cleavage product in the biological sample. The therapeutic nucleic acid may be an antagomir, and the activity level of the antagomir is assayed by measuring the amount of target miRNA in the biological sample. The therapeutic nucleic acid may also be a microRNA mimic, and the activity level of the miRNA mimic is assayed by measuring the amount of target RNA in the biological sample. The therapeutic nucleic acid may be a gene therapy, and activity of the gene therapy may be assayed by detecting expression of the gene product, e.g., an mRNA or protein product, in the biological sample.

For example, an assay is disclosed for determining activity of an iRNA agent in a subject. One such assay includes, for example: i) contacting a biological sample obtained from a subject who was administered an iRNA agent with a probe for detecting the amount of the target RNA, ii) measuring the amount of the target RNA or a cleavage product thereof, and iii) comparing the amount of the target RNA or a cleavage product thereof measured in step (ii) with a reference sample. A change in the amount of the target RNA or an increased amount of the cleavage product compared to the reference sample is indicative of iRNA agent activity in the subject.

Also disclosed herein are methods for determining activity of a modulator in a subject. One such method includes, for example: i) providing a biological sample from a subject who was administered a modulator, such as a modulator that targets a protein or RNA in a tissue, where the tissue is separate from the biological sample, and ii) detecting levels of the target RNA or a cleavage product thereof, or an RNA of a downstream target of the protein encoded by the target RNA in the biological sample. A change in the level of the target RNA or cleavage product thereof, or an RNA of a downstream target of the protein encoded by the target RNA, as compared to a reference sample, is indicative that the modulator is active in the subject. The target RNA can be, for example, proprotein convertase subtilisin/kexin type 9 (PCSK9), transthyretin (TTR), vascular endothelial growth factor A (VEGF), kinesin-like protein (KSP), parotid proline-rich salivary protein Pc (PCS), Hepcidin (SLC11A2), egl nine homolog (EGLN), hepatitis C virus (HCV), hepatitis B virus (HBV), polo-like kinase 1 (PLK), alpha-antitrypsin (AAT), activated protein C (APC), transmembrane protease, serine 6 (TMPRSS6), Kruppel-like factor 4 (KLF), B-cell CLL/lymphoma 11A (BCL11A), p53, caspase 2, β-catenin, protein kinase N3 (PKN3) huntingtin (HTT), or synuclein (SNCA).

A modulator can target a protein, or an RNA that expresses a protein. A modulator can inhibit or activate expression of the desired protein at the mRNA or protein level. A modulator can be, for example, a small molecule, a protein, a peptide, an antibody, a Fab fragment, an scFv fragment, an aptamer, a dsRNA, short hairpin RNA, an antisense oligonucleotide, a ribozyme, an antagomir, a microRNA mimic or a gene therapy.

Another aspect disclosed herein is a method for detecting a target RNA, which includes, for example: i) providing a biological sample from a subject administered a modulator, and ii) detecting levels of a target RNA or a cleavage product thereof in the biological sample. A change in the level of the target RNA, or a cleavage product thereof, or an RNA of a downstream target of the protein encoded by the target RNA, as compared to a reference sample is indicative that the modulator is active in the subject. The target can be for example, proprotein convertase subtilisin/kexin type 9 (PCSK9), transthyretin (TTR), vascular endothelial growth factor A (VEGF), kinesin-like protein (KSP), parotid proline-rich salivary protein Pc (PCS), Hepcidin (SLC11A2), egl nine homolog (EGLN), hepatitis C virus (HCV), hepatitis B virus (HBV), polo-like kinase 1 (PLK), alpha-antitrypsin (AAT), activated protein C (APC), transmembrane protease, serine 6 (TMPRSS6), Kruppel-like factor 4 (KLF), B-cell CLL/lymphoma 11A (BCL11A), p53, caspase 2, β-catenin, protein kinase N3 (PKN3) huntingtin (HTT), or synuclein (SNCA).

Also disclosed herein is a composition comprising an exosome-enriched biological sample from a subject who was administered an iRNA agent. The exosome can contain, for example, a cleavage product resulting from activity of an iRNA agent or target mRNA.

Also provided are kits for performing the methods described herein. The kit may include one or more primers and at least one additional reagent for performing an assay to detect a target RNA or cleavage product thereof in a biological sample of a subject. The target RNA can be an mRNA, e.g., when the therapeutic agent is a dsRNA or a gene therapy or an miRNA mimic, or the target RNA can be an miRNA, such as when the therapeutic RNA is an antagomir.

The kit can further include instructions for use, and the instructions may require that the subject has been administered an iRNA agent.

The kit may further include reagents for isolating the RNA or cleavage product from a biological sample from the subject. The kit can also include a column, beads, and one or more buffers or reagents. One or more primers, such as primers that hybridize to the target mRNA, may also be included in a kit. The primers can include an oligo-dT primer or gene specific primer or random hexamer for reverse transcription and optionally a forward and a reverse primer for PCR. one or more primers may be for 5' RACE.

The kit may include one or more reagents to increase RNA yield such as lithium chloride (LiCl) or sodium acetate. The LiCl can be included at a concentration ready for use, such as at a concentration of 0.1 to 5M, *e.g.,* 1M, or the LiCl can be provided in a concentrated form. The reagent for isolating the RNA or cleavage product may be TRIzol™ reagent, chloroform, phenol/chloroform, or a combination thereof. A kit can also include a reagent to enhance precipitation of RNA, *e.g.*, glycogen or polyethylene glycol (PEG). The kit may include a reagent that inhibits RNAse activity, such as an RNAse inhibitor or EDTA. The kit may include one or more tools for isolating the biological sample from the subject. The kit may include some reagents (detergents) to increase RNA yield from biological sample.

The kit may include an antibody, and/or antibody detection reagents, such as to monitor activity of a gene therapy or dsRNA.

Administration of the inhibitory RNA effector molecule may reduce at least one symptom of disease by at least 5%, *e.g.,* by at least 10%, by at least 15%, at least 20%, at least 25%, at least 30%, at least 40%, at least 50%, or at least 60%, or more. At least one symptom of the disease to be treated may be decreased by at least 5%, at least 10%, at least 15%, at least 20%, at least 25%, at least 30%, at least 40%, at least 50%, or at least 60%, or more, as compared to pretreatment levels. The decrease in at least one symptom of disease may be maintained for at least 5, 10, 20, 30, or 40 days or longer.

An iRNA as described herein may target a wildtype target RNA transcript, and in another embodiment, the iRNA targets a mutant transcript. For example, an iRNA can target a polymorphic variant, such as a single nucleotide polymorphism (SNP). The iRNA may target both a wildtype and a mutant transcript. Also the iRNA may target a transcript variant.

An iRNA featured herein may target a non-coding region of a target RNA transcript, such as the 5' or 3' untranslated region.

Also described herein is pharmaceutical composition for inhibiting the expression of a target gene in an organism, generally a human subject. The composition typically includes one or more of the iRNAs described herein and a pharmaceutically acceptable carrier or delivery vehicle. The composition may be used for treating a disorder.

The pharmaceutical composition may be formulated for administration of a dosage regimen described herein, e.g., not more than once every two months, not more than once per month, not more than twice per month, not more than once every four weeks, not more than once every three weeks, not more than once every two weeks, or not more than once every week. Administration of the pharmaceutical composition can be maintained for a month or longer, e.g., one, two, three, or six months, or one year, or five years, or ten years, or longer, including the remaining lifetime of a subject.

The pharmaceutical composition may be formulated for continuous administration into the CNS (*e.g.*, continuous infusion) for at least 5 minutes; in other embodiments, the composition is administered by continuous infusion into the CNS for at least 10 min, at least 20 min, at least 30 min, at least 40 min, at least 50 min, at least 60 min, at least 90 min, at least 2 hours, at least 3 hours, at least 4 hours, at least 5 hours, at least 6 hours, at least 7 hours, at least 8 hours, at least 12 hours, at least 24 hours, at least 36 hours, at least 48 hours or more.

A composition containing an iRNA described herein, *e.g.,* a dsRNA targeting a desired gene, may be administered with a non-iRNA therapeutic agent, such as an agent known to treat the disorder to be targeted with the iRNA agent.

An iRNA may be administered to a patient, and then the non iRNA agent is administered to the patient (or vice versa). An iRNA and the non-iRNA therapeutic agent may be administered at the same time.

The iRNA or dsRNA administered to a subject can inhibit gene expression in any tissue where gene silencing is desired. For example, the iRNA can target RNA in liver, kidney, brain, spinal cord, choroid plexus, peripheral neurons or nerve, muscle, endothelial cells, heart, immune cells, adipocytes, bladder, skin, eye, pancreas, lung, stomach, small or large intestines, colon, adrenal gland, tumors, cancer lesions, or spleen. In one embodiment, the iRNA administered to the subject inhibits gene expression in a liver cell, such as a hepatocyte, stellate cell, or a Kupffer cell. In another embodiment, the iRNA administered to the subject activates gene expression in a liver cell.

Also provided herein is a method for assaying for activity of a gene in a tissue of a subject by, for example, providing a biological sample from the subject, wherein the biological sample is separate from the tissue, and detecting the level of an RNA in the biological sample, where the RNA is expressed from the gene in the tissue of the subject. A change in the level of the RNA, or an RNA of a downstream target of the target RNA, as compared to a reference sample, is indicative of a change in activity of the gene, and a change in activity of the gene is indicative of, for example, disease progression or recession, an effect of a modulator on the subject, or an increased or decreased risk of disease in the subject. The methods provided herein are useful for, *e.g.,* diagnostic, prognostic, and pharmacogenetic purposes, and the like.

One aspect, described herein features a method of treating a subject by, for example, acquiring knowledge of activity of an iRNA agent in a subject, such as by detecting levels of a target RNA or a cleavage product thereof in a biological sample, where the biological sample comprises exosomes, and wherein the iRNA agent targets an RNA in a tissue separate from the biological sample, and where a change in the level of the targeted or expressed RNA as compared to a reference sample is indicative that the iRNA agent is active in the subject, and administering to the subject an effective amount of iRNA agent, wherein the effective amount is determined on the basis of the knowledge of the activity of the iRNA agent in the subject.

The method may further included increasing the dose of iRNA agent administered to the subject based on the determination that the iRNA agent is inactive in the subject, and also the method may further include increasing or decreasing the dose of iRNA agent administered to the subject based on the determination that the iRNA agent is active in the subject.

The biological sample may have undergone centrifugation prior to detecting levels of cleavage product, such as at 100,000 g to 150,000 g, e.g., about 110,000 g to about 140,000 g.

The biological sample may be blood or CSF, and also iRNA agent may target an RNA expressed in liver or the CNS. The target RNA may be TTR and the subject treated for transthyretin (TTR) amyloidosis.One

One aspect, described herein features a method for evaluating the activity of a target gene in a tissue of a subject, where the method includes (i) acquiring a non-biopsy sample comprising exosomes from the subject, and (ii) detecting the level of an expression product of said target gene in the sample. The method may include detecting the level of a mRNA cleavage product of mRNA for the target gene. The target gene may be the TTR gene, and also the exosomes may be not purified from the non-biopsy sample prior to detecting levels of RNA in the non-biopsy sample.

The method further include (iii) determining that activity of a gene has changed when the level of target RNA or a downstream RNA is increased or decreased, or the level of target RNA cleavage product is increased or decreased, as compared to a reference sample.

The method may further include (iv) determining that a disease has progressed or regressed, or the subject's risk of disease has worsened or improved if the activity of the gene is determined to be changed.

The non-biopsy sample may be from a subject who was administered an iRNA agent that targets an RNA in the tissue. The method can include, for example, administering to the subject, an iRNA agent that targets the RNA.

The administration may be performed prior to acquiring the sample, and in another embodiment, the administration is performed after acquiring the sample. In one embodiment, the iRNA agent is determined to be inactive, and the dose of iRNA agent administered to the subject is increased, and also the iRNA agent may be determined to be active, and the dose of iRNA agent is adjusted to increase or decrease activity of the iRNA agent in the subject.

The biological sample may include microvesicles as well as exosomes, and a fraction of the RNA is in the microvesicles.

A infraction of the RNA may be in the exosomes.

The biological sample may have undergone centrifugation prior to detecting levels of target RNA or cleavage product, and also the centrifugation may be performed at 100,000 g to 150,000 g, e.g., 110,000 g to 140,000 g.

The iRNA agent may target an RNA in liver, kidney, brain, spinal cord, choroid plexus, peripheral neurons or nerve, muscle, endothelial cells, heart, immune cells, skin, eye, pancreas, lung, stomach, small or large intestines, colon, adrenal gland, tumors, cancer lesions, or spleen.

The iRNA agent may target an RNA in liver, and in another embodiment, the iRNA agent targets an RNA expressed in the central nervous system.

The biological sample may be selected from the group consisting of: blood, urine, cerebrospinal fluid (CSF), amniotic fluid, saliva, breast milk, bronchoalveolar lavage fluid, synovial fluid, and malignant ascites. In one embodiment, the biological sample is a serum sample.

Also disclosed herein, are methods and assays for detecting gene delivery of, *e.g*., an exogenous gene such as a therapeutic exogenous gene, in a subject. For example, providing a biological sample from the subject, wherein the biological sample is separate from the target tissue for exogenous gene expression, and detecting the presence and/or the level of the RNA in the biological sample, wherein the RNA is expressed from the exogenous gene delivered to the subject. Thus, the methods and assays described herein are useful in detecting the level of expression of an exogenous gene delivered using, *e.g.,* a gene therapy method such as viral delivery, plasmid DNA delivery *etc.*

The details of various embodiments of the invention are set forth in the description below.

## Description of the Drawings

**FIG. 1** is a graph depicting the detection of TTR, GAPDH, and Rplp2 mRNAs in mouse serum using quantitative RT-PCR.

**FIGs. 2A and 2B** are graphs depicting the detection of TTR, FVII, GAPDH, beta actin, and Acta2 mRNAs in rat serum. FIG. 2A shows raw expression data for each mRNA. FIG. 2B shows mRNA expression levels relative to GAPDH expression.

**FIG. 3** is a bar graph showing the levels of full-length TTR mRNA (*e.g.,* uncleaved mRNA) in rat serum using two different TaqMan® assays.

**FIGs. 4A and 4B** are bar graphs depicting the detection of TTR and 18s mRNA in serum from cynomolgus monkey (*Macaca fascicularis*; "cyno"). FIG. 4A shows data relating to TTR mRNA levels normalized to total RNA in the starting material. FIG. 4B shows TTR mRNA expression levels normalized to 18s RNA.

**FIGs. 5A and 5B** are graphs depicting the detection of ubiquitous (*e.g.*, 18s RNA, GAPDH) and liver specific (*e.g.*, TTR, AAT, albumin) mRNA transcripts in cynomolgus monkey serum and in human serum. FIG. 5A shows mRNA expression of GAPDH, 18s RNA, TTR and AIAT in cynomolgus monkey serum. FIG. 5B shows mRNA expression of GAPDH, 18s RNA, P0, TTR, AAT and albumin in human serum.

**FIGs. 6A and 6B** are bar graphs depicting the detection of TTR, AAT, and 18s mRNA in human serum using quantitative RT-PCR. FIG. 6A shows TTR mRNA expression relative to AAT mRNA expression. FIG. 6B shows TTR mRNA expression relative to 18s mRNA expression.

**FIG. 7** is a bar graph showing the detection of TTR, SNCA, HTT, and DrD2 mRNAs in cerebral spinal fluid (CSF).

**FIGs. 8A and 8B** are bar graphs showing that serum RNA is protected from nuclease digestion via exosomes. FIG. 8A shows the normalized concentration of RNA in isolated rat exosomes that are untreated, treated with RNase or treated with RNase enzyme in the presence of Tx-100. FIG. 8B shows the normalized concentration of RNA in isolated human exosomes that are untreated, treated with RNase enzyme or treated with RNase enzyme in the presence of Tx-100.

**FIGs. 9A and 9B** are graphs depicting the detection of RNAi mediated silencing of TTR mRNA in rat serum using AF-011-18534 double-stranded RNA (dsRNA). FIG. 9A shows TTR mRNA expression normalized to GAPDH expression in liver tissue. FIG. 9B shows TTR mRNA expression relative to GAPDH expression in serum.

**FIGs. 10A to 10D** are a series of graphs depicting that AF-011-18534-mediated silencing of TTR mRNA can be detected in rat serum. FIGs. 10A-10B show silencing of serum TTR mRNA expression relative to serum mRNA of the hepatocyte-specific genes AAT (FIG. 10A) and albumin (FIG. 10B). FIGs. 10C-10D shows silencing of serum TTR mRNA expression relative to serum mRNA of the ubiquitous genes GAPDH (FIG. 10C) and 18s (FIG. 10D).

**FIGs. 11A to 11D** are a series of graphs showing the effect of AF-011-18534 on mRNA expression levels of the hepatocyte-specific genes AAT or albumin in rat serum. FIG. 11A shows the mRNA expression of AAT normalized to GAPDH mRNA levels in rat serum. FIG. 11B shows mRNA expression of AAT normalized to GAPDH mRNA levels in serum samples from individual animals. FIG. 11C shows the mRNA expression of albumin normalized to GAPDH mRNA levels in rat serum. FIG. 11D shows mRNA expression of albumin normalized to GAPDH mRNA levels in serum samples from individual animals.

**FIGs. 12A and 12B** are graphs depicting the dose-response relationship of AF-011-18534 mediated silencing of TTR mRNA in rat liver tissue.

**FIGs. 13A and 13B** are graphs depicting the dose-response relationship of AF-011-18534 mediated silencing of TTR mRNA in rat serum.

**FIGs. 14A and 14B** are graphs depicting the detection of RNAi mediated silencing of TTR mRNA in cynomolgus monkey liver tissue and serum using SNALP-18324 double-stranded RNA (dsRNA). FIG. 14A shows data for TTR mRNA expression relative to GAPDH mRNA in cynomolgus monkey liver tissue. FIG. 14B shows TTR mRNA expression relative to 18s mRNA expression in cynomolgus monkey serum.

FIG. 15 is a bar graph showing serum concentrations of TTR protein measured using ELISA at 48h following administration of SNALP-18324.

**FIGs. 16A and 16B** are graphs depicting the silencing of TMPRSS6 in a rat model as detected using mRNA levels in the liver and serum.

**FIG. 17A and 17B** are bar graphs showing TTR mRNA levels at specified time intervals post TTR targeting or control siRNA administration. FIG. 17A shows the post-silencing recovery of mRNA levels in rat serum and liver. FIG. 17B shows TTR mRNA reduction within the first 24 hours post siRNA administration.

**FIG. 18** is a bar graph showing the effect of isolating RNA in the presence or absence of 1M lithium chloride (LiCl).

The relative mRNA expression levels of GAPDH, albumin, beta actin, and TTR are shown.

**FIGs. 19A and 19B** show the alignment of 5' RACE products of TTR mRNA with the full lengthTTR sequence. FIG. 19A shows the alignment of two 5' RACE products of rat TTR mRNA with the full length rat TTR mRNA. FIG. 19B shows the alignment of two 5' RACE products of cynomolgus monkey TTR mRNA with the full length cynomolgus monkey TTR mRNA.

**FIG. 20** is a bar graph showing the expression of human ApoE in rats injected with an adenovirus expressing GFP (Ad-GFP) or an adenovirus expressing human ApoE (Ad-hApoE).

**FIG. 21** is a bar graph depicting the expression of GFP in rats injected with an adenovirus expressing GFP (Ad-GFP) or an adenovirus expressing human ApoE (Ad-hApoE).

**FIGs. 22A and 22B** demonstrate the applicability of detecting circulating exosome mRNA in monitoring the activity of miRNA-targeting oligonucleotides. FIG. 22A is a bar graph showing the detection of serum and liver miR-122 in rats injected with oligonucleotide directed against miRNA, miR-122, or mismatched oligonucleotide control. FIG. 22B is a bar graph showing the detection of the miR-122 target aldolase A mRNA in the serum and liver of rats injected with oligonucleotide directed against miR-122, or mismatched oligonucleotide control.

**FIG. 23** is a bar graph depicting the detection of striatum and CSF SNCA mRNA levels in rats infused with *Snca* targeting siRNA.

**FIG. 24** is a bar graph depicting levels of extracellular RNA identified in rat serum.

## Detailed Description

The assays, compositions and methods described herein are based, in part, on the discovery that iRNA-mediated (*e.g.*, dsRNA-mediated) gene silencing can be detected by measuring levels of a target RNA in blood or cerebrospinal fluid (CSF), even if the target RNA to be silenced is expressed in a different biological compartment (*e.g.*, liver tissue). Thus, provided herein are assays and methods for determining the activity or efficacy of an iRNA by detecting RNA expression in a biological sample (*e.g.*, blood, serum, cerebral spinal fluid (CSF), breast milk, saliva, urine, *etc.*) obtained from a subject administered an iRNA agent, wherein the iRNA targets an RNA in a tissue that is separate (*e.g.*, distal) from the biological sample. The assays described herein are useful for monitoring the activity of agents other than iRNA agents. For example, the activity of other therapeutic nucleic acids, such as antagomirs, miRNA mimics and gene therapy agents can be assayed by detecting target RNA levels, or gene therapy expression products, in a biological sample, such as a serum, blood, urine or CSF sample, even when the therapeutic nucleic acid is active in a particular tissue distant from the site of sample collection.

A tissue that is separate from the biological sample can be distal to the sample. In one embodiment, the tissue contacts the biological sample, but is separated from the biological sample by a membrane, such as a lipid bilayer. For example, the tissue can be an organ, and the biological sample can be a fluid that contacts the tissue, such as in the case where the tissue is bathed in the biological sample. For example, the tissue can be the liver, and the biological sample used to monitor RNA levels in the liver can be a blood sample. The blood sample can be collected by methods known in the art, such as from a vein in the subject. RNA levels in brain tissue can be monitored by assaying for RNA levels in cerebrospinal fluid.

An iRNA agent that targets the brain can be monitored for silencing activity by assaying target RNAs or cleavage products in the cerebrospinal fluid; an iRNA agent that targets the liver, kidney or heart, for example, can be monitored for silencing activity by assaying target RNAs or cleavage products in the blood, serum, or urine. The methods and assays described herein have the advantage of non-invasively determining the activity of an iRNA administered to a subject.

Double-stranded RNA molecules (dsRNA) have been shown to block gene expression in a highly conserved regulatory mechanism known as RNA interference (RNAi). WO 99/32619 (Fire et al.) disclosed the use of a dsRNA of at least 25 nucleotides in length to inhibit the expression of genes in C. elegans. dsRNA has also been shown to degrade target RNA in other organisms, including plants (see, *e.g.,* WO 99/53050, Waterhouse et al.; and WO 99/61631, Heifetz et al.), Drosophila (see, *e.g.,* Yang, D., et al., Curr. Biol. (2000) 10:1191-1200), and mammals (see WO 00/44895, Limmer; and DE 101 00 586.5, Kreutzer et al.). This natural mechanism has now become the focus for the development of a new class of pharmaceutical agents for treating disorders that are caused by the aberrant or unwanted regulation of a gene.

The iRNAs administered to a subject to treat a disorder can include an RNA strand (the antisense strand) having a region which is 30 nucleotides or less in length, *i.e.,* 15-30 nucleotides in length, generally 19-24 nucleotides in length, which region is substantially complementary to at least part of an mRNA transcript of a target gene. The use of these iRNAs enables the targeted degradation of mRNAs of genes that are implicated in a particular disease. Very low dosages of iRNAs in particular can specifically and efficiently mediate RNAi, resulting in significant inhibition of expression of a gene.

Examples of the pharmaceutical compositions useful for treating a subject also include an iRNA having an

antisense strand having a region that is 30 nucleotides or less in length, generally 19-24 nucleotides in length, which region is substantially complementary to at least part of an RNA transcript of a target gene, together with a pharmaceutically acceptable carrier. Examples of compositions featured herein also include an iRNA having an antisense strand having a region of complementarity which is 30 nucleotides or less in length, generally 19-24 nucleotides in length, and is substantially complementary to at least part of an RNA transcript of a target gene. WO 2009/015357 is an example of a method for determining the activity of a therapeutic agent in exosomes derived from a serum sample.

I. Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A," "T" and "U" each generally stand for a nucleotide that contains guanine, cytosine, adenine, thymidine and uracil as a base, respectively. However, it will be understood that the term "ribonucleotide" or "nucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine can be replaced in the nucleotide sequences of dsRNA featured herein by a nucleotide containing, for example, inosine. In another example, adenine and cytosine anywhere in the oligonucleotide can be replaced with guanine and uracil, respectively to form G-U Wobble base pairing with the target mRNA. Sequences containing such replacement moieties are suitable for the compositions and methods described herein.

As used herein, the term "iRNA" refers to an agent that contains RNA as that term is defined herein, and which mediates the targeted cleavage of an RNA transcript via an RNA induced silencing complex (RISC) pathway. An iRNA as described herein may effect inhibition of target gene expression.

The iRNAs included in the compositions featured herein encompass a double stranded RNA (dsRNA) having an RNA strand (the antisense strand) having a region that is 30 nucleotides or less, generally 19-24 nucleotides in length, that is substantially complementary to at least part of an mRNA transcript of a target gene.

The dsRNA may comprise a region of at least 15 contiguous nucleotides.

In one embodiment, an iRNA for inhibiting expression of the target gene includes at least two sequences that are complementary to each other. The iRNA includes a sense strand having a first sequence and an antisense strand having a second sequence. The antisense strand includes a nucleotide sequence that is substantially complementary to at least part of an mRNA encoding the target gene, and the region of complementarity is 30 nucleotides or less, and at least 15 nucleotides in length. Generally, the iRNA is 19 to 24, *e.g.*, 19 to 21 nucleotides in length. The iRNA may be from about 15 to about 25 nucleotides in length, and also the iRNA may be from about 25 to about 30 nucleotides in length. The iRNA, upon contacting with a cell expressing the target gene, inhibits the expression of the target gene by at least 10%, at least 20%, at least 25%, at least 30%, at least 35% or at least 40% or more, such as when assayed by a method as described herein. The iRNA may be formulated in a stable nucleic acid lipid particle (SNALP).

An iRNA featured herein may include a first sequence of a dsRNA that is selected from the group consisting of the sense sequences of Table 2, and a second sequence that is selected from the group consisting of the corresponding antisense sequences of Table 2. An iRNA featured herein may include a first sequence of a dsRNA that is selected from the group consisting of the sense sequences of Table 3, and a second sequence that is selected from the group consisting of the corresponding antisense sequences of Table 3.

As used herein, the term "antisense oligonucleotide" refers to a single-stranded oligonucleotide that is substantially complementary to at least part of an mRNA encoding the target gene. Antisense oligonucleotides can inhibit translation in a stoichiometric manner by base pairing to the mRNA and physically obstructing the translation machinery, see Dias, N. et al., (2002) Mol Cancer Ther 1:347-355. Antisense oligonucleotides can also inhibit target protein expression by binding to the mRNA target and promoting mRNA target destruction via RNase-H.

As used herein, the term "miRNA," or "microRNA" refers to a short RNA sequence (*e.g.*, about 22 nucleotides) produced by a eukaryotic cell that acts as a post-transcriptional regulator by binding to complementary sequences on target mRNAs. MicroRNAs typically induce translational repression and gene silencing.

As used herein, "target sequence" refers to a contiguous portion of the nucleotide sequence of an mRNA molecule formed during the transcription of a gene to be inhibited, including messenger RNA (mRNA) that is a product of RNA processing of a primary transcription product. The target portion of the sequence will be at least long enough to serve as a substrate for iRNA-directed cleavage at or near that portion. For example, the target sequence will generally be from 9-36 nucleotides in length, *e.g.,* 15-30 nucleotides in length, including all sub ranges therebetween. As non-limiting examples, the target sequence can be from 15-30 nucleotides, 15-26 nucleotides, 15-23 nucleotides, 15-22

nucleotides, 15-21 nucleotides, 15-20 nucleotides, 15-19 nucleotides, 15-18 nucleotides, 15-17 nucleotides, 18-30 nucleotides, 18-26 nucleotides, 18-23 nucleotides, 18-22 nucleotides, 18-21 nucleotides, 18-20 nucleotides, 19-30 nucleotides, 19-26 nucleotides, 19-23 nucleotides, 19-22 nucleotides, 19-21 nucleotides, 19-20 nucleotides, 20-30 nucleotides, 20-26 nucleotides, 20-25 nucleotides, 20-24 nucleotides, 20-23 nucleotides, 20-22 nucleotides, 20-21 nucleotides, 21-30 nucleotides, 21-26 nucleotides, 21-25 nucleotides, 21-24 nucleotides, 21-23 nucleotides, or 21-22 nucleotides.

As used herein, the term "strand comprising a sequence" refers to an oligonucleotide comprising a chain of nucleotides that is described by the sequence referred to using the standard nucleotide nomenclature.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions may include: 400 mM NaCl, 40 mM PIPES pH 6.4, 1 mM EDTA, 50°C or 70°C for 12-16 hours followed by washing. Other conditions, such as physiologically relevant conditions as can be encountered inside an organism, can apply. The skilled person will be able to determine the set of conditions most appropriate for a test of complementarity of two sequences in accordance with the ultimate application of the hybridized nucleotides.

Complementary sequences within an iRNA, *e.g.,* within a dsRNA as described herein, include base-pairing of the oligonucleotide or polynucleotide comprising a first nucleotide sequence to an oligonucleotide or polynucleotide comprising a second nucleotide sequence over the entire length of one or both nucleotide sequences. Such sequences can be referred to as "fully complementary" with respect to each other herein. However, where a first sequence is referred to as "substantially complementary" with respect to a second sequence herein, the two sequences can be fully complementary, or they can form one or more, but generally not more than 5, 4, 3 or 2 mismatched base pairs upon hybridization for a duplex up to 30 base pairs (bp), while retaining the ability to hybridize under the conditions most relevant to their ultimate application, *e.g.,* inhibition of gene expression via a RISC pathway. However, where two oligonucleotides are designed to form, upon hybridization, one or more single stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, may yet be referred to as "fully complementary" for the purposes described herein.

"Complementary" sequences, as used herein, can also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs include, but are not limited to, G:U Wobble or Hoogstein base pairing.

The terms "complementary," "fully complementary" and "substantially complementary" herein can be used with respect to the base matching between the sense strand and the antisense strand of a dsRNA, or between the antisense strand of an iRNA agent and a target sequence, as will be understood from the context of their use.

As used herein, a polynucleotide that is "substantially complementary to at least part of" a messenger RNA (an mRNA) refers to a polynucleotide that is substantially complementary to a contiguous portion of the mRNA of interest. For example, a polynucleotide is complementary to at least a part of a target mRNA if the sequence is substantially complementary to a non-interrupted portion of an mRNA encoding the target gene.

The term "double-stranded RNA" or "dsRNA," as used herein, refers to an iRNA that includes an RNA molecule or complex of molecules having a hybridized duplex region that comprises two anti-parallel and substantially complementary nucleic acid strands, which will be referred to as having "sense" and "antisense" orientations with respect to a target RNA. The duplex region can be of any length that permits specific degradation of a desired target RNA through a RISC pathway, but will typically range from 9 to 36 base pairs in length, *e.g.,* 15-30 base pairs in length. Considering a duplex between 9 and 36 base pairs, the duplex can be any length in this range, for example, 9, 10, 11, 12, 13, 14, 15, 16, 17, 18, 19, 20, 21, 22, 23, 24, 25, 26, 27, 28, 29, 30, 31, 32, 33, 34, 35, or 36 and any sub-range therein between, including, but not limited to 15-30 base pairs, 15-26 base pairs, 15-23 base pairs, 15-22 base pairs, 15-21 base pairs, 15-20 base pairs, 15-19 base pairs, 15-18 base pairs, 15-17 base pairs, 18-30 base pairs, 18-26 base pairs, 18-23 base pairs, 18-22 base pairs, 18-21 base pairs, 18-20 base pairs, 19-30 base pairs, 19-26 base pairs, 19-23 base pairs, 19-22 base pairs, 19-21 base pairs, 19-20 base pairs, 20-30 base pairs, 20-26 base pairs, 20-25 base pairs, 20-24 base pairs, 20-23 base pairs, 20-22 base pairs, 20-21 base pairs, 21-30 base pairs, 21-26 base pairs, 21-25 base pairs, 21-24 base pairs, 21-23 base pairs, or 21-22 base pairs. dsRNAs generated in the cell by processing with Dicer and similar enzymes are generally in the range of 19-22 base pairs in length. One strand of the duplex region of a dsDNA comprises a sequence that is substantially complementary to a region of a target RNA. The two strands forming the duplex structure can be from a single RNA molecule having at least one self-complementary region, or can be formed from two or more separate RNA molecules.

Where the duplex region is formed from two strands of a single molecule, the molecule can have a duplex

region separated by a single stranded chain of nucleotides (herein referred to as a "hairpin loop") between the 3'-end of one strand and the 5'-end of the respective other strand forming the duplex structure. The hairpin loop can comprise at least one unpaired nucleotide; in some embodiments the hairpin loop can comprise at least 3, at least 4, at least 5, at least 6, at least 7, at least 8, at least 9, at least 10, at least 20, at least 23 or more unpaired nucleotides. Where the two substantially complementary strands of a dsRNA are comprised by separate RNA molecules, those molecules need not, but can be covalently connected. Where the two strands are connected covalently by means other than a hairpin loop, the connecting structure is referred to as a "linker." The term "siRNA" is also used herein to refer to a dsRNA as described above.

In one aspect, an RNA interference agent includes a single stranded RNA that interacts with a target RNA sequence to direct the cleavage of the target RNA. Without wishing to be bound by theory, long double stranded RNA introduced into plants and invertebrate cells is broken down into siRNA by a Type III endonuclease known as Dicer (Sharp et al., Genes Dev. 2001, 15:485). Dicer, a ribonuclease-III-like enzyme, processes the dsRNA into 19-23 base pair short interfering RNAs with characteristic two base 3' overhangs (Bernstein, et al., (2001) Nature 409:363). The siRNAs are then incorporated into an RNA-induced silencing complex (RISC) where one or more helicases unwind the siRNA duplex, enabling the complementary antisense strand to guide target recognition (Nykanen, et al., (2001) Cell 107:309). Upon binding to the appropriate target mRNA, one or more endonucleases within the RISC cleaves the target to induce silencing (Elbashir, et al., (2001) Genes Dev. 15:188). Thus, one aspect described herein relates to a single stranded RNA that promotes the formation of a RISC complex to effect silencing of the target gene.

As used herein, the term "nucleotide overhang" refers to at least one unpaired nucleotide that protrudes from the duplex structure of an iRNA, *e.g.,* a dsRNA. For example, when a 3'-end of one strand of a dsRNA extends beyond the 5'-end of the other strand, or vice versa, there is a nucleotide overhang. A dsRNA can comprise an overhang of at least one nucleotide; alternatively the overhang can comprise at least two nucleotides, at least three nucleotides, at least four nucleotides, at least five nucleotides or more. A nucleotide overhang can comprise or consist of a nucleotide/nucleoside analog, including a deoxynucleotide/nucleoside. The overhang(s) may be on the sense strand, the antisense strand or any combination thereof. Furthermore, the nucleotide(s) of an overhang can be present on the 5' end, 3' end or both ends of either an antisense or sense strand of a dsRNA.

The antisense strand of a dsRNA may have a 1-10 nucleotide overhang at the 3' end and/or the 5' end. The sense strand of a dsRNA may have a 1-10 nucleotide overhang at the 3' end and/or the 5' end. One or more of the nucleotides in the overhang may be replaced with a nucleoside thiophosphate.

The terms "blunt" or "blunt ended" as used herein in reference to a dsRNA mean that there are no unpaired nucleotides or nucleotide analogs at a given terminal end of a dsRNA, *i.e.,* no nucleotide overhang. One or both ends of a dsRNA can be blunt. Where both ends of a dsRNA are blunt, the dsRNA is said to be blunt ended. To be clear, a "blunt ended" dsRNA is a dsRNA that is blunt at both ends, *i.e.,* no nucleotide overhang at either end of the molecule. Most often such a molecule will be double-stranded over its entire length.

The term "antisense strand" or "guide strand" refers to the strand of an iRNA, *e.g.,* a dsRNA, which includes a region that is substantially complementary to a target sequence. As used herein, the term "region of complementarity" refers to the region on the antisense strand that is substantially complementary to a sequence, for example a target sequence, as defined herein. Where the region of complementarity is not fully complementary to the target sequence, the mismatches may be in the internal or terminal regions of the molecule. Generally, the most tolerated mismatches are in the terminal regions, *e.g.*, within 5, 4, 3, or 2 nucleotides of the 5' and/or 3' terminus.

The term "sense strand," or "passenger strand" as used herein, refers to the strand of an iRNA that includes a region that is substantially complementary to a region of the antisense strand as that term is defined herein.

As used herein, the term "SNALP" may refer to a stable nucleic acid lipid particle. A SNALP represents a vesicle of lipids coating a reduced aqueous interior comprising a nucleic acid such as an iRNA or a plasmid from which an iRNA is transcribed. SNALPs are described, *e.g.*, in U.S. Patent Application Publication Nos. 20060240093, 20070135372, and in International Application No. WO 2009082817. Examples of "SNALP" formulations are described elsewhere herein and are known in the art.

"Introducing into a cell," when referring to an iRNA, means facilitating or effecting uptake or absorption into the cell, as is understood by those skilled in the art. Absorption or uptake of an iRNA can occur through unaided diffusive or active cellular processes, or by auxiliary agents or devices. The meaning of this term is not limited to cells *in vitro*; an iRNA can also be "introduced into a cell," wherein the cell is part of a living organism. In such an instance, introduction into the cell will include the delivery to the organism. For example, for *in vivo* delivery, iRNA can be injected into a tissue site or administered systemically. *In vivo* delivery can also be by a β-glucan delivery system, such as those described in U.S. Patent Nos. 5,032,401 and 5,607,677, and U.S. Publication No. 2005/0281781. *In vitro* introduction into a cell includes methods known in the art such as electroporation and lipofection. Further approaches are described herein below or are known in the art.

As used herein, the term "modulate the expression of," refers to at an least partial "inhibition" or partial "activation" of target gene expression in a cell treated with an composition as described herein compared to the expression of the

target gene in an untreated cell. As such, a "modulator" refers to an RNA or DNA effector molecule that modulates (*e.g.*, inhibits or activates) expression of the target gene.

The terms "activate," "enhance," "up-regulate the expression of," "increase the expression of," and the like, in so far as they refer to a target gene, herein refer to the at least partial activation of the expression of the target gene, as manifested by an increase in the amount of target mRNA, which can be isolated from or detected in a first cell or group of cells in which a target gene is transcribed and which has or have been treated such that the expression of the target gene is increased, as compared to a second cell or group of cells substantially identical to the first cell or group of cells but which has or have not been so treated (control cells).

Expression of a target gene may be activated by at least about 10%, 15%, 20%, 25%, 30%, 35%, 40% 45%, or 50% by administration of an iRNA. The target gene may be activated by at least about 60%, 70%, or 80% by administration of an iRNA. Expression of the target gene may be activated by at least about 85%, 90%, or 95% or more by administration of an iRNA as described herein. The target gene expression may be increased by at least 1-fold, at least 2-fold, at least 5-fold, at least 10-fold, at least 50-fold, at least 100-fold, at least 500-fold, at least 1000 fold or more in cells treated with an iRNA as described herein compared to the expression in an untreated cell. Activation of expression by small dsRNAs is described, for example, in Li et al., 2006 Proc. Natl. Acad. Sci. U.S.A. 103:17337-42, and in US2007/0111963 and US2005/226848.

The terms "silence," "inhibit the expression of," "down-regulate the expression of," "suppress the expression of," and the like, in so far as they refer to a target gene, herein refer to the at least partial suppression of the expression of a target gene, as manifested by a reduction of the amount of target mRNA which can be isolated from or detected in a first cell or group of cells in which the target gene is transcribed and which has or have been treated such that the expression of the target gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition can be given in terms of a reduction of a parameter that is functionally linked to target gene expression, *e.g.*, the amount of protein encoded by the gene, or the number of cells displaying a certain phenotype. In principle, gene silencing can be determined in any cell expressing the target gene, either constitutively or by genomic engineering, and by any appropriate assay.

For example, in certain instances, expression of a target gene is suppressed by at least about 10%, 15%, 20%, 25%, 30%, 35%, 40%, 45%, or 50% by administration of an iRNA. In some embodiments, the target gene is suppressed by at least about 60%, 70%, or 80% by administration of an iRNA described herein. The target gene may be suppressed by at least about 85%, 90%, 95%, 98%, 99%, or more, by administration of an iRNA as described herein.

As used herein in the context of target gene expression, the terms "treat," "treatment," and the like, refer to relief from or alleviation of pathological processes mediated by expression of the target gene. In the context described herein insofar as it relates to any of the other conditions recited herein below, the terms "treat," "treatment," and the like mean to relieve or alleviate at least one symptom associated with the condition to be treated, or to slow or reverse the progression or anticipated progression of such condition.

By "lower" in the context of a disease marker or symptom is meant a statistically significant decrease in such level. The decrease can be, for example, at least 10%, at least 20%, at least 30%, at least 40% or more, *e.g.*, down to a level accepted as within the range of normal for an individual without such disorder.

As used herein, the phrases "therapeutically effective amount" and "prophylactically effective amount" refer to an amount that provides a therapeutic benefit in the treatment, prevention, or management of pathological processes mediated by expression of the target gene or an overt symptom of pathological processes mediated by such expression. The specific amount that is therapeutically effective can be readily determined by an ordinary medical practitioner, and can vary depending on factors known in the art, such as, for example, the type of pathological processes mediated by expression of the target gene, the patient's history and age, the stage of pathological processes mediated by target gene expression, and the administration of other agents that inhibit pathological processes mediated by target gene expression.

As used herein, a "pharmaceutical composition" comprises a pharmacologically effective amount of a therapeutic nucleic acid, e.g., an iRNA, and a pharmaceutically acceptable carrier. As used herein, "pharmacologically effective amount," "therapeutically effective amount" or simply "effective amount" refers to that amount of an iRNA effective to produce the intended pharmacological, therapeutic or preventive result. For example, if a given clinical treatment is considered effective when there is at least a 10% reduction in a measurable parameter associated with a disease or disorder, a therapeutically effective amount of a drug for the treatment of that disease or disorder is the amount necessary

to effect at least a 10% reduction in that parameter.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract. Agents included in drug formulations are described further herein below.

As used herein, a "subject" is a mammal, *e.g.* a dog, horse, cat, and other non-human primates. In one embodiment, a subject is a human.

As used herein, the term "RNA activation or RNAa" refers to the use of an RNA activating agent (*e.g.*, dsRNA) to up-regulate the expression of the target gene in a cell or mammal.

As used herein, the terms "distal from the biological sample" and "separate from the biological sample" refer to a site distinct from the the biological sample obtained from a subject for analysis; that is, the biological sample obtained is not the intended site at which the therapeutic nucleic acid modulates expression of a desired gene product (*e.g.*, RNA effector molecule, DNA effector molecule etc.). The biological sample may not be obtained from the same tissue in which the therapeutic gene is targeted for expression. For example, a therapeutic nucleic acid can be expressed in liver tissue and the biological sample obtained from the subject is blood or serum. The tissue may contact the biological sample, but may be separated from the biological sample by a membrane, such as a lipid bilayer. For example, the tissue can be an organ, and the biological sample can be a fluid that contacts the tissue, such as in the case where the tissue is bathed in the biological sample. For example, the tissue can be the liver, and the biological sample used to monitor RNA levels in the liver can be a blood sample. The biological sample can be collected by methods known in the art. For example, a blood sample can be collected from a vein in the subject. RNA levels in brain tissue may be monitored by assaying for RNA levels in cerebrospinal fluid.

An iRNA agent that targets the brain can be monitored for silencing activity by assaying target RNAs or cleavage products in the cerebrospinal fluid; an iRNA agent that targets the liver, kidney or heart, for example, can be monitored for silencing activity by assaying target RNAs or cleavage products in the blood, serum, or urine. The methods and assays described herein have the advantage of determining the activity of an iRNA administered to a subject by using minimally invasive techniques.

As used herein, the term "LNPXX", wherein the "XX" are numerals, is also referred to as "AF-0XX" herein. For example, LNP11 is also referred to AF-011.

"Acquire" or "acquiring" as the terms are used herein, refer to obtaining possession of a physical entity, or a value, *e.g.*, a numerical value, by "directly acquiring" or "indirectly acquiring" the physical entity or value. "Directly acquiring" means performing a process (*e.g.*, performing a synthetic or analytical method) to obtain the physical entity or value. "Indirectly acquiring" refers to receiving the physical entity or value from another party or source (*e.g.*, a third party laboratory that directly acquired the physical entity or value). Directly acquiring a physical entity includes performing a process that includes a physical change in a physical substance, *e.g.*, a starting material. Exemplary changes include making a physical entity from two or more starting materials, shearing or fragmenting a substance, separating or purifying a substance, combining two or more separate entities into a mixture, performing a chemical reaction that includes breaking or forming a covalent or non-covalent bond. Directly acquiring a value includes performing a process that includes a physical change in a sample or another substance, *e.g.*, performing an analytical process which includes a physical change in a substance, *e.g.*, a sample, analyte, or reagent (sometimes referred to herein as "physical analysis"), performing an analytical method, *e.g.*, a method which includes one or more of the following: separating or purifying a substance, *e.g.*, an analyte, or a fragment or other derivative thereof, from another substance; combining an analyte, or fragment or other derivative thereof, with another substance, *e.g.*, a buffer, solvent, or reactant; or changing the structure of an analyte, or a fragment or other derivative thereof, *e.g.*, by breaking or forming a covalent or non-covalent bond, between a first and a second atom of the analyte; or by changing the structure of a reagent, or a fragment or other derivative thereof, *e.g.*, by breaking or forming a covalent or non-covalent bond, between a first and a second atom of the reagent.

"Acquiring a sample" as the term is used herein, refers to obtaining possession of a sample, *e.g.*, a tissue sample or nucleic acid sample, by "directly acquiring" or "indirectly acquiring" the sample. "Directly acquiring a sample" means performing a process (*e.g.*, performing a physical method such as a surgery or extraction) to obtain the sample. "Indirectly acquiring a sample" refers to receiving the sample from another party or source (*e.g.*, a third party laboratory that directly acquired the sample). Directly acquiring a sample includes performing a process that includes a physical change in a physical substance, *e.g.*, a starting material, such as a tissue, *e.g.*, a tissue in a human patient or a tissue

that has was previously isolated from a patient. Exemplary changes include making a physical entity from a starting material, dissecting or scraping a tissue; separating or purifying a substance (*e.g.*, a sample tissue or a nucleic acid sample); combining two or more separate entities into a mixture; performing a chemical reaction that includes breaking or forming a covalent or non-covalent bond. Directly acquiring a sample includes performing a process that includes a physical change in a sample or another substance, *e.g.*, as described above.

As used herein, the term "comprising" or "comprises" is used in reference to compositions, methods, and respective component(s) thereof, that are essential to the invention, yet open to the inclusion of unspecified elements, whether essential or not.

As used herein, the term "consisting essentially of' refers to those elements required for a given aspect.

The term "consisting of' refers to compositions, methods, and respective components thereof as described herein, which are exclusive of any element not recited in that description of the aspect.

II. Assays

Disclosed herein are assays for determining the activity of an iRNA agent by detecting expression of a target RNA in a biological sample obtained from a subject administered an iRNA agent. The methods described herein permit the detection of iRNA activity in a biological sample that is separate from the tissue where the iRNA-mediated gene silencing occurs. Such assays have the advantage of obtaining a biological sample in a non-invasive manner to confirm the activity and/or efficacy of iRNA action in a desired tissue.

*Biological Samples.* Essentially any biological sample can be used to measure mRNA expression levels of a target gene to be silenced. Typically, the biological sample is a biological fluid such as blood, serum, plasma, urine, cerebrospinal fluid (CSF), amniotic fluid, saliva, breast milk, bronchoalveolar lavage fluid, synovial fluid, sputum, spinal fluid, pleural fluid, nipple aspirates, lymph fluid, fluid of the respiratory, intestinal, and genitourinary tracts, tear fluid, fluid from the lymphatic system, semen, intra- organ system fluid, malignant ascites tumor cyst fluid and combinations thereof. The biological sample may be serum and in another embodiment, the biological sample is CSF.

A biological sample that can be obtained using methods having minimal invasiveness (*e.g.*, plasma, urine, serum, blood) may be desired. The methods and assays provided herein provide an advantage of permitting detection of successful iRNA- or dsRNA-mediated silencing using minimally invasive techniques and avoiding costly and/or risky tissue biopsies (*e.g.*, liver biopsy).

A biological sample can be obtained from a subject using methods known to those of skill in the art, *e.g.*, a physician or the like. For example, the sample can be obtained using a syringe or a swab.

A biological sample can be obtained, or provided, directly or indirectly. Directly obtaining, or providing, means performing a process to obtain the sample. Indirectly obtaining, or providing, refers to receiving the sample from another party or source, such as from a third party laboratory that directly obtained the sample.

The biological sample can be stored prior to detecting target RNA levels. Typically, the lower the temperature the longer the sample can stably be stored. In one embodiment, the temperature is between -5° C and -80° C. The storage temperature may be between -15° C and -20° C. The storage temperature may be -20° C or -80° C prior to isolation of RNA from the sample. The sample may also be stored at 4° C for, *e.g.*, 2 hours to 168 hours prior to RNA isolation from the sample.

The biological sample can be subjected to filtration, such as through a 0.2 μM to 0.5 μM filter, e.g., through a 0.4 μM filter, prior to RNA isolation. Further, or in the alternative, the biological sample can be subjected to centrifugation, such as high speed centrifugation, prior to RNA isolation from the sample. For example, the sample can be centrifuged at 1000 g to 10,000 g, e.g., 2000 g to 5000 g. The sample may be centrifuged (e.g., is further centrifuged) at 100,000 g to 150,000 g, e.g., 110,000 g to 140,000 g. LiCl may be added to the sample before one or more of the centrifugation steps. LiCl can be added at a concentration of, for example, 0.5 M, 1 M, 1.5 M, 2 M or more.

*Exosomes.* The biological sample may contain exosomes. The term "exosome" as used herein is a nanometer-sized (30 nm to 150 nm, e.g., 40 nm to 100 nm) vesicle that originates as an internal vesicle of a multivesicular body (MVB), present in endocytic and secretory pathways. Exosomes are formed by an invagination process or inward budding which causes a membrane-enclosed compartment in which the lumen is topologically equivalent of cytoplasm. "Microvesicles" are larger vesicles (*e.g.*, 100 nm to 1000 nm) produced by a budding process from lipid raft regions of the plasma membrane.

RNAs, including mRNA and miRNA are internalized within exosomes. Exosomes are released in a regulated fashion into the extracellular environment by different cell types under both normal and pathological conditions, and contain RNA, protein and intracellular organelles from the cells where they originated. Cell types that express exosomes include neural cells, liver cells, and cells of most other organs. Their protein, RNA (*e.g.*, mRNA and miRNA) and lipid composition are a consequence of sorting events at the level of the microvesicle body. Exosomes include common as well as cell-type specific proteins and RNA. Exosomes are present in many types of bodily fluids, including blood, plasma, serum, cerebrospinal fluid, urine, saliva, amniotic fluid, breast milk, bronchoalveolar lavage and synovial fluids, and

malignant ascites.

A fraction of the target RNA or cleavage product thereof in the biological sample may be present in exosomes. For example, typically at least 0.000001% of the target RNA or cleavage product thereof in the biological sample is present in exosomes; For example at least 0.00001%, at least 0.001%, at least 0.01%, at least 1%, at least 2%, at least 5%, at least 10%, at least 15%, at least 20%, at least 25%, at least 30%, at least 35%, at least 40%, at least 50%, at least 55%, at least 60%, at least 65%, at least 70%, at least 75%, at least 80%, at least 85%, at least 90%, at least 95%, at least 99%, or even 100% (*i.e.*, the entire fraction) of the target RNA or cleavage product may be present in exosomes. For example, exosomal RNA for liver markers is approximately $10^5$ to $10^6$ lower than the liver cellular RNA (*e.g.*, 0.00001% to 0.000001% of the RNA is in the exosomes).

A biological sample may be enriched for exosomes. A biological sample can be enriched for exosomes by, for example, centrifugation and/or chromatography, such as size-exclusion chromatrography. A biological sample can be enriched for exosomes, but may not include exosomes that have been "purified." Exosomes are purified through the use of exosome-binding molecules, such as antibodies, leptins or other protein ligands.

Exosomes may not be purified prior to detection of the target RNA (*e.g.*, mRNA) or cleavage product thereof (*e.g.*, RISC cleavage product). Exosomes that have not been purified from a biological sample have not been selected for using antibodies or leptins or other protein ligands. For example, the exosomes are not purified by selecting for proteins that are enriched on exosomes, such as by using antibodies or leptins or other protein ligands. Proteins enriched on exosomes include, e.g., chaperones, tetraspanins, adhesion molecules, rab proteins, cytoskeletal proteins and metabolic enzymes. Exemplary proteins associated with exosomes include, e.g., CD63, CD9, beta1-integrin, CD81, ICAM-1, Mfg-E8, transferrin receptor, sialic acid, mucins, Tsg101 (Tumor susceptibility gene 101), Aip1/Alix, annexin II, EF1a (Transcription Elongation factor 1a), CD82, ceramide, and sphingomyelin (e.g., Conde-Vancells et al., J. Proteome Res. 7:5157-5166, 2008).

"Enriched" as used herein refers to a sample that is selected, processed, or manipulated to contain a greater percentage of a particular component (*e.g.*, a biological sample enriched for exosomes, or enriched for more liver-specific exosomes) than the sample contained prior to the manipulation. For example, an enriched sample comprises at least 10% of the desired component (*e.g.*, exosomes); in other embodiments, the enriched sample comprises at least 20%, at least 30%, at least 40%, at least 50%, at least 60%, at least 70%, at least 80%, at least 90%, at least 95%, or at least 99% of the desired component. Thus, an exosome-enriched sample refers to a sample that comprises at least 10% exosomes as determined by, *e.g.*, measuring the level of an exosome cell surface antigen such as those described in *e.g.*, U.S. Patent No. 7,198,923. An exosome-enriched sample may comprise *e.g.*, at least 20%, at least 30%, at least 40%, at least 50%, at least 60%, at least 70%, at least 80%, at least 90%, at least 95%, or at least 99% exosomes.

As used herein, "purified" means substantially separated from other components of the biological sample, *e.g.*, cells and cell-free proteins and nucleic acids. Components are purified, for example, using antibodies or leptins or other protein ligands to separate the purified component from other components in the biological sample.

Exosomes derived from a tissue where gene silencing may be targeted are not selected for prior to detecting the RNA. As used herein, the term "selected for" refers to purification of vesicles by a marker, such as a cell surface marker. For example, an antibody, such as a monoclonal or polyclonal antibody, or a peptide ligand, is not used to select for a tissue specific marker on the surface of an exosome, such as to purify the exosome from a specific tissue, such as from the liver. Exosomes are not purified by selecting for proteins expressed on the surface of exosomes.

In some instances, such as where an siRNA cleavage product is assayed, exosomes derived from a tissue where gene silencing may be targeted are selected for prior to detecting the RNA. For example, an antibody, such as a monoclonal or polyclonal antibody, or a peptide ligand, is used to select for a tissue specific marker on the surface of an exosome, such as to purify the exosome from a specific tissue, such as from the liver. For example, SRBI is a hepatocyte marker that can be used to select for exosomes from liver. The exosomes in the biological samples featured herein may be purified by other methods known in the art. For example, differential centrifugation can be used to purify exosomes (see *e.g.*, Raposo et al., J Exp Med (1996) 183:1161-1172). Other methods include anion exchange chromatography, gel permeation chromatography (*e.g.*, U.S. Patent Nos. 6,899,863 and 6,812,023), sucrose density gradients, organelle electrophoresis (*e.g.*, U.S. Patent No. 7,198,923) magnetic activated cell sorting (MACS) (Taylor and Gercel-Taylor, Gynecology Oncology (2008) 110(1): 13-21) and nanomembrane ultrafiltration concentrators (Cheruvanky et al., Am J Physiol Renal Physiol (2007) 292(5):F1657-61). Other methods of purifying exosomes are described in Gibbings et al. (U.S. 2011/0177054).

Exosomes in a biological sample may be enriched for those originating from a specific cell type, such as, for example, liver, lung, stomach, intestine, bladder, kidney, ovary, testis, skin, colorectal, breast, prostate, brain, spinal cord, choroid plexus, peripheral neurons or nerve, esophagus, adrenal gland, spleen, placenta, tumors, cancer lesions, or fetus cells. Because the exosomes carry surface molecules such as antigens from their donor cells, surface molecules may be used to identify exosomes from a specific donor cell type (Al-Nedawi et al., Nat Cell Biol (2008) 10:619-624; Taylor and Gercel-Taylor, Gynecology Oncology (2008) 110(1): 13-21). In this way, exosomes originating from distinct cell populations can be analyzed for their nucleic acid (*e.g.*, RNA) content. For example, tumor (malignant and non-

malignant) exosomes carry tumor-associated surface antigens and can be detected via tumor-associated surface antigens.

The purification of exosomes from specific cell types, such as for assaying for RNA cleavage products, can be accomplished, for example, by using antibodies, aptamers, aptamer analogs or molecularly imprinted polymers specific for a desired surface antigen. The surface antigen may be specific for a cancer type. The surface antigen may also be specific for a cell type that is not necessarily cancerous. One example of a method of exosome separation based on cell surface antigen is provided in U.S. Patent No. 7,198,923. As described in, *e.g.*, U.S. Patent Nos. 5,840,867 and 5,582,981, and WO/2003/050290, aptamers and their analogs specifically bind surface molecules and can be used as a separation tool for retrieving cell type-specific exosomes. Molecularly imprinted polymers also specifically recognize surface molecules as described in, *e.g.*, U.S. Patent Nos. 6,525,154; 7,332,553; and 7,384,589 and are a tool for retrieving and isolating cell type-specific exosomes. Exosomes can also be identified and purified from a biological sample by a microchip technology that uses a microfluidic platform to efficiently and selectively separate tumor derived microvesicles (Nagrath et al., Nature (2007) 450:1235-9).

It may be beneficial or otherwise desirable to extract the nucleic acid (*e.g.*, RNA) from a biological sample prior to the analysis. Nucleic acid molecules can be isolated from the sample using any number of procedures, which are well-known in the art, the particular isolation procedure chosen being appropriate for the particular biological sample. In some instances, with some techniques, it may also be possible to analyze the nucleic acid without extraction from the biological sample.

The extracted nucleic acids, including RNA, may be analyzed directly without an amplification step. Direct analysis may be performed with different methods including, but not limited to branched DNA (bDNA) or nanostring technology. NanoString technology enables identification and quantification of individual target molecules in a biological sample by attaching a color-coded fluorescent reporter to each target molecule. This approach is similar to the concept of measuring inventory by scanning barcodes. Reporters can be made with hundreds or even thousands of different codes allowing for highly multiplexed analysis. The technology is described by Geiss *et al*. (Geiss et al., Nat Biotech (2008) 26:317-325).

"Microvesicles" are membrane fragments that are shed or pinched-off from the plasma membrane. Microvesicles are generally thought to be larger than exosomes (*e.g.*, 100 nm to 1000nm), but their features and biogenesis are believed to be similar. RNAs, including mRNA and miRNA are present in microvesicles, which are released in a regulated fashion similar to that of exosomes. A fraction of the target RNA or cleavage product thereof in the biological sample may be present in microvesicles. Microvesicles are typically not isolated from the biological sample prior to detection of the target RNA.

Target RNA or a cleavage product thereof may be isolated from a biological sample comprising exosomes and microvesicles (*e.g.*, extracellular vesicles). Exosomes may be separated from microvesicles, *e.g.*, by size using, *e.g.*, density gradient centrifugation or other methods known in the art.

*Isolation and amplification of RNA.* RNA obtained from a biological sample can be isolated by any standard means known to a skilled artisan. Standard methods of RNA isolation, as well as recombinant nucleic acid methods used herein generally, are described in Sambrook et al., Molecular Biology: A laboratory Approach, Cold Spring Harbor, N.Y. 1989; Ausubel, et al., Current protocols in Molecular Biology, Greene Publishing, Y, 1995.

Nucleic acids can be recovered from the biological samples by extraction with an organic solvent, chloroform extraction, phenol-chloroform extraction, precipitation with ethanol, isopropanol or any other lower alcohol, by chromatography including ion exchange chromatography, size exclusion chromatography, silica gel chromatography and reversed phase chromatography, or by electrophoretic methods, including polyacrylamide gel electrophoresis and agarose gel electrophoresis, as will be apparent to one of skill in the art.

Target RNA may be isolated from the biological sample using phenol chloroform extraction. The RNA may be isolated from the biological sample using TRIzol™ reagent (available from Invitrogen™, Carlsbad, Calif.).

Following isolation, RNA can optionally be purified by techniques which are well known in the art. Purification may result in RNA that is substantially free from contaminating DNA or proteins. Further purification can be accomplished by any of the aforementioned techniques for nucleic acid recovery. RNA can be purified by precipitation using a lower alcohol, especially with ethanol or with isopropanol. Precipitation can be carried out in the presence of a carrier such as glycogen that facilitates precipitation.

RNA from the biological sample can be amplified by a variety of mechanisms, some of which may employ PCR. See, *e.g.*, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19:4967 (1991); Eckert et al., PCR Methods and Applications 1:17 (1991); PCR (Eds. McPherson et al., IRL Press, Oxford); and U.S. Patent Nos. 4,683,202; 4,683,195; 4,800,159; 4,965,188; and 5,333,675. The sample can be amplified on an array. See, for example, U.S. Pat. No 6,300,070 and U.S. patent application Ser. No. 09/513,300.

Other suitable amplification methods include the ligase chain reaction (LCR) (*e.g.*, Wu and Wallace, Genomics

4:560 (1989), Landegren et al., Science 241:1077 (1988) and Barringer et al. Gene 89:117 (1990)), transcription amplification (Kwoh et al., Proc. Natl. Acad. Sci. USA 86:1173 (1989) and WO88/10315), self-sustained sequence replication (Guatelli et al., Proc. Nat. Acad. Sci. USA, 87:1874 (1990) and WO90/06995), selective amplification of target polynucleotide sequences (U.S. Pat. No 6,410,276), consensus sequence primed polymerase chain reaction (CP-PCR) (U.S. Pat. No. 4,437,975), arbitrarily primed polymerase chain reaction (AP-PCR) (U.S. Pat. No. 5,413,909; 5,861,245) and nucleic acid based sequence amplification (NABSA). (See, U.S. Pat. Nos. 5,409,818; 5,554,517; and 6,063,603. Other amplification methods that can be used are described in, U.S. Pat. Nos. 5,242,794; 5,494,810; 4,988,617; and 6,582,938.

RNA isolated by the method described herein can include messenger RNA (mRNA), transfer RNA (tRNA), and ribosomal RNA (rRNA). The RNA may be mRNA. The RNA may be an mRNA cleavage product. The RNA may also be a RISC-cleavage product.

Typically, isolation of the RNA requires a process that includes a physical change in a physical substance, *e.g.*, a starting material, such as biological sample and the contents of the sample (*e.g.*, the RNA). Exemplary changes include making a physical entity from two or more starting materials (*e.g.*, by polymerase chain reaction), shearing or fragmenting a substance, separating or purifying a substance, combining two or more separate entities into a mixture, or performing a chemical reaction that includes breaking or forming a covalent or non-covalent bond.

*Methods for detecting target RNA or a cleavage product thereof.* Methods for assessing RNA levels, *e.g.*, mRNA levels are well known to those skilled in the art. Detection of RNA transcripts can be accomplished using known amplification methods. For example, it is within the scope described herein to reverse transcribe mRNA into cDNA followed by polymerase chain reaction (RT-PCR); or, to use a single enzyme for both steps as described in U.S. Pat. No. 5,322,770, or reverse transcribed mRNA into cDNA followed by symmetric gap ligase chain reaction (RT-AGLCR) as described by R. L. Marshall, et al., PCR Methods and Applications 4: 80-84 (1994).

Gene expression may be measured using quantitative real time PCR. Quantitative real-time PCR refers to a polymerase chain reaction which is monitored, usually by fluorescence, over time during the amplification process, to measure a parameter related to the extent of amplification of a particular sequence. The amount of fluorescence released during the amplification cycle is proportional to the amount of product amplified in each PCR cycle.

Hybridization methods can also be employed, for example, a radioactively or fluorescently labeled antisense RNA probe is hybridized to isolated RNA, washed, cleaved with RNase and visualized. Methods for conducting polynucleotide hybridization assays have been well developed in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y., 1989); Berger and Kimmel Methods in Enzymology, Vol. 152, Guide to Molecular Cloning Techniques (Academic Press, Inc., San Diego, Calif., 1987); Young and Davism, P.N.A.S, 80: 1194 (1983). Methods and apparatus for carrying out repeated and controlled hybridization reactions have been described, for example, in U.S. Pat. Nos. 5,871,928; 5,874,219; 6,045,996 and 6,386,749; 6,391,623. RNA expression, including mRNA expression, can be detected on a DNA array, chip or a microarray. Oligonucleotides corresponding to a target RNA are immobilized on a chip which is then hybridized with labeled RNA of a biological sample obtained from a subject. A positive hybridization signal is obtained with the sample containing transcripts of the target gene. Methods of preparing DNA arrays and their use are well known in the art. (See, for example, U.S. Pat. Nos: 6,618,6796; 6,379,897; 6,664,377; 6,451,536; 548,257; U.S. 20030157485 and Schena et al. 1995 Science 20:467-470; Gerhold et al. 1999 Trends in Biochem. Sci. 24, 168-173; and Lennon et al. 2000 Drug discovery Today 5: 59-65). Serial Analysis of Gene Expression (SAGE) can also be performed (See for example U.S. Patent Application 20030215858).

5' RACE ("Rapid Amplification of cDNA Ends") techniques can be employed in detection of a cleavage product and can be used *e.g.*, to obtain the full-length RNA associated with the cleavage product. 5' RACE techniques typically involve producing a cDNA copy of the RNA sequence of interest using reverse transcription followed by PCR amplification of the cDNA copies. The amplified cDNA copies are sequenced can be mapped to a full-length mRNA sequence. Methods and kits for performing 5' RACE are known to those of skill in the art.

Typically, detecting or analyzing or measuring RNA (*e.g.*, RNA levels) requires a process that includes a physical change in a physical substance, *e.g.*, a starting material, such as isolated RNA. Exemplary changes include making a physical entity from two or more starting materials (*e.g.*, by polymerase chain reaction), shearing or fragmenting a substance, separating or purifying a substance, combining two or more separate entities into a mixture, or performing a chemical reaction that includes breaking or forming a covalent or non-covalent bond. Analyzing a sample can include performing an analytical process which includes a physical change in a substance, *e.g.*, an RNA sample, an analyte, or a reagent (sometimes referred to herein as "physical analysis"), performing an analytical method, *e.g.*, a method which includes one or more of the following: combining a nucleic acid, an analyte, or fragment or other derivative thereof, with another substance, *e.g.*, a buffer, solvent, enzyme or reactant; changing the structure of a nucleic acid, an analyte, or a fragment or other derivative thereof, *e.g.*, by breaking or forming a covalent or non-covalent bond, between a first and a second atom of the nucleic acid or analyte; or changing the structure of a reagent, or a fragment or other derivative thereof, *e.g.*, by breaking or forming a covalent or non-covalent bond, between a first and a second atom of the reagent;

or separating or purifying a substance, *e.g.*, a nucleic acid an analyte, or a fragment or other derivative thereof, from another substance.

*Reference Samples.* A "reference sample" as used herein, is, for example, a biological sample obtained from a patient who has not received an iRNA, or obtained from a patient prior to receiving an iRNA. The results of an assay described herein, *e.g.*, to measure target RNA levels in a sample following administration of an iRNA or to determine the activity of an iRNA on target RNA degradation, can be compared to target RNA levels in a biological sample isolated from the patient prior to receiving the iRNA.

Similarly, the results of an assay described herein, *e.g.*, to determine gene therapy effectiveness and/or to measure the level of expression of the exogenous gene (*e.g.*, the transgene), can be compared to RNA levels, *e.g.*, a corresponding gene in a biological sample isolated from the patient prior to receiving the exogenous gene (*i.e.*, baseline or background level of the gene). For example, the corresponding gene can have a mutation, and the corresponding gene is transcribed into an RNA, or is not transcribed in the patient. The level of the corresponding gene prior to gene therapy may be absent or the expression may be so low as to be undetectable in the serum and/or tissue samples. The gene corresponding to the exogenous gene may not be expressed in the patient (e.g., the exogenous gene encodes a synthetic peptide or a viral peptide). Expression of the exogenous RNA can be monitored by comparison to expression of an endogenous RNA (e.g., from a housekeeping gene) in the patient before and after administration of the exogenous gene.

The target RNA levels measured (*e.g.*, at a first and second time point) can be compared to the RNA levels of a gene that is not expected to vary with the iRNA treatment (*e.g.*, a housekeeping gene or non-targeted tissue-specific gene). The target RNA levels in a sample following administration of an iRNA can be compared to levels of a ubiquitous control RNA (*e.g.*, GAPDH, β-actin or ribosomal (*e.g*, 18S)) before and after administration of the iRNA. The target RNA levels in a sample following administration of an iRNA can be compared to levels of a non-target tissue-specific RNA before and after administration of the iRNA. For example, levels of a non-target liver RNA, *e.g.*, alpha antitrypsin (AAT), can be compared to TTR target RNA levels following administration of an iRNA that target TTR. In some embodiments, the target RNA levels are normalized to the RNA levels of a gene that is not expected to vary with iRNA treatment.

The reference sample may be a population standard determined, *e.g.*, by averaging the target RNA levels measured in a biological sample among individuals in a normal population and/or in a diseased population. The diseased population can be further separated into untreated and iRNA treated groups for measuring target RNA levels and determining the population standard. Such population standards are useful in determining an acceptable range or threshold value of target RNA levels that can be used, *e.g.*, in the clinic for comparison with an individual's target RNA levels. Thus, the reference sample may be a number (*e.g.*, a threshold value, a cut-off value, an acceptable range, etc.).

*Kits.* Components for the methods and assays discussed herein can be provided in a kit. The kit can include, for example, primers, *e.g.*, to reverse-transcribe and/or amplify a target RNA from a biological sample. The kit can include, for example, primers for 5' RACE amplification of an RNA.

The kit may provide one or more primer pairs, each pair capable of amplifying a desired target transcript thereby providing a kit for analysis of expression of one or more targets in a biological sample in one reaction or several parallel reactions. Primers in the kits may be labeled, for example fluorescently labeled, to facilitate detection of the amplification products and consequent analysis of the expression levels of the target RNA or cleavage product thereof.

More than one target gene can be detected in one analysis. A combination kit will therefore comprise primers capable of amplifying different target RNA. The primers may be differentially labeled, for example using different fluorescent labels, so as to differentiate between the target RNAs to be detected.

The primers contained within the kit may include one or more exemplary primers shown herein in Example 6. Alternatively, primers can be designed for 5' RACE, reverse transcription or TaqMan® quantitative PCR using a sequence for one or more of the following target RNAs: proprotein convertase subtilisin/kexin type 9 (PCSK9) (GenBank Accession No. NM_174936), transthyretin (TTR) (GenBank reference NM_000371.3), vascular endothelial growth factor A (VEGF) (GenBank Accession No. NM_001025366.2), kinesin-like protein (KSP) (GenBank Accession No. NM_004523),Parotid proline-rich salivary protein Pc (PCS) (GenBank Accession No. NM_020872.1), angiopoietin-like 3 (ANGPTL3) (GenBank Accession No. NM_014495.2), apolipoprotein C-III (ApoC3) (GenBank Accession No. NM_000040.1), Hepcidin (SLC11A2) (GenBank Accession No. NM_001174129.1), egl nine homolog (EGLN) (GenBank Accession No. BC111057.1), hepatitis C Virus (HCV) (GenBank Accession No. AK367384.1), Hepatitis B Virus (HBV) (GenBank Accession No. AB602818.1), polo-like kinase 1 (PLK) (GenBank Accession No. NM_005030.3), alpha-antitrypsin (AAT) (GenBank Accession No. NM_000295.4), activated protein C, transmembrane protease, serine 6 (TMPRSS6) (GenBank Accession No. NM_153609.2), Kruppel-like factor 4 (KLF) (GenBank Accession No. DQ658241.1), B-cell CLL/lymphoma 11A (BCL11A) (GenBank Accession No. NM_022893.3), p53 (GenBank Accession No. NM_001126114.1), caspase 2 (GenBank Accession No. NM_001224.4), β-catenin (GenBank Accession No. NM_001904.3), protein kinase N3 (PKN3) (GenBank Accession No. NM_013355.3), huntingtin (HTT) (Genbank Accession No. NM_002111.6) or synuclein, alpha (SNCA) (GenBank Accession No. NM_007308.2).

In addition to the one or more primers, the kit can include other ingredients, such as a solvent or buffer, a

stabilizer, or a preservative. The agent can be provided in any form, *e.g.*, liquid, dried or lyophilized form, and in substantially pure and/or sterile form. When the agents are provided in a liquid solution, the liquid solution is, for example, an aqueous solution. When the agents are provided as a dried form, reconstitution generally is by the addition of a suitable solvent. The solvent, *e.g.*, sterile water or buffer, can optionally be provided in the kit.

The kit can also include instructions, such as for amplification protocols and analysis of the results. The instructions may also specify that the person have been administered an iRNA agent. The kit may alternatively also comprise buffers, enzymes, and containers for performing the amplification and analysis of the amplification products. The informational material of the kits is not limited in its form. For example, the informational material can include information about how to perform an assay, concentrations of required reagents, date of expiration, batch or production site information, and so forth. In one embodiment, the informational material relates to methods of obtaining a biological sample, and isolating and measuring levels of a target RNA or cleavage product thereof. The information can be provided in a variety of formats, including printed text, computer readable material, video recording, or audio recording, or information that provides a link or address (*e.g.*, a URL) to substantive material.

The kit can include informational material for performing and interpreting the assay. The kit can provide guidance as to where to report the results of the assay, *e.g.*, to a research facility, treatment center or healthcare provider. The kit can include forms for reporting the results of an assay described herein, and address and contact information regarding where to send such forms or other related information; or a URL (Uniform Resource Locator) address for reporting the results in an online database or an online application (*e.g.*, an app). The informational material can include guidance regarding whether a patient should receive treatment or continue to receive treatment with an iRNA agent, depending on the results of the assay.

The kit may also include reagents for isolating RNA from a biological sample including, for example, phenol/chloroform or TRIzol™ reagent. Reagents that increase RNA yield during isolation may also be included in the kit. A reagent to increase RNA yield may be lithium chloride, *e.g.*, which can be added to the sample at a final concentration of 0.1-5 M (*e.g.*, 0.5, 1, 1.5, 2, 2.5 or 3M). The kit can further include a reagent such as glycogen that acts as a co-precipitant to help pellet small volumes of RNA. The kit may include reagents to inhibit RNAse activity, or detergents to lyse RNA carrying vesicles (exosomes).

The kit can further include an agent to inhibit RNAse activity in the biological sample or to enhance RNA precipitation. For example, the kit can include RNAse inhibitor or EDTA to inhibit RNAse activity, and/or can include PEG or lithium chloride to enhance RNA precipitation.

The kit may also be a component of a screening, diagnostic or prognostic kit comprising other tools such as DNA microarrays. The kit can also include one or more control templates, such as a target RNA isolated from a normal serum or tissue sample or a recombinant target RNA. Alternatively, a biological sample isolated from the subject prior to being administered an iRNA agent or modulator can be used as the control.

The kit can include one or more containers for the composition or compositions containing the agents. The kit may contain separate containers, dividers or compartments for the composition and informational material. For example, the composition can be contained in a bottle, vial, or syringe, and the informational material can be contained in a plastic sleeve or packet. The separate elements of the kit are contained within a single, undivided container. For example, the composition may be contained in a bottle, vial or syringe that has attached thereto the informational material in the form of a label. The kit may include a plurality (*e.g.*, a pack) of individual containers, each containing one or more unit dosage forms (*e.g.*, a dosage form described herein) of the agents. The containers can include a combination unit, *e.g.*, a unit that includes primers for RT-PCR, or primers that target more than one RNA. In one example, the kit includes a plurality of syringes, ampoules, foil packets, blister packs, or medical devices, *e.g.*, each containing a single combination unit, such as for obtaining a biological sample, and isolating and detecting target RNA from the sample. The containers of the kits can be air tight, waterproof (*e.g.*, impermeable to changes in moisture or evaporation), and/or light-tight.

## III. Therapeutic Nucleic Acids

As used herein, the term "therapeutic nucleic acid" refers to a nucleic acid, such as a DNA or RNA effector molecule, that is administed to a subject, such as for treatment of a disease or disorder. The therapeutic nucleic acid can be used to increase expression of a desired gene product or to decrease expression of the desired gene product. For example, a therapeutic nucleic acid can increase expression of a desired gene product when the therapeutic nucleic acid is an exogenous DNA that expresses a transgene, for example, or when the nucleic acid functions by RNA activation. A therapeutic nucleic acid can decrease expression of a desired gene product, such as when the therapeutic nucleic acid is an inhibitory RNA effector molecule, a nucleic acid that expresses a domininant negative mutant, or an antisense oligonucleotide.

Therapeutic nucleic acids can include ribonucleotides only, deoxyribonucleotides only (*e.g.*, oligodeoxynucleotides), or both deoxyribonucleotides and ribonucleotides. Any of the DNA or RNA effector molecules described below can include both deoxyribonucleotides and ribonucleotides.

*DNA effector molecules*

As used herein the term "DNA effector molecule" refers to deoxyribonucleotide agents that are capable of increasing the expression of a gene product (*e.g.*, therapeutic gene) or decreasing the activity of a protein (*e.g.*, a dominant negative mutant) within a host cell. Such DNA effector molecules can include, *e.g.*, plasmid- or vector-encoded DNA, such as an expressed cDNA. DNA effector molecules can be used to express a transgene, an exogenous gene product, a xenobiotic gene product, a mutated gene product, a dominant negative mutant, or a modified gene product, etc. A DNA effector molecule can be delivered in a course of a gene therapy.

A DNA effector molecule can be used to insert a desired gene into the genome of the subject, *e.g.*, to replace a non-functional gene. Such genes can be inserted randomly into the genome, or swapped for the non-functional gene using homologous recombination techniques.

A number of different vector technologies and gene delivery methods have been proposed and tested for delivering genes *in vivo*, including viral vectors and various nucleic acid encapsulation techniques. For example, DNA effector molecules can be delivered using a viral vector, such as, an adenoviral vector, a retroviral vector, a pseudotyped vector, an Epstein-Barr virus, or a herpes simplex virus. Alternative viral delivery vehicles for genes include murine retroviruses, recombinant adenoviral vectors, adeno-associated virus, HSV, HIV vectors, and baculovirus. Modification of such viral vectors for delivery of a gene to a host cell are known to those of skill in the art.

Nonviral gene delivery methods include use of cationic or neutral liposomes or polymers. For example, the efficiency of gene transfer can be enhanced, e.g., through the use of fusogenic peptides in combination with liposomes or polymers to facilitate the release of plasmid DNA from endosomes.

A DNA effector molecule can be delivered as naked DNA or plasmid DNA. For example, naked DNA can be injected into the tissue where expression of encoded gene is desired. In addition, several physical methods can be utilized to induce uptake of naked DNA or plasmid DNA into a host cell or tissue including, but not limited to, a gene gun, electroporation, sonoporation and magnetofection.

DNA effector molecules can be delivered using lipoplexes (*e.g.*, cationic lipids), polyplexes (*e.g.*, cationic lipid complexes), or dendrimers (*e.g.*, spherical, highly branched macromolecules that are taken up by the cell via endocytosis).

DNA effector molecules can also be delivered by localized injection, such as into muscle tissue or into tumours. Targeted delivery to particular tissues or cells can be accomplished using conjugates of DNA with targeting groups, e.g., plasmid DNA/polylysine/liver targeting group, to target the liver. In one embodiment, conjugates of a therapeutic plasmid (*e.g.*, with a tumour suppressor gene) can be introduced directly into the intestine for gene therapy to treat colon cancer, or plasmid DNA conjugated with liposomes can be delivered topically, directly to skin cells, for gene therapy to treat melanoma or haemophilia B. Targeted delivery of DNA effector molecules can be by intravenous delivery.

Receptor-mediated delivery vehicles can also be employed to deliver a nucleic acid encoding a therapeutic gene into cells, *e.g.*, by taking advantage of the selective uptake of macromolecules by receptor-mediated endocytosis. Because of the cell type-specific distribution of various receptors, the delivery can be highly specific.

A vector or plasmid for gene delivery can include a constitutive or inducible promoter to control the expression of a gene product from a DNA effector molecule in a host cell. Constitutive promoters include, but are not limited to, immediate early cytomegalovirus (CMV) promoter, herpes simplex virus 1 (HSV1) immediate early promoter, SV40 promoter, lysozyme promoter, early and late CMV promoters, early and late HSV promoters, an actin promoter, tubulin promoter, Rous-Sarcoma virus (RSV) promoter, and heat-shock protein (HSP) promoter.

Inducible promoters are regulated by substances either produced or recognized by the transcription control elements within the cell in which the gene is incorporated. An inducible promoter may be a promoter which is inactive or exhibits relatively low activity except in the presence of an inducer substance (or vice versa). Some examples of inducible promoters include, but are not limited to, MT II, MMTV, collagenase, stromelysin, SV40, murine MX gene, $\alpha$-2-macroglobulin, MHC class I gene h-2 kb, proliferin, tumor necrosis factor, or thyroid stimulating hormone $\alpha$ gene.

Inducible promoters also include tissue-specific promoters, developmentally-regulated promoters, and chemically inducible promoters. Examples of tissue-specific promoters include the glucose-6-phosphatase (G6P) promoter, vitellogenin promoter, ovalbumin promoter, ovomucoid promoter, conalbumin promoter, ovotransferrin promoter, prolactin promoter, kidney uromodulin promoter, and placental lactogen promoter.

Examples of chemically inducible promoters include reproductive hormone induced promoters and antibiotic inducible promoters such as the tetracycline inducible promoter and the zinc-inducible metallothionine promoter. Other inducible promoter systems include the Lac operator repressor system inducible by IPTG (isopropyl beta-D-thiogalactoside), ecdysone-based inducible systems; estrogen-based inducible systems; progesterone-based inducible systems using a chimeric regulator, GLVP, which is a hybrid protein consisting of the GAL4 binding domain and the herpes simplex virus transcriptional activation domain, VP16, and a truncated form of the human progesterone receptor that retains the ability to bind ligand and can be turned on by RU486. Chemical substances that activate the chemically inducible promoters can be administered to the animal containing the transgene of interest via any method known to those of skill in the art. Additional promoters known to those of skill in the art for controlling gene expression can also

be practiced with the methods and assays described herein.

Described herein are assays and methods for determining activity of a DNA effector molecule that expresses or encodes a gene product.

*RNA effector molecules*

As used herein, the term "RNA effector molecule" refers to ribonucleotide agents that are capable of reducing or preventing the expression of a target gene within a host cell, or ribonucleotide agents capable of forming a molecule that can reduce the expression level of a target gene within a host cell. RNA effector molecules also include ribonucleotide agents that can increase expression of a desired gene product, such as by RNA activation. RNA effector molecules include, e.g., iRNAs, dsRNAs, antagomirs, miRNAs, miRNA mimics, antisense RNAs, promoter-directed RNAs (pdRNAs), Piwi-interacting RNa (piRNA), RNA activating molecules, expressed interfering RNA (eiRNA), short hairpin RNAs (shRNAs), decoy RNAs and aptamers.

A portion of a RNA effector molecule, wherein the portion is at least 10, at least 12, at least 15, at least 17, at least 18, at least 19, or at least 20 nucleotides long, is substantially complementary to the target gene. Preferably, at least 15 contiguous nucleotides of the RNA effector molecule are complementary to the target sequence (e.g., at least 16, 17, at least 18, at least 19, or more, contiguous nucleotides of the RNA effector molecule are complementary to the target sequence). The RNA effector molecules interact with RNA transcripts of target genes and mediate their selective degradation or otherwise prevent their translation.

RNA effector molecules can comprise a single RNA strand or more than one RNA strand. Examples of RNA effector molecules include, *e.g.*, double stranded RNA (dsRNA), microRNA (miRNA), antisense RNA, promoter-directed RNA (pdRNA), Piwi-interacting RNA (piRNA), expressed interfering RNA (eiRNA), short hairpin RNA (shRNA), antagomirs, decoy RNA, and aptamers. The RNA effector molecule can be single-stranded or double-stranded. A single-stranded RNA effector molecule can have double-stranded regions and a double-stranded RNA effector can have single-stranded regions. Preferably, the RNA effector molecules are double-stranded RNA, wherein the antisense strand comprises a sequence that is substantially complementary to the target gene.

Complementary sequences within a RNA effector molecule, *e.g.*, within a dsRNA (a double-stranded ribonucleic acid) may be fully complementary or substantially complementary. Generally, for a duplex up to 30 base pairs, the dsRNA comprises no more than 5, 4, 3 or 2 mismatched base pairs upon hybridization, while retaining the ability to regulate the expression of its target gene.

The RNA effector molecule may comprise a single-stranded oligonucleotide that interacts with and directs the cleavage of RNA transcripts of a target gene. For example, single stranded RNA effector molecules comprise a 5' modification including one or more phosphate groups or analogs thereof to protect the effector molecule from nuclease degradation. The RNA effector molecule can be a single-stranded antisense nucleic acid having a nucleotide sequence that is complementary to a "sense" nucleic acid of a target gene, *e.g.*, the coding strand of a double-stranded cDNA molecule or a RNA sequence, *e.g.*, a pre-mRNA, mRNA, miRNA, or pre-miRNA. Accordingly, an antisense nucleic acid can form hydrogen bonds with a sense nucleic acid target.

Given a coding strand sequence (*e.g.*, the sequence of a sense strand of a cDNA molecule), antisense nucleic acids can be designed according to the rules of Watson-Crick base pairing. The antisense nucleic acid can be complementary to the coding or noncoding region of a RNA, *e.g.*, the region surrounding the translation start site of a pre-mRNA or mRNA, *e.g.*, the 5' UTR. An antisense oligonucleotide can be, for example, about 10 to 25 nucleotides in length (*e.g.*, 10, 11, 12, 13, 14, 15, 16, 17, 18, 19, 20, 21, 22, 23, or 24 nucleotides in length). The antisense oligonucleotide may comprised one or more modified nucleotides, *e.g.*, phosphorothioate derivatives and/or acridine substituted nucleotides, designed to increase the biological stability of the molecule and/or the physical stability of the duplexes formed between the antisense and target nucleic acids. Antisense oligonucleotides can comprise ribonucleotides only, deoxyribonucleotides only (*e.g.*, oligodeoxynucleotides), or both deoxyribonucleotides and ribonucleotides. For example, an antisense agent consisting only of ribonucleotides can hybridize to a complementary RNA and prevent access of the translation machinery to the target RNA transcript, thereby preventing protein synthesis. An antisense molecule including only deoxyribonucleotides, or deoxyribonucleotides and ribonucleotides, can hybridize to a complementary RNA and the RNA target can be subsequently cleaved by an enzyme, *e.g.*, RNAse H, to prevent translation. The flanking RNA sequences can include 2'-O-methylated nucleotides, and phosphorothioate linkages, and the internal DNA sequence can include phosphorothioate internucleotide linkages. The internal DNA sequence is preferably at least five nucleotides in length when targeting by RNAseH activity is desired.

The RNA effector may comprise a double-stranded ribonucleic acid (dsRNA), wherein said dsRNA (a) comprises a sense strand and an antisense strand that are substantially complementary to each other; and (b) wherein said antisense strand comprises a region of complementarity that is substantially complementary to one of the target genes, and wherein said region of complementarity is from 10 to 30 nucleotides in length.

RNA effector molecule may be a double-stranded oligonucleotide. Typically, the duplex region formed by the

two strands is small, about 30 nucleotides or less in length. Such dsRNA is also referred to as siRNA. For example, the siRNA may be from 15 to 30 nucleotides in length, from 10 to 26 nucleotides in length, from 17 to 28 nucleotides in length, from 18 to 25 nucleotides in length, or from 19 to 24 nucleotides in length, etc.

The duplex region can be of any length that permits specific degradation of a desired target RNA through a RISC pathway, but will typically range from 9 to 36 base pairs in length, *e.g.*, 15 to 30 base pairs in length. For example, the duplex region may be 9, 10, 11, 12, 13, 14, 15, 16, 17, 18, 19, 20, 21, 22, 23, 24, 25, 26, 27, 28, 29, 30, 31, 32, 33, 34, 35, or 36 base pairs in length, or any sub-range there between, including, *e.g.*, 15 to 30 base pairs, 15 to 26 base pairs, 15 to 23 base pairs, 15 to 22 base pairs, 15 to 21 base pairs, 15 to 20 base pairs, 15 to 19 base pairs, 15 to 18 base pairs, 15 to 17 base pairs, 18 to 30 base pairs, 18 to 26 base pairs, 18 to 23 base pairs, 18 to 22 base pairs, 18 to 21 base pairs, 18 to 20 base pairs, 19 to 30 base pairs, 19 to 26 base pairs, 19 to 23 base pairs, 19 to 22 base pairs, 19 to 21 base pairs, 19 to 20 base pairs, 20 to 30 base pairs, 20 to 26 base pairs, 20 to 25 base pairs, 20 to 24 base pairs, 20 to 23 base pairs, 20 to 22 base pairs, 20 to 21 base pairs, 21 to 30 base pairs, 21 to 26 base pairs, 21 to 25 base pairs, 21 to 24 base pairs, 21 to 23 base pairs, or 21 to 22 base pairs.

The two strands forming the duplex structure of a dsRNA can be from a single RNA molecule having at least one self-complementary region, or can be formed from two or more separate RNA molecules. Where the duplex region is formed from two strands of a single molecule, the molecule can have a duplex region separated by a single stranded chain of nucleotides (a "hairpin loop") between the 3'-end of one strand and the 5'-end of the respective other strand forming the duplex structure. The hairpin loop can comprise at least one unpaired nucleotide; in some embodiments the hairpin loop can comprise at least 3, at least 4, at least 5, at least 6, at least 7, at least 8, at least 9, at least 10, at least 20, at least 23 or more unpaired nucleotides. Where the two substantially complementary strands of a dsRNA are formed by separate RNA strands, the two strands can be optionally covalently linked. Where the two strands are connected covalently by means other than a hairpin loop, the connecting structure is referred to as a "linker."

It is known that dsRNAs having a duplex structure of between 20 and 23, but specifically 21, base pairs have been hailed as particularly effective in inducing RNA interference. Elbashir et al., 20 EMBO 6877-88 (2001).

A double-stranded oligonucleotide can include one or more single-stranded nucleotide overhangs, which are one or more unpaired nucleotide that protrudes from the terminus of a duplex structure of a double-stranded oligonucleotide, *e.g.*, a dsRNA. A double-stranded oligonucleotide can comprise an overhang of at least one nucleotide; alternatively the overhang can comprise at least two nucleotides, at least three nucleotides, at least four nucleotides, at least five nucleotides or more. The overhang(s) can be on the sense strand, the antisense strand or any combination thereof. Furthermore, the nucleotide(s) of an overhang can be present on the 5' end, 3' end, or both ends of either an antisense or sense strand of a dsRNA.

At least one end of a dsRNA may have a single-stranded nucleotide overhang of 1 to 4, generally 1 or 2 nucleotides.

The overhang can comprise a deoxyribonucleoside or a nucleoside analog. Further, one or more of the internucloside linkages in the overhang can be replaced with a phosphorothioate. The overhang may comprise one or more deoxyribonucleoside or the overhang comprises one or more dT, *e.g.*, the sequence 5'-dTdT-3' or 5'-dTdTdT-3'. Overhang may comprise the sequence 5'-dT*dT-3, wherein * is a phosphorothioate internucleoside linkage.

An RNA effector molecule as described herein can contain one or more mismatches to the target sequence. Preferably, a RNA effector molecule as described herein contains no more than three mismatches. If the antisense strand of the RNA effector molecule contains one or more mismatches to a target sequence, it is preferable that the mismatch(s) is (are) not located in the center of the region of complementarity, but are restricted to be within the last 5 nucleotides from either the 5' or 3' end of the region of complementarity. For example, for a 23-nucleotide RNA effector molecule agent RNA, the antisense strand generally does not contain any mismatch within the central 13 nucleotides.

The dsRNA can be synthesized by standard methods known in the art as further discussed below, *e.g.*, by use of an automated DNA synthesizer, such as are commercially available from, for example, Biosearch Technologies (Novato, CA).

The RNA effector molecule may be a promoter-directed RNA (pdRNA) which is substantially complementary to a noncoding region of an mRNA transcript of a target gene. The pdRNA may be substantially complementary to the promoter region of a target gene mRNA at a site located upstream from the transcription start site, *e.g.*, more than 100, more than 200, or more than 1,000 bases upstream from the transcription start site. The pdRNA may be substantially complementary to the 3'-UTR of a target gene mRNA transcript. The pdRNA may comprise dsRNA of 18-28 bases optionally having 3' di- or tri-nucleotide overhangs on each strand. The pdRNA may comprise a gapmer consisting of a single stranded polynucleotide comprising a DNA sequence which is substantially complementary to the promoter or the 3'-UTR of a target gene mRNA transcript, and flanking the polynucleotide sequences (*e.g.*, comprising the 5 terminal bases at each of the 5' and 3' ends of the gapmer) comprises one or more modified nucleotides, such as 2' MOE, 2'OMe, or Locked Nucleic Acid bases (LNA), which protect the gapmer from cellular nucleases.

pdRNA can be used to selectively increase, decrease, or otherwise modulate expression of a target gene. Without being limited to theory, it is believed that pdRNAs modulate expression of target genes by binding to endogenous

antisense RNA transcripts which overlap with noncoding regions of a target gene mRNA transcript, and recruiting Argonaute proteins (in the case of dsRNA) or host cell nucleases (*e.g.*, RNase H) (in the case of gapmers) to selectively degrade the endogenous antisense RNAs. The endogenous antisense RNA may negatively regulated expression of the target gene and the pdRNA effector molecule activates expression of the target gene. Thus, in some embodiments, pdRNAs can be used to selectively activate the expression of a target gene by inhibiting the negative regulation of target gene expression by endogenous antisense RNA. Methods for identifying antisense transcripts encoded by promoter sequences of target genes and for making and using promoter-directed RNAs are known, *see*, *e.g.*, WO 2009/046397.

The RNA effector molecule may comprise an aptamer which binds to a non-nucleic acid ligand, such as a small organic molecule or protein, *e.g.*, a transcription or translation factor, and subsequently modifies (*e.g.*, inhibits) activity. An aptamer can fold into a specific structure that directs the recognition of a targeted binding site on the non-nucleic acid ligand. Aptamers can contain any of the modifications described herein.

The RNA effector molecule may comprise an antagomir. Antagomirs are single stranded, double stranded, partially double stranded or hairpin structures that target a microRNA. An antagomir consists essentially of or comprises at least 10 or more contiguous nucleotides substantially complementary to an endogenous miRNA and more particularly a target sequence of an miRNA or pre-miRNA nucleotide sequence. Antagomirs preferably have a nucleotide sequence sufficiently complementary to a miRNA target sequence of about 12 to 25 nucleotides, such as about 15 to 23 nucleotides, to allow the antagomir to hybridize to the target sequence. More preferably, the target sequence differs by no more than 1, 2, or 3 nucleotides from the sequence of the antagomir. In some embodiments, the antagomir includes a non-nucleotide moiety, *e.g.*, a cholesterol moiety, which can be attached, *e.g.*, to the 3' or 5' end of the oligonucleotide agent.

Antagomirs may be stabilized against nucleolytic degradation by the incorporation of a modification, e.g., a nucleotide modification. For example, in some embodiments, antagomirs contain a phosphorothioate comprising at least the first, second, and/or third internucleotide linkages at the 5' or 3' end of the nucleotide sequence. In further embodiments, antagomirs include a 2'-modified nucleotide, e.g., a 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), or 2'-O-N-methylacetunido (2'-O-NMA). In some embodiments, antagomirs include at least one 2'-O-methyl-modified nucleotide.

The RNA effector molecule may be a promoter-directed RNA (pdRNA) which is substantially complementary to a noncoding region of an mRNA transcript of a target gene. The pdRNA can be substantially complementary to the promoter region of a target gene mRNA at a site located upstream from the transcription start site, *e.g.*, more than 100, more than 200, or more than 1,000 bases upstream from the transcription start site. Also, the pdRNA can substantially complementary to the 3'-UTR of a target gene mRNA transcript. For example, the pdRNA comprises dsRNA of 18 to 28 bases optionally having 3' di- or tri-nucleotide overhangs on each strand. The dsRNA is substantially complementary to the promoter region or the 3'-UTR region of a target gene mRNA transcript. The pdRNA may comprise a gapmer consisting of a single stranded polynucleotide comprising a DNA sequence which is substantially complementary to the promoter or the 3'-UTR of a target gene mRNA transcript, and flanking the polynucleotide sequences (*e.g.*, comprising the five terminal bases at each of the 5' and 3' ends of the gapmer) comprising one or more modified nucleotides, such as 2'MOE, 2'OMe, or Locked Nucleic Acid bases (LNA), which protect the gapmer from cellular nucleases.

Expressed interfering RNA (eiRNA) can be used to selectively increase, decrease, or otherwise modulate expression of a target gene. Typically, eiRNA, the dsRNA is expressed in the first transfected cell from an expression vector. In such a vector, the sense strand and the antisense strand of the dsRNA can be transcribed from the same nucleic acid sequence using *e.g.*, two convergent promoters at either end of the nucleic acid sequence or separate promoters transcribing either a sense or antisense sequence. Alternatively, two plasmids can be cotransfected, with one of the plasmids designed to transcribe one strand of the dsRNA while the other is designed to transcribe the other strand. Methods for making and using eiRNA effector molecules are known in the art. *See*, *e.g.*, WO 2006/033756; U.S. Patent Pubs. No. 2005/0239728 and No. 2006/0035344.

The RNA effector molecule may comprise a small single-stranded Piwi-interacting RNA (piRNA effector molecule) which is substantially complementary to a target gene, and which selectively binds to proteins of the Piwi or Aubergine subclasses of Argonaute proteins. A piRNA effector molecule can be about 10 to 50 nucleotides in length, about 25 to 39 nucleotides in length, or about 26 to 31 nucleotides in length. *See*, *e.g.*, U.S. Patent Application Pub. No. 2009/0062228.

MicroRNAs are a highly conserved class of small RNA molecules that are transcribed from DNA in the genomes of plants and animals, but are not translated into protein. Pre-microRNAs are processed into miRNAs. Processed microRNAs are single stranded ~17 to 25 nucleotide (nt) RNA molecules that become incorporated into the RNA-induced silencing complex (RISC) and have been identified as key regulators of development, cell proliferation, apoptosis and differentiation. They are believed to play a role in regulation of gene expression by binding to the 3'-untranslated region of specific mRNAs. MicroRNAs cause post-transcriptional silencing of specific target genes, *e.g.*, by inhibiting translation or initiating degradation of the targeted mRNA. The miRNA may be completely complementary with the target nucleic acid. The miRNA may also have a region of noncomplementarity with the target nucleic acid, resulting in a "bulge" at

the region of non-complementarity. The region of noncomplementarity (the bulge) may be flanked by regions of sufficient complementarity, *e.g.*, complete complementarity, to allow duplex formation. For example, the regions of complementarity are at least 8 to 10 nucleotides long (e.g., 8, 9, or 10 nucleotides long).

miRNA can inhibit gene expression by, *e.g.*, repressing translation, such as when the miRNA is not completely complementary to the target nucleic acid, or by causing target RNA degradation, when the miRNA binds its target with perfect or a high degree of complementarity. The RNA effector molecule can include an oligonucleotide agent which targets an endogenous miRNA or pre-miRNA. For example, the RNA effector can target an endogenous miRNA which negatively regulates expression of a target gene, such that the RNA effector alleviates miRNA-based inhibition of the target gene.

The miRNA can comprise naturally occurring nucleobases, sugars, and covalent internucleotide (backbone) linkages, or comprise one or more non-naturally-occurring features that confer desirable properties, such as enhanced cellular uptake, enhanced affinity for the endogenous miRNA target, and/or increased stability in the presence of nucleases. In some embodiments, an miRNA designed to bind to a specific endogenous miRNA has substantial complementarity, e.g., at least 70%, 80%, 90%, or 100% complementary, with at least 10, 20, or 25 or more bases of the target miRNA. Exemplary oligonucleotide agents that target miRNAs and pre-miRNAs are described, for example, in U.S. Patent Pubs. No. 20090317907, No. 20090298174, No. 20090291907, No. 20090291906, No. 20090286969, No. 20090236225, No. 20090221685, No. 20090203893, No. 20070049547, No. 20050261218, No. 20090275729, No. 20090043082, No. 20070287179, No. 20060212950, No. 20060166910, No. 20050227934, No. 20050222067, No. 20050221490, No. 20050221293, No. 20050182005, and No. 20050059005.

A miRNA or pre-miRNA can be 10 to 200 nucleotides in length, for example from 16 to 80 nucleotides in length. Mature miRNAs can have a length of 16 to 30 nucleotides, such as 21 to 25 nucleotides, particularly 21, 22, 23, 24, or 25 nucleotides in length. miRNA precursors can have a length of 70 to 100 nucleotides and can have a hairpin conformation. miRNAs may be generated *in vivo* from pre-miRNAs by the enzymes cDicer and Drosha. miRNAs or pre-miRNAs can be synthesized *in vivo* by a cell-based system or can be chemically synthesized. miRNAs can comprise modifications which impart one or more desired properties, such as superior stability, hybridization thermodynamics with a target nucleic acid, targeting to a particular tissue or cell-type, and/or cell permeability, *e.g.*, by an endocytosis-dependent or -independent mechanism. Modifications can also increase sequence specificity, and consequently decrease off-site targeting.

Described herein are assays and methods for determining activity of a therapeutic nucleic acid, such as an RNA effector molecule, that modulates the expression of a target gene in a subject.

## IV. Double-Stranded RNA

The iRNA agent may include double-stranded ribonucleic acid (dsRNA) molecules for inhibiting the expression of the target gene in a cell or mammal. The dsRNA includes an antisense strand having a region of complementarity which is complementary to at least a part of an mRNA formed in the expression of a target gene. The region of complementarity is 30 nucleotides or less in length, generally 19-24 nucleotides in length, and where the dsRNA, upon contact with a cell expressing the target gene, inhibits the expression of the target gene by at least 10% as assayed by, for example, a PCR or branched DNA (bDNA)-based method, or by a protein-based method, such as by Western blot. The iRNA agent may activate the expression of a target gene in a cell or mammal. Expression of the target gene in cell culture, such as in COS cells, HeLa cells, primary hepatocytes, HepG2 cells, primary cultured cells or in a biological sample from a subject, can be assayed by measuring mRNA levels, such as by bDNA or TaqMan® assay, or by measuring protein levels, such as by immunofluorescence analysis, using, for example, Western blotting or flow cytometric techniques.

A dsRNA includes two RNA strands that are complementary and hybridize to form a duplex structure under conditions in which the dsRNA will be used. One strand of a dsRNA (the antisense strand) includes a region of complementarity that is substantially complementary, and generally fully complementary, to a target sequence. The target sequence can be derived from the sequence of an mRNA formed during the expression of a target gene. The other strand (the sense strand) includes a region that is complementary to the antisense strand, such that the two strands hybridize and form a duplex structure when combined under suitable conditions. Generally, the duplex structure is between 15 and 30 base pairs inclusive, more generally between 18 and 25 inclusive, yet more generally between 19 and 24 inclusive, and most generally between 19 and 21 base pairs in length, inclusive. Similarly, the region of complementarity to the target sequence is between 15 and 30 base pairs in length inclusive, more generally between 18 and 25 inclusive, yet more generally between 19 and 24 inclusive, and most generally between 19 and 21 nucleotides in length, inclusive. The dsRNA may be between 15 and 20 nucleotides in length, inclusive, and also the dsRNA is between 25 and 30 nucleotides in length, inclusive. As the ordinarily skilled person will recognize, the targeted region of an RNA targeted for cleavage will most often be part of a larger RNA molecule, often an mRNA molecule. Where relevant, a "part" of an mRNA target is a contiguous sequence of an mRNA target of sufficient length to be a substrate for RNAi-directed cleavage (*i.e.*, cleavage through a RISC pathway). dsRNAs having duplexes as short as 9 base pairs can, under some circumstances, mediate RNAi-directed RNA cleavage. Most often a target will be at least 15 nucleotides in length, *e.g.*, 15-30 nucleotides in length.

One of skill in the art will also recognize that the duplex region is a primary functional portion of a dsRNA, *e.g.*, a duplex region of 9 to 36, *e.g.*, 15-30 base pairs. Thus, in one embodiment, to the extent that it becomes processed to a functional duplex of, *e.g.*, 15-30 base pairs that targets a desired RNA for cleavage, an RNA molecule or complex of RNA molecules having a duplex region greater than 30 base pairs is a dsRNA. Thus, an ordinarily skilled artisan will recognize an miRNA may be a dsRNA. A dsRNA may not be naturally occurring miRNA. An iRNA agent useful to target target gene expression may not be generated in the target cell by cleavage of a larger dsRNA.

A dsRNA as described herein can further include one or more single-stranded nucleotide overhangs. The dsRNA can be synthesized by standard methods known in the art as further discussed below, *e.g.*, by use of an automated DNA synthesizer, such as are commercially available from, for example, Biosearch, Applied Biosystems, Inc. A target gene may be a human gene. The target gene may also be a mouse or a rat gene.

The dsRNA may target proprotein convertase subtilisin/kexin type 9 (PCSK9) (GenBank Accession No. NM_174936), transthyretin (TTR) (GenBank reference NM_000371.3), vascular endothelial growth factor A (VEGF) (GenBank Accession No. NM_001025366.2), kinesin-like protein (KSP) (GenBank Accession No. NM_004523), Parotid proline-rich salivary protein Pc (PCS) (GenBank Accession No. NM_020872.1), angiopoietin-like 3 (ANGPTL3) (GenBank Accession No. NM_014495.2), apolipoprotein C-III (ApoC3) (GenBank Accession No. NM_000040.1), Hepcidin (SLC11A2) (GenBank Accession No. NM_001174129.1), egl nine homolog (EGLN) (GenBank Accession No. BC111057.1), hepatitis C Virus (HCV) (GenBank Accession No. AK367384.1), Hepatitis B Virus (HBV) (GenBank Accession No. AB602818.1), polo-like kinase 1 (PLK) (GenBank Accession No. NM_005030.3), alpha-antitrypsin (AAT) (GenBank Accession No. NM_000295.4), activated protein C, transmembrane protease, serine 6 (TMPRSS6) (GenBank Accession No. NM_153609.2), Kruppel-like factor 4 (KLF) (GenBank Accession No. DQ658241.1), B-cell CLL/lymphoma 11A (BCL11A) (GenBank Accession No. NM_022893.3), p53 (GenBank Accession No. NM_001126114.1), caspase 2 (GenBank Accession No. NM_001224.4), β-catenin (GenBank Accession No. NM_001904.3), protein kinase N3 (PKN3) (GenBank Accession No. NM_013355.3), huntingtin (HTT) (Genbank Accession No. NM_002111.6) or synuclein, alpha (SNCA) (GenBank Accession No. NM_007308.2).

Thus, in one aspect, a dsRNA will include at least two nucleotide sequences, a sense and an antisense sequence, whereby the sense strand is selected from the groups of sequences provided in Table 2 or Table 3. In this aspect, one of the two sequences is complementary to the other of the two sequences, with one of the sequences being substantially complementary to a sequence of an mRNA generated in the expression of a target gene. As such, in this aspect, a dsRNA will include two oligonucleotides, where one oligonucleotide is described as the sense strand in Table 2 or Table 3, and the second oligonucleotide is described as the corresponding antisense strand of the sense strand from Table 2 or Table 3. As described elsewhere herein and as known in the art, the complementary sequences of a dsRNA can also be contained as self complementary regions of a single nucleic acid molecule, as opposed to being on separate oligonucleotides.

The skilled person is well aware that dsRNAs having a duplex structure of between 20 and 23, but specifically 21, base pairs have been hailed as particularly effective in inducing RNA interference (Elbashir et al., EMBO 2001, 20:6877-6888). However, others have found that shorter or longer RNA duplex structures can be effective as well. In the aspects described above, by virtue of the nature of the oligonucleotide sequences provided in Table 2 or Table 3, dsRNAs described herein can include at least one strand of a length of minimally 21 nt. It can be reasonably expected that shorter duplexes having one of the sequences of Table 2 or Table 3 minus only a few nucleotides on one or both ends may be similarly effective as compared to the dsRNAs described above. Hence, dsRNAs having a partial sequence of at least 15, 16, 17, 18, 19, 20, or more contiguous nucleotides from one of the sequences of Table 2 or Table 3, and differing in their ability to inhibit the expression of a target gene by not more than 5, 10, 15, 20, 25, or 30 % inhibition from a dsRNA comprising the full sequence, are also described herein.

In addition, the RNAs provided in Table 2 or Table 3 identify a site in a TTR or HTT transcript that is susceptible to RISC-mediated cleavage. As such, also described herein are iRNAs that target within one of such sequences. As used herein, an iRNA is said to target within a particular site of an RNA transcript if the iRNA promotes cleavage of the transcript anywhere within that particular site. Such an iRNA will generally include at least 15 contiguous nucleotides from one of the sequences provided in Table 2 or Table 3 coupled to additional nucleotide sequences taken from the region contiguous to the selected sequence in a TTR or HTT gene.

While a target sequence is generally 15-30 nucleotides in length, there is wide variation in the suitability of particular sequences in this range for directing cleavage of any given target RNA. Various software packages and the guidelines set out herein provide guidance for the identification of optimal target sequences for any given gene target, but an empirical approach can also be taken in which a "window" or "mask" of a given size (as a non-limiting example, 21 nucleotides) is literally or figuratively (including, *e.g.*, *in silico*) placed on the target RNA sequence to identify sequences in the size range that may serve as target sequences. By moving the sequence "window" progressively one nucleotide

upstream or downstream of an initial target sequence location, the next potential target sequence can be identified, until the complete set of possible sequences is identified for any given target size selected. This process, coupled with systematic synthesis and testing of the identified sequences (using assays as described herein or as known in the art) to identify those sequences that perform optimally can identify those RNA sequences that, when targeted with an iRNA agent, mediate the best inhibition of target gene expression. Thus, while the sequences identified, for example, in Table 2 and Table 3 represent effective target sequences to TTR and HTT, it is contemplated that further optimization of inhibition efficiency can be achieved by progressively "walking the window" one nucleotide upstream or downstream of the given sequences to identify sequences with equal or better inhibition characteristics.

Further, it is contemplated that for any sequence identified, *e.g.*, in Table 2 or Table 3, further optimization could be achieved by systematically either adding or removing nucleotides to generate longer or shorter sequences and testing those sequences generated by walking a window of the longer or shorter size up or down the target RNA from that point. Again, coupling this approach to generating new candidate targets with testing for effectiveness of iRNAs based on those target sequences in an inhibition assay as known in the art or as described herein can lead to further improvements in the efficiency of inhibition. Further still, such optimized sequences can be adjusted by, *e.g.*, the introduction of modified nucleotides as described herein or as known in the art, addition or changes in overhang, or other modifications as known in the art and/or discussed herein to further optimize the molecule (*e.g.*, increasing serum stability or circulating half-life, increasing thermal stability, enhancing transmembrane delivery, targeting to a particular location or cell type, increasing interaction with silencing pathway enzymes, increasing release from endosomes, etc.) as an expression inhibitor.

An iRNA as described herein can contain one or more mismatches to the target sequence. An iRNA as described herein may contain no more than 3 mismatches. If the antisense strand of the iRNA contains mismatches to a target sequence, it is typical that the area of mismatch is not located in the center of the region of complementarity. If the antisense strand of the iRNA contains mismatches to the target sequence, it is typical that the mismatch be restricted to be within the last 5 nucleotides from either the 5' or 3' end of the region of complementarity. For example, for a 23 nucleotide iRNA agent RNA strand which is complementary to a region of a target gene, the RNA strand generally does not contain any mismatch within the central 13 nucleotides. The methods described herein or methods known in the art can be used to determine whether an iRNA containing a mismatch to a target sequence is effective in inhibiting the expression of a target gene. Consideration of the efficacy of iRNAs with mismatches in inhibiting expression of the target gene is important, especially if the particular region of complementarity in a target gene is known to have polymorphic sequence variation within the population.

At least one end of a dsRNA may have a single-stranded nucleotide overhang of 1 to 4, generally 1 or 2 nucleotides. Such dsRNAs having at least one nucleotide overhang have unexpectedly superior inhibitory properties relative to their blunt-ended counterparts. The RNA of an iRNA, *e.g.*, a dsRNA, may be chemically modified to enhance stability or other beneficial characteristics. The nucleic acids featured herein may be synthesized and/or modified by methods well established in the art, such as those described in "Current protocols in nucleic acid chemistry," Beaucage, S.L. et al. (Edrs.), John Wiley & Sons, Inc., New York, NY, USA. Any modification known to those of skill in the art can be used in the design of a therapeutic iRNA for treatment of a disease in a subject. Modifications include, for example, (a) end modifications, *e.g.*, 5' end modifications (phosphorylation, conjugation, inverted linkages, etc.) 3' end modifications (conjugation, DNA nucleotides, inverted linkages, etc.), (b) base modifications, *e.g.*, replacement with stabilizing bases, destabilizing bases, or bases that base pair with an expanded repertoire of partners, removal of bases (abasic nucleotides), or conjugated bases, (c) sugar modifications (*e.g.*, at the 2' position or 4' position) or replacement of the sugar, as well as (d) backbone modifications, including modification or replacement of the phosphodiester linkages.

An iRNA may contain a ligand that alters the distribution, targeting or lifetime of the iRNA agent. A ligand may provide an enhanced affinity for a selected target, *e.g.*, molecule, cell or cell type (*e.g.*, a liver cell, such as a hepatocyte), compartment, *e.g.*, a cellular or organ compartment, tissue, organ or region of the body, as, *e.g.*, compared to a species absent such a ligand. Certain ligands will not take part in duplex pairing in a duplexed nucleic acid.

Ligands can include a naturally occurring substance, such as a protein (*e.g.*, human serum albumin (HSA), low-density lipoprotein (LDL), or globulin); carbohydrate (*e.g.*, a dextran, pullulan, chitin, chitosan, inulin, cyclodextrin or hyaluronic acid); or a lipid. The ligand may also be a recombinant or synthetic molecule, such as a synthetic polymer, *e.g.*, a synthetic polyamino acid. Examples of polyamino acids include polyamino acid is a polylysine (PLL), poly L aspartic acid, poly L-glutamic acid, styrenemaleic acid anhydride copolymer, poly(L-lactide-co-glycolide) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Examples of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an $\alpha$ helical peptide.

Ligands can also include targeting groups, *e.g.*, a cell or tissue targeting agent, *e.g.*, a lectin, glycoprotein, lipid or protein, *e.g.*, an antibody, that binds to a specified cell type such as a kidney cell. A targeting group can be a thyrotropin,

melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, vitamin A, biotin, or an RGD peptide or RGD peptide mimetic.

Other examples of ligands include dyes, intercalating agents (*e.g.* acridines), cross-linkers (*e.g.* psoralene, mitomycin C), porphyrins (TPPC4, texaphyrin, Sapphyrin), polycyclic aromatic hydrocarbons (*e.g.*, phenazine, dihydrophenazine), artificial endonucleases (*e.g.* EDTA), lipophilic molecules, *e.g.*, cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid,O3-(oleoyl)lithocholic acid, O3-(oleoyl)cholenic acid, dimethoxytrityl, or phenoxazine)and peptide conjugates (*e.g.*, antennapedia peptide, Tat peptide), alkylating agents, phosphate, amino, mercapto, PEG (*e.g.*, PEG-40K), MPEG, [MPEG]2, polyamino, alkyl, substituted alkyl, radiolabeled markers, enzymes, haptens (*e.g.* biotin), transport/absorption facilitators (*e.g.*, aspirin, vitamin E, folic acid), synthetic ribonucleases (*e.g.*, imidazole, bisimidazole, histamine, imidazole clusters, acridine-imidazole conjugates, Eu3+ complexes of tetraazamacrocycles), dinitrophenyl, HRP, or AP.

Ligands can be proteins, *e.g.*, glycoproteins, or peptides, *e.g.*, molecules having a specific affinity for a co-ligand, or antibodies *e.g.*, an antibody, that binds to a specified cell type such as a cancer cell, endothelial cell, or bone cell. Ligands may also include hormones and hormone receptors. They can also include non-peptidic species, such as lipids, lectins, carbohydrates, vitamins, cofactors, multivalent lactose, multivalent galactose, N-acetyl-galactosamine, N-acetyl-gulucosamine multivalent mannose, or multivalent fucose. The ligand can be, for example, a lipopolysaccharide, an activator of p38 MAP kinase, or an activator of NF-κB.

The ligand can be a substance, *e.g.*, a drug, which can increase the uptake of the iRNA agent into the cell, for example, by disrupting the cell's cytoskeleton, *e.g.,* by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

A ligand attached to an iRNA as described herein may act as a PK modulator. As used herein, a "PK modulator" refers to a pharmacokinetic modulator. PK modulators include lipophiles, bile acids, steroids, phospholipid analogues, peptides, protein binding agents, PEG, vitamins etc. Exemplary PK modulators include, but are not limited to, cholesterol, fatty acids, cholic acid, lithocholic acid, dialkylglycerides, diacylglyceride, phospholipids, sphingolipids, naproxen, ibuprofen, vitamin E, biotin etc. Oligonucleotides that comprise a number of phosphorothioate linkages are also known to bind to serum protein, thus short oligonucleotides, *e.g.*, oligonucleotides of about 5 bases, 10 bases, 15 bases or 20 bases, comprising multiple phosphorothioate linkages in the backbone are also amenable to the present disclosure as ligands (*e.g.*, as PK modulating ligands). In addition, aptamers that bind serum components (*e.g.*, serum proteins) are also suitable for use as PK modulating ligands in the embodiments described herein.

The iRNA may comprise a formulation that prevents entrapment of the iRNA in an endosomal or lysosomal compartment of the cell. The endosomolytic components described herein can be polyanionic peptides or peptidomimetics which show pH-dependent membrane activity and/or fusogenicity. A peptidomimetic can be a small protein-like chain designed to mimic a peptide. A peptidomimetic can arise from modification of an existing peptide in order to alter the molecule's properties, or the synthesis of a peptide-like molecule using unnatural amino acids or their analogs. They may have improved stability and/or biological activity when compared to a peptide. In certain embodiments, the endosomolytic component assumes its active conformation at endosomal pH (*e.g.*, pH 5-6). The "active" conformation is that conformation in which the endosomolytic component promotes lysis of the endosome and/or transport of the modular composition featured herein, or its any of its components (*e.g.*, a nucleic acid), from the endosome to the cytoplasm of the cell.

Exemplary endosomolytic components include the GALA peptide (Subbarao et al., Biochemistry, 1987, 26: 2964-2972), the EALA peptide (Vogel et al., J. Am. Chem. Soc., 1996, 118: 1581-1586), and their derivatives (Turk et al., Biochem. Biophys. Acta, 2002, 1559: 56-68). The endosomolytic component can contain a chemical group (*e.g.*, an amino acid) which will undergo a change in charge or protonation in response to a change in pH. The endosomolytic component may be linear or branched. Exemplary primary sequences of endosomolytic components include H2N-(AALEALAEALEALAEALEALAEAAAAGGC) CO2H (SEQ ID NO:2); H2N-(AALAEALAEALAEALAEALAEALAAAAGGC)-CO2H (SEQ ID NO:3); and H2N-(ALEALAEALEALAEA)-CONH2 (SEQ ID NO:4).

More than one endosomolytic component can be incorporated into the iRNA agent featured herein. This may entail incorporating more than one of the same endosomolytic component into the iRNA agent. This may also entail incorporating two or more different endosomolytic components into iRNA agent.

*Lipid Conjugates.* The ligand or conjugate may be lipid or lipid-based molecule. Such a lipid or lipid-based molecule typically binds a serum protein, *e.g.*, human serum albumin (HSA). An HSA binding ligand allows for distribution of the conjugate to a target tissue, *e.g.*, a non-kidney target tissue of the body. For example, the target tissue can be the liver, including parenchymal cells of the liver. Other molecules that can bind HSA can also be used as ligands. For example, neproxin or aspirin can be used. A lipid or lipid-based ligand can (a) increase resistance to degradation of the

conjugate, (b) increase targeting or transport into a target cell or cell membrane, and/or (c) can be used to adjust binding to a serum protein, *e.g.*, HSA.

A lipid based ligand can be used to modulate, *e.g.*, control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

The lipid based ligand may bind HSA. Typically, it binds HSA with a sufficient affinity such that the conjugate will be distributed to a non-kidney tissue. However, the affinity should not be so strong that the HSA-ligand binding cannot be reversed.

The lipid based ligand may bind HSA weakly or not at all, such that the conjugate will be distributed to the kidney. Other moieties that target to kidney cells can also be used in place of or in addition to the lipid based ligand.

In another aspect, the ligand is a moiety, *e.g.,* a vitamin, which is taken up by a target cell, *e.g.,* a proliferating cell. These are particularly useful for treating disorders characterized by unwanted cell proliferation, *e.g.,* of the malignant or non-malignant type, *e.g.*, cancer cells. Exemplary vitamins include vitamin A, E, and K. Other exemplary vitamins include are B vitamin, *e.g.*, folic acid, B12, riboflavin, biotin, pyridoxal or other vitamins or nutrients taken up by cancer cells. Also included are HSA and low density lipoprotein (LDL).

*Cell Permeation Peptides.* Peptides suitable for use in administering an iRNA to a subject can be a natural peptide, *e.g.,* tat or antennapedia peptide, a synthetic peptide, or a peptidomimetic. Furthermore, the peptide can be a modified peptide, for example, a peptide can comprise non-peptide or pseudo-peptide linkages, and D-amino acids. A peptidomimetic (also referred to herein as an oligopeptidomimetic) is a molecule capable of folding into a defined three-dimensional structure similar to a natural peptide. The attachment of peptide and peptidomimetics to iRNA agents can affect pharmacokinetic distribution of the iRNA, such as by enhancing cellular recognition and absorption. The peptide or peptidomimetic moiety can be about 5-50 amino acids long, *e.g.*, about 5, 10, 15, 20, 25, 30, 35, 40, 45, or 50 amino acids long.

A peptide or peptidomimetic can be, for example, a cell permeation peptide, cationic peptide, amphipathic peptide, or hydrophobic peptide (*e.g.*, consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP (SEQ ID NO:5). An RFGF analogue (*e.g.*, amino acid sequence AALLPVLLAAP (SEQ ID NO:6)) containing a hydrophobic MTS can also be a targeting moiety. The peptide moiety can be a "delivery" peptide, which can carry large polar molecules including peptides, oligonucleotides, and protein across cell membranes. For example, sequences from the HIV Tat protein (GRKKRRQRRRPPQ (SEQ ID NO:7)) and the Drosophila Antennapedia protein (RQIKIWFQNRRMKWKK (SEQ ID NO: 8)) have been found to be capable of functioning as delivery peptides. A peptide or peptidomimetic can be encoded by a random sequence of DNA, such as a peptide identified from a phage-display library, or one-bead-one-compound (OBOC) combinatorial library (Lam et al., Nature, 354:82-84, 1991). For example, the peptide or peptidomimetic tethered to the lipid can be a cell-targeting peptide such as an arginine-glycine-aspartic acid (RGD)-peptide, or RGD mimic. A peptide moiety can range in length from about 5 amino acids to about 40 amino acids. The peptide moieties can have a structural modification, such as to increase stability or direct conformational properties. Any of the structural modifications described below can be utilized.

An RGD peptide moiety can be used to target a tumor cell, such as an endothelial tumor cell or a breast cancer tumor cell (Zitzmann et al., Cancer Res., 62:5139-43, 2002). An RGD peptide can facilitate targeting of an dsRNA agent to tumors of a variety of other tissues, including the lung, kidney, spleen, or liver (Aoki et al., Cancer Gene Therapy 8:783-787, 2001). The RGD peptide may facilitate targeting of an iRNA agent to the kidney. The RGD peptide can be linear or cyclic, and can be modified, *e.g.*, glycosylated or methylated to facilitate targeting to specific tissues. For example, a glycosylated RGD peptide can deliver a iRNA agent to a tumor cell expressing $\alpha V\beta 3$ (Haubner et al., Jour. Nucl. Med., 42:326-336, 2001).

Peptides that target markers enriched in proliferating cells can be used. *E.g.*, RGD containing peptides and peptidomimetics can target cancer cells, in particular cells that exhibit an $\alpha v\beta 3$ integrin. Thus, one could use RGD peptides, cyclic peptides containing RGD, RGD peptides that include D-amino acids, as well as synthetic RGD mimics. In addition to RGD, one can use other moieties that target the $\alpha v\beta 3$ integrin ligand. Generally, such ligands can be used to control proliferating cells and angiogeneis.

A "cell permeation peptide" is capable of permeating a cell, *e.g.,* a microbial cell, such as a bacterial or fungal cell, or a mammalian cell, such as a human cell. A microbial cell-permeating peptide can be, for example, an $\alpha$-helical linear peptide (*e.g.*, LL-37 or Ceropin PI), a disulfide bond-containing peptide (*e.g.*, $\alpha$-defensin, $\beta$-defensin or bactenecin), or a peptide containing only one or two dominating amino acids (*e.g.*, PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV-1 gp41 and the NLS of SV40 large T antigen (Simeoni et al., Nucl. Acids Res. 31:2717-2724, 2003).

*Carbohydrate Conjugates.* The iRNA oligonucleotides described herein may further comprise carbohydrate conjugates. The carbohydrate conjugates are advantageous for the *in vivo* delivery of nucleic acids, as well as compositions suitable for *in vivo* therapeutic use, as described herein. As used herein, "carbohydrate" refers to a compound which is either a carbohydrate per se made up of one or more monosaccharide units having at least 6 carbon atoms (which may be linear, branched or cyclic) with an oxygen, nitrogen or sulfur atom bonded to each carbon atom; or a compound having as a part thereof a carbohydrate moiety made up of one or more monosaccharide units each having at least six carbon atoms (which may be linear, branched or cyclic), with an oxygen, nitrogen or sulfur atom bonded to each carbon atom. Representative carbohydrates include the sugars (mono-, di-, tri- and oligosaccharides containing from about 4-9 monosaccharide units), and polysaccharides such as starches, glycogen, cellulose and polysaccharide gums. Specific monosaccharides include C5 and above (*e.g.*, C5 -C8) sugars; di- and trisaccharides include sugars having two or three monosaccharide units (*e.g.,* C5 -C8). The carbohydrate conjugate may further comprised other ligands such as, but not limited to, PK modulator, endosomolytic ligand, and cell permeation peptide.

*Linkers.* The conjugates described herein can be attached to the iRNA oligonucleotide with various linkers that can be cleavable or non cleavable. The term "linker" or "linking group" means an organic moiety that connects two parts of a compound. Linkers typically comprise a direct bond or an atom such as oxygen or sulfur, a unit such as NR8, C(O), C(O)NH, SO, SO2, SO2NH or a chain of atoms, such as, but not limited to, substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl, which one or more methylenes can be interrupted or terminated by O, S, S(O), SO2, N(R8), C(O), substituted or unsubstituted aryl, substituted or unsubstituted heteroaryl, substituted or unsubstituted heterocyclic; where R8 is hydrogen, acyl, aliphatic or substituted aliphatic. The linker may be between 1-24 atoms, *e.g.*, 4-24 atoms, 6-18 atoms, 8-18 atoms, or 8-16 atoms.

*Other Modifications.* In certain instances, the RNA of an iRNA can be modified by a non-ligand group. A number of non-ligand molecules have been conjugated to iRNAs in order to enhance the activity, cellular distribution or cellular uptake of the iRNA, and procedures for performing such conjugations are available in the scientific literature. Such non-ligand moieties have included lipid moieties, such as cholesterol (Kubo, T. et al., Biochem. Biophys. Res. Comm., 2007, 365(1):54-61; Letsinger et al., Proc. Natl. Acad. Sci. USA, 1989, 86:6553), cholic acid (Manoharan et al., Bioorg. Med. Chem. Lett., 1994, 4:1053), a thioether, *e.g.,* hexyl-S-tritylthiol (Manoharan et al., Ann. N.Y. Acad. Sci., 1992, 660:306; Manoharan et al., Bioorg. Med. Chem. Let., 1993, 3:2765), a thiocholesterol (Oberhauser et al., Nucl. Acids Res., 1992, 20:533), an aliphatic chain, *e.g.,* dodecandiol or undecyl residues (Saison-Behmoaras et al., EMBO J., 1991, 10:111; Kabanov et al., FEBS Lett., 1990, 259:327; Svinarchuk et al., Biochimie, 1993, 75:49), a phospholipid, *e.g.*, di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids Res., 1990, 18:3777), a polyamine or a polyethylene glycol chain (Manoharan et al., Nucleosides & Nucleotides, 1995, 14:969), or adamantane acetic acid (Manoharan et al., Tetrahedron Lett., 1995, 36:3651), a palmityl moiety (Mishra et al., Biochim. Biophys. Acta, 1995, 1264:229), or an octadecylamine or hexylamino-carbonyl-oxycholesterol moiety (Crooke et al., J. Pharmacol. Exp. Ther., 1996, 277:923).

Representative United States patents that teach the preparation of such RNA conjugates have been listed above. Typical conjugation protocols involve the synthesis of an RNA bearing an amino linker at one or more positions of the sequence. The amino group is then reacted with the molecule being conjugated using appropriate coupling or activating reagents. The conjugation reaction may be performed either with the RNA still bound to the solid support or following cleavage of the RNA, in solution phase. Purification of the RNA conjugate by HPLC typically affords the pure conjugate.

*Delivery of iRNA.* The delivery of an iRNA to a subject in need thereof can be achieved in a number of different ways. *In vivo* delivery can be performed directly by administering a composition comprising an iRNA, *e.g.,* a dsRNA, to a subject. Alternatively, delivery can be performed indirectly by administering one or more vectors that encode and direct the expression of the iRNA.

*Direct Delivery of an iRNA composition.* In general, any method of delivering a nucleic acid molecule can be adapted for use with an iRNA (see *e.g.,* Akhtar S. and Julian RL. (1992) Trends Cell. Biol. 2(5):139-144 and WO94/02595. Non-specific effects of an iRNA can be minimized by local administration, for example by direct injection or implantation into a tissue (as a non-limiting example, a tumor) or topically administering the preparation. Local administration to a treatment site maximizes local concentration of the agent, limits the exposure of the agent to systemic tissues that may otherwise be harmed by the agent or that may degrade the agent, and permits a lower total dose of the iRNA molecule

to be administered.

For administering an iRNA systemically for the treatment of a disease, the RNA can be modified or alternatively delivered using a drug delivery system; both methods act to prevent the rapid degradation of the dsRNA by endo- and exo-nucleases *in vivo.* Modification of the RNA or the pharmaceutical carrier can also permit targeting of the iRNA composition to the target tissue and avoid undesirable off-target effects. iRNA molecules can be modified by chemical conjugation to lipophilic groups such as cholesterol to enhance cellular uptake and prevent degradation.

In an alternative aspect the iRNA can be delivered using drug delivery systems such as a nanoparticle, a dendrimer, a polymer, a liposome, or a cationic delivery system. Positively charged cationic delivery systems facilitate binding of an iRNA molecule (negatively charged) and also enhance interactions at the negatively charged cell membrane to permit efficient uptake of an iRNA by the cell. Cationic lipids, dendrimers, or polymers can either be bound to an iRNA, or induced to form a vesicle or micelle (see *e.g.,* Kim SH., et al (2008) Journal of Controlled Release 129(2):107-116) that encases an iRNA. The formation of vesicles or micelles further prevents degradation of the iRNA when administered systemically.

Methods for making and administering cationic- iRNA complexes are well within the abilities of one skilled in the art (see *e.g.,* Sorensen, DR., et al (2003) J. Mol. Biol 327:761-766; Verma, UN., et al (2003) Clin. Cancer Res. 9:1291-1300; Arnold, AS et al (2007) J. Hypertens. 25:197-205). Some non-limiting examples of drug delivery systems useful for systemic delivery of iRNAs include DOTAP (Sorensen, DR., *et al* (2003), supra; Verma, UN., *et al* (2003), supra), Oligofectamine, "solid nucleic acid lipid particles" (Zimmermann, TS., et al (2006) Nature 441:111-114), cardiolipin (Chien, PY., et al (2005) Cancer Gene Ther. 12:321-328; Pal, A., et al (2005) Int J. Oncol. 26:1087-1091), polyethyleneimine (Bonnet ME., et al (2008) Pharm. Res. Aug 16 Epub ahead of print; Aigner, A. (2006) J. Biomed. Biotechnol. 71659), Arg-Gly-Asp (RGD) peptides (Liu, S. (2006) Mol. Pharm. 3:472-487), and polyamidoamines (Tomalia, DA., et al (2007) Biochem. Soc. Trans. 35:61-67; Yoo, H., et al (1999) Pharm. Res. 16:1799-1804). An iRNA may form a complex with cyclodextrin for systemic administration. Methods for administration and pharmaceutical compositions of iRNAs and cyclodextrins can be found in U.S. Patent No. 7, 427, 605.

*Vector encoded iRNAs.* In one aspect, the assays and methods described herein can be used to determine iRNA activity by measuring target RNAs in a sample obtained from a subject treated with a vector-expressed iRNA. The vector may included at least one regulatory sequence operably linked to a nucleotide sequence that encodes at least one strand of an iRNA as described herein.

In another aspect, iRNA targeting the desired gene can be expressed from transcription units inserted into DNA or RNA vectors (see, *e.g.,* Couture, A, et al., TIG. (1996), 12:5-10; Skillern, A., et al., International PCT Publication No. WO 00/22113, Conrad, PCT Publication No. WO 00/22114, and Conrad, U.S. Pat. No. 6,054,299). Expression can be transient (on the order of hours to weeks) or sustained (weeks to months or longer), depending upon the specific construct used and the target tissue or cell type. These transgenes can be introduced as a linear construct, a circular plasmid, or a viral vector, which can be an integrating or non-integrating vector. The transgene can also be constructed to permit it to be inherited as an extrachromosomal plasmid (Gassmann, et al., Proc. Natl. Acad. Sci. USA (1995) 92:1292).

The individual strand or strands of an iRNA can be transcribed from a promoter on an expression vector. Where two separate strands are to be expressed to generate, for example, a dsRNA, two separate expression vectors can be co-introduced (*e.g.*, by transfection or infection) into a target cell. Alternatively each individual strand of a dsRNA can be transcribed by promoters both of which are located on the same expression plasmid. The strands of a dsRNA may be expressed as inverted repeat polynucleotides joined by a linker polynucleotide sequence such that the dsRNA has a stem and loop structure.

iRNA expression vectors are generally DNA plasmids or viral vectors. Expression vectors compatible with eukaryotic cells, such as those compatible with vertebrate cells, can be used to produce recombinant constructs for the expression of an iRNA as described herein. Eukaryotic cell expression vectors are well known in the art and are available from a number of commercial sources. Typically, such vectors are provided containing convenient restriction sites for insertion of the desired nucleic acid segment. Delivery of iRNA expressing vectors can be systemic, such as by intravenous or intramuscular administration, by administration to target cells ex-planted from the patient followed by reintroduction into the patient, or by any other means that allows for introduction into a desired target cell.

iRNA expression plasmids can be transfected into target cells as a complex with cationic lipid carriers (*e.g.*, Oligofectamine) or non-cationic lipid-based carriers (*e.g.*, Transit-TKOTM). Multiple lipid transfections for iRNA-mediated knockdowns targeting different regions of a target RNA over a period of a week or more are also described herein. Successful introduction of vectors into host cells can be monitored using various known methods. For example, transient transfection can be signaled with a reporter, such as a fluorescent marker, such as Green Fluorescent Protein (GFP). Stable transfection of cells *ex vivo* can be ensured using markers that provide the transfected cell with resistance to specific environmental factors (*e.g.*, antibiotics and drugs), such as hygromycin B resistance.

Viral vector systems which can be utilized with the methods and compositions described herein include, but are not limited to, (a) adenovirus vectors; (b) retrovirus vectors, including but not limited to lentiviral vectors, moloney murine leukemia virus, etc.; (c) adeno-associated virus vectors; (d) herpes simplex virus vectors; (e) SV40 vectors; (f)

polyoma virus vectors; (g) papilloma virus vectors; (h) picornavirus vectors; (i) pox virus vectors such as an orthopox, *e.g.,* vaccinia virus vectors or avipox, *e.g.* canary pox or fowl pox; and (j) a helper-dependent or gutless adenovirus. Replication-defective viruses can also be advantageous. Different vectors will or will not become incorporated into the cells' genome. The constructs can include viral sequences for transfection, if desired. Alternatively, the construct may be incorporated into vectors capable of episomal replication, *e.g.* EPV and EBV vectors. Constructs for the recombinant expression of an iRNA will generally require regulatory elements, *e.g.*, promoters, enhancers, etc., to ensure the expression of the iRNA in target cells. Other aspects to consider for vectors and constructs are further described below.

Vectors useful for the delivery of an iRNA will include regulatory elements (promoter, enhancer, etc.) sufficient for expression of the iRNA in the desired target cell or tissue. The regulatory elements can be chosen to provide either constitutive or regulated/inducible expression.

Expression of the iRNA can be precisely regulated, for example, by using an inducible regulatory sequence that is sensitive to certain physiological regulators, *e.g.*, circulating glucose levels, or hormones (Docherty et al., 1994, FASEB J. 8:20-24). Such inducible expression systems, suitable for the control of dsRNA expression in cells or in mammals include, for example, regulation by ecdysone, by estrogen, progesterone, tetracycline, chemical inducers of dimerization, and isopropyl-β-D1-thiogalactopyranoside (IPTG). A person skilled in the art would be able to choose the appropriate regulatory/promoter sequence based on the intended use of the iRNA transgene.

Viral vectors that contain nucleic acid sequences encoding an iRNA can be used. For example, a retroviral vector can be used (see Miller et al., Meth. Enzymol. 217:581-599 (1993)). These retroviral vectors contain the components necessary for the correct packaging of the viral genome and integration into the host cell DNA. The nucleic acid sequences encoding an iRNA are cloned into one or more vectors, which facilitates delivery of the nucleic acid into a patient. More detail about retroviral vectors can be found, for example, in Boesen et al., Biotherapy 6:291-302 (1994), which describes the use of a retroviral vector to deliver the mdr1 gene to hematopoietic stem cells in order to make the stem cells more resistant to chemotherapy. Other references illustrating the use of retroviral vectors in gene therapy are: Clowes et al., J. Clin. Invest. 93:644-651 (1994); Kiem et al., Blood 83:1467-1473 (1994); Salmons and Gunzberg, Human Gene Therapy 4:129-141 (1993); and Grossman and Wilson, Curr. Opin. in Genetics and Devel. 3:110-114 (1993). Lentiviral vectors contemplated for use include, for example, the HIV based vectors described in U.S. Patent Nos. 6,143,520; 5,665,557; and 5,981,276.

Adenoviruses are also contemplated for use in delivery of iRNAs. Adenoviruses are attractive vehicles, *e.g.,* for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells.

Use of Adeno-associated virus (AAV) vectors is also contemplated (Walsh et al., Proc. Soc. Exp. Biol. Med. 204:289-300 (1993); U.S. Pat. No. 5,436,146). In one embodiment, the iRNA can be expressed as two separate, complementary single-stranded RNA molecules from a recombinant AAV vector having, for example, either the U6 or HI RNA promoters, or the cytomegalovirus (CMV) promoter. Suitable AAV vectors for expressing the dsRNA featured herein, methods for constructing the recombinant AV vector, and methods for delivering the vectors into target cells are described in Samulski R et al. (1987), J. Virol. 61: 3096-3101; Fisher K J et al. (1996), J. Virol, 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641.

Another exemplary viral vector is a pox virus such as a vaccinia virus, for example an attenuated vaccinia such as Modified Virus Ankara (MVA) or NYVAC, an avipox such as fowl pox or canary pox.

The tropism of viral vectors can be modified by pseudotyping the vectors with envelope proteins or other surface antigens from other viruses, or by substituting different viral capsid proteins, as appropriate. For example, lentiviral vectors can be pseudotyped with surface proteins from vesicular stomatitis virus (VSV), rabies, Ebola, Mokola, and the like. AAV vectors can be made to target different cells by engineering the vectors to express different capsid protein serotypes; see, *e.g.,* Rabinowitz J E et al. (2002), J Virol 76:791-801.

The pharmaceutical preparation of a vector can include the vector in an acceptable diluent, or can include a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

V. <u>Pharmaceutical compositions containing therapeutic nucleic acids</u>

The assays described herein are useful for detecting delivery of a therapeutic nucleic acid, e.g., an iRNA agent, to a target tissue and for monitoring activity of the therapeutic nucleic acid. The therapeutic nucleic acids to be administered to a subject are formulated as pharmaceutical compositions containing a therapeutic nucleic acid, such as an iRNA, and a pharmaceutically acceptable carrier. For example, a pharmaceutical composition containing an iRNA is useful for treating a disease or disorder associated with the expression or activity of the target gene, such as pathological processes

mediated by target gene expression. Such pharmaceutical compositions are formulated based on the mode of delivery. One example includes compositions are formulated for systemic administration via parenteral delivery, *e.g.*, by intravenous (IV) delivery.

Pharmaceutical compositions are administered in dosages sufficient to inhibit expression of the target gene. In general, a suitable dose of a therapeutic nucleic acid, such as an iRNA, will be in the range of 0.001 to 200.0 milligrams per kilogram body weight of the recipient per day, generally in the range of 0.01 to 50 mg per kilogram body weight per day. For example, an iRNA can be administered at 0.01, 0.05 mg/kg, 0.5 mg/kg, 1 mg/kg, 1.5 mg/kg, 2 mg/kg, 3 mg/kg, 10 mg/kg, 20 mg/kg, 30 mg/kg, 40 mg/kg, or 50 mg/kg per single dose. The pharmaceutical composition may be administered once daily, or once weekly, or once monthly, or once every other month. The composition can alternatively be administered twice per week or twice per month, or once every two, three or four weeks. The therapeutic nucleic acid may be administered as two, three, or more sub doses at appropriate intervals throughout the day or even using continuous infusion or delivery through a controlled release formulation. In that case, the therapeutic nucleic acid contained in each sub-dose must be correspondingly smaller in order to achieve the total daily dosage. In another case, the therapeutic nucleic acid can be administered with continuous infusion, *e.g.* with continuous intracranial infusion into the CNS *e.g.,* into brain parenchyma, continuous intracerebroventricular infusion, or continuous intrathecal infusion. The dosage unit can also be compounded for delivery over several days, *e.g.*, using a conventional sustained release formulation which provides sustained release of the therapeutic nucleic acid over a several day period. Sustained release formulations are well known in the art and are particularly useful for delivery of agents at a particular site, such as could be used with the agents described herein. The dosage unit may contain a corresponding multiple of daily dose.

The effect of a single dose of an iRNA on target mRNA levels can be long lasting, such that subsequent doses are administered at not more than 3, 4, or 5 day intervals, or at not more than 1, 2, 3, or 4 week intervals.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and *in vivo* half-lives for the individual iRNAs described herein can be made using conventional methodologies or on the basis of *in vivo* testing using an appropriate animal model, as described elsewhere herein.

Advances in mouse genetics have generated a number of mouse models for the study of various human diseases. Such models can be used for *in vivo* testing of a therapeutic nucleic acid, as well as for determining a therapeutically effective dose. A suitable mouse model is, for example, a mouse containing a transgene expressing the human target gene.

Pharmaceutical compositions may be administered in a number of ways depending upon whether local or systemic treatment is desired and upon the area to be treated. Administration may be topical (*e.g.,* by a transdermal patch), pulmonary (*e.g.,* by inhalation or insufflation of powders or aerosols, including by nebulizer); intratracheal, intranasal, epidermal and transdermal, oral or parenteral. Parenteral administration includes intravenous, intraarterial, subcutaneous, intraperitoneal or intramuscular injection or infusion; subdermal, *e.g.,* via an implanted device; or intracranial, *e.g.,* by intraparenchymal, intrathecal or intraventricular, administration.

The therapeutic nucleic acid, such as an iRNA, can be delivered in a manner to target a particular tissue, such as the liver (*e.g.*, the hepatocytes of the liver).

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Suitable topical formulations include those in which the iRNAs featured herein are in admixture with a topical delivery agent such as lipids, liposomes, fatty acids, fatty acid esters, steroids, chelating agents and surfactants. Suitable lipids and liposomes include neutral (*e.g.*, dioleoylphosphatidyl DOPE ethanolamine, dimyristoylphosphatidyl choline DMPC, distearolyphosphatidyl choline) negative (*e.g.,* dimyristoylphosphatidyl glycerol DMPG) and cationic (*e.g.*, dioleoyltetramethylaminopropyl DOTAP and dioleoylphosphatidyl ethanolamine DOTMA). iRNAs featured herein may be encapsulated within liposomes or may form complexes thereto, in particular to cationic liposomes. Alternatively, iRNAs may be complexed to lipids, in particular to cationic lipids. Suitable fatty acids and esters include but are not limited to arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a C1-20 alkyl ester (*e.g.*, isopropylmyristate IPM), monoglyceride, diglyceride or pharmaceutically acceptable salt thereof. Topical formulations are described in detail in U.S. Patent No. 6,747,014.

*Liposomal formulations.* The RNA or DNA effector molecule or the iRNA formulation may comprise liposomes. Some non-limiting examples of different liposomal formulations are discussed below.

Liposomes fall into two broad classes. Cationic liposomes are positively charged liposomes which interact with the negatively charged DNA molecules to form a stable complex. The positively charged DNA/liposome complex binds

to the negatively charged cell surface and is internalized in an endosome. Due to the acidic pH within the endosome, the liposomes are ruptured, releasing their contents into the cell cytoplasm (Wang et al., Biochem. Biophys. Res. Commun., 1987, 147, 980-985).

Liposomes which are pH-sensitive or negatively-charged, entrap nucleic acids rather than complex with it. Since both the DNA and the lipid are similarly charged, repulsion rather than complex formation occurs. Nevertheless, some DNA is entrapped within the aqueous interior of these liposomes. pH-sensitive liposomes have been used to deliver nucleic acids encoding the thymidine kinase gene to cell monolayers in culture. Expression of the exogenous gene was detected in the target cells (Zhou et al., Journal of Controlled Release, 1992, 19, 269-274).

One major type of liposomal composition includes phospholipids other than naturally derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Non-ionic liposomal systems have also been examined to determine their utility in the delivery of drugs to the skin, in particular systems comprising non-ionic surfactant and cholesterol. Non-ionic liposomal formulations comprising Novasome™ I (glyceryl dilaurate/cholesterol/polyoxyethylene-10-stearyl ether) and Novasome™ II (glyceryl distearate/cholesterol/polyoxyethylene-10-stearyl ether) were used to deliver cyclosporin-A into the dermis of mouse skin. Results indicated that such non-ionic liposomal systems were effective in facilitating the deposition of cyclosporine A into different layers of the skin (Hu et al., S.T.P.Pharma. Sci., 1994, 4, 6, 466).

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome (A) comprises one or more glycolipids, such as monosialoganglioside GM1, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Letters, 1987, 223, 42; Wu et al., Cancer Research, 1993, 53, 3765).

Various liposomes comprising one or more glycolipids are known in the art. Papahadjopoulos et al. (Ann. N.Y. Acad. Sci., 1987, 507, 64) reported the ability of monosialoganglioside GM1, galactocerebroside sulfate and phosphatidylinositol to improve blood half-lives of liposomes. These findings were expounded upon by Gabizon et al. (Proc. Natl. Acad. Sci. U.S.A., 1988, 85, 6949). U.S. Pat. No. 4,837,028 and WO 88/04924, both to Allen et al., disclose liposomes comprising (1) sphingomyelin and (2) the ganglioside GM1 or a galactocerebroside sulfate ester. U.S. Pat. No. 5,543,152 (Webb et al.) discloses liposomes comprising sphingomyelin. Liposomes comprising 1,2-sn-dimyristoylphosphatidylcholine are disclosed in WO 97/13499 (Lim et al).

Many liposomes comprising lipids derivatized with one or more hydrophilic polymers, and methods of preparation thereof, are known in the art. Sunamoto et al. (Bull. Chem. Soc. Jpn., 1980, 53, 2778) described liposomes comprising a nonionic detergent, 2C1215G, that contains a PEG moiety. Illum et al. (FEBS Lett., 1984, 167, 79) noted that hydrophilic coating of polystyrene particles with polymeric glycols results in significantly enhanced blood half-lives. Synthetic phospholipids modified by the attachment of carboxylic groups of polyalkylene glycols (*e.g.,* PEG) are described by Sears (U.S. Pat. Nos. 4,426,330 and 4,534,899). Klibanov et al. (FEBS Lett., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, e.g., DSPE-PEG, formed from the combination of distearoylphosphatidylethanolamine (DSPE) and PEG. Liposomes having covalently bound PEG moieties on their external surface are described in European Patent No. EP 0 445 131 B1 and WO 90/04384 to Fisher. Liposome compositions containing 1-20 mole percent of PE derivatized with PEG, and methods of use thereof, are described by Woodle et al. (U.S. Pat. Nos. 5,013,556 and 5,356,633) and Martin et al. (U.S. Pat. No. 5,213,804 and European Patent No. EP 0 496 813 B1). Liposomes comprising a number of other lipid-polymer conjugates are disclosed in WO 91/05545 and U.S. Pat. No. 5,225,212 (both to Martin et al.) and in WO 94/20073 (Zalipsky et al.) Liposomes comprising PEG-modified ceramide lipids are described in WO 96/10391 (Choi et al). U.S. Pat. No. 5,540,935 (Miyazaki et al*.)* and U.S. Pat. No. 5,556,948 (Tagawa et al*.)* describes PEG-containing liposomes that can be further derivatized with functional moieties on their surfaces.

A number of liposomes comprising nucleic acids are known in the art. WO 96/40062 to Thierry et al. discloses methods for encapsulating high molecular weight nucleic acids in liposomes. U.S. Pat. No. 5,264,221 to Tagawa et al. discloses protein-bonded liposomes and asserts that the contents of such liposomes may include a dsRNA. U.S. Pat. No. 5,665,710 to Rahman et al. describes certain methods of encapsulating oligodeoxynucleotides in liposomes. WO

97/04787 to Love et al. discloses liposomes comprising dsRNAs targeted to the raf gene.

Transfersomes are yet another type of liposomes, and are highly deformable lipid aggregates which are attractive candidates for drug delivery vehicles. Transfersomes may be described as lipid droplets which are so highly deformable that they are easily able to penetrate through pores which are smaller than the droplet. Transfersomes are adaptable to the environment in which they are used, *e.g.,* they are self-optimizing (adaptive to the shape of pores in the skin), self-repairing, frequently reach their targets without fragmenting, and often self-loading. To make transfersomes it is possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known as the "head") provides the most useful means for categorizing the different surfactants used in formulations (Rieger, in Pharmaceutical Dosage Forms, Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides. The use of surfactants in drug products, formulations and in emulsions has been reviewed (Rieger, in Pharmaceutical Dosage Forms, Marcel Dekker, Inc., New York, N.Y., 1988, p. 285).

*Nucleic acid lipid particles.* A dsRNA administered to a subject may be fully encapsulated in the lipid formulation, *e.g*., to form a SPLP, pSPLP, SNALP, or other nucleic acid-lipid particle.

As used herein, the term "SNALP" refers to a stable nucleic acid-lipid particle, including SPLP. As used herein, the term "SPLP" refers to a nucleic acid-lipid particle comprising plasmid DNA encapsulated within a lipid vesicle. SNALPs and SPLPs typically contain a cationic or ionizable lipid, a non-cationic lipid, and a lipid that prevents aggregation of the particle (*e.g.,* a PEG-lipid conjugate). SNALPs and SPLPs are extremely useful for systemic applications, as they exhibit extended circulation lifetimes following intravenous (i.v.) injection and accumulate at distal sites (*e.g*., sites physically separated from the administration site). SPLPs include "pSPLP," which include an encapsulated condensing agent-nucleic acid complex as set forth in PCT Publication No. WO 00/03683.

The particles typically have a mean diameter of about 50 nm to about 150 nm, more typically about 60 nm to about 130 nm, more typically about 70 nm to about 110 nm, most typically about 70 nm to about 90 nm, and are substantially nontoxic. In addition, the nucleic acids when present in the nucleic acid- lipid particles described herein are resistant in aqueous solution to degradation with a nuclease. Nucleic acid-lipid particles and their method of preparation are disclosed in, *e.g.,* U.S. Patent Nos. 5,976,567; 5,981,501; 6,534,484; 6,586,410; 6,815,432; and PCT Publication No. WO96/40964.

The lipid to drug ratio (mass/mass ratio) (*e.g*., lipid to dsRNA ratio) may be be in the range of from about 1:1 to about 50:1, from about 1:1 to about 25:1, from about 3:1 to about 15:1, from about 4:1 to about 10:1, from about 5:1 to about 9:1, or about 6:1 to about 9:1.

The cationic and ionizable lipids can be, for example, N,N-dioleyl-N,N-dimethylammonium chloride (DODAC), N,N-distearyl-N,N-dimethylammonium bromide (DDAB), N-(I -(2,3- dioleoyloxy)propyl)-N,N,N-trimethylammonium chloride (DOTAP), N-(I -(2,3- dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylaminopropane (DLin-2-DMAP), 1,2-Dilinoleyloxy-3-trimethylaminopropane chloride salt (DLin-TMA.Cl), 1,2-Dilinoleoyl-3-trimethylaminopropane chloride salt (DLin-TAP.Cl), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA), 1,2-Dilinolenyloxy-N,N-dimethylaminopropane (DLinDMA), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), or a mixture thereof. The cationic or ionizable lipid may comprise from about 20 mol % to about 50 mol % or about 40 mol % of the total lipid present in the particle.

The compound 2,2-Dilinoleyl-4-dimethylaminoethyl-[1,3]-dioxolane can be used to prepare lipid-siRNA nanoparticles. Synthesis of 2,2-Dilinoleyl-4-dimethylaminoethyl-[1,3]-dioxolane is described in United States provisional patent application number 61/107,998 filed on October 23, 2008,

The lipid-siRNA particlemay include 40% 2, 2-Dilinoleyl-4-dimethylaminoethyl-[1,3]-dioxolane: 10% DSPC: 40% Cholesterol: 10% PEG-C-DOMG (mole percent) with a particle size of 63.0 $\pm$ 20 nm and a 0.027 siRNA/Lipid Ratio.

The non-cationic lipid may be an anionic lipid or a neutral lipid including, but not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl- phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1 -trans PE, 1-stearoyl-2-oleoyl-phosphatidyethanolamine (SOPE), cholesterol, or a mixture thereof. The non-cationic lipid may be from about 5 mol % to about 90 mol %, about 10 mol %, or about 58 mol % if cholesterol is included, of the total lipid present in the particle.

The conjugated lipid that inhibits aggregation of particles may be, for example, a polyethyleneglycol (PEG)-lipid including, without limitation, a PEG-diacylglycerol (DAG), a PEG-dialkyloxypropyl (DAA), a PEG-phospholipid, a PEG-ceramide (Cer), or a mixture thereof. The PEG-DAA conjugate may be, for example, a PEG-dilauryloxypropyl (Ci2), a PEG-dimyristyloxypropyl (Ci4), a PEG-dipalmityloxypropyl (Ci6), or a PEG- distearyloxypropyl (C]8). The conjugated lipid that prevents aggregation of particles may be from 0 mol % to about 20 mol % or about 2 mol % of the total lipid present in the particle.

The nucleic acid-lipid particle may further include cholesterol at, *e.g.,* about 10 mol % to about 60 mol % or about 48 mol % of the total lipid present in the particle.

*LNP01.* The lipidoid ND98·4HCl (MW 1487) (see U.S. Patent Application No. 12/056,230, filed 3/26/2008, Cholesterol (Sigma-Aldrich), and PEG-Ceramide C16 (Avanti Polar Lipids) can be used to prepare lipid-dsRNA nanoparticles (*i.e.*, LNP01 particles). Stock solutions of each in ethanol can be prepared as follows: ND98, 133 mg/ml; Cholesterol, 25 mg/ml, PEG-Ceramide C16, 100 mg/ml. The ND98, Cholesterol, and PEG-Ceramide C16 stock solutions can then be combined in a, *e.g.*, 42:48:10 molar ratio. The combined lipid solution can be mixed with aqueous dsRNA (*e.g.,* in sodium acetate pH 5) such that the final ethanol concentration is about 35-45% and the final sodium acetate concentration is about 100-300 mM. Lipid-dsRNA nanoparticles typically form spontaneously upon mixing. Depending on the desired particle size distribution, the resultant nanoparticle mixture can be extruded through a polycarbonate membrane (*e.g.*, 100 nm cut-off) using, for example, a thermobarrel extruder, such as Lipex Extruder (Northern Lipids, Inc). In some cases, the extrusion step can be omitted. Ethanol removal and simultaneous buffer exchange can be accomplished by, for example, dialysis or tangential flow filtration. Buffer can be exchanged with, for example, phosphate buffered saline (PBS) at about pH 7, *e.g.,* about pH 6.9, about pH 7.0, about pH 7.1, about pH 7.2, about pH 7.3, or about pH 7.4.

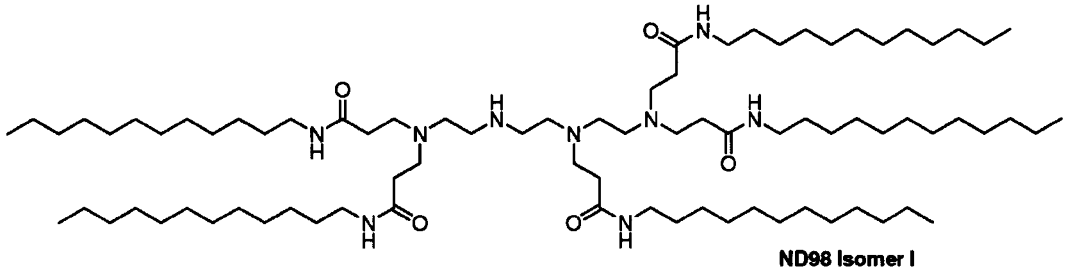

ND98 Isomer I

Formula I

LNP01 formulations are described, *e.g.,* in International Application Publication No. WO 2008/042973, which is hereby incorporated by reference in its entirety.

Additional exemplary lipid-dsRNA formulations include the following:

| | **Cationic Lipid** | **cationic lipid/non-cationic lipid/ cholesterol/PEG-lipid conjugate Lipid:siRNA ratio** |
|---|---|---|
| SNALP-1 | 1,2-Dilinolenyloxy-N,N-dimethylaminopropane (DLinDMA) | DLinDMA/DPPC/Cholesleroll/PEG-cDMA (57.1/7.1/34.4/1.4) lipid:siRNA ~ 7:1 |
| S-XTC | 2,2-Dilinoleyl-4-dimethylaminoethyl-[1,31-dioxolane (XTC) | XTC/DPPC/Cholesterol/PEG-cDMA 57.1/7.1/34.4/1.4 lipid:siRNA ~ 7:1 |
| LNP09 | 2,2-Dilinoleyl-4-dimethylaminoethyl-[1,3]-dioxolane (XTC) | XTC/DSPC/Cholesterol/PEG-DMG 50/10/38.5/1.5 Lipid:siRNA 10:1 |
| LNP10 | (3aR,5s,6aS)-N,N -dimethyl- 2,2-di((9Z, 12Z)-octadeca-9, 12-dienyl)tetrahydro-3aH-cyclopenta[d][1,3]dioxol-5-amine (ALN100) | ALN100/DSPC/Cholesterol/PEG-DMG 50/10/38.5/1.5 Lipid:siRNA 10:1 |
| LNP11 | (6Z,9Z,28Z,31Z)-heptatriacoata-6,9,28,31-tetraen-19-yl 4-(dimcthylamino)butanoatc (MC3) | MC-3/DSPC/Cholesterol/PEG-DMG 50/10/38.5/1.5 Lipid:siRNA 10:1 |
| LNP12 | 1,1'-(2-(4-(2-((2-(bis(2-hydroxydodecyl)amino)ethyl)(2-hydroxydodecyl)amino)ethyl)piperazin-1-yl) ethylazanediyl)didodecan-2-ol (C12-200) | C12-200/DSPC/Cholesterol/PEG-DMG 50/10/38.5/1.5 Lipid:siRNA 10:1 |

DSPC: distearoylphosphatidylcholine
DPPC: dipalmitoylphosphatidylcholine
PEG-DMG: PEG-didimyristoyl glycerol (C14-PEG, or PEG-C14) (PEG with avg mol wt of 2000)
PEG-DSG: PEG-distyryl glycerol (C18-PEG, or PEG-C18) (PEG with avg mol wt of 2000)
PEG-cDMA: PEG-carbamoyl-1,2-dimyristyloxypropylamine (PEG with avg mol wt of 2000)
SNALP (1,2-Dilinolenyloxy-N,N-dimethylaminopropane (DLinDMA)) comprising formulations are described in International Publication No. WO2009/127060, filed April 15, 2009.
XTC comprising formulations are described, *e.g.*, in U.S. Provisional Serial No. 61/239,686, filed September 3, 2009.
MC3 comprising formulations are described, *e.g.*, in U.S. Provisional Serial No. 61/244,834, filed September 22, 2009, U.S. Provisional Serial No. 61/185,800, filed June 10, 2009, and International Application No. PCT/US10/28224, filed June 10, 2010.
ALNY-100 comprising formulations are described, *e.g.,* International patent application number PCT/US09/63933, filed on November 10, 2009.
C12-200 comprising formulations are described in U.S. Provisional Serial No. 61/175,770, filed May 5, 2009 and International Application No. PCT/US10/33777, filed May 5, 2010.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Oral formulations may be those in which dsRNAs featured herein are administered in conjunction with one or more penetration enhancers surfactants and chelators. Suitable surfactants include fatty acids and/or esters or salts thereof, bile acids and/or salts thereof. Suitable bile acids/salts include chenodeoxycholic acid (CDCA) and ursodeoxychenodeoxycholic acid (UDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate and sodium glycodihydrofusidate. Suitable fatty acids include arachidonic acid, undecanoic acid, oleic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a monoglyceride, a diglyceride or a pharmaceutically acceptable salt thereof (*e.g.*, sodium). In some embodiments, combinations of penetration enhancers are used, for example, fatty acids/salts in combination with bile acids/salts. One exemplary combination is the sodium salt of lauric acid, capric acid and UDCA. Further penetration enhancers include polyoxyethylene-9-lauryl ether, polyoxyethylene-20-cetyl ether. DsRNAs featured herein may be delivered orally, in granular form including sprayed dried particles, or complexed to form micro or nanoparticles. DsRNA complexing agents include polyamino acids; polyimines; polyacrylates; polyalkylacrylates, polyoxethanes, polyalkylcyanoacrylates; cationized gelatins, albumins, starches, acrylates, polyethyleneglycols (PEG) and starches; polyalkylcyanoacrylates; DEAE-derivatized polyimines, pollulans, celluloses and starches. Suitable complexing agents include chitosan, N-trimethylchitosan, poly-L-lysine, polyhistidine, polyomithine, polyspermines, protamine, polyvinylpyridine, polythiodiethylaminomethylethylene P(TDAE), polyaminostyrene (*e.g.*, p-amino), poly(methylcyanoacrylate), poly(ethylcyanoacrylate), poly(butylcyanoacrylate), poly(isobutylcyanoacrylate), poly(isohexylcynaoacrylate), DEAE-methacrylate, DEAE-hexylacrylate, DEAE-acrylamide, DEAE-albumin and DEAE-dextran, polymethylacrylate, polyhexylacrylate, poly(D,L-lactic acid), poly(DL-lactic-co-glycolic acid (PLGA), alginate, and polyethyleneglycol (PEG). Oral formulations for dsRNAs and their preparation are described in detail in U.S. Patent 6,887,906, US Publn. No. 20030027780, and U.S. Patent No. 6,747,014.

Compositions and formulations for parenteral, intraparenchymal (into the brain), intrathecal, intraventricular or intrahepatic administration may include sterile aqueous solutions which may also contain buffers, diluents and other suitable additives such as, but not limited to, penetration enhancers, carrier compounds and other pharmaceutically acceptable carriers or excipients.

Pharmaceutical compositions described herein include, but are not limited to, solutions, emulsions, and liposome-containing formulations. These compositions may be generated from a variety of components that include, but are not limited to, preformed liquids, self-emulsifying solids and self-emulsifying semisolids. Typical formulations will target the liver when hepatic disorders such as hepatic carcinoma are treated.

The pharmaceutical formulations can conveniently be presented in unit dosage form, and may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions described herein may be formulated into any of many possible dosage forms such as, but not limited to, tablets, capsules, gel capsules, liquid syrups, soft gels, suppositories, and enemas. The compositions described herein may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

*Additional Formulations*

*Emulsions.* The iRNA to be administered to a subject can be prepared and formulated as an emulsion. Emulsions are typically heterogeneous systems of one liquid dispersed in another in the form of droplets usually exceeding 0.1 μm in diameter (see *e.g.*, Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199; Rosoff, in Pharmaceutical Dosage Forms, Lieberman, Rieger and Banker (Eds.), 1988, Marcel Dekker, Inc., New York, N.Y., Volume 1, p. 245; Block in Pharmaceutical Dosage Forms, Lieberman, Rieger and Banker (Eds.), 1988, Marcel Dekker, Inc., New York, N.Y., volume 2, p. 335; Higuchi et al., in Remington's Pharmaceutical Sciences, Mack Publishing Co., Easton, Pa., 1985, p. 301). Emulsions are often biphasic systems comprising two immiscible liquid phases intimately mixed and dispersed with each other. In general, emulsions may be of either the water-in-oil (w/o) or the oil-in-water (o/w) variety. When an aqueous phase is finely divided into and dispersed as minute droplets into a bulk oily phase, the resulting composition is called a water-in-oil (w/o) emulsion. Alternatively, when an oily phase is finely divided into and dispersed as minute droplets into a bulk aqueous phase, the resulting composition is called an oil-in-water (o/w) emulsion. Emulsions may contain additional components in addition to the dispersed phases, and the active drug which may be present as a solution in either the aqueous phase, oily phase or itself as a separate phase. Pharmaceutical excipients such as emulsifiers, stabilizers, dyes, and anti-oxidants may also be present in emulsions as needed. Pharmaceutical emulsions may also be multiple emulsions that are comprised of more than two phases such as, for example, in the case of oil-in-water-in-oil (o/w/o) and water-in-oil-in-water (w/o/w) emulsions. Such complex formulations often provide certain advantages that simple binary emulsions do not. Multiple emulsions in which individual oil droplets of an o/w emulsion enclose small water droplets constitute a w/o/w emulsion. Likewise a system of oil droplets enclosed in globules of water stabilized in an oily continuous phase provides an o/w/o emulsion.

Emulsions are characterized by little or no thermodynamic stability. Often, the dispersed or discontinuous phase of the emulsion is well dispersed into the external or continuous phase and maintained in this form through the means of emulsifiers or the viscosity of the formulation. Either of the phases of the emulsion may be a semisolid or a solid, as is the case of emulsion-style ointment bases and creams. Other means of stabilizing emulsions entail the use of emulsifiers that may be incorporated into either phase of the emulsion. Emulsifiers may broadly be classified into four categories: synthetic surfactants, naturally occurring emulsifiers, absorption bases, and finely dispersed solids (see *e.g.,* Ansel's Pharmaceutical Dosage Forms and Drug Delivery Systems, Allen, LV., Popovich NG., and Ansel HC., 2004, Lippincott

Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199).

Synthetic surfactants, also known as surface active agents, have found wide applicability in the formulation of emulsions and have been reviewed in the literature (see *e.g*., Ansel's Pharmaceutical Dosage Forms and Drug Delivery Systems, Allen, LV., Popovich NG., and Ansel HC., 2004, Lippincott Williams & Wilkins (8th ed.), New York, NY; Rieger, in Pharmaceutical Dosage Forms, Lieberman, Rieger and Banker (Eds.), 1988, Marcel Dekker, Inc., New York, N.Y., volume 1, p. 285; Idson, in Pharmaceutical Dosage Forms, Lieberman, Rieger and Banker (Eds.), Marcel Dekker, Inc., New York, N.Y., 1988, volume 1, p. 199). Surfactants are typically amphiphilic and comprise a hydrophilic and a hydrophobic portion. The ratio of the hydrophilic to the hydrophobic nature of the surfactant has been termed the hydrophile/lipophile balance (HLB) and is a valuable tool in categorizing and selecting surfactants in the preparation of formulations. Surfactants may be classified into different classes based on the nature of the hydrophilic group: nonionic, anionic, cationic and amphoteric (see *e.g*., Ansel's Pharmaceutical Dosage Forms and Drug Delivery Systems, Allen, LV., Popovich NG., and Ansel HC., 2004, Lippincott Williams & Wilkins (8th ed.), New York, NY Rieger, in Pharmaceutical Dosage Forms, Lieberman, Rieger and Banker (Eds.), 1988, Marcel Dekker, Inc., New York, N.Y., volume 1, p. 285).

Naturally occurring emulsifiers used in emulsion formulations include lanolin, beeswax, phosphatides, lecithin and acacia. Absorption bases possess hydrophilic properties such that they can soak up water to form w/o emulsions yet retain their semisolid consistencies, such as anhydrous lanolin and hydrophilic petrolatum. Finely divided solids have also been used as good emulsifiers especially in combination with surfactants and in viscous preparations. These include polar inorganic solids, such as heavy metal hydroxides, nonswelling clays such as bentonite, attapulgite, hectorite, kaolin, montmorillonite, colloidal aluminum silicate and colloidal magnesium aluminum silicate, pigments and nonpolar solids such as carbon or glyceryl tristearate.

A large variety of non-emulsifying materials are also included in emulsion formulations and contribute to the properties of emulsions. These include fats, oils, waxes, fatty acids, fatty alcohols, fatty esters, humectants, hydrophilic colloids, preservatives and antioxidants (Block, in Pharmaceutical Dosage Forms, Lieberman, Rieger and Banker (Eds.), 1988, Marcel Dekker, Inc., New York, N.Y., volume 1, p. 335; Idson, in Pharmaceutical Dosage Forms, Lieberman, Rieger and Banker (Eds.), 1988, Marcel Dekker, Inc., New York, N.Y., volume 1, p. 199).

Hydrophilic colloids or hydrocolloids include naturally occurring gums and synthetic polymers such as polysaccharides (for example, acacia, agar, alginic acid, carrageenan, guar gum, karaya gum, and tragacanth), cellulose derivatives (for example, carboxymethylcellulose and carboxypropylcellulose), and synthetic polymers (for example, carbomers, cellulose ethers, and carboxyvinyl polymers). These disperse or swell in water to form colloidal solutions that stabilize emulsions by forming strong interfacial films around the dispersed-phase droplets and by increasing the viscosity of the external phase.

Since emulsions often contain a number of ingredients such as carbohydrates, proteins, sterols and phosphatides that may readily support the growth of microbes, these formulations often incorporate preservatives. Commonly used preservatives included in emulsion formulations include methyl paraben, propyl paraben, quaternary ammonium salts, benzalkonium chloride, esters of p-hydroxybenzoic acid, and boric acid. Antioxidants are also commonly added to emulsion formulations to prevent deterioration of the formulation. Antioxidants used may be free radical scavengers such as tocopherols, alkyl gallates, butylated hydroxyanisole, butylated hydroxytoluene, or reducing agents such as ascorbic acid and sodium metabisulfite, and antioxidant synergists such as citric acid, tartaric acid, and lecithin.

The application of emulsion formulations via dermatological, oral and parenteral routes and methods for their manufacture have been reviewed in the literature (see *e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199). Emulsion formulations for oral delivery have been very widely used because of ease of formulation, as well as efficacy from an absorption and bioavailability standpoint (see *e.g*., Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245; Idson, in Pharmaceutical Dosage Forms, Lieberman, Rieger and Banker (Eds.), 1988, Marcel Dekker, Inc., New York, N.Y., volume 1, p. 199). Mineral-oil base laxatives, oil-soluble vitamins and high fat nutritive preparations are among the materials that have commonly been administered orally as o/w emulsions.

The compositions of iRNAs and nucleic acids may be formulated as microemulsions. A microemulsion may be defined as a system of water, oil and amphiphile which is a single optically isotropic and thermodynamically stable liquid solution (see *e.g*., Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245). Typically microemulsions are systems that are prepared by first dispersing an oil in an aqueous surfactant solution and then adding a sufficient amount of a fourth component, generally an intermediate chain-length alcohol to form a transparent system. Therefore, microemulsions have also been described as thermodynamically stable, isotropically clear dispersions of two

immiscible liquids that are stabilized by interfacial films of surface-active molecules (Leung and Shah, in: Controlled Release of Drugs: Polymers and Aggregate Systems, Rosoff, M., Ed., 1989, VCH Publishers, New York, pages 185-215). Microemulsions commonly are prepared via a combination of three to five components that include oil, water, surfactant, cosurfactant and electrolyte. Whether the microemulsion is of the water-in-oil (w/o) or an oil-in-water (o/w) type is dependent on the properties of the oil and surfactant used and on the structure and geometric packing of the polar heads and hydrocarbon tails of the surfactant molecules (Schott, in Remington's Pharmaceutical Sciences, Mack Publishing Co., Easton, Pa., 1985, p. 271).

The phenomenological approach utilizing phase diagrams has been extensively studied and has yielded a comprehensive knowledge, to one skilled in the art, of how to formulate microemulsions (see *e.g.*, Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245; Block, in Pharmaceutical Dosage Forms, Lieberman, Rieger and Banker (Eds.), 1988, Marcel Dekker, Inc., New York, N.Y., volume 1, p. 335). Compared to conventional emulsions, microemulsions offer the advantage of solubilizing water-insoluble drugs in a formulation of thermodynamically stable droplets that are formed spontaneously.

Surfactants used in the preparation of microemulsions include, but are not limited to, ionic surfactants, non-ionic surfactants, Brij 96, polyoxyethylene oleyl ethers, polyglycerol fatty acid esters, tetraglycerol monolaurate (ML310), tetraglycerol monooleate (MO310), hexaglycerol monooleate (PO310), hexaglycerol pentaoleate (PO500), decaglycerol monocaprate (MCA750), decaglycerol monooleate (MO750), decaglycerol sequioleate (SO750), decaglycerol decaoleate (DAO750), alone or in combination with cosurfactants. The cosurfactant, usually a short-chain alcohol such as ethanol, 1-propanol, and 1-butanol, serves to increase the interfacial fluidity by penetrating into the surfactant film and consequently creating a disordered film because of the void space generated among surfactant molecules. Microemulsions may, however, be prepared without the use of cosurfactants and alcohol-free self-emulsifying microemulsion systems are known in the art. The aqueous phase may typically be, but is not limited to, water, an aqueous solution of the drug, glycerol, PEG300, PEG400, polyglycerols, propylene glycols, and derivatives of ethylene glycol. The oil phase may include, but is not limited to, materials such as Captex 300, Captex 355, Capmul MCM, fatty acid esters, medium chain (C8-C12) mono, di, and tri-glycerides, polyoxyethylated glyceryl fatty acid esters, fatty alcohols, polyglycolized glycerides, saturated polyglycolized C8-C10 glycerides, vegetable oils and silicone oil.

Microemulsions are particularly of interest from the standpoint of drug solubilization and the enhanced absorption of drugs. Lipid based microemulsions (both o/w and w/o) have been proposed to enhance the oral bioavailability of drugs, including peptides (see *e.g.,* U.S. Patent Nos. 6,191,105; 7,063,860; 7,070,802; 7,157,099; Constantinides et al., Pharmaceutical Research, 1994, 11, 1385-1390; Ritschel, Meth. Find. Exp. Clin. Pharmacol., 1993, 13, 205). Microemulsions afford advantages of improved drug solubilization, protection of drug from enzymatic hydrolysis, possible enhancement of drug absorption due to surfactant-induced alterations in membrane fluidity and permeability, ease of preparation, ease of oral administration over solid dosage forms, improved clinical potency, and decreased toxicity (see *e.g.,* U.S. Patent Nos. 6,191,105; 7,063,860; 7,070,802; 7,157,099; Constantinides et al., Pharmaceutical Research, 1994, 11, 1385; Ho et al., J. Pharm. Sci., 1996, 85, 138-143). Often microemulsions may form spontaneously when their components are brought together at ambient temperature. This may be particularly advantageous when formulating thermolabile drugs, peptides or iRNAs. Microemulsions have also been effective in the transdermal delivery of active components in both cosmetic and pharmaceutical applications. It is expected that the microemulsion compositions and formulations herein will facilitate the increased systemic absorption of iRNAs and nucleic acids from the gastrointestinal tract, as well as improve the local cellular uptake of iRNAs and nucleic acids.

Microemulsions described herein may also contain additional components and additives such as sorbitan monostearate (Grill 3), Labrasol, and penetration enhancers to improve the properties of the formulation and to enhance the absorption of the iRNAs and nucleic acids described herein. Penetration enhancers used in the microemulsions described herein may be classified as belonging to one of five broad categories-surfactants, fatty acids, bile salts, chelating agents, and non-chelating non-surfactants (Lee et al., Critical Reviews in Therapeutic Drug Carrier Systems, 1991, p. 92). Each of these classes has been discussed above.

*Penetration Enhancers.* In one aspect, the present disclosure employs various penetration enhancers to effect the efficient delivery of nucleic acids, particularly iRNAs, to the skin of animals. Most drugs are present in solution in both ionized and nonionized forms. However, usually only lipid soluble or lipophilic drugs readily cross cell membranes. It has been discovered that even non-lipophilic drugs may cross cell membranes if the membrane to be crossed is treated with a penetration enhancer. In addition to aiding the diffusion of non-lipophilic drugs across cell membranes, penetration enhancers also enhance the permeability of lipophilic drugs.

Some non-limiting examples of penetration enhancers include surfactants, fatty acids, bile salts, chelating agents, and non-chelating non-surfactants (see *e.g.*, Malmsten, M. Surfactants and polymers in drug delivery, Informa Health Care, New York, NY, 2002; Lee et al., Critical Reviews in Therapeutic Drug Carrier Systems, 1991, p.92).

Surfactants: Surfactants (or "surface-active agents") reduce the surface tension of the solution or the interfacial

tension between the aqueous solution and another liquid, with the result that absorption of iRNAs through the mucosa is enhanced. In addition to bile salts and fatty acids, these penetration enhancers include, for example, sodium lauryl sulfate, polyoxyethylene-9-lauryl ether and polyoxyethylene-20-cetyl ether) (see *e.g.,* Malmsten, M. Surfactants and polymers in drug delivery, Informa Health Care, New York, NY, 2002; Lee et al., Critical Reviews in Therapeutic Drug Carrier Systems, 1991, p.92); and perfluorochemical emulsions, such as FC-43. Takahashi et al., J. Pharm. Pharmacol., 1988, 40, 252).

Fatty acids: Various fatty acids and their derivatives which act as penetration enhancers include, for example, oleic acid, lauric acid, capric acid (n-decanoic acid), myristic acid, palmitic acid, stearic acid, linoleic acid, linolenic acid, dicaprate, tricaprate, monoolein (1-monooleoyl-rac-glycerol), dilaurin, caprylic acid, arachidonic acid, glycerol 1-monocaprate, 1-dodecylazacycloheptan-2-one, acylcarnitines, acylcholines, C1-20 alkyl esters thereof (*e.g.*, methyl, isopropyl and t-butyl), and mono- and diglycerides thereof (*i.e.*, oleate, laurate, caprate, myristate, palmitate, stearate, linoleate, etc.) (see *e.g.,* Touitou, E., et al. Enhancement in Drug Delivery. CRC Press, Danvers, MA, 2006; Lee et al., Critical Reviews in Therapeutic Drug Carrier Systems, 1991, p.92; Muranishi, Critical Reviews in Therapeutic Drug Carrier Systems, 1990, 7, 1-33; El Hariri et al., J. Pharm. Pharmacol., 1992, 44, 651-654).

Bile salts: The physiological role of bile includes the facilitation of dispersion and absorption of lipids and fat-soluble vitamins (see *e.g.,* Malmsten, M. Surfactants and polymers in drug delivery, Informa Health Care, New York, NY, 2002; Brunton, Chapter 38 in: Goodman & Gilman's The Pharmacological Basis of Therapeutics, 9th Ed., Hardman et al. Eds., McGraw-Hill, New York, 1996, pp. 934-935). The term "bile salts" includes any of the naturally occurring components of bile as well as any of their synthetic derivatives. Suitable bile salts include, for example, cholic acid (or its pharmaceutically acceptable sodium salt, sodium cholate), dehydrocholic acid (sodium dehydrocholate), deoxycholic acid (sodium deoxycholate), glucholic acid (sodium glucholate), glycholic acid (sodium glycocholate), glycodeoxycholic acid (sodium glycodeoxycholate), taurocholic acid (sodium taurocholate), taurodeoxycholic acid (sodium taurodeoxycholate), chenodeoxycholic acid (sodium chenodeoxycholate), ursodeoxycholic acid (UDCA), sodium tauro-24,25-dihydro-fusidate (STDHF), sodium glycodihydrofusidate and polyoxyethylene-9-lauryl ether (POE) (see *e.g.*, Malmsten, M. Surfactants and polymers in drug delivery, Informa Health Care, New York, NY, 2002; Lee et al., Critical Reviews in Therapeutic Drug Carrier Systems, 1991, page 92; Swinyard, Chapter 39 In: Remington's Pharmaceutical Sciences, 18th Ed., Gennaro, ed., Mack Publishing Co., Easton, Pa., 1990, pages 782-783; Muranishi, Critical Reviews in Therapeutic Drug Carrier Systems, 1990, 7, 1-33; Yamamoto el a/., J. Pharm. Exp. Ther., 1992, 263, 25; Yamashita et al., J. Pharm. Sci., 1990, 79, 579-583).

Chelating Agents: Chelating agents can be defined as compounds that remove metallic ions from a solution by forming complexes, with the result that absorption of iRNAs through the mucosa is enhanced. With regards to their use as penetration enhancers chelating agents have the added advantage of also serving as DNase inhibitors, as most characterized DNA nucleases require a divalent metal ion for catalysis and are thus inhibited by chelating agents (Jarrett, J. Chromatogr., 1993, 618, 315-339). Suitable chelating agents include but are not limited to disodium ethylenediaminetetraacetate (EDTA), citric acid, salicylates (*e.g.,* sodium salicylate, 5-methoxysalicylate and homovanilate), N-acyl derivatives of collagen, laureth-9 and N-amino acyl derivatives of β-diketones (enamines)(see *e.g.,* Katdare, A. et al., Excipient development for pharmaceutical, biotechnology, and drug delivery, CRC Press, Danvers, MA, 2006; Lee et al., Critical Reviews in Therapeutic Drug Carrier Systems, 1991, page 92; Muranishi, Critical Reviews in Therapeutic Drug Carrier Systems, 1990, 7, 1-33; Buur et al., J. Control Rel., 1990, 14,43-51).

Non-chelating non-surfactants: As used herein, non-chelating non-surfactant penetration enhancing compounds are compounds that demonstrate insignificant activity as chelating agents or as surfactants but that nonetheless enhance absorption of iRNAs through the alimentary mucosa (see *e.g.,* Muranishi, Critical Reviews in Therapeutic Drug Carrier Systems, 1990, 7, 1-33). This class of penetration enhancers includes, for example, unsaturated cyclic ureas, 1-alkyl- and 1-alkenylazacyclo-alkanone derivatives (Lee et al., Critical Reviews in Therapeutic Drug Carrier Systems, 1991, page 92); and non-steroidal anti-inflammatory agents such as diclofenac sodium, indomethacin and phenylbutazone (Yamashita et al., J. Pharm. Pharmacol., 1987, 39, 621-626). Agents that enhance uptake of iRNAs at the cellular level may also be added to the pharmaceutical and other compositions described herein. For example, cationic lipids, such as lipofectin (Junichi et al, U.S. Pat. No. 5,705,188), cationic glycerol derivatives, and polycationic molecules, such as polylysine (Lollo et al., PCT Application WO 97/30731), are also known to enhance the cellular uptake of dsRNAs. Examples of commercially available transfection reagents include, for example Lipofectamine™ (Invitrogen; Carlsbad, CA), Lipofectamine 2000™ (Invitrogen; Carlsbad, CA), 293fectin™ (Invitrogen; Carlsbad, CA), Cellfectin™ (Invitrogen; Carlsbad, CA), DMRIE-C™ (Invitrogen; Carlsbad, CA), FreeStyle™ MAX (Invitrogen; Carlsbad, CA), Lipofectamine™ 2000 CD (Invitrogen; Carlsbad, CA), Lipofectamine™ (Invitrogen; Carlsbad, CA), RNAiMAX (Invitrogen; Carlsbad, CA), Oligofectamine™ (Invitrogen; Carlsbad, CA), Optifect™ (Invitrogen; Carlsbad, CA), X-tremeGENE Q2 Transfection Reagent (Roche; Grenzacherstrasse, Switzerland), DOTAP Liposomal Transfection Reagent (Grenzacherstrasse, Switzerland), DOSPER Liposomal Transfection Reagent (Grenzacherstrasse, Switzerland), or Fugene (Grenzacherstrasse, Switzerland), Transfectam® Reagent (Promega; Madison, WI), TransFast™ Transfection Reagent (Promega; Madison, WI), Tfx™-20 Reagent (Promega; Madison, WI), Tfx™-50 Reagent (Promega; Madison, WI), DreamFect™ (OZ Biosciences; Marseille, France), EcoTransfect (OZ Biosciences; Marseille, France), TransPass[a] D1 Transfection Reagent (New England Biolabs; Ipswich, MA, USA), LyoVec™/LipoGen™ (Invivogen; San Diego, CA, USA), PerFectin Transfection Reagent (Genlantis; San Diego, CA, USA), NeuroPORTER Transfection Reagent (Genlantis; San Diego, CA, USA), GenePORTER Transfection reagent (Genlantis; San Diego, CA, USA), GenePORTER 2 Transfection reagent (Genlantis; San Diego, CA, USA), Cytofectin Transfection Reagent (Genlantis; San Diego, CA, USA), BaculoPORTER Transfection Reagent (Genlantis; San Diego, CA, USA), TroganPORTER™ transfection Reagent (Genlantis; San Diego, CA, USA), RiboFect (Bioline; Taunton, MA, USA), PlasFect (Bioline; Taunton, MA, USA), UniFECTOR (B-Bridge International; Mountain View, CA, USA), SureFECTOR (B-Bridge International; Mountain View, CA, USA), or HiFect™ (B-Bridge International, Mountain View, CA, USA), among others.

Other agents may be utilized to enhance the penetration of the administered nucleic acids, including glycols such as ethylene glycol and propylene glycol, pyrrols such as 2-pyrrol, azones, and terpenes such as limonene and menthone.

*Carriers.* Certain compositions described herein also incorporate carrier compounds in the formulation. As used herein, "carrier compound" or "carrier" can refer to a nucleic acid, or analog thereof, which is inert (*i.e.*, does not possess biological activity per se) but is recognized as a nucleic acid by *in vivo* processes that reduce the bioavailability of a nucleic acid having biological activity by, for example, degrading the biologically active nucleic acid or promoting its removal from circulation. The coadministration of a nucleic acid and a carrier compound, typically with an excess of the latter substance, can result in a substantial reduction of the amount of nucleic acid recovered in the liver, kidney or other extracirculatory reservoirs, presumably due to competition between the carrier compound and the nucleic acid for a common receptor. For example, the recovery of a partially phosphorothioate dsRNA in hepatic tissue can be reduced when it is coadministered with polyinosinic acid, dextran sulfate, polycytidic acid or 4-acetamido-4'isothiocyano-stilbene-2,2'-disulfonic acid (Miyao et al., DsRNA Res. Dev., 1995, 5, 115-121; Takakura et al., DsRNA & Nucl. Acid Drug Dev., 1996, 6, 177-183.

*Excipients.* In contrast to a carrier compound, a "pharmaceutical carrier" or "excipient" is a pharmaceutically acceptable solvent, suspending agent or any other pharmacologically inert vehicle for delivering one or more nucleic acids to an animal. The excipient may be liquid or solid and is selected, with the planned manner of administration in mind, so as to provide for the desired bulk, consistency, etc., when combined with a nucleic acid and the other components of a given pharmaceutical composition. Typical pharmaceutical carriers include, but are not limited to, binding agents (*e.g.*, pregelatinized maize starch, polyvinylpyrrolidone or hydroxypropyl methylcellulose, etc.); fillers (*e.g.*, lactose and other sugars, microcrystalline cellulose, pectin, gelatin, calcium sulfate, ethyl cellulose, polyacrylates or calcium hydrogen phosphate, etc.); lubricants (*e.g.*, magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (*e.g.*, starch, sodium starch glycolate, etc.); and wetting agents (*e.g.*, sodium lauryl sulphate, etc).

Pharmaceutically acceptable organic or inorganic excipients suitable for non-parenteral administration which do not deleteriously react with nucleic acids can also be used to formulate the compositions described herein. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

Formulations for topical administration of nucleic acids may include sterile and non-sterile aqueous solutions, non-aqueous solutions in common solvents such as alcohols, or solutions of the nucleic acids in liquid or solid oil bases. The solutions may also contain buffers, diluents and other suitable additives. Pharmaceutically acceptable organic or inorganic excipients suitable for non-parenteral administration which do not deleteriously react with nucleic acids can be used.

Suitable pharmaceutically acceptable excipients include, but are not limited to, water, salt solutions, alcohol, polyethylene glycols, gelatin, lactose, amylose, magnesium stearate, talc, silicic acid, viscous paraffin, hydroxymethylcellulose, polyvinylpyrrolidone and the like.

*Other Components.* The compositions may additionally contain other adjunct components conventionally found in pharmaceutical compositions, at their art-established usage levels. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions described herein, such as dyes, flavoring agents, preservatives, antioxidants, opacifiers, thickening agents and stabilizers. However, such materials, when added, should not unduly interfere with the biological activities of the components of the compositions described herein. The formulations can be sterilized and, if desired, mixed with auxiliary agents, *e.g.*, lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Aqueous suspensions can contain substances that increase the viscosity of the suspension including, for example, sodium carboxymethylcellulose, sorbitol and/or dextran. The suspension may also contain stabilizers.

A pharmaceutical composition can include (a) one or more iRNA compounds and (b) one or more anti-cytokine biologic agents which function by a non-RNAi mechanism. Examples of such biologics include, biologics that target IL1β (*e.g.,* anakinra), IL6 (*e.g.,* tocilizumab), or TNF (*e.g.,* etanercept, infliximab, adlimumab, or certolizumab).

Toxicity and therapeutic efficacy of such compounds can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are typical.

The data obtained from cell culture assays and animal studies can be used in formulating a range of dosage for use in humans. The dosage of compositions featured herein lies generally within a range of circulating concentrations that include the ED50 with little or no toxicity. The dosage can vary within this range depending upon the dosage form employed and the route of administration utilized. For any compound used in the methods featured herein, the therapeutically effective dose can be estimated initially from cell culture assays. A dose can be formulated in animal models to achieve a circulating plasma concentration range of the compound or, when appropriate, of the polypeptide product of a target sequence (*e.g.*, achieving a decreased concentration of the polypeptide) that includes the IC50 (*i.e.*, the concentration of the test compound which achieves a half-maximal inhibition of symptoms) as determined in cell culture. Such information can be used to more accurately determine useful doses in humans. Levels in plasma may be measured, for example, by high performance liquid chromatography.

In addition to their administration, as discussed above, the iRNAs described herein can be administered in combination with other known agents effective in treatment of pathological processes mediated by target expression. In any event, the administering physician can adjust the amount and timing of iRNA administration on the basis of results observed using standard measures of efficacy known in the art or described herein.

VI. Antagomirs

The methods described herein can be useful for monitoring antagomir activity, e.g., after administering an antagomir to a subject.

Antagomirs are RNA-like oligonucleotides that harbor various modifications for RNAse protection and pharmacologic properties, such as enhanced tissue and cellular uptake. They differ from normal RNA by, for example, complete 2'-O-methylation of sugar, phosphorothioate backbone and, for example, a cholesterol-moiety at the 3'-end. Antagomirs may be used to efficiently silence endogenous miRNAs by forming duplexes comprising the antagomir and endogenous miRNA, thereby preventing miRNA-induced gene silencing. An example of antagomir-mediated miRNA silencing is the silencing of miR-122, described in Krutzfeldt et al, Nature, 2005, 438: 685-689. Antagomir RNAs may be synthesized using standard solid phase oligonucleotide synthesis protocols. See U.S. patent application Ser. No. 11/502,158 and 11/657,341. An antagomir can include ligand-conjugated monomer subunits and monomers for oligonucleotide synthesis. Exemplary monomers are described in U.S.20050107325. An antagomir can have a ZXY structure, such as is described in WO 2004/080406. An antagomir can be complexed with an amphipathic moiety. Exemplary amphipathic moieties for use with oligonucleotide agents are described in WO 2004/080406.

Antagomirs may be single stranded, double stranded, partially double stranded or hairpin-structured, chemically modified oligonucleotides that target a microRNA. An antagomir may consist essentially of or comprise about 12 or more contiguous nucleotides substantially complementary to an endogenous miRNA, and more particularly, agents that include about 12 or more contiguous nucleotides substantially complementary to a target sequence of an miRNA or pre-miRNA nucleotide sequence. An antagomir featured herein may include a nucleotide sequence sufficiently complementary to hybridize to a miRNA target sequence of about 12 to 25 nucleotides, in some instances about 15 to 23 nucleotides.

Antagomirs are further described, for example, in WO2007/021896.

The activity of an antagomir can be assayed, for example, by detecting target miRNA levels in a biological sample, such as a biological fluid, e.g., a blood, serum, urine or CSF sample, before and after administration of the antagomir. For example, an antagomir can target, e.g., miR-122, miR-16, miR-192, or miR-194, and the efficacy of the antagomir can be assessed by monitoring the level of the target miRNA in the biological sample.

VII. miRNA Mimics

The methods described herein can be useful for monitoring activity of an miRNA mimic, e.g., after administering the miRNA mimic to a subject.

miRNA mimics represent a class of molecules that can be used to imitate the gene silencing ability of one or more miRNAs. Thus, the term "microRNA mimic" refers to synthetic non-coding RNAs (*i.e.*, the miRNA is not obtained by purification from a source of the endogenous miRNA) that are capable of entering the RNAi pathway and regulating gene expression. miRNA mimics can be designed as mature molecules (*e.g.*, single stranded) or mimic precursors (*e.g.*,

pri- or pre-miRNAs). miRNA mimics can be comprised of nucleic acid (modified or modified nucleic acids) including oligonucleotides comprising, without limitation, RNA, modified RNA, DNA, modified DNA, locked nucleic acids, or 2'-O,4'-C-ethylene-bridged nucleic acids (ENA), or any combination of the above (including DNA-RNA hybrids). In addition, miRNA mimics can comprise conjugates that can affect delivery, intracellular compartmentalization, stability, specificity, functionality, strand usage, and/or potency. In one design, miRNA mimics are double stranded molecules (*e.g.*, with a duplex region of between about 16 and about 31 nucleotides in length) and contain one or more sequences that have identity with the mature strand of a given miRNA. Modifications can comprise 2' modifications (including 2'-O methyl modifications and 2' F modifications) on one or both strands of the molecule and internucleotide modifications (*e.g.,* phorphorthioate modifications) that enhance nucleic acid stability and/or specificity.

In addition, miRNA mimics can include overhangs. The overhangs can consist of 1-6 nucleotides on either the 3' or 5' end of either strand and can be modified to enhance stability or functionality. A miRNA mimic may comprise a duplex region of between 16 and 31 nucleotides and one or more of the following chemical modification patterns: the sense strand contains 2'-O-methyl modifications of nucleotides 1 and 2 (counting from the 5' end of the sense oligonucleotide), and all of the Cs and Us; the antisense strand modifications can comprise 2' F modification of all of the Cs and Us, phosphorylation of the 5' end of the oligonucleotide, and stabilized internucleotide linkages associated with a 2 nucleotide 3' overhang.

The activity of an miRNA mimic can be assayed by detecting levels of target mRNA in a biological sample, e.g., a blood or serum, or urine or CSF sample before and after administration of the miRNA mimic.

VIII. Gene Therapy

Described herein are assays and methods for detecting the introduction of a therapeutic (*e.g.*, exogenous) gene therapy to a subject, and for detecting and monitoring activity (e.g., expression) of the gene therapy. For example, an RNA product of a gene therapy can be detected using the methods and assays described herein. The activity of the gene therapy may be monitored by detecting protein expressed from the gene therapy in a biological sample of the subject, e.g., a blood, serum, urine or CSF sample. A gene therapy is useful for delivery of a DNA effector molecule.

Gene therapy generally involves the introduction of one or more therapeutic genes (*e.g.*, transgenes) into cells, whose expression results in amelioration or treatment of genetic disorders. The therapeutic genes involved may be those that encode proteins, structural or enzymatic RNAs, inhibitory products such as antisense RNA or DNA, or any other gene product. Expression is the generation of such a gene product or the resultant effects of the generation of such a gene product. Thus, enhanced expression includes the greater production of any therapeutic gene or the augmentation of that product's role in determining the condition of the cell, tissue, organ or organism.

The techniques for introducing nucleic acids into cells vary depending upon whether the nucleic acid is transferred into a cultured cell *in vitro* or injected *in vivo* directly into the cells of the intended host. Techniques suitable for the transfer of nucleic acid into mammalian cells *in vitro* include the use of liposomes, electroporation (Luxembourg A., et al., Expert Opinion Biol. Ther. 7(11):1647-1664 (2007); Kesaraju P, et al., Mol. Ther. 14(3):416-422 (2006); Luxembourg, et al., Vaccine (24(21):4490-4493 (2006)), microinjection, cell fusion, DEAE-dextran, the calcium phosphate precipitation method, etc.

Other exemplary gene transfer techniques include transfection with viral (typically, retroviral) vectors and viral coat protein-liposome mediated transfection (Dzau, et al., Trends in Biotechnology 11(5):205-10 (1993)), stem cell therapy, and vaccination with cDNA

Administering a polynucleotide to a mammal *in vivo,* such that a cellular immune response element or fragment thereof is expressed in the mammal, can be achieved using methods known in the art for mammalian gene expression. For example such methods for administering expressible polynucleotides to mammals including expression systems and delivery systems can be found in U.S. Pat. Nos. 6,875,748, 5,763,270, 5,580,859, 6,040,295, and 6,034,072.

A therapeutic gene can be administered as a plasmid, which contains the necessary elements for expression of the nucleic acid within the cassette. Expression includes the efficient transcription of an inserted gene, nucleic acid sequence, or nucleic acid cassette with the plasmid. Expression products may be proteins, polypeptides or RNA. The nucleic acid sequence can be contained in a nucleic acid cassette. Expression of the nucleic acid can be continuous or regulated.

Disclosed herein are methods for monitoring the activity of a gene therapy. For example, an RNA (e.g., an mRNA or iRNA agent) expressed by a gene therapy can be detected in a biological sample in a subject as a means to determine whether the gene therapy is being expressed and to what extent. Efficacy of the gene therapy may be monitored by detecting a protein product expressed from the gene therapy.

Methods for Treating Diseases Caused by Expression of the Target Gene

A disease to be treated using an iRNA agent or a modulator typically includes a disease having excess activity

or deficient activity of a gene product. Thus, an iRNA agent or modulator can be administered to inhibit expression of a gene that has excess activity. Alternatively, a therapeutic gene, a DNA effector molecule, or an RNA activating agent that activates gene expression can be used to correct deficient activity of a gene product. Essentially any disease, for which a target gene can be identified as having involvement in the pathogenesis or propagation of the disease, can be treated using an iRNA agent or a modulator. The assays described herein are useful for monitoring the efficacy of an iRNA agent or modulator in a subject.

An RNA or DNA effector molecule can be administered in combination with an additional therapeutic agent. The RNA or DNA effector molecule and the additional therapeutic agent can be administered in the same composition, e.g., parenterally, or the additional therapeutic agent can be administered as part of a separate composition or by another method described herein. Administration of the therapeutic gene and the additional therapeutic agent can be at the same time, or at different times and, in any order. An assay described herein can be performed at any stage of administration of the combination therapy, such as before and after administration of the RNA or DNA effector molecule; after administration of a combination of the RNA or DNA effector molecule and the additional therapeutic agent; or after administration of the RNA or DNA effector molecule and before and/or after further administration of the additional therapeutic agent, such as when the RNA or DNA effector molecule and the additional therapeutic agent are administered sequentially.

Also described herein is a method of administering an iRNA agent targeting a desired gene to a patient having a disease or disorder mediated by the target expression, and further assaying for an effect of the iRNA agent on target gene expression. Administration of the dsRNA can lower at least one symptom of the disease to be treated by 5%, 10%, 15%, 20%, 25% or more. The level of at least one symptm can be lowered to below 50%, below 45%, below 40%, below 35%, below 35% or lower.

By "lower" in this context is meant a statistically significant decrease in such level. The decrease can be, for example, at least 10%, at least 20%, at least 30%, at least 40% or more, *e.g.*, down to a level accepted as within the range of normal for an individual without such disorder.

In addition to assaying for an effect of the RNA or DNA effector on target gene expression, efficacy of treatment or prevention of disease can be assessed, for example by measuring disease progression, disease remission, symptom severity, reduction in pain, quality of life, dose of a medication required to sustain a treatment effect, level of a disease marker or any other measurable parameter appropriate for a given disease being treated or targeted for prevention. It is well within the ability of one skilled in the art to monitor efficacy of treatment or prevention by measuring any one of such parameters, or any combination of parameters.

A treatment or preventive effect is evident when there is a statistically significant improvement in one or more parameters of disease status, or by a failure to worsen or to develop symptoms where they would otherwise be anticipated. As an example, a favorable change of at least 10% in a measurable parameter of disease, *e.g.*, at least 20%, 30%, 40%, 50% or more can be indicative of effective treatment. Efficacy for a given iRNA drug or formulation of that drug can also be judged using an experimental animal model for the given disease as known in the art. When using an experimental animal model, efficacy of treatment is evidenced when a statistically significant reduction in a marker or symptom is observed.

Alternatively, the efficacy can be measured by a reduction in the severity of disease as determined by one skilled in the art of diagnosis based on a clinically accepted disease severity grading scale.

Patients can be administered a therapeutic amount of an RNA or DNA effector molecule, such as an iRNA, at e.g., 0.01 mg/kg, 0.05 mg/kg, 0.1 mg/kg, 0.5 mg/kg, 1.0 mg/kg, 1.5 mg/kg, 2.0 mg/kg, or 2.5 mg/kg dsRNA. The iRNA can be administered by intravenous infusion over a period of time, such as over a 5 minute, 10 minute, 15 minute, 20 minute, 25 minute, 30 minute, 60 minute, 90 minute or 120 minute period. The administration is repeated, for example, on a regular basis, such as biweekly (*i.e.*, every two weeks) for one month, two months, three months, four months or longer. After an initial treatment regimen, the treatments can be administered on a less frequent basis. For example, after administration biweekly for three months, administration can be repeated once per month, for six months or a year or longer. In another example, the administration is repeated, for example, on a regular basis, such as every four weeks) for two months, three months, four months or longer. After an initial treatment regimen, the treatments can be administered on a less frequent basis. For example, after administration every four weeks for three months, administration can be repeated once per two months, for six months or a year or longer. Administration of the iRNA can reduce gene expression levels, *e.g.,* in a cell, tissue, blood, urine or other compartment of the patient by at least 10%, at least 15%, at least 20%, at least 25%, at least 30%, at least 40%, at least 50%, at least 60%, at least 70%, at least 80 % or at least 90% or more.

Before administration of a full dose of the iRNA, patients can be administered a smaller dose, such as a 5% infusion reaction, and monitored for adverse effects, such as an allergic reaction or a worsening of symptoms. In another example, the patient can be monitored for unwanted immunostimulatory effects, such as increased cytokine (*e.g.*, TNF-$\alpha$ or INF-$\alpha$) levels.

Methods for Modulating Expression of a Target Gene

In yet another aspect, a subject is administered an agent for modulating (*e.g.*, inhibiting or activating) the expression of a target gene in a mammal, and the methods disclosed herein are useful for monitoring the effect of the agent on target gene expression.

The method may include administering an RNA effector molecule, such as an iRNA, such that expression of the target gene is decreased, such as for an extended duration, *e.g.,* at least two, three, four days or more, *e.g.,* one week, two weeks, three weeks, or four weeks or longer. Assays described herein are useful to monitor target gene expression over such duration.

The method may include administering a composition as described herein to a mammal such that expression of the target gene is increased by, e.g., at least 10% compared to an untreated animal. The activation of the target gene may occur over an extended duration, *e.g.*, at least two, three, four days or more, *e.g.,* one week, two weeks, three weeks, four weeks, or more. Without wishing to be bound by theory, an RNA activating agent can activate target gene expression by stabilizing the target mRNA transcript, interacting with a promoter in the genome, and/or inhibiting an inhibitor of target gene expression.

iRNAs useful for the methods and compositions featured herein can specifically target RNAs (primary or processed) of the target gene. Compositions and methods for inhibiting the expression of these genes using iRNAs can be prepared and performed as described elsewhere herein.

The method may include administering a composition containing an iRNA, where the iRNA includes a nucleotide sequence that is complementary to at least a part of an RNA transcript of the target gene of the mammal to be treated. When the organism to be treated is a mammal such as a human, the composition may be administered by any means known in the art including, but not limited to oral, intraperitoneal, or parenteral routes, including intracranial (*e.g.*, intraventricular, intraparenchymal and intrathecal), intravenous, intramuscular, subcutaneous, transdermal, airway (aerosol), nasal, rectal, and topical (including buccal and sublingual) administration. The compositions are administered by intravenous infusion or injection.

Unless otherwise defined, all technical and scientific terms used herein have the same meaning as commonly understood by one of ordinary skill in the art to which this invention belongs. In case of conflict, the present specification, including definitions, will control. In addition, the examples are illustrative only and not intended to be limiting.

## Examples

### Example 1. Interference RNA (iRNA) synthesis

*Source of reagents*

Where the source of a reagent is not specifically given herein, such reagent may be obtained from any supplier of reagents for molecular biology at a quality/purity standard for application in molecular biology.

*Oligonucleotide Synthesis.*

Applicants have used several different methods to generate iRNA molecules useful for treating a subject. This Example describes one approach that has been used. The ordinarily skilled artisan can use any method known in the art to prepare iRNAs as described herein.

Oligonucleotides are synthesized on an AKTAoligopilot synthesizer. Commercially available controlled pore glass solid support (dT-CPG, 500Å, Prime Synthesis) and RNA phosphoramidites with standard protecting groups, 5'-O-dimethoxytrityl N6-benzoyl-2'-t-butyldimethylsilyl-adenosine-3'-O-N,N'-diisopropyl-2-cyanoethylphosphoramidite, 5'-O-dimethoxytrityl-N4-acetyl-2'-t-butyldimethylsilyl-cytidine-3'-O-N,N'-diisopropyl-2-cyanoethylphosphoramidite, 5'-O-dimethoxytrityl-N2-isobutryl-2'-t-butyldimethylsilyl-guanosine-3'-O-N,N'-diisopropyl-2-cyanoethylphosphoramidite, and 5'-O-dimethoxytrityl-2'-t-butyldimethylsilyl-uridine-3'-O-N,N'-diisopropyl-2-cyanoethylphosphoramidite (Pierce Nucleic Acids Technologies) were used for the oligonucleotide synthesis. The 2'-F phosphoramidites, 5'-O-dimethoxytrityl-N4-acetyl-2'-fluro-cytidine-3'-O-N,N'-diisopropyl-2-cyanoethylphosphoramidite and 5'-O-dimethoxytrityl-2'-fluro-uridine-3'-O-N,N'-diisopropyl-2-cyanoethyl-phosphoramidite are purchased from (Promega). All phosphoramidites are used at a concentration of 0.2M in acetonitrile ($CH3CN$) except for guanosine which is used at 0.2M concentration in 10% THF/ANC (v/v). Coupling/recycling time of 16 minutes is used. The activator is 5-ethyl thiotetrazole (0.75M, American International Chemicals); for the PO-oxidation iodine/water/pyridine is used and for the PS-oxidation PADS (2%) in 2,6-lutidine/ACN (1:1 v/v) is used.

3'-ligand conjugated strands are synthesized using solid support containing the corresponding ligand. For example, the introduction of cholesterol unit in the sequence is performed from a hydroxyprolinol-cholesterol phosphoramidite. Cholesterol is tethered to trans-4-hydroxyprolinol via a 6-aminohexanoate linkage to obtain a hydroxyprolinol-cholesterol moiety. 5'-end Cy-3 and Cy-5.5 (fluorophore) labeled iRNAs are synthesized from the corresponding Quasar-570 (Cy-3) phosphoramidite are purchased from Biosearch Technologies. Conjugation of ligands to 5'-end and or internal position is achieved by using appropriately protected ligand-phosphoramidite building block. An extended 15 min coupling of 0.1 M solution of phosphoramidite in anhydrous CH3CN in the presence of 5-(ethylthio)-1H-tetrazole activator to a solid-support-bound oligonucleotide. Oxidation of the internucleotide phosphite to the phosphate is carried out using standard iodine-water as reported (1) or by treatment with tert-butyl hydroperoxide/acetonitrile/water (10: 87: 3) with 10 min oxidation wait time conjugated oligonucleotide. Phosphorothioate is introduced by the oxidation of phosphite to phosphorothioate by using a sulfur transfer reagent such as DDTT (purchased from AM Chemicals), PADS and or Beaucage reagent. The cholesterol phosphoramidite is synthesized in house and used at a concentration of 0.1 M in dichloromethane. Coupling time for the cholesterol phosphoramidite is 16 minutes.

*Deprotection I (Nucleobase Deprotection)*

After completion of synthesis, the support is transferred to a 100 mL glass bottle (VWR). The oligonucleotide is cleaved from the support with simultaneous deprotection of base and phosphate groups with 80 mL of a mixture of ethanolic ammonia [ammonia: ethanol (3:1)] for 6.5 h at 55oC. The bottle is cooled briefly on ice and then the ethanolic ammonia mixture is filtered into a new 250-mL bottle. The CPG is washed with 2 x 40 mL portions of ethanol/water (1:1 v/v). The volume of the mixture is then reduced to ~ 30 mL by roto-vap. The mixture is then frozen on dry ice and dried under vacuum on a speed vac.

*Deprotection II (Removal of 2'-TBDMS group)*

The dried residue is resuspended in 26 mL of triethylamine, triethylamine trihydrofluoride (TEA•3HF) or pyridine-HF and DMSO (3:4:6) and heated at 60°C for 90 minutes to remove the tert-butyldimethylsilyl (TBDMS) groups at the 2' position. The reaction is then quenched with 50 mL of 20 mM sodium acetate and the pH is adjusted to 6.5. Oligonucleotide is stored in a freezer until purification.

***Analysis***

The oligonucleotides are analyzed by high-performance liquid chromatography (HPLC) prior to purification and selection of buffer and column depends on nature of the sequence and or conjugated ligand.

*HPLC Purification*

The ligand-conjugated oligonucleotides are purified by reverse-phase preparative HPLC. The unconjugated oligonucleotides are purified by anion-exchange HPLC on a TSK gel column packed in house. The buffers are 20 mM sodium phosphate (pH 8.5) in 10% CH3CN (buffer A) and 20 mM sodium phosphate (pH 8.5) in 10% CH3CN, 1M NaBr (buffer B). Fractions containing full-length oligonucleotides are pooled, desalted, and lyophilized. Approximately 0.15 OD of desalted oligonucleotides are diluted in water to 150 μL and then pipetted into special vials for CGE and LC/MS analysis. Compounds are then analyzed by LC-ESMS and CGE.

*iRNA preparation*

For the general preparation of iRNA, equimolar amounts of sense and antisense strand are heated in 1xPBS at 95°C for 5 min and slowly cooled to room temperature. Integrity of the duplex is confirmed by HPLC analysis.

Nucleic acid sequences are represented below using standard nomenclature, and specifically the abbreviations of Table 1.

**Table 1: Abbreviations of nucleotide monomers used in nucleic acid sequence representation. It will be understood that these monomers, when present in an oligonucleotide, are mutually linked by 5'-3'-phosphodiester bonds.**

| Abbreviation | Nucleotide(s) |
|---|---|
| A | adenosine |
| C | cytidine |
| G | guanosine |

(continued)

| Abbreviation | Nucleotide(s) |
|---|---|
| T | thymidine |
| U | uridine |
| N | any nucleotide (G, A, C, T or U) |
| a | 2'-O-methyladenosine |
| c | 2'-O-methylcytidine |
| g | 2'-O-methylguanosine |
| u | 2'-O-methyluridine |
| dT | 2'-deoxythymidine |
| s | phosphorothioate linkage |

**Table 2: Exemplary iRNA molecules targeting human transthyretin (TTR) mRNA.**

| Position relative to (NM_000371.3, SEQ ID NO:1) | Sense Sequence (5' to 3') | SEQ ID NO: | Antisense Sequence (5' to 3') | SEQ ID NO: |
|---|---|---|---|---|
| 100 | CCGGUGAAUCCAAGUGUCCdTdT | 9 | GGACACUUGGAUUCACCGGdTdT | 10 |
| 11 | ACUCAUUCUUGGCAGGAUGdTdT | 11 | CAUCCUGCCAAGAAUGAGUdTdT | 12 |
| 111 | AAGUGUCCUCUGAUGGUCAdTdT | 13 | UGACCAUCAGAGGACACUUdTdT | 14 |
| 13 | UCAUUCUUGGCAGGAUGGCdTdT | 15 | GCCAUCCUGCCAAGAAUGAdTdT | 16 |
| 130 | AAGUUCUAGAUGCUGUCCGdTdT | 17 | CGGACAGCAUCUAGAACUUdTdT | 18 |
| 132 | GUUCUAGAUGCUGUCCGAGdTdT | 19 | CUCGGACAGCAUCUAGAACdTdT | 20 |
| 135 | CUAGAUGCUGUCCGAGGCAdTdT | 21 | UGCCUCGGACAGCAUCUAGdTdT | 22 |
| 138 | GAUGCUGUCCGAGGCAGUCdTdT | 23 | GACUGCCUCGGACAGCAUCdTdT | 24 |
| 14 | CAUUCUUGGCAGGAUGGCUdTdT | 25 | AGCCAUCCUGCCAAGAAUGdTdT | 26 |
| 140 | UGCUGUCCGAGGCAGUCCUdTdT | 27 | AGGACUGCCUCGGACAGCAdTdT | 28 |
| 146 | CCGAGGCAGUCCUGCCAUCdTdT | 29 | GAUGGCAGGACUGCCUCGGdTdT | 30 |
| 152 | CAGUCCUGCCAUCAAUGUGdTdT | 31 | CACAUUGAUGGCAGGACUGdTdT | 32 |
| 164 | CAAUGUGGCCGUGCAUGUGdTdT | 33 | CACAUGCACGGCCACAUUGdTdT | 34 |
| 178 | AUGUGUUCAGAAAGGCUGCdTdT | 35 | GCAGCCUUUCUGAACACAUdTdT | 36 |
| 2 | CAGAAGUCCACUCAUUCUUdTdT | 37 | AAGAAUGAGUGGACUUCUGdTdT | 38 |
| 21 | GGCAGGAUGGCUUCUCAUCdTdT | 39 | GAUGAGAAGCCAUCCUGCCdTdT | 40 |
| 210 | GAGCCAUUUGCCUCUGGGAdTdT | 41 | UCCCAGAGGCAAAUGGCUCdTdT | 42 |
| 23 | CAGGAUGGCUUCUCAUCGUdTdT | 43 | ACGAUGAGAAGCCAUCCUGdTdT | 44 |
| 24 | AGGAUGGCUUCUCAUCGUCdTdT | 45 | GACGAUGAGAAGCCAUCCUdTdT | 46 |
| 245 | AGAGCUGCAUGGGCUCACAdTdT | 47 | UGUGAGCCCAUGCAGCUCUdTdT | 48 |
| 248 | GCUGCAUGGGCUCACAACUdTdT | 49 | AGUUGUGAGCCCAUGCAGCdTdT | 50 |
| 25 | GGAUGGCUUCUCAUCGUCUdTdT | 51 | AGACGAUGAGAAGCCAUCCdTdT | 52 |
| 251 | GCAUGGGCUCACAACUGAGdTdT | 53 | CUCAGUUGUGAGCCCAUGCdTdT | 54 |

(continued)

| Position relative to (NM_000371.3, SEQ ID NO:1) | Sense Sequence (5' to 3') | SEQ ID NO: | Antisense Sequence (5' to 3') | SEQ ID NO: |
|---|---|---|---|---|
| 253 | AUGGGCUCACAACUGAGGAdTdT | 55 | UCCUCAGUUGUGAGCCCAUdTdT | 56 |
| 254 | UGGGCUCACAACUGAGGAGdTdT | 57 | CUCCUCAGUUGUGAGCCCAdTdT | 58 |
| 270 | GAGGAAUUUGUAGAAGGGAdTdT | 59 | UCCCUUCUACAAAUUCCUCdTdT | 60 |
| 276 | UUUGUAGAAGGGAUAUACAdTdT | 61 | UGUAUAUCCCUUCUACAAAdTdT | 62 |
| 277 | UUGUAGAAGGGAUAUACAAdTdT | 63 | UUGUAUAUCCCUUCUACAAdTdT | 64 |
| 278 | UGUAGAAGGGAUAUACAAAdTdT | 65 | UUUGUAUAUCCCUUCUACAdTdT | 66 |
| 281 | AGAAGGGAUAUACAAAGUGdTdT | 67 | CACUUUGUAUAUCCCUUCUdTdT | 68 |
| 295 | AAGUGGAAAUAGACACCAAdTdT | 69 | UUGGUGUCUAUUUCCACUUdTdT | 70 |
| 299 | GGAAAUAGACACCAAAUCUdTdT | 71 | AGAUUUGGUGUCUAUUUCCdTdT | 72 |
| 300 | GAAAUAGACACCAAAUCUUdTdT | 73 | AAGAUUUGGUGUCUAUUUCdTdT | 74 |
| 303 | AUAGACACCAAAUCUUACUdTdT | 75 | AGUAAGAUUUGGUGUCUAUdTdT | 76 |
| 304 | UAGACACCAAAUCUUACUGdTdT | 77 | CAGUAAGAUUUGGUGUCUAdTdT | 78 |
| 305 | AGACACCAAAUCUUACUGGdTdT | 79 | CCAGUAAGAUUUGGUGUCUdTdT | 80 |
| 317 | UUACUGGAAGGCACUUGGCdTdT | 81 | GCCAAGUGCCUUCCAGUAAdTdT | 82 |
| 32 | UUCUCAUCGUCUGCUCCUCdTdT | 83 | GAGGAGCAGACGAUGAGAAdTdT | 84 |
| 322 | GGAAGGCACUUGGCAUCUCdTdT | 85 | GAGAUGCCAAGUGCCUUCCdTdT | 86 |
| 326 | GGCACUUGGCAUCUCCCCAdTdT | 87 | UGGGGAGAUGCCAAGUGCCdTdT | 88 |
| 333 | GGCAUCUCCCCAUUCCAUGdTdT | 89 | AUGGAAUGGGGAGAUGCCTTdTdT | 90 |
| 334 | GCAUCUCCCCAUUCCAUGAdTdT | 91 | UCAUGGAAUGGGGAGAUGCdTdT | 92 |
| 335 | CAUCUCCCCAUUCCAUGAGdTdT | 93 | CUCAUGGAAUGGGGAGAUGdTdT | 94 |
| 336 | AUCUCCCCAUUCCAUGAGCdTdT | 95 | GCUCAUGGAAUGGGGAGAUdTdT | 96 |
| 338 | CUCCCCAUUCCAUGAGCAUdTdT | 97 | AUGCUCAUGGAAUGGGGAGdTdT | 98 |
| 341 | CCCAUUCCAUGAGCAUGCAdTdT | 99 | UGCAUGCUCAUGGAAUGGGdTdT | 100 |
| 347 | CCAUGAGCAUGCAGAGGUGdTdT | 101 | CACCUCUGCAUGCUCAUGGdTdT | 102 |
| 352 | AGCAUGCAGAGGUGGUAUUdTdT | 103 | AAUACCACCUCUGCAUGCUdTdT | 104 |
| 354 | CAUGCAGAGGUGGUAUUCAdTdT | 105 | UGAAUACCACCUCUGCAUGdTdT | 106 |
| 355 | AUGCAGAGGUGGUAUUCACdTdT | 107 | GUGAAUACCACCUCUGCAUdTdT | 108 |
| 362 | GGUGGUAUUCACAGCCAACdTdT | 109 | GUUGGCUGUGAAUACCACCdTdT | 110 |
| 363 | GUGGUAUUCACAGCCAACGdTdT | 111 | CGUUGGCUGUGAAUACCACdTdT | 112 |
| 364 | UGGUAUUCACAGCCAACGAdTdT | 113 | UCGUUGGCUGUGAAUACCAdTdT | 114 |
| 365 | GGUAUUCACAGCCAACGACdTdT | 115 | GUCGUUGGCUGUGAAUACCdTdT | 116 |
| 366 | GUAUUCACAGCCAACGACUdTdT | 117 | AGUCGUUGGCUGUGAAUACdTdT | 118 |
| 367 | UAUUCACAGCCAACGACUCdTdT | 119 | GAGUCGUUGGCUGUGAAUAdTdT | 120 |
| 370 | UCACAGCCAACGACUCCGGdTdT | 121 | CCGGAGUCGUUGGCUGUGAdTdT | 122 |
| 390 | CCCCGCCGCUACACCAUUGdTdT | 123 | CAAUGGUGUAGCGGCGGGGdTdT | 124 |

(continued)

| Position relative to (NM_000371.3, SEQ ID NO:1) | Sense Sequence (5' to 3') | SEQ ID NO: | Antisense Sequence (5' to 3') | SEQ ID NO: |
|---|---|---|---|---|
| 4 | GAAGUCCACUCAUUCUUGGdTdT | 125 | CCAAGAAUGAGUGGACUUCdTdT | 126 |
| 412 | CCCUGCUGAGCCCCUACUCdTdT | 127 | GAGUAGGGGCUCAGCAGGGdTdT | 128 |
| 417 | CUGAGCCCCUACUCCUAUUdTdT | 129 | AAUAGGAGUAGGGGCUCAGdTdT | 130 |
| 418 | UGAGCCCCUACUCCUAUUCdTdT | 131 | GAAUAGGAGUAGGGGCUCAdTdT | 132 |
| 422 | CCCCUACUCCUAUUCCACCdTdT | 133 | GGUGGAAUAGGAGUAGGGGdTdT | 134 |
| 425 | CUACUCCUAUUCCACCACGdTdT | 135 | CGUGGUGGAAUAGGAGUAGdTdT | 136 |
| 426 | UACUCCUAUUCCACCACGGdTdT | 137 | CCGUGGUGGAAUAGGAGUAdTdT | 138 |
| 427 | ACUCCUAUUCCACCACGGCdTdT | 139 | GCCGUGGUGGAAUAGGAGUdTdT | 140 |
| 429 | UCCUAUUCCACCACGGCUGdTdT | 141 | CAGCCGUGGUGGAAUAGGAdTdT | 142 |
| 432 | UAUUCCACCACGGCUGUCGdTdT | 143 | CGACAGCCGUGGUGGAAUAdTdT | 144 |
| 433 | AUUCCACCACGGCUGUCGUdTdT | 145 | ACGACAGCCGUGGUGGAAUdTdT | 146 |
| 437 | CACCACGGCUGUCGUCACCdTdT | 147 | GGUGACGACAGCCGUGGUGdTdT | 148 |
| 438 | ACCACGGCUGUCGUCACCAdTdT | 149 | UGGUGACGACAGCCGUGGUdTdT | 150 |
| 439 | CCACGGCUGUCGUCACCAAdTdT | 151 | UUGGUGACGACAGCCGUGGdTdT | 152 |
| 441 | ACGGCUGUCGUCACCAAUCdTdT | 153 | GAUUGGUGACGACAGCCGUdTdT | 154 |
| 442 | CGGCUGUCGUCACCAAUCCdTdT | 155 | GGAUUGGUGACGACAGCCGdTdT | 156 |
| 449 | CGUCACCAAUCCCAAGGAAdTdT | 157 | UUCCUUGGGAUUGGUGACGdTdT | 158 |
| 455 | CAAUCCCAAGGAAUGAGGGdTdT | 159 | CCCUCAUUCCUUGGGAUUGdTdT | 160 |
| 491 | CCUGAAGGACGAGGGAUGGdTdT | 161 | CCAUCCCUCGUCCUUCAGGdTdT | 162 |
| 497 | GGACGAGGGAUGGGAUUUCdTdT | 163 | GAAAUCCCAUCCCUCGUCCdTdT | 164 |
| 5 | AAGUCCACUCAUUCUUGGCdTdT | 165 | GCCAAGAAUGAGUGGACUUdTdT | 166 |
| 508 | GGGAUUUCAUGUAACCAAGdTdT | 167 | CUUGGUUACAUGAAAUCCCdTdT | 168 |
| 509 | GGAUUUCAUGUAACCAAGAdTdT | 169 | UCUUGGUUACAUGAAAUCCdTdT | 170 |
| 514 | UCAUGUAACCAAGAGUAUUdTdT | 171 | AAUACUCUUGGUUACAUGAdTdT | 172 |
| 516 | AUGUAACCAAGAGUAUUCCdTdT | 173 | GGAAUACUCUUGGUUACAUdTdT | 174 |
| 517 | UGUAACCAAGAGUAUUCCAdTdT | 175 | UGGAAUACUCUUGGUUACAdTdT | 176 |
| 518 | GUAACCAAGAGUAUUCCAUdTdT | 177 | AUGGAAUACUCUUGGUUACdTdT | 178 |
| 54 | UGCCUUGCUGGACUGGUAUdTdT | 179 | AUACCAGUCCAGCAAGGCAdTdT | 180 |
| 543 | UAAAGCAGUGUUUUCACCUdTdT | 181 | AGGUGAAAACACUGCUUUAdTdT | 182 |
| 55 | GCCUUGCUGGACUGGUAUUdTdT | 183 | AAUACCAGUCCAGCAAGGCdTdT | 184 |
| 551 | UGUUUUCACCUCAUAUGCUdTdT | 185 | AGCAUAUGAGGUGAAAACAdTdT | 186 |
| 552 | GUUUUCACCUCAUAUGCUAdTdT | 187 | UAGCAUAUGAGGUGAAAACdTdT | 188 |
| 553 | UUUUCACCUCAUAUGCUAUdTdT | 189 | AUAGCAUAUGAGGUGAAAAdTdT | 190 |
| 555 | UUCACCUCAUAUGCUAUGUdTdT | 191 | ACAUAGCAUAUGAGGUGAAdTdT | 192 |
| 557 | CACCUCAUAUGCUAUGUUAdTdT | 193 | UAACAUAGCAUAUGAGGUGdTdT | 194 |

(continued)

| Position relative to (NM_000371.3, SEQ ID NO:1) | Sense Sequence (5' to 3') | SEQ ID NO: | Antisense Sequence (5' to 3') | SEQ ID NO: |
|---|---|---|---|---|
| 56 | CCUUGCUGGACUGGUAUUUdTdT | 195 | AAAUACCAGUCCAGCAAGGdTdT | 196 |
| 563 | AUAUGCUAUGUUAGAAGUCdTdT | 197 | GACUUCUAACAUAGCAUAUdTdT | 198 |
| 564 | UAUGCUAUGUUAGAAGUCCdTdT | 199 | GGACUUCUAACAUAGCAUAdTdT | 200 |
| 566 | UGCUAUGUUAGAAGUCCAGdTdT | 201 | CUGGACUUCUAACAUAGCAdTdT | 202 |
| 57 | CUUGCUGGACUGGUAUUUGdTdT | 203 | CAAAUACCAGUCCAGCAAGdTdT | 204 |
| 578 | AGUCCAGGCAGAGACAAUAdTdT | 205 | AUUGUCUCUGCCUGGACUTTdTdT | 206 |
| 580 | UCCAGGCAGAGACAAUAAAdTdT | 207 | UUUAUUGUCUCUGCCUGGAdTdT | 208 |
| 607 | GUGAAAGGCACUUUUCAUUdTdT | 209 | AAUGAAAAGUGCCUUUCACdTdT | 210 |
| 62 | UGGACUGGUAUUUGUGUCUdTdT | 211 | AGACACAAAUACCAGUCCAdTdT | 212 |
| 77 | GUCUGAGGCUGGCCCUACGdTdT | 213 | CGUAGGGCCAGCCUCAGACdTdT | 214 |
| 79 | CUGAGGCUGGCCCUACGGGdTdT | 215 | CCCGUAGGGCCAGCCUCAGdTdT | 216 |
| 81 | GAGGCUGGCCCUACGGGCAdTdT | 217 | UGCCCGUAGGGCCAGCCUCdTdT | 218 |
| 82 | AGGCUGGCCCUACGGGCACdTdT | 219 | GUGCCCGUAGGGCCAGCCUdTdT | 220 |
| 84 | GCUGGCCCUACGGGCACCGdTdT | 221 | CGGUGCCCGUAGGGCCAGCdTdT | 222 |
| 85 | CUGGCCCUACGGGCACCGGdTdT | 223 | CCGGUGCCCGUAGGGCCAGdTdT | 224 |
| 87 | GGCCCUACGGGCACCGGUGdTdT | 225 | CACCGGUGCCCGUAGGGCCdTdT | 226 |
| 9 | CCACUCAUUCUUGGCAGGAdTdT | 227 | UCCUGCCAAGAAUGAGUGGdTdT | 228 |
| 90 | CCUACGGGCACCGGUGAAUdTdT | 229 | AUUCACCGGUGCCCGUAGGdTdT | 230 |
| 91 | CUACGGGCACCGGUGAAUCdTdT | 231 | GAUUCACCGGUGCCCGUAGdTdT | 232 |
| 92 | UACGGGCACCGGUGAAUCCdTdT | 233 | GGAUUCACCGGUGCCCGUAdTdT | 234 |
| 93 | ACGGGCACCGGUGAAUCCAdTdT | 235 | UGGAUUCACCGGUGCCCGUdTdT | 236 |
| 97 | GCACCGGUGAAUCCAAGUGdTdT | 237 | CACUUGGAUUCACCGGUGCdTdT | 238 |
| 98 | CACCGGUGAAUCCAAGUGUdTdT | 239 | ACACUUGGAUUCACCGGUGdTdT | 240 |
| 167 | UGUGGCCAUGCAUGUGUUCdTdT | 241 | GAACACAUGCAUGGCCACAdTdT | 242 |
| 168 | GUGGCCAUGCAUGUGUUCAdTdT | 243 | UGAACACAUGCAUGGCCACdTdT | 244 |
| 171 | GCCAUGCAUGUGUUCAGAAdTdT | 245 | UUCUGAACACAUGCAUGGCdTdT | 246 |
| 432 | UAUUCCACCACGGCUGUCAdTdT | 247 | UGACAGCCGUGGUGGAAUAdTdT | 248 |
| 447 | GUCAUCACCAAUCCCAAGGdTdT | 249 | CCUUGGGAUUGGUGAUGACdTdT | 250 |
| 115 | GUCCUCUGAUGGUCAAAGUdTdT | 251 | ACUUUGACCAUCAGAGGACdTdT | 252 |
| 122 | GAUGGUCAAAGUUCUAGAUdTdT | 253 | AUCUAGAACUUUGACCAUCdTdT | 254 |
| 139 | AUGCUGUCCGAGGCAGUCCdTdT | 255 | GGACUGCCUCGGACAGCAUdTdT | 256 |
| 172 | CCGUGCAUGUGUUCAGAAAdTdT | 257 | UUUCUGAACACAUGCACGGdTdT | 258 |
| 238 | AGUCUGGAGAGCUGCAUGGdTdT | 259 | CCAUGCAGCUCUCCAGACUdTdT | 260 |
| 252 | CAUGGGCUCACAACUGAGGdTdT | 261 | CCUCAGUUGUGAGCCCAUGdTdT | 262 |
| 33 | UCUCAUCGUCUGCUCCUCCdTdT | 263 | GGAGGAGCAGACGAUGAGAdTdT | 264 |

(continued)

| Position relative to (NM_000371.3, SEQ ID NO:1) | Sense Sequence (5' to 3') | SEQ ID NO: | Antisense Sequence (5' to 3') | SEQ ID NO: |
|---|---|---|---|---|
| 340 | CCCCAUUCCAUGAGCAUGCdTdT | 265 | GCAUGCUCAUGGAAUGGGGdTdT | 266 |
| 421 | GCCCCUACUCCUAUUCCACdTdT | 267 | GUGGAAUAGGAGUAGGGGCdTdT | 268 |
| 431 | CUAUUCCACCACGGCUGUCdTdT | 269 | GACAGCCGUGGUGGAAUAGdTdT | 270 |
| 440 | CACGGCUGUCGUCACCAAUdTdT | 271 | AUUGGUGACGACAGCCGUGdTdT | 272 |
| 496 | AGGACGAGGGAUGGGAUUUdTdT | 273 | AAAUCCCAUCCCUCGUCCUdTdT | 274 |
| 556 | UCACCUCAUAUGCUAUGUUdTdT | 275 | AACAUAGCAUAUGAGGUGAdTdT | 276 |
| 559 | CCUCAUAUGCUAUGUUAGAdTdT | 277 | UCUAACAUAGCAUAUGAGGdTdT | 278 |
| 570 | AUGUUAGAAGUCCAGGCAGdTdT | 279 | CUGCCUGGACUUCUAACAUdTdT | 280 |
| 78 | UCUGAGGCUGGCCCUACGGdTdT | 281 | CCGUAGGGCCAGCCUCAGAdTdT | 282 |
| 87 | GGCCCUACGGGCACCGGUGdTdT | 283 | CACCGGUGCCCGUAGGGCCdTdT | 284 |
| 95 | GGGCACCGGUGAAUCCAAGdTdT | 285 | CUUGGAUUCACCGGUGCCCdTdT | 286 |
| 167 | CCAUGCAUGUGUUCAGAAAdTdT | 287 | UUUCUGAACACAUGCAUGGdTdT | 288 |

**Table 3: Exemplary iRNA molecules targeting huntingtin (HTT) mRNA.**

| Duplex name | Sense strand sequence (5'-3') | SEQ ID NO: | Antisense strand sequence (5'-3') | SEQ ID NO: |
|---|---|---|---|---|
| AD-10894 | gaaucgagaucggaugucaTT | 289 | ugacauccgaucucgauucTT | 290 |
| AD-10895 | aaauccugcuuuagucgagTT | 291 | cucgacuaaagcaggauuuTT | 292 |
| AD-10896 | agucaguccggguagaacuTT | 293 | aguucuacccggacugacuTT | 294 |
| AD-10897 | gguuuaugaacugacguuaTT | 295 | uaacgucaguucauaaaccTT | 296 |
| AD-10898 | guuacggguuaauuacuguTT | 297 | acaguaauuaacccguaacTT | 298 |
| AD-10899 | ugcuuuagucgagaaccaaTT | 299 | uugguucucgacuaaagcaTT | 300 |
| AD-10900 | ucuguaccguugagucccaTT | 301 | ugggacucaacgguacagaTT | 302 |
| AD-10901 | aaauuguguuagacgguacTT | 303 | guaccgucuaacacaauuuTT | 304 |
| AD-10902 | uggccggaaacuugcuugcTT | 305 | gcaagcaaguuuccggccaTT | 306 |
| AD-10903 | guucaguuacggguuaauuTT | 307 | aauuaacccguaacugaacTT | 308 |
| AD-10904 | gcgggcucguuccaugaucTT | 309 | gaucauggaacgagcccgcTT | 310 |
| AD-10905 | gacuccgagcacuuaacguTT | 311 | acguuaagugcucggagucTT | 312 |
| AD-10906 | cgcauggucgacauccuugTT | 313 | caaggaugucgaccaugcgTT | 314 |
| AD-10907 | aagacgagauccucgcucaTT | 315 | ugagcgaggaucucgucuuTT | 316 |
| AD-10908 | aagucaguccggguagaacTT | 317 | guucuacccggacugacuuTT | 318 |
| AD-10909 | aaggccuucauagcgaaccTT | 319 | gguucgcuaugaaggccuuTT | 320 |
| AD-10910 | aggccuucauagcgaaccuTT | 321 | agguucgcuaugaaggccuTT | 322 |
| AD-10911 | acuccgagcacuuaacgugTT | 323 | cacguuaagugcucggaguTT | 324 |
| AD-10912 | uaaaggccuucauagcgaaTT | 325 | uucgcuaugaaggccuuuaTT | 326 |
| AD-10913 | ucugaaucgagaucggaugTT | 327 | cauccgaucucgauucagaTT | 328 |

(continued)

| Duplex name | Sense strand sequence (5'-3') | SEQ ID NO: | Antisense strand sequence (5'-3') | SEQ ID NO: |
|---|---|---|---|---|
| AD-10914 | ugaaauuguguuagacgguTT | 329 | accgucuaacacaauuucaTT | 330 |
| AD-10915 | uggcucgcauggucgacauTT | 331 | augucgaccaugcgagccaTT | 332 |
| AD-10916 | aaagucaguccggguagaaTT | 333 | uucuacccggacugacuuuTT | 334 |
| AD-10917 | gagugcccgugucgguucuTT | 335 | agaaccgacacgggcacucTT | 336 |
| AD-10918 | ggagcucgggacggauaguTT | 337 | acuauccgucccgagcuccTT | 338 |
| AD-10919 | agaaaacaagccuugccgcTT | 339 | gcggcaaggcuuguuuucuTT | 340 |
| AD-10920 | auaaucacauucguuuguuTT | 341 | aacaaacgaaugugauuauTT | 342 |
| AD-10921 | ucugggcaucgcuauggaaTT | 343 | uuccauagcgaugcccagaTT | 344 |
| AD-10922 | ggccuucauagcgaaccugTT | 345 | cagguucgcuaugaaggccTT | 346 |
| AD-10923 | cuaaaugugcucuuaggcuTT | 347 | agccuaagagcacauuuagTT | 348 |
| AD-10924 | guuuaugaacugacguuacTT | 349 | guaacgucaguucauaaacTT | 350 |
| AD-10925 | uuuaugaacugacguuacaTT | 351 | uguaacgucaguucauaaaTT | 352 |
| AD-10926 | augaacugacguuacaucaTT | 353 | ugauguaacgucaguucauTT | 354 |
| AD-10927 | ccacaauguugugaccggaTT | 355 | uccggucacaacauuguggTT | 356 |
| AD-10928 | cugguggccgaagccguagTT | 357 | cuacggcuucggccaccagTT | 358 |
| AD-10929 | aauuguguuagacgguaccTT | 359 | gguaccgucuaacacaauuTT | 360 |
| AD-10930 | uuguguuagacgguaccgaTT | 361 | ucgguaccgucuaacacaaTT | 362 |
| AD-10931 | aaaacaagccuugccgcauTT | 363 | augcggcaaggcuuguuuuTT | 364 |
| AD-10932 | aagagcuguaccguugggaTT | 365 | ucccaacgguacagcucuuTT | 366 |
| AD-10933 | auaccucagguccuguuacTT | 367 | guaacaggaccugagguauTT | 368 |
| AD-10934 | uccugcuuuagucgagaacTT | 369 | guucucgacuaaagcaggaTT | 370 |
| AD-10935 | cauaaucacauucguuuguTT | 371 | acaaacgaaugugauuaugTT | 372 |
| AD-10936 | aagcgacugucucgacagaTT | 373 | ucugucgagacagucgcuuTT | 374 |
| AD-10937 | ccgagcacuuaacguggcuTT | 375 | agccacguuaagugcucggTT | 376 |
| AD-10938 | cuggcucgcauggucgacaTT | 377 | ugucgaccaugcgagccagTT | 378 |
| AD-10939 | uugucgccggguagaaaugTT | 379 | cauuucuacccggcgacaaTT | 380 |
| AD-10940 | ugcaagacucacuuaguccTT | 381 | ggacuaagugagucuugcaTT | 382 |
| AD-10941 | gaaacagugaguccggacaTT | 383 | uguccggacucacuguuucTT | 384 |
| AD-10942 | aaaucccaguguuggaccaTT | 385 | ugguccaacacugggauuuTT | 386 |
| AD-10943 | gcuagcuccaugcuuaagcTT | 387 | gcuuaagcauggagcuagcTT | 388 |
| AD-10944 | uccaugcuuaagccuagggTT | 389 | cccuaggcuuaagcauggaTT | 390 |
| AD-10945 | ccaugcuuaagccuagggaTT | 391 | ucccuaggcuuaagcauggTT | 392 |
| AD-10946 | ucaacagcuacacacguguTT | 393 | acacguguguagcuguugaTT | 394 |
| AD-10947 | augugugccacugcguuuuTT | 395 | aaaacgcaguggcacacauTT | 396 |
| AD-10948 | ugugugccacugcguuuuaTT | 397 | uaaaacgcaguggcacacaTT | 398 |
| AD-10949 | ucaguccggguagaacuucTT | 399 | gaaguucuacccggacugaTT | 400 |
| AD-10950 | aguccggguagaacuucagTT | 401 | cugaaguucuacccggacuTT | 402 |
| AD-10951 | gauuguugcuauggagcggTT | 403 | ccgcuccauagcaacaaucTT | 404 |

(continued)

| Duplex name | Sense strand sequence (5'-3') | SEQ ID NO: | Antisense strand sequence (5'-3') | SEQ ID NO: |
|---|---|---|---|---|
| AD-10952 | acuuguuuacgaaauguccTT | 405 | ggacauuucguaaacaaguTT | 406 |
| AD-10953 | cuuguuuacgaaauguccaTT | 407 | uggacauuucguaaacaagTT | 408 |
| AD-10954 | gcuuccgcacaugccgcggTT | 409 | ccgcggcaugugcggaagcTT | 410 |
| AD-10955 | uaauuuuaacguaacucuuTT | 411 | aagaguuacguuaaaauuaTT | 412 |
| AD-10956 | cuuucuaugcccguguaaaTT | 413 | uuuacacgggcauagaaagTT | 414 |
| AD-10957 | aaagggaaggacugacgagTT | 415 | cucgucaguccuucccuuuTT | 416 |
| AD-10958 | gcuggcucgcauggucgacTT | 417 | gucgaccaugcgagccagcTT | 418 |
| AD-10959 | ugacguuacaucauacacaTT | 419 | uguguaugauguaacgucaTT | 420 |
| AD-10960 | acgguaccgacaaccaguaTT | 421 | uacugguugucgguaccguTT | 422 |
| AD-10961 | gguaccgacaaccaguauuTT | 423 | aauacugguugucgguaccTT | 424 |
| AD-10962 | acgagugcucaauaauguuTT | 425 | aacauuauugagcacucguTT | 426 |
| AD-10963 | caucggagaguuucuguccTT | 427 | ggacagaaacucuccgaugTT | 428 |
| AD-10964 | gcgaaccugaagucaagcuTT | 429 | agcuugacuucagguucgcTT | 430 |
| AD-10965 | cugaaucgagaucggauguTT | 431 | acauccgaucucgauucagTT | 432 |
| AD-10966 | cgguaccgacaaccaguauTT | 433 | auacugguugucgguaccgTT | 434 |
| AD-10967 | acugaaccgggugaucaagTT | 435 | cuugaucacccgguucaguTT | 436 |
| AD-10968 | ccuugccgcaucaaaggugTT | 437 | caccuuugaugcggcaaggTT | 438 |
| AD-10969 | cuuuggcggauugcauuccTT | 439 | ggaaugcaauccgccaaagTT | 440 |
| AD-10970 | cuguaccguugagucccaaTT | 441 | uugggacucaacgguacagTT | 442 |
| AD-10971 | uguaccguugagucccaagTT | 443 | cuugggacucaacgguacaTT | 444 |
| AD-10972 | agucgagaaccaaugauggTT | 445 | ccaucauugguucucgacuTT | 446 |
| AD-10973 | ccgacuaccgcugguggcTT | 447 | gcccaccagcgguagucggTT | 448 |
| AD-10974 | auaucaccggcugcugacuTT | 449 | agucagcagccggugauauTT | 450 |
| AD-10975 | ugcauaucgcugggcucaaTT | 451 | uugagcccagcgauaugcaTT | 452 |
| AD-10976 | uuguuuacgacgugaucuaTT | 453 | uagaucacgucguaaacaaTT | 454 |
| AD-10977 | guguuagacgguaccgacaTT | 455 | ugucgguaccgucuaacacTT | 456 |
| AD-10978 | cuugaacuacaucgaucauTT | 457 | augaucgauguaguucaagTT | 458 |
| AD-10979 | ggccggaaacuugcuugcaTT | 459 | ugcaagcaaguuuccggccTT | 460 |
| AD-10980 | cugucucgacagauagcugTT | 461 | cagcuaucugucgagacagTT | 462 |
| AD-10981 | gcaucgcuauggaacuuuuTT | 463 | aaaaguuccauagcgaugcTT | 464 |
| AD-10982 | acugacguuacaucauacaTT | 465 | uguaugauguaacgucaguTT | 466 |
| AD-10983 | cugacguuacaucauacacTT | 467 | guguaugauguaacgucagTT | 468 |
| AD-10984 | ugaaucgagaucggaugucTT | 469 | gacauccgaucucgauucaTT | 470 |
| AD-10985 | uagacgguaccgacaaccaTT | 471 | ugguugucgguaccgucuaTT | 472 |
| AD-10986 | uugccgcaucaaaggugacTT | 473 | gucaccuuugaugcggcaaTT | 474 |
| AD-10987 | aacuacaucgaucauggagTT | 475 | cuccaugaucgauguaguuTT | 476 |
| AD-10988 | uuuggcggauugcauuccuTT | 477 | aggaaugcaauccgccaaaTT | 478 |
| AD-10989 | gcuuuagucgagaaccaauTT | 479 | auugguucucgacuaaagcTT | 480 |

(continued)

| Duplex name | Sense strand sequence (5'-3') | SEQ ID NO: | Antisense strand sequence (5'-3') | SEQ ID NO: |
|---|---|---|---|---|
| AD-10990 | uuuagucgagaaccaaugaTT | 481 | ucauugguucucgacuaaaTT | 482 |
| AD-10991 | uagucgagaaccaaugaugTT | 483 | caucauugguucucgacuaTT | 484 |
| AD-10992 | aagugucuacccaguugaaTT | 485 | uucaacuggguagacacuuTT | 486 |
| AD-10993 | ucaguuacggguuaauuacTT | 487 | guaauuaacccguaacugaTT | 488 |
| AD-10994 | uuacggguuaauuacugucTT | 489 | gacaguaauuaacccguaaTT | 490 |
| AD-10995 | uacggguuaauuacugucuTT | 491 | agacaguaauuaacccguaTT | 492 |
| AD-10996 | gucucgacagauagcugacTT | 493 | gucagcuaucugucgagacTT | 494 |
| AD-10997 | ucucgacagauagcugacaTT | 495 | ugucagcuaucugucgagaTT | 496 |
| AD-10998 | ugcgggcucguuccaugauTT | 497 | aucauggaacgagcccgcaTT | 498 |
| AD-10999 | uucagucucguugugaaaaTT | 499 | uuuucacaacgagacugaaTT | 500 |
| AD-11000 | ugucgccggguagaaaugcTT | 501 | gcauuucuacccggcgacaTT | 502 |
| AD-11001 | ucggaguucaaccuaagccTT | 503 | ggcuuagguugaacuccgaTT | 504 |
| AD-11002 | caugcuuaagccuagggauTT | 505 | aucccuaggcuuaagcaugTT | 506 |
| AD-11003 | ccgcugagucuggaucuccTT | 507 | ggagauccagacucagcggTT | 508 |
| AD-11004 | ugucaacagcuacacacguTT | 509 | acguguguagcuguugacaTT | 510 |
| AD-11005 | guggccggcaacccagcugTT | 511 | cagcuggguugccggccacTT | 512 |
| AD-11006 | gaaagggaucgcccacugcTT | 513 | gcagugggcgaucccuuucTT | 514 |
| AD-11007 | aaagggaucgcccacugcgTT | 515 | cgcagugggcgaucccuuuTT | 516 |
| AD-11008 | cggguagaacuucagacccTT | 517 | gggucugaaguucuacccgTT | 518 |
| AD-11009 | gcucgaccgcagggccuucTT | 519 | gaaggcccugcggucgagcTT | 520 |
| AD-11010 | agcccauaucaccggcugcTT | 521 | gcagccggugauaugggcuTT | 522 |
| AD-11011 | uucuaugcccguguaaaguTT | 523 | acuuuacacgggcauagaaTT | 524 |
| AD-11012 | cccuuuuagucaggagaguTT | 525 | acucuccugacuaaaagggTT | 526 |
| AD-11013 | gguuggcgacugucaugugTT | 527 | cacaugacagucgccaaccTT | 528 |
| AD-11014 | acugucucgacagauagcuTT | 529 | agcuaucugucgagacaguTT | 530 |
| AD-11015 | uugucugacaauaugugaaTT | 531 | uucacauauugucagacaaTT | 532 |
| AD-11016 | cugggcaucgcuauggaacTT | 533 | guuccauagcgaugcccagTT | 534 |
| AD-11017 | cucggaguuugcgugcugcTT | 535 | gcagcacgcaaacuccgagTT | 536 |
| AD-11018 | uguuaaaggccuucauagcTT | 537 | gcuaugaaggccuuuaacaTT | 538 |
| AD-11019 | uuaaaggccuucauagcgaTT | 539 | ucgcuaugaaggccuuuaaTT | 540 |
| AD-11020 | gccuucauagcgaaccugaTT | 541 | ucagguucgcuaugaaggcTT | 542 |
| AD-11021 | aaggcagcuucggagugacTT | 543 | gucacuccgaagcugccuuTT | 544 |
| AD-11022 | agguuuaugaacugacguuTT | 545 | aacgucaguucauaaaccuTT | 546 |
| AD-11023 | aacugacguuacaucauacTT | 547 | guaugauguaacgucaguuTT | 548 |
| AD-11024 | cacaauguugugaccggagTT | 549 | cuccggucacaacauugugTT | 550 |
| AD-11025 | caauguugugaccggagccTT | 551 | ggcuccggucacaacauugTT | 552 |
| AD-11026 | agcagcucuucagaacgccTT | 553 | ggcguucugaagagcugcuTT | 554 |
| AD-11027 | guggccgaagccguaguggTT | 555 | ccacuacggcuucggccacTT | 556 |

(continued)

| Duplex name | Sense strand sequence (5'-3') | SEQ ID NO: | Antisense strand sequence (5'-3') | SEQ ID NO: |
|---|---|---|---|---|
| AD-11028 | cguagugggaguauuguggTT | 557 | ccacaauacucccacuacgTT | 558 |
| AD-11029 | ggaguauuguggaacuuauTT | 559 | auaaguuccacaauacuccTT | 560 |
| AD-11030 | aguauuguggaacuuauagTT | 561 | cuauaaguuccacaauacuTT | 562 |
| AD-11031 | gagaucggaugucagcagcTT | 563 | gcugcugacauccgaucucTT | 564 |
| AD-11032 | cagcgccgucccaucugacTT | 565 | gucagaugggacggcgcugTT | 566 |
| AD-11033 | ccaccgaagggccugauucTT | 567 | gaaucaggcccuucgguggTT | 568 |
| AD-11034 | auuguguuagacgguaccgTT | 569 | cgguaccgucuaacacaauTT | 570 |
| AD-11035 | ccgacaaccaguauuugggTT | 571 | cccaaauacugguugucggTT | 572 |
| AD-11036 | aaacaagccuugccgcaucTT | 573 | gaugcggcaaggcuuguuuTT | 574 |
| AD-11037 | gccuugccgcaucaaagguTT | 575 | accuuugaugcggcaaggcTT | 576 |
| AD-11038 | aucuugaacuacaucgaucTT | 577 | gaucgauguaguucaagauTT | 578 |
| AD-11039 | aucgaucauggagacccacTT | 579 | gugggucuccaugaucgauTT | 580 |
| AD-11040 | uggagacccacagguucgaTT | 581 | ucgaaccugugggucuccaTT | 582 |
| AD-11041 | ggagacccacagguucgagTT | 583 | cucgaaccugugggucuccTT | 584 |
| AD-11042 | ccgcuuccacgugggagauTT | 585 | aucucccacguggaagcggTT | 586 |
| AD-11043 | ucuuuggcggauugcauucTT | 587 | gaaugcaauccgccaaagaTT | 588 |
| AD-11044 | uuggcggauugcauuccuuTT | 589 | aaggaaugcaauccgccaaTT | 590 |
| AD-11045 | agcagcuacagugaguuagTT | 591 | cuaacucacuguagcugcuTT | 592 |
| AD-11046 | cgagugcucaauaauguugTT | 593 | caacauuauugagcacucgTT | 594 |
| AD-11047 | aauuaggcuugucccaaagTT | 595 | cuuugggacaagccuaauuTT | 596 |
| AD-11048 | uggaguuuagguuggcacuTT | 597 | agugccaaccuaaacuccaTT | 598 |
| AD-11049 | cuugguucccauuggaucuTT | 599 | agauccaaugggaaccaagTT | 600 |
| AD-11050 | uuuuggccggaaacuugcuTT | 601 | agcaaguuuccggccaaaaTT | 602 |
| AD-11051 | ugccuucucuaacaaacccTT | 603 | ggguuuguuagagaaggcaTT | 604 |
| AD-11052 | uaagucccauccgacgaaaTT | 605 | uuucgucggaugggacuuaTT | 606 |
| AD-11053 | ugauaccucagguccuguuTT | 607 | aacaggaccugagguaucaTT | 608 |
| AD-11054 | gauaccucagguccuguuaTT | 609 | uaacaggaccugagguaucTT | 610 |
| AD-11055 | uguuacaacaaguaaauccTT | 611 | ggauuuacuuguuguaacaTT | 612 |
| AD-11056 | cuaggauaccugaaauccuTT | 613 | aggauuucagguauccuagTT | 614 |
| AD-11057 | cuuuagucgagaaccaaugTT | 615 | cauugguucucgacuaaagTT | 616 |
| AD-11058 | acuguuuguguucaacaauTT | 617 | auuguugaacacaaacaguTT | 618 |
| AD-11059 | caauuguugaagacucucuTT | 619 | agagagucuucaacaauugTT | 620 |
| AD-11060 | caagucacaaggccgagcaTT | 621 | ugcucggccuugugacuugTT | 622 |
| AD-11061 | aagucacaaggccgagcacTT | 623 | gugcucggccuugugacuuTT | 624 |
| AD-11062 | ggcuuguaccacuacugcuTT | 625 | agcaguagugguacaagccTT | 626 |
| AD-11063 | acgacaccucgggaugguuTT | 627 | aaccaucccgaggugucguTT | 628 |
| AD-11064 | caccucgggaugguuugauTT | 629 | aucaaaccaucccgaggugTT | 630 |
| AD-11065 | cucgggaugguuugaugucTT | 631 | gacaucaaaccaucccgagTT | 632 |

(continued)

| Duplex name | Sense strand sequence (5'-3') | SEQ ID NO: | Antisense strand sequence (5'-3') | SEQ ID NO: |
|---|---|---|---|---|
| AD-11066 | agugucacaaagaaccgugTT | 633 | cacgguucuuugugacacuTT | 634 |
| AD-11067 | gugucacaaagaaccgugcTT | 635 | gcacgguucuuugugacacTT | 636 |
| AD-11068 | aaccgugcagauaagaaugTT | 637 | cauucuuaucugcacgguuTT | 638 |
| AD-11069 | accgugcagauaagaaugcTT | 639 | gcauucuuaucugcacgguTT | 640 |
| AD-11070 | ccgugcagauaagaaugcuTT | 641 | agcauucuuaucugcacggTT | 642 |
| AD-11071 | gcagauaagaaugcuauucTT | 643 | gaauagcauucuuaucugcTT | 644 |
| AD-11072 | acauucguuuguuugaaccTT | 645 | gguucaaacaaacgaauguTT | 646 |
| AD-11073 | ugaaccucuuguuauaaaaTT | 647 | uuuuauaacaagagguucaTT | 648 |
| AD-11074 | uuuagauuugcuggcgcagTT | 649 | cugcgccagcaaaucuaaaTT | 650 |
| AD-11075 | ugguucaguuacggguuaaTT | 651 | uuaacccguaacugaaccaTT | 652 |
| AD-11076 | gggccaguucagggaaucaTT | 653 | ugauucccugaacuggcccTT | 654 |
| AD-11077 | uggaagcgacugucucgacTT | 655 | gucgagacagucgcuuccaTT | 656 |
| AD-11078 | ggaagcgacugucucgacaTT | 657 | ugucgagacagucgcuuccTT | 658 |
| AD-11079 | gaagcgacugucucgacagTT | 659 | cugucgagacagucgcuucTT | 660 |
| AD-11080 | gcgacugucucgacagauaTT | 661 | uaucugucgagacagucgcTT | 662 |
| AD-11081 | ugucucgacagauagcugaTT | 663 | ucagcuaucugucgagacaTT | 664 |
| AD-11082 | cucgacagauagcugacauTT | 665 | augucagcuaucugucgagTT | 666 |
| AD-11083 | agguggaaaugagugagcaTT | 667 | ugcucacucauuuccaccuTT | 668 |
| AD-11084 | agugagcagcaacauacuuTT | 669 | aaguauguugcugcucacuTT | 670 |
| AD-11085 | guuccgcagugauggcuguTT | 671 | acagccaucacugcggaacTT | 672 |
| AD-11086 | caaccacaccgacuaccgcTT | 673 | gcgguagucggugugguugTT | 674 |
| AD-11087 | aaccacaccgacuaccgcuTT | 675 | agcgguagucggugugguuTT | 676 |
| AD-11088 | accacaccgacuaccgcugTT | 677 | cagcgguagucggugugguTT | 678 |
| AD-11089 | cccgaaaagacacagucugTT | 679 | cagacugugucuuuucgggTT | 680 |
| AD-11090 | uccagcacaaaguuacuuaTT | 681 | uaaguaacuuugugcuggaTT | 682 |
| AD-11091 | uuggaaugugcaauagagaTT | 683 | ucucuauugcacauuccaaTT | 684 |
| AD-11092 | agaucugaucagccuuuccTT | 685 | ggaaaggcugaucagaucuTT | 686 |
| AD-11093 | caggcaauucagucucguuTT | 687 | aacgagacugaauugccugTT | 688 |
| AD-11094 | ggcaauucagucucguuguTT | 689 | acaacgagacugaauugccTT | 690 |
| AD-11095 | gcaauucagucucguugugTT | 691 | cacaacgagacugaauugcTT | 692 |
| AD-11096 | aauucagucucguugugaaTT | 693 | uucacaacgagacugaauuTT | 694 |
| AD-11097 | ucagucucguugugaaaacTT | 695 | guuuucacaacgagacugaTT | 696 |
| AD-11098 | aaaccuuucaacuccaaccTT | 697 | gguuggaguugaaagguuuTT | 698 |
| AD-11099 | cuuuccgugugcuggcucgTT | 699 | cgagccagcacacggaaagTT | 700 |
| AD-11100 | ccgugugcuggcucgcaugTT | 701 | caugcgagccagcacacggTT | 702 |
| AD-11101 | ucgacauccuugcuugucgTT | 703 | cgacaagcaaggaugucgaTT | 704 |
| AD-11102 | ugcuugucgccgguagaaTT | 705 | uucuacccggcgacaagcaTT | 706 |
| AD-11103 | gcuugucgccgguagaaaTT | 707 | uuucuacccggcgacaagcTT | 708 |

(continued)

| Duplex name | Sense strand sequence (5'-3') | SEQ ID NO: | Antisense strand sequence (5'-3') | SEQ ID NO: |
|---|---|---|---|---|
| AD-11104 | cuugucgccggguagaaauTT | 709 | auuucuacccggcgacaagTT | 710 |
| AD-11105 | ggcccaguugccaauggaaTT | 711 | uuccauuggcaacugggccTT | 712 |
| AD-11106 | cagguuucgucucuccaccTT | 713 | gguggagagacgaaaccugTT | 714 |
| AD-11107 | ggcacgugucacuggaaacTT | 715 | guuuccagugacacgugccTT | 716 |
| AD-11108 | cuggaaacagugaguccggTT | 717 | ccggacucacuguuuccagTT | 718 |
| AD-11109 | caaaucccaguguuggaccTT | 719 | gguccaacacugggauuugTT | 720 |
| AD-11110 | acucggaguucaaccuaagTT | 721 | cuuagguugaacuccgaguTT | 722 |
| AD-11111 | cucggaguucaaccuaagcTT | 723 | gcuuagguugaacuccgagTT | 724 |
| AD-11112 | agccuagggaugagugaaaTT | 725 | uuucacucaucccuaggcuTT | 726 |
| AD-11113 | gucaacagcuacacacgugTT | 727 | cacguguguagcuguugacTT | 728 |
| AD-11114 | gauggucacccaaaccgggTT | 729 | cccgguuugggugaccaucTT | 730 |
| AD-11115 | ugacagaacugcgaaggguTT | 731 | acccuucgcaguucugucaTT | 732 |
| AD-11116 | gaagacgagauccucgcucTT | 733 | gagcgaggaucucgucuucTT | 734 |
| AD-11117 | acgagauccucgcucaguaTT | 735 | uacugagcgaggaucucguTT | 736 |
| AD-11118 | aaccugaaagggaucgcccTT | 737 | gggcgaucccuuucagguuTT | 738 |
| AD-11119 | gaucgcccacugcgugaacTT | 739 | guucacgcagugggcgaucTT | 740 |
| AD-11120 | cacugcgugaacauucacaTT | 741 | ugugaauguucacgcagugTT | 742 |
| AD-11121 | agaacuauccucuggacguTT | 743 | acguccagaggauaguucuTT | 744 |
| AD-11122 | gucaguccggguagaacuuTT | 745 | aaguucuacccggacugacTT | 746 |
| AD-11123 | ugaacaaagucaucggagaTT | 747 | ucuccgaugacuuuguucaTT | 748 |
| AD-11124 | aagucaucggagaguuucuTT | 749 | agaaacucuccgaugacuuTT | 750 |
| AD-11125 | gucaucggagaguuucuguTT | 751 | acagaaacucuccgaugacTT | 752 |
| AD-11126 | ggccaccgugguguauaagTT | 753 | cuuauacaccacgguggccTT | 754 |
| AD-11127 | accgugguguauaagguguTT | 755 | acaccuuauacaccacgguTT | 756 |
| AD-11128 | cugacuuguuuacgaaaugTT | 757 | cauuucguaaacaagucagTT | 758 |
| AD-11129 | uguuuacgaaauguccacaTT | 759 | uguggacauuucguaaacaTT | 760 |
| AD-11130 | ccaccgagccagcuuggucTT | 761 | gaccaagcuggcucgguggTT | 762 |
| AD-11131 | caccgagccagcuugguccTT | 763 | ggaccaagcuggcucggugTT | 764 |
| AD-11132 | caggcaacgugcgugucucTT | 765 | gagacacgcacguugccugTT | 766 |
| AD-11133 | aacgugcgugucucugccaTT | 767 | uggcagagacacgcacguuTT | 768 |
| AD-11134 | uuaauuuuaacguaacucuTT | 769 | agaguuacguuaaaauuaaTT | 770 |
| AD-11135 | uuaacguaacucuuucuauTT | 771 | auagaaagaguuacguuaaTT | 772 |
| AD-11136 | uaacguaacucuuucuaugTT | 773 | cauagaaagaguuacguuaTT | 774 |
| AD-11137 | aacguaacucuuucuaugcTT | 775 | gcauagaaagaguuacguuTT | 776 |
| AD-11138 | guaacucuuucuaugcccgTT | 777 | cgggcauagaaagaguuacTT | 778 |
| AD-11139 | uaugcccguguaaaguaugTT | 779 | cauacuuuacacgggcauaTT | 780 |
| AD-11140 | ugcccguguaaaguaugugTT | 781 | cacauacuuuacacgggcaTT | 782 |
| AD-11141 | ugagcacccgcugacauuuTT | 783 | aaaugucagcgggugcucaTT | 784 |

(continued)

| Duplex name | Sense strand sequence (5'-3') | SEQ ID NO: | Antisense strand sequence (5'-3') | SEQ ID NO: |
|---|---|---|---|---|
| AD-11142 | cacccgcugacauuuccguTT | 785 | acggaaaugucagcgggugTT | 786 |
| AD-11143 | uuuuagucaggagagugcaTT | 787 | ugcacucuccugacuaaaaTT | 788 |
| AD-11144 | agccaagucauuaaaauggTT | 789 | ccauuuuaaugacuuggcuTT | 790 |
| AD-11145 | guuggcgacugucauguggTT | 791 | ccacaugacagucgccaacTT | 792 |
| AD-11146 | gcccuuaagggaagcuacuTT | 793 | aguagcuucccuuaagggcTT | 794 |
| AD-11147 | gcauaucgcugggcucaacTT | 795 | guugagcccagcgauaugcTT | 796 |
| AD-11148 | aauaugagcucauuaguaaTT | 797 | uuacuaaugagcucauauuTT | 798 |
| AD-11149 | gugcccgugucgguucuucTT | 799 | gaagaaccgacacgggcacTT | 800 |
| AD-11150 | aaugaaaccaggguagaauTT | 801 | auucuacccugguuucauuTT | 802 |
| AD-11151 | cacccagaauguagcaucuTT | 803 | agaugcuacauucugggugTT | 804 |
| AD-11152 | gagcucgggacggauaguaTT | 805 | uacuauccgucccgagcucTT | 806 |
| AD-11153 | ugacaacugaaggcaaccuTT | 807 | agguugccuucaguugucaTT | 808 |
| AD-11154 | caacguggaccugccuacgTT | 809 | cguaggcagguccacguugTT | 810 |
| AD-11155 | gacugacgagagauguauaTT | 811 | uauacaucucucgucagucTT | 812 |
| AD-11156 | acgagagauguauauuuaaTT | 813 | uuaaauauacaucucucguTT | 814 |

## Example 2: Detection of tissue-specific mRNA in serum

RNAi activity in the liver is presently monitored through molecular analysis of liver tissue samples, and, for secreted proteins, through quantitation of circulating levels of target protein in serum or plasma samples. In clinical studies, obtaining liver biopsies from human subjects carries associated risks such as hemorrhage. As a result, in normal human volunteers, the procedure is not justified, and liver biopsies cannot be obtained. Amongst patients, liver biopsies are acceptable only in a small subset of disease states, such as liver cancer, and even amongst those patients, consent rates can be low.

In the absence of liver tissue samples, quantitation of circulating target protein is the primary method of monitoring RNAi activity in the liver. This approach requires that the target protein be secreted into the blood, which excludes a number of molecular targets in the liver, as well as in other tissues, where the corresponding protein is not secreted. For these non-secreted proteins, there is currently no method of monitoring RNAi, in the absence of liver tissue biopsies. The invention described here - detection of target RNA or a cleavage product thereof in serum samples - provides the ability to monitor RNAi for non-secreted protein targets through the molecular evaluation of serum.

For secreted proteins, in order to monitor RNAi in the liver, the target protein must reach blood levels that can be readily detected and quantified. The protein assay for the target of interest must be sufficiently sensitive, selective, accurate and reproducible, in order to rely on circulating levels of target protein as a key readout for RNAi activity in the tissue of interest. In addition, circulating levels of target protein may or may not exhibit the same dose-response to iRNA administration as levels of liver mRNA; in this case, if target RNA levels are more sensitive to iRNA administration than corresponding protein levels, then it would be useful to have the ability to monitor target RNA levels, which may reveal RNAi activity at lower iRNA dose levels.

If multiple tissues contribute to circulating levels of a given target protein, and RNAi occurs only in one or a subset of these tissues, as a consequence of the delivery system, then RNAi activity may not result in measurable lowering of the total target protein in the circulation. However, detection in the serum of the cleavage product from the target RNA provides a qualitative indicator of RNAi activity in the tissue. Furthermore, through isolation of exosomes derived from a particular tissue such as the liver, or through isolation of a biological sample (such as a blood or serum sample) enriched for such exosomes, target RNA from only that particular tissue may be quantified, independent of other tissue contributions.

The methods and assays described herein detect a biomarker for evaluating RNA interference *in vivo* without the need of sampling the tissue which serves as the site of action, enabling clinical monitoring of gene silencing for non-secreted proteins, and facilitating clinical monitoring of gene silencing for secreted proteins.

*RNA isolation from serum*

Serum samples obtained from mouse, rat, cyno, or human (fresh or stored) were subjected to filtration using a 0.4 μm filter or spin at 2000-5000g for 10minutes. In some cases, lithium chloride was added to the filtered samples to a final concentration of 1 M LiCl. The samples were incubated at 4 °C for 2 hours and then centrifuged at 110,000-140,000 g for 1h and 35min.

RNA was isolated from the resulting pellet using a TRIzol™ Chloroform method. RNA was precipitated using isopropanol in the presence of glycogen, which was a coprecipitant. Linear polyacrylamide can be used in place of glycogen as coprecipitants.

This protocol results in the isolation of ubiquitous, liver and tissue-specific RNA as shown in FIG. 24.

*RNA quantification*

RNA was quantified using Nanodrop technology. ~100-1000ng of RNA was used for cDNA synthesis using Applied Biosystems High Capacity cDNA synthesis kit, using the manufacturer's instructions. Quantitative PCR was performed with Roche Master Mix and TaqMan® probes in a Roche Lightcycler 480.

*Serum samples*

Serum was obtained from mouse, rat, cyno and human subjects. In some experiments, individual serum samples were analyzed separately, while in other experiments individual serum samples were pooled prior to analysis.

4 ml of serum was obtained from each cyno subject and stored frozen prior to analysis. 2ml of pooled cyno serum per group was used for analysis. 4-5 ml of human serum was used.

*RNA detection in serum samples*

*Mouse*

14 mL of frozen serum from 129Sv male mice was pooled for analysis of hepatocyte-specific (*e.g.*, TTR, FVII) and ubiquitous (GAPDH, Rplp2) mRNAs. FIG. 1 shows that Rplp2, GAPDH, and TTR mRNA were each detectable in the pooled serum samples.

*Rat*

14 mL of fresh serum from rats were pooled for analysis of tissue specific mRNAs. FIGs. 2A and 2B show that hepatocyte specific (TTR, FVII), smooth muscle-specific (Acta2) and ubiquitous (GAPDH, beta actin) mRNAs can be detected in rat serum. mRNAs corresponding to the liver expressed genes AAT and albumin were detected by reverse transcription quantitative PCR, along with the ubiquitously expressed gene 18s. To assess whether the TTR mRNA detected in serum represents a full-length TTR mRNA, two different TaqMan® assays were employed in which one assay utilized primers that amplified the 5' end of the mRNA and a second assay utilized primers that amplified the 3' terminus of the mRNA. FIG. 3 shows that the levels of the 5' and 3' ends of the TTR were similar, thereby indicating that at least some of the detected TTR mRNA was the full length mRNA.

*Cyno and Human*

4 mL of frozen serum from individual cyno subjects were analyzed for hepatocyte-specific RNA in the serum. Two technical replicates were performed for each sample. FIGs. 4A and 4B show that hepatocyte-specific (TTR) and ubiquitous (18s) mRNAs can be detected in cyno serum. FIG. 5A shows data depicting the detection of GAPDH, 18s, TTR and AIAT mRNA in cynomolgus monkey serum. FIG. 5B shows data depicting the detection of GAPDH, 18s, TTR, AAT and albumin mRNA in human serum.

Fresh serum samples (4-5mL each) were obtained from human subjects. Two technical replicates were performed per sample. The levels of mRNA varied 8-10 fold between samples when TTR mRNA was normalized to 18s. The levels of mRNA between samples varied only 3-fold with normalization to liver-specific mRNA (*i.e.,* alpha antitrypsin (AAT)). 4-5mL of serum was obtained from each subject and two technical replicates were performed per sample.

FIG. 6A shows TTR mRNA normalized to AAT in three individual serum samples. FIG. 6B shows TTR mRNA normalized to 18s ribosomal RNA in three individual serum samples. FIG. 5B shows the expression of additional mRNA markers in serum, including GAPDH, 18s RNA, P0, TTR, AAT and albumin.

*RNA detection in cerebrospinal fluid (CSF)*

A total of 8 mL of CSF was obtained by pooling CSF samples from 30 naive rats. The total concentration of RNA (as determined using nanodrop technology at an OD of 260) was approximately 2 μg. The CSF was centrifuged at 10,000g for 10min to remove blood and cells from the sample. The supernatant was then centrifuged at 110,000g for 1h and 35min to obtain a pellet comprising nucleic acids. The pellet was resuspended in TRIzol™ reagent for isolation of RNA. RNA was precipitated using glycogen.

FIG. 7 shows that SNCA, TTR, HTT and DRD2 mRNA was detectable in CSF samples from rat. CSF samples were obtained from 30 animals and pooled for analysis.

## Example 3: Serum RNA is associated with exosomes

Serum RNA can be associated with exosomes, which can protect the RNA from RNase digestion. Therefore, in some embodiments one of skill in the art may isolate exosomes prior to detecting mRNA expression levels.

*Exosome isolation and detection of mRNA*

Rat serum was collected and subjected to filtration through a 40μM filter, followed by a centrifugation step at 110,000 x g. The resulting pellet was resuspended and loaded onto a 0.25M-2M sucrose gradient and subjected to centrifugation. 1mL fractions were collected from the density gradient.

The intracellular components of the pellet were resolved into four main fractions by density gradient centrifugation. The least dense fraction is at the top of the tube and the densest fraction at the bottom; the order of the intracellular component fractions from top to bottom is as follows: (i) a vesicle fraction, (ii) a lysosomal fraction, (iii) a peroxisomal fraction, and (iv) a nuclei-containing fraction.

RNA was detected in each sucrose gradient fraction. However, the highest levels of RNA were detected in fractions 2-7 (density -1.075-1.13). This pattern of detection was consistent with reported density values for exosomes (density -1.05-1.19), indicating that much of the RNA present in serum is present in exosomes.

To determine whether the RNA in serum is encapsulated within the exosome, exosomes were isolated by centrifugation to produce a pellet, which was subjected to RNase digestion in the presence or absence of a membrane-disruptive detergent Triton X-100 (Tx-100).

FIGs. 8A and 8B show that a majority of the RNA in isolated exosomes is protected from RNase degradation, indicating that much of the RNA is encapsulated within exosome vesicles. In samples pretreated with 0.2% Tx-100 prior to RNase treatment, RNA degradation by RNase was increased indicating that the membrane was disrupted and the RNase enzyme had greater access to the RNA in the sample. It was determined that 40-50% of the RNA is protected from nuclease digestion by RNAse enzymes. Taken together, these data indicate that serum RNA is present within exosomes and is protected from nuclease digestion by the exosomal membrane.

*Exosome markers*

Western blotting for exosome markers Alix and CD9 was performed on the exosome pellet or on pulverized rat liver powder that was resuspended in RIPA buffer with SDS. Protein content was determined by a BCA assay and each lane was loaded with an equal amount of protein.

The exosomal markers Alix and CD9 were present in isolated human, rat and mouse exosomal fractions, but were not detected in the rat liver sample. The hepatocyte marker SRBI was also detected in the exosome fractions of human, rat and mouse serum.

*Exosome characteristics*

Freeze-fracture electron micropscopy of the exosome pellet indicated the presence of vesicles with an average size of ~100nm.

Vesicles with similar characteristics as exosomes were detected by dynamic light scattering. The dynamic light scattering measurement for the particles for mouse, rat and human samples are as follows:

| | | |
|---|---|---|
| Mouse | Z-Ave: 79.8nm | PDI 0.577 |
| Rat | Z-Ave: 134nm | PDI 0.944 |
| Human | Z-Ave: 97nm | PDI 0.907 |

**Example 4: Detection of gene silencing in serum samples**

AF-011-18534, comprising a dsRNA targeting rodent TTR, formulated in a lipid nanoparticle for delivery to liver hepatocytes was used to silence the liver gene, TTR, in naive rats. SNALP-18324, comprising a dsRNA targeting primate TTR, formulated in a lipid nanoparticle for delivery to liver hepatocytes was used to silence the liver gene, TTR, in naive cynos. AF-011-1955 and SNALP-1955, targeting the non-mammalian gene luciferase, are used herein as dsRNA controls and are not known to have any gene silencing effects when administered *in vivo.*

*Gene silencing in naïve rats*

*Experiment 1:* A single IV bolus of 0.3 mpk (mg/kg) AF-011-18534 (n=6) or AF-011-1955 (n=6) was administered to rats to determine if liver gene silencing could be detected by measuring mRNA levels in serum samples. Liver and serum TTR mRNA levels were measured 24h after bolus administration. 14mL of fresh serum samples were pooled for each group. 2-4 technical replicates were performed per group.

The data in FIG. 9A shows the reduction in liver mRNA of TTR following administratin of AF-011-18534. FIG. 9B shows a similar reduction in the TTR mRNA level in pooled serum from animals treated with AF-011-18534 compared to pooled serum from animals treated with the control dsRNA AF-011-1955. Thus, TTR gene silencing in the liver mediated by AF-011-18534 is detectable as TTR mRNA lowering in serum samples.

FIGs. 10A-10D show TTR mRNA levels in rat serum as normalized to AAT, albumin, GAPDH, and 18s mRNA, respectively. The data in each of FIGs. 10A-10D shows that AF-011-18534 mediated TTR gene silencing in the liver can be detected as similar TTR mRNA lowering in serum samples, when normalized to either hepatocyte-specific or ubiquitous genes.

FIGs. 11A-11D show that AF-011-18534 administered to the rat does not alter mRNA levels of non-targeted hepatocyte-specific genes (*i.e.,* AAT or albumin) in serum samples.

In animals treated with a single IV bolus of an anti-TTR RNAi agent, there was no apparent knockdown of TTR mRNA in pancreas.

*Experiment 2:* A single IV bolus of 0.001 mpk, 0.003 mpk, 0.01 mpk, 0.03 mpk or 0.1 mpk of AF-011-18534 was administered to rats (n = 4-5) to determine if a dose-response relationship of liver gene silencing could be detected by measuring mRNA levels in serum samples. In the control groups, a single IV bolus of 0.1 mpk AF-011-1955 (n = 3) was used as a dsRNA control and PBS (n = 3) was administered as a vehicle control. Liver and serum TTR mRNA levels were measured 24h following administration of the IV bolus.

A dose-response relationship of AF-011-18534-mediated TTR gene silencing was evident as expected in liver samples as shown in FIGs. 12A and 12B. A similar dose-response relationship of AF-011-18534-mediated gene silencing was observed with TTR mRNA levels measured in serum samples, as shown in FIGs. 13A and 13B.

*Gene silencing in naïve cynos*

*Experiment 3:* A single 15 minute IV infusion of 0.3 mpk, 1 mpk or 3 mpk SNALP-18324 (n = 3), or 3 mpk SNALP-1955 (n = 3) was administered to cynos to determine if liver gene silencing could be detected by measuring mRNA levels in serum samples. Liver and serum TTR mRNA levels were measured 48h after IV administration.

FIG. 14A shows the dose-response relationship for SNALP-18324 mediated silencing of TTR mRNA in liver tissue. FIG. 14B shows the dose-response relationship observed for SNALP-18324 mediated silencing of TTR mRNA in serum samples.

FIG. 15 shows the concentration of TTR protein (as measured using ELISA) in serum samples at 48h after administration of SNALP-18324.

At 3 mg/kg SNALP-18324, the extent of TTR mRNA suppression in the serum was indistinguishable from the extent of TTR mRNA suppression in the liver, whereas the extent of TTR protein suppression in the serum was less pronounced, by approximately 20%.

*Serum detection of Silencing of TMPRSS6*

Detection of gene silencing using serum mRNA expression levels can be extended to genes other than TTR. For example, FIGs. 16A and 16B indicate that silencing of TMPRSS6 mRNA can be detected in both liver and serum samples.

In this experiment, a single IV bolus at 0.3mg/kg was administered to male rats (n=4 per group). Liver and serum TMPRSS6 mRNA levels were measured 24h post dose.

*Duration of silencing following bolus administration of RNAi*

Rats (6-8 animals per group) were administered a single IV bolus of 0.1mg/kg of an anti-TTR siRNA preparation or a control anti-luciferase siRNA preparation. Liver and serum were collected at different time points of animal sacrifice (e.g., Day 1, Day 2, Day 5, Day 8, Day 10, and Day 14) and analyzed for TTR mRNA expression. FIG. 17A shows that the recovery of TTR mRNA levels in serum parallels the recovery of TTR mRNA in liver samples. These data indicate that measuring TTR levels in serum is a viable method for detecting clinical effect of an administered RNAi agent.

In a second study, reductions in liver and serum TTR mRNA were measured at shorter time points following siRNA administration. Within 3 hours of treatment, both liver and serum TTR mRNA levels were reduced by 40-50%, with peak target reductions of approximately 90% achieved in liver and serum by 12 hours (Figure 17B). Thus, there was remarkable concordance of the relative TTR mRNA levels in the liver and in serum over time following treatment with LNP-siRNA, with respect to both onset and duration of target gene silencing. These results indicate that monitoring circulating mRNA levels of a liver gene by circulating exosome mRNA detection provides an accurate representation of tissue-specific target gene silencing.

## Example 5: Improving RNA yield

In some experiments, lithium chloride (LiCl) was used at a final concentration of 1M to increase RNA yield during isolation. In these experiments, the serum samples were incubated at 4°C in the presence or absence of 1M LiCl for one hour post filtration. The RNA was subsequently isolated via sucrose density gradient ultracentrifugation as described above, and analyzed via TaqMan® quantitative PCR. As shown in FIG. 18, the relative mRNA expression levels of GAPDH, albumin, beta actin, and TTR increase in the presence of LiCl. The incubation with LiCl likely enhances precipitation of RNA during the ultracentrifugation step, thus enhancing overall RNA yield during isolation.

## Example 6: Q-PCR and 5' RACE

The quantitive PCR was performed using Applied Biosystems TaqMan® primer system. The primers/assays used can be obtained commercially from Applied Biosystems, and include human transthyretin (TTR) (Cat. No. Hs00174914_m1), human alpha antitrypsin (AAT) (Cat. No. Hs01097800_m1), and human albumin (Cat. No. Hs00910225_m1).

Custom TaqMan® primers were designed for *Macaca fascicularis* ("cyno") TTR and include the following:

cyno TTR forward primer: TGG CAT CTC CCC ATT CCA
cyno TTR reverse primer: CGG AAT CGT TGG CTG TGA A
cyno TTR TaqMan® probe: 6FAMAGC ATG CAG AGG TGGMGBNFQ

Custom primers for reverse-transcription or 5'RACE (in combination with other primers) of human transthyretin (TTR) were designed and include the following:

Reverse primers:

5' ttttacagaatgtgtctttttaatggctgc 3'
5' gctgcagatcatatttaatacgtgc 3'
5' gcttgcaagacaatggaaatttggatc 3'

Forward primer:
5' gactaagtcaataatcagaatcagcag 3'

Custom 5' RACE primers were designed using the rat TTR sequence and include the following:

5' tttttgaatgaaataaaggtgg 3'
5' gaatgaaataaaggtgg 3'
5' ggtggtttaataagaacg 3'
5' taataagaacgtttcacggc 3'
5' tttttttgaatgaaataaaggt 3'
5' ccactgctgtcgtcagtaacc 3'
5' gaactgagggacccagcccac 3'
5' ccacgaggaccaagatcttgc 3'

*5'-RACE*

Rats (n=6 per group) were injected with LNP-siTTR or LNP-siLuc (at 0.1mg/kg) and sacrificed for analysis 24h later. Serum from the animals in each group was pooled for analysis. RNA was extracted from the pooled serum and processed for 5'RACE (5' Rapid Amplification of cDNA Ends). Bands from nested PCR of the correct size were eluted, cloned and sequenced.

20% of the sequences demonstrated the correct cleavage product in the siTTR-treated group while there were no sequences with the correct cleavage product detected in the control LNP-siLuc group (Table 4). In addition, FIG. 19A shows the alignment of two sequences for a cleavage product with the full length rat TTR mRNA.

**Table 4: Number of correct cleavage products in rat LNP-siTTR and control LNP-siLuc treatment groups, as detected by 5' RACE.**

| Treatment group | # correct cleavage site | Total |
|---|---|---|
| **LNP-siTTR** | 15 | 51 |
| **LNP-siLuc** | 0 | 45 |

The results of the 5' RACE analysis described above in rats were also consistent in a non-human primate model, in which 84% of the sequences demonstrated the correct cleavage product in the siTTR-treated group while there were no sequences with the correct cleavage product detected in the control LNP-siLuc group (Table 5). In addition, FIG. 19B shows the alignment of two sequences for a cleavage product with the full length cynomolgus monkey TTR mRNA.

**Table 5: Number of correct cleavage products in cynomolgus monkey LNP-siTTR and control LNP-siLuc treatment groups, as detected by 5' RACE.**

| Treatment group | # correct cleavage site | Total |
|---|---|---|
| **LNP-siTTR** (Animal 1) | 16 | 22 |
| **LNP-siTTR** (Animal 2) | 20 | 23 |
| **LNP-siLuc** | 0 | 14 |

These data indicate that 5'-RACE can be used to detect a TTR cleavage product in the serum of RNAi treated rats. That is, liver RNAi can be confirmed by detection of a specific 5'-RACE product in serum.

**Example 7: Detection of mRNA in serum and liver following gene therapy**

It was determined that expression of an exogenous gene can be detected in the serum of a rat model injected with an adenovirus expressing GFP (Ad-GFP) or an adenovirus expressing human ApoE (Ad-hApoE) by measuring serum levels of mRNA. Similarly, expression of an exogenous or therapeutic gene can be detected in the liver of a rat model by measuring liver mRNA levels of the therapeutic gene.

Rats were injected intravenously with either Ad-GFP or Ad-hApoE (moi of $1x10^{11}$) on Day 1; serum and the entire liver were harvested for analysis on Day 5. Two separate RNA preps were prepared from each liver to increase tissue sampling, since adenovirus expression is not uniform throughout the liver.

FIG. 20 and FIG. 21 show that the mRNA of exogenous GFP and ApoE delivered using an adenovirus can be detected in both liver and serum of the treated animals. Expression levels varied from animal to animal, as expected. The data also indicated that there is a correlation between relative mRNA levels in liver and serum of the treated animals.

Taken together, these data indicate that the methods and assays described herein can be used to assess mRNA levels of exogenous genes delivered using gene therapy methods in serum or liver. Thus, such assays and methods can be used to determine and/or monitor the success of a gene therapy regimen in a patient in a relatively non-invasive manner, *e.g.,* by measuring mRNA levels in a serum sample.

**Example 8: Detection of miRNA inhibition in serum and liver samples.**

In addition to mRNAs and proteins, microRNAs (miRNA) have been detected in circulating exosomes. To test the applicability of circulating exosome mRNA detection to monitor the activity of miRNA-targeting oligonucleotides, rats were administered an LNP-formulated oligonucleotide directed against the liver-specific miRNA, *miR-122.* Control animals received a mis-matched oligonucleotide. Three days later, *miR-122* levels were measured in total RNA isolated from liver and serum. As shown in FIG. 22A, treatment with the *miR122*-specific oligonucleotide reduced liver *miR-122* levels by 88%, with a similar decrease of 86% in serum. To further confirm the reduction in *miR-122* levels was not caused by non-specific downregualtion of *miR-122,* the expression level of the *miR-122* target, aldolase A, was measured. As shown in FIG. 22B, treatment with the *miR122*-specific oligonucleotide caused an increase target aldolase A mRNA expression in liver. This upregulation, however, could not be detected in serum, probably because aldolase A is ubiquitously expressed. Together these results establish the use of circulating exosome mRNA detection to monitor miRNA inhibition in liver.

**Example 9: Detection of gene silencing in cerebrospinal fluid.**

Exosomes have been detected in cerebrospinal fluid (CSF). To determine whether circulating exosome mRNA detection could be applied to CSF to monitor gene silencing in the brain, siRNA targeting the Parkinson's Disease associated gene, *Snca*, was infused bilaterally into the striatum of rats. Male Sprague-Dawley rats were anesthetized and placed into a stereotaxic frame (Benchmark™ Digital Stereotaxic, myNeuroLab). A 30 gauge osmotic pump infusion cannula (Plastics One, Wallingford, CN) was implanted into the right and left hemisphere, targeting the striatum (stereotaxic coordinates AP 0.5, ML 3 and DV 5.1 relative to bregma; incisor bar 3.3 mm below the interaural line). Osmotic pumps (1 μl/hr flow rate, Alzet) containing Luc or SNCA siRNA formulated in PBS were primed in 0.9% saline overnight at 37°C according to the manufacturer's instructions, and then connected to the cannula and implanted subcutaneously. A concentration of 4 mg/ml siRNAs administered to rats at a flow rate of 1 ul/hr corresponds to a dose of 0.096 mg/day for each site of striatum. After 7 days of infusion, rats were anesthetized and mounted in a stereotaxic frame. Atlanto-occipital membrane was exposed and CSF was collected using a syringe with a 30 gauge need through atlanto-occipital membrane. After the CSF collection, the rats were euthanized and brains were removed. For mRNA quantitation, coronal slices, 1 mm thick, through the rat brain from anterior to posterior were obtained using a brain matrix (Braintree Scientific, Braintree, MA) and striatum was dissected from each slice, snap-frozen in liquid nitrogen, and then stored at -80°C until mRNA quantitation was performed. To prepare striatal RNA, tissue was homogenized in Qiazol (Qiagen, Inc.) and total RNA isolated using the Qiagen RNeasy kit (Qiagen, Inc.). Synthesis of cDNA was performed with the TaqMan® Reverse Transcription kit (Applied Biosystems), and rat *Snca* and *Gapdh* expression were quantitified using TaqMan® assays (Applied Biosystems). To isolate RNA from CSF, samples were pooled (5ml each) and LiCl added to a final concentration of 1M. After 1h incubations at 4o C, samples were centrifuged at 110,000 x g for 100 minutes, and RNA was prepared from the pellets by Trizol extraction and isopropanol precipitation. Synthesis of cDNA and TaqMan® analysis were performed per standard protocol.

At the end of the 7 day infusion period, brains and CSF were collected for analysis. A 33% reduction in striatal *Snca* mRNA was measured in animals receiving the *Snca* siRNA compared to naive animals (Figure 23). To analyze CSF exosomal RNA, pooled samples were subjected to high speed centrifugation, and total RNA was isolated from the pellets. A 61% reduction in *Snca* mRNA was measured in CSF from *Snca* siRNA treated animals vs controls (FIG. 23). The greater relative degree of *Snca* mRNA reduction in the CSF compared to striatum could be due to variability in secretion of exosomes by different regions of the brain. These results confirm the suitability of circulating exosome mRNA detection to monitor silencing of a brain-expressed gene by assaying CSF RNA.

Other embodiments are in the claims.

## Claims

**1.** A method for determining the activity of an iRNA agent targeting transthyretin (TTR) RNA in a subject who was administered said iRNA agent in a liver tissue comprising:

(a) detecting the level of the TTR RNA or a cleavage product thereof in a serum sample acquired from the subject, and,
(b) comparing the level obtained in (a) to a reference sample;

wherein a decreased level of the TTR RNA or an increased level of the cleavage product as compared to the reference sample is indicative that the iRNA agent is active, and an increased or static level of the TTR RNA or a decreased or static level of cleavage product as compared to the reference sample is indicative that the iRNA agent is inactive in the subject.

**2.** The method of claim 1, wherein based on the determination that the iRNA agent is inactive in the subject the dose of iRNA agent to be administered to the subject is increased.

**3.** The method of claim 1, wherein based on the determination that the iRNA agent is active in the subject the dose of iRNA agent to be administered to the subject is increased or decreased.

**4.** The method of any one of claims 1 to 3, wherein the target RNA or RNA cleavage product is measured by a 5' RACE, hybridization, polymerase chain reaction (PCR), quantitative PCR (qPCR), branched DNA (bDNA) assay, or reverse transcription-PCR (RT-PCR).

## Patentansprüche

**1.** Verfahren zur Bestimmung der Aktivität eines iRNA Agens, das auf Transthyretin (TTR) RNA zielt, in einem Individuum, dem das iRNA Agens in ein Lebergewebe verabreicht wurde, umfassend:

(a) Nachweis des Levels der TTR RNA oder eines Spaltprodukts davon in einer Serumprobe, die von dem Individuum erlangt wurde, und
(b) Vergleich des in (a) erhaltenen Levels mit einer Referenzprobe;

wobei ein erniedrigtes Level der TTR RNA oder ein erhöhtes Level des Spaltprodukts im Vergleich zu der Referenzprobe anzeigt, dass das iRNA Agens aktiv ist, und ein erhöhtes oder gleichbleibendes Level der TTR RNA oder ein erniedrigtes oder gleichbleibendes Level des Spaltprodukts im Vergleich zu der Referenzprobe anzeigt, dass das iRNA Agens in dem Individuum inaktiv ist.

**2.** Verfahren nach Anspruch 1, wobei basierend auf der Bestimmung, dass das iRNA Agens in dem Individuum inaktiv ist, die Dosierung des an das Individuum zu verabreichenden iRNA Agens erhöht wird.

**3.** Verfahren nach Anspruch 1, wobei basierend auf der Bestimmung, dass das iRNA Agens in dem Individuum aktiv ist, die Dosierung des an das Individuum zu verabreichenden iRNA Agens erhöht oder erniedrigt wird.

**4.** Verfahren nach einem der Ansprüche 1 bis 3, wobei die Ziel-RNA oder das RNA Spaltprodukt mittels einer/m 5' RACE, Hybridisierung, Polymerasekettenreaktion (PCR), quantitativen PCR (qPCR), verzweigten DNA (bDNA) Assay oder reversen Transkription-PCR (RT-PCR) gemessen wird.

## Revendications

**1.** Méthode pour déterminer l'activité d'un agent ARNi ciblant l'ARN de transthyrétine (TTR) chez un sujet auquel a été administré ledit agent ARNi dans un tissu hépatique, comprenant :

(a) la détection du niveau d'ARN de TTR ou d'un produit de clivage de celui-ci dans un échantillon de sérum obtenu du sujet, et
(b) la comparaison du niveau obtenu en (a) avec un échantillon de référence ;

dans lequel un niveau réduit de l'ARN de TTR ou un niveau accru du produit de clivage, en comparaison avec l'échantillon de référence, est indicatif du fait que l'agent ARNi est actif, et un niveau accru ou statique de l'ARN de TTR ou un niveau réduit ou statique du produit de clivage, en comparaison avec l'échantillon de référence, est indicatif du fait que l'agent ARNi est inactif chez le sujet.

**2.** Méthode selon la revendication 1, dans lequel, sur la base de la détermination du fait que l'agent ARNi est inactif chez le sujet, la dose de l'agent ARNi à administrer au sujet est augmentée.

**3.** Méthode selon la revendication 1, dans lequel, sur la base de la détermination du fait que l'agent ARNi est actif chez le sujet, la dose de l'agent ARNi à administrer au sujet est augmentée ou réduite.

**4.** Méthode selon l'une quelconque des revendications 1 à 3, dans lequel l'ARN cible ou le produit de clivage d'ARN est mesuré par 5' RACE, hybridation, amplification en chaîne par polymérase (PCR), PCR quantitative (qPCR), dosage d'ADN ramifié (bDNA), ou transcription inverse-PCR (RT-PCR).

FIG. 1

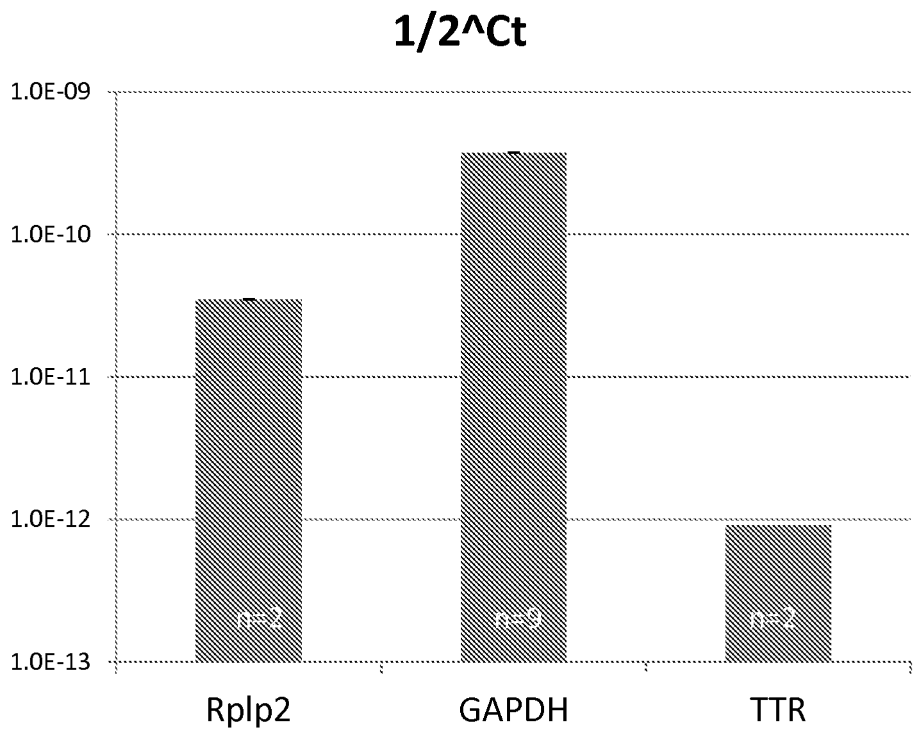

FIG. 2A

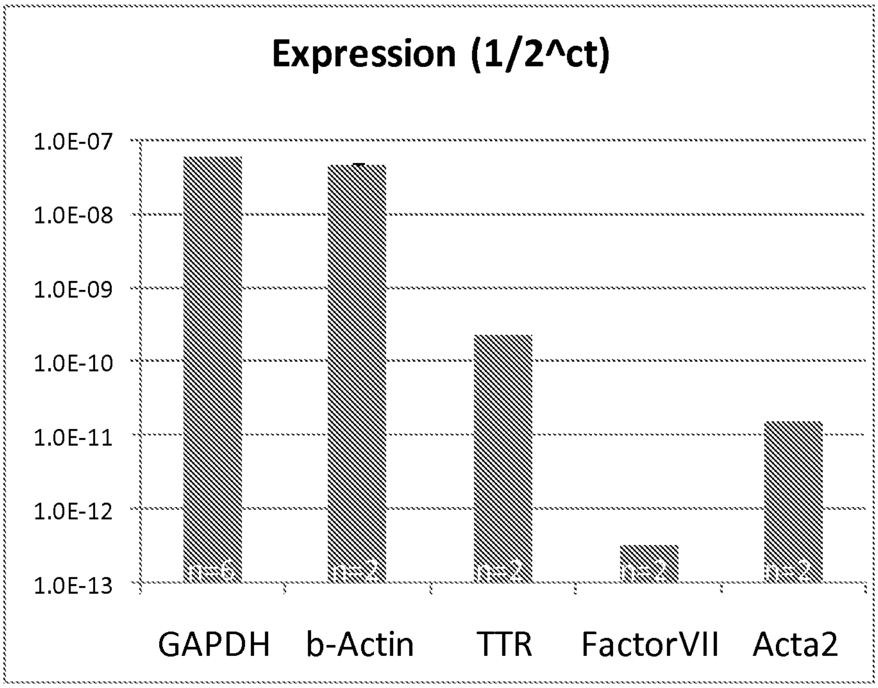
Expression (1/2^ct)
1.0E-07
1.0E-08
1.0E-09
1.0E-10
1.0E-11
1.0E-12
1.0E-13
n=6
n=2
n=2
n=2
n=2
GAPDH
b-Actin
TTR
FactorVII
Acta2

FIG. 2B

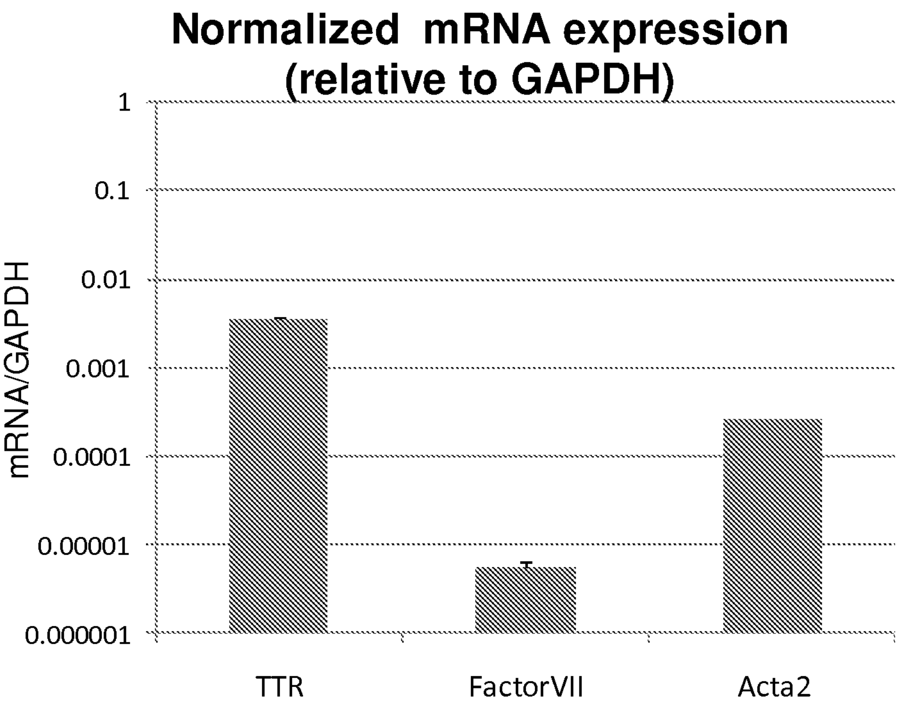
Normalized mRNA expression (relative to GAPDH)
mRNA/GAPDH
1
0.1
0.01
0.001
0.0001
0.00001
0.000001
TTR
FactorVII
Acta2

FIG. 3

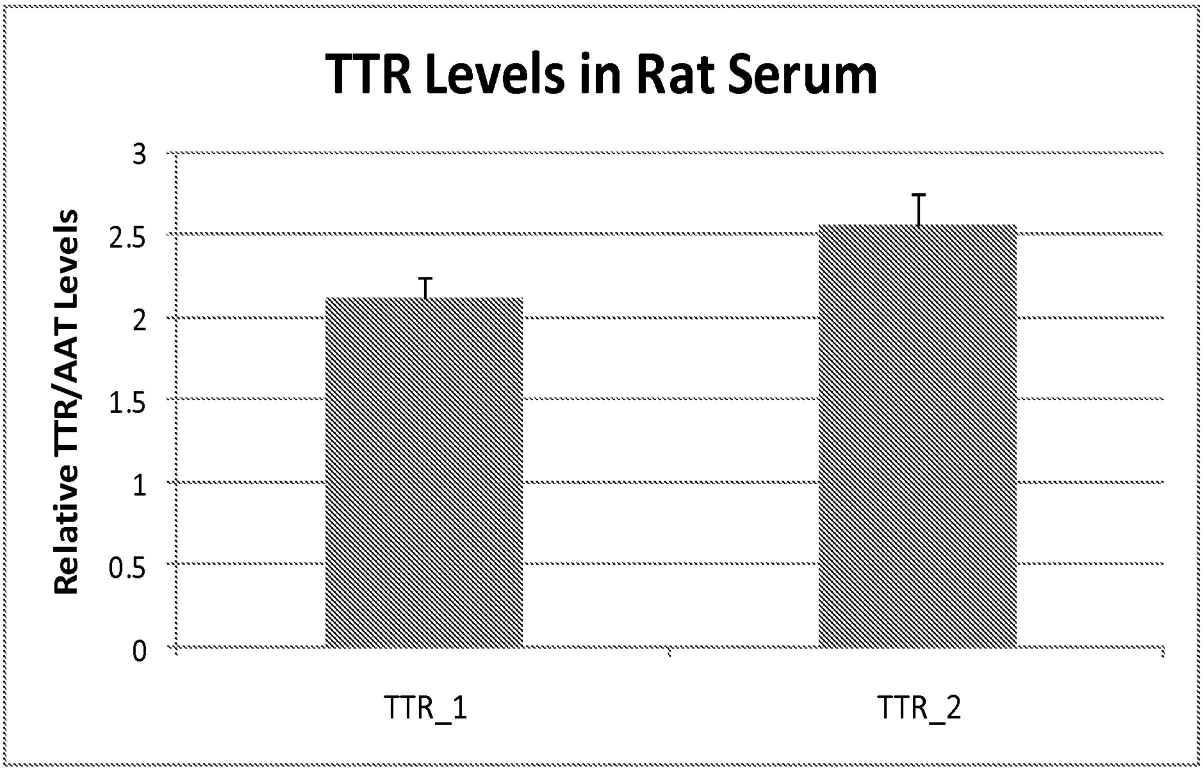
TTR Levels in Rat Serum
Relative TTR/AAT Levels
3
2.5
2
1.5
1
0.5
0
TTR_1
TTR_2

FIG. 4A

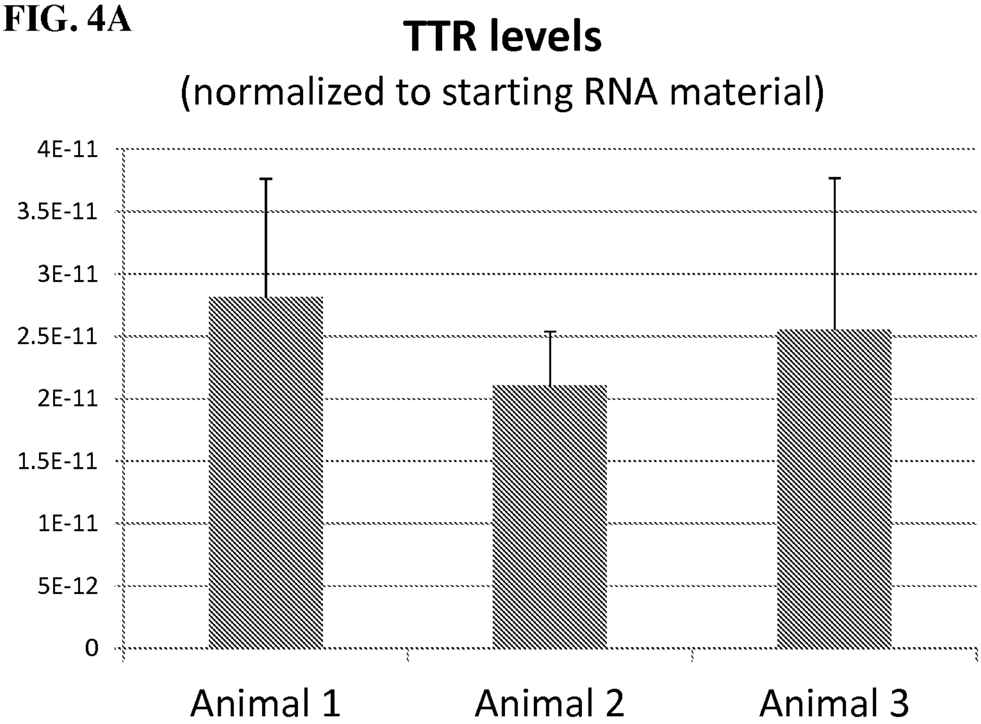
TTR levels
(normalized to starting RNA material)
4E-11
3.5E-11
3E-11
2.5E-11
2E-11
1.5E-11
1E-11
5E-12
0
Animal 1
Animal 2
Animal 3

FIG. 4B

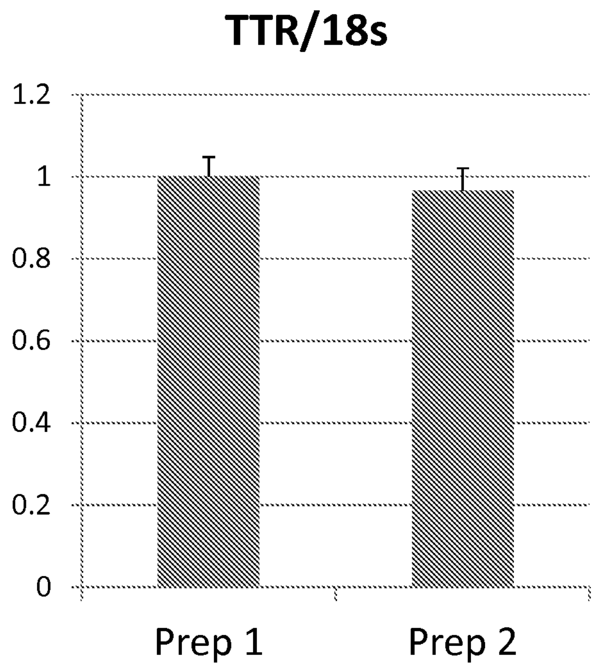
TTR/18s
1.2
1
0.8
0.6
0.4
0.2
0
Prep 1
Prep 2

FIG. 5A

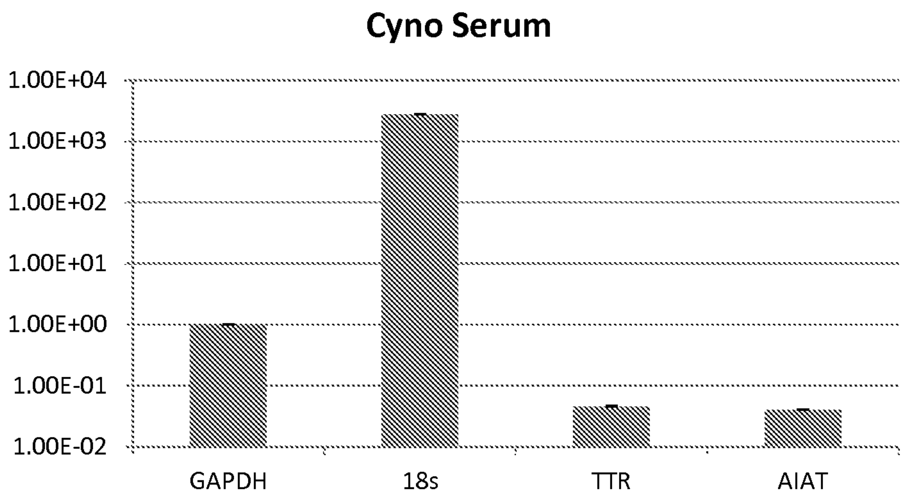
Cyno Serum
1.00E+04
1.00E+03
1.00E+02
1.00E+01
1.00E+00
1.00E-01
1.00E-02
GAPDH
18s
TTR
AIAT

FIG. 5B

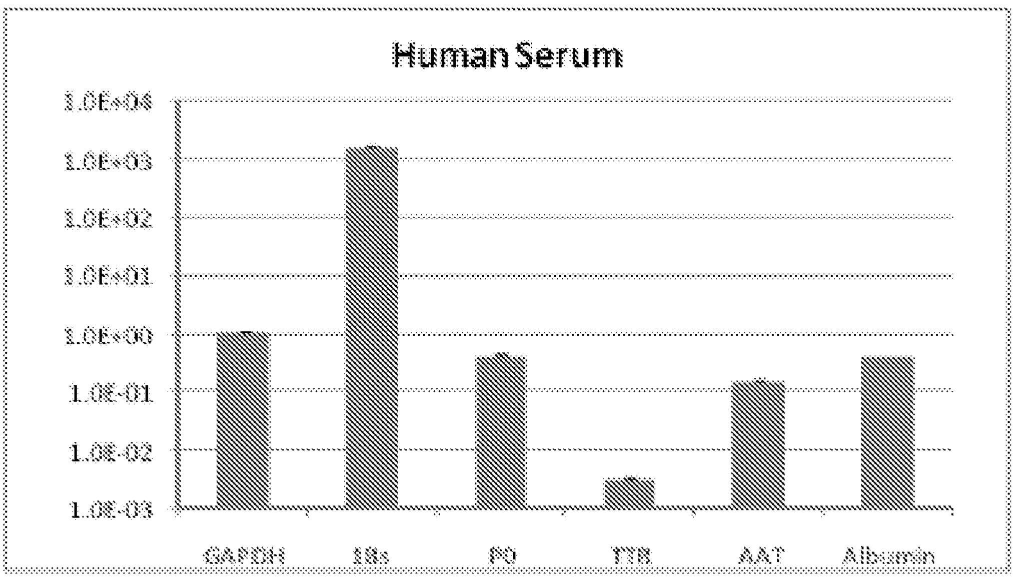
Human Serum
1.0E+04
1.0E+03
1.0E+02
1.0E+01
1.0E+00
1.0E-01
1.0E-02
1.0E-03
GAPDH
18s
P0
TTR
AAT
Albumin

FIG. 6A

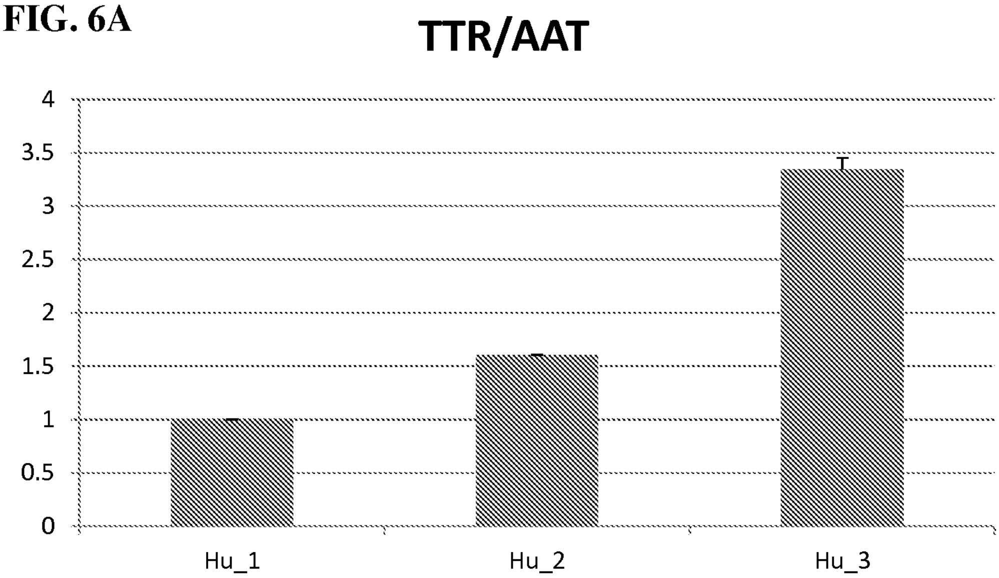
TTR/AAT
4
3.5
3
2.5
2
1.5
1
0.5
0
Hu_1
Hu_2
Hu_3

FIG. 6B

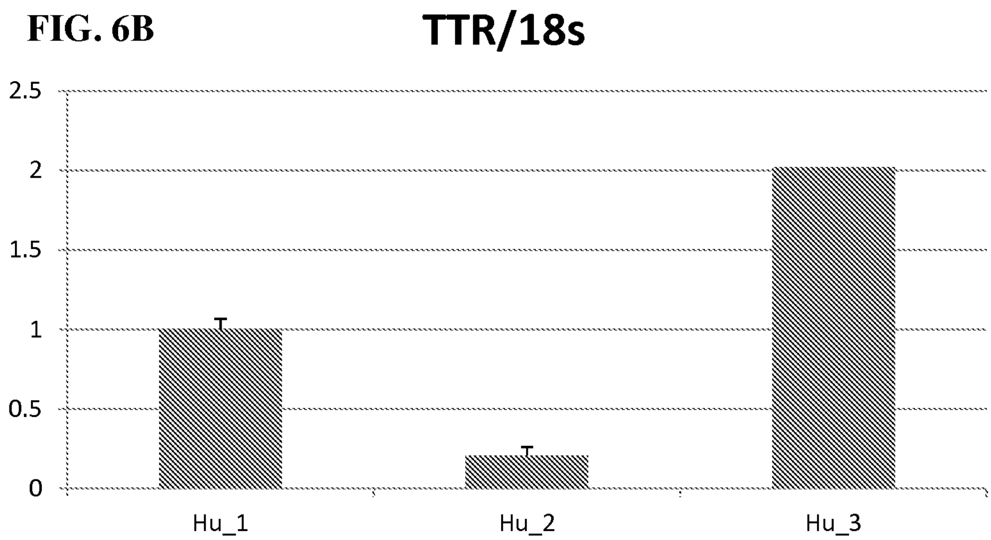
TTR/18s
2.5
2
1.5
1
0.5
0
Hu_1
Hu_2
Hu_3

FIG. 7

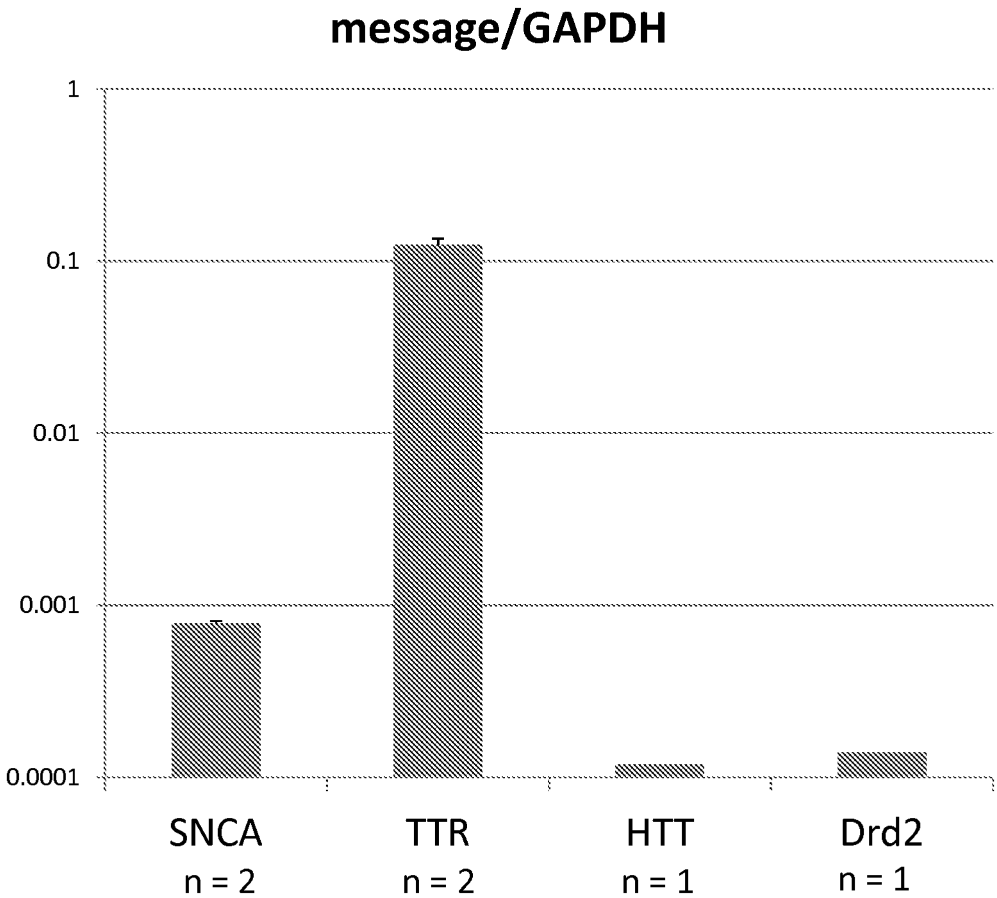

FIG. 8A

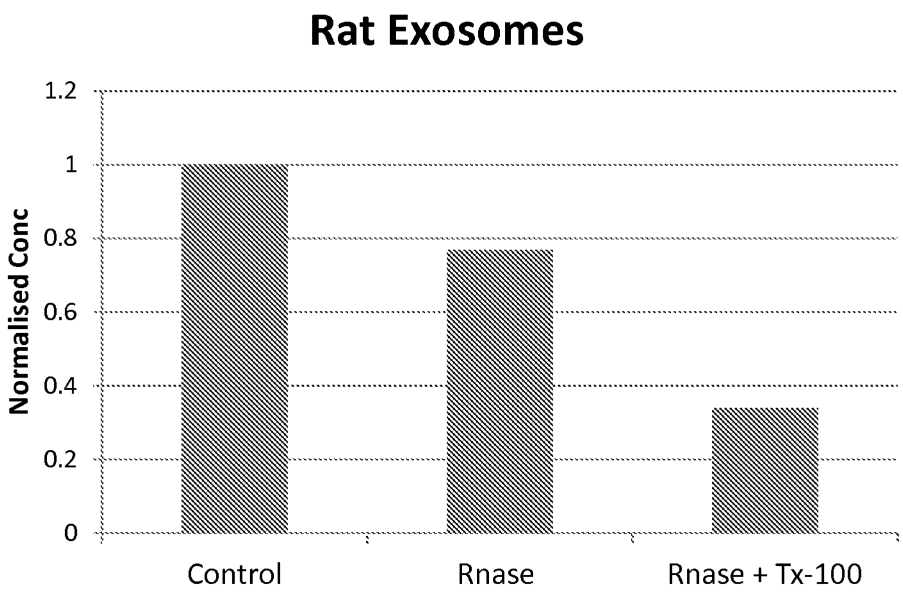
Rat Exosomes
Normalised Conc
1.2
1
0.8
0.6
0.4
0.2
0
Control
Rnase
Rnase + Tx-100

FIG. 8B

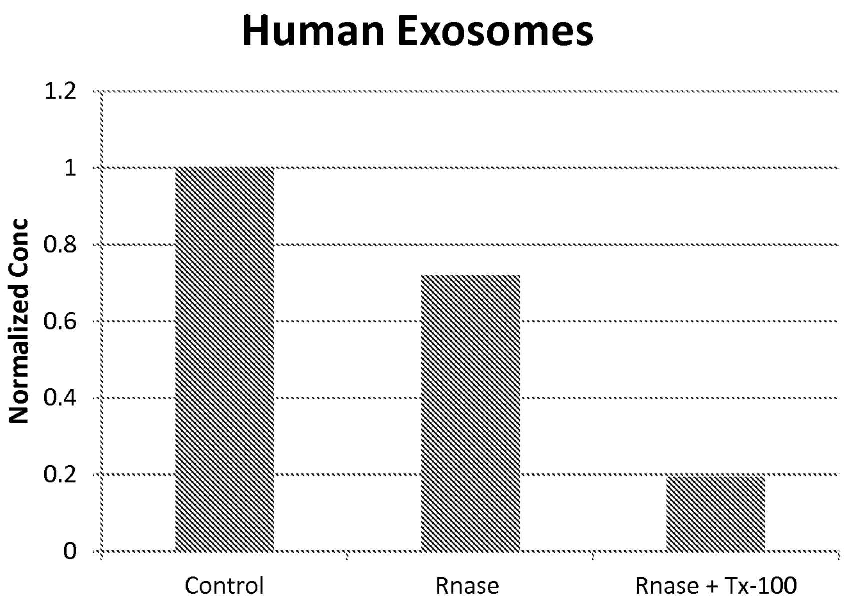
Human Exosomes
Normalized Conc
1.2
1
0.8
0.6
0.4
0.2
0
Control
Rnase
Rnase + Tx-100

FIG. 9A

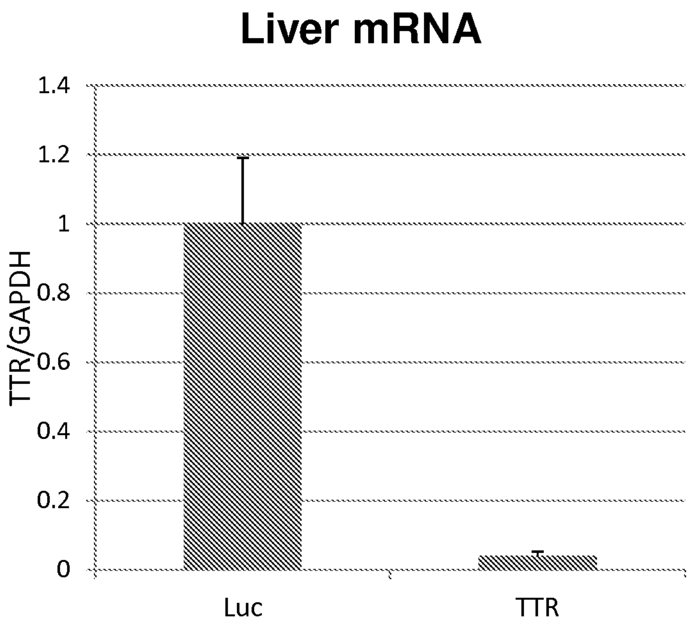
Liver mRNA
1.4
1.2
1
0.8
0.6
0.4
0.2
0
TTR/GAPDH
Luc
TTR

FIG. 9B

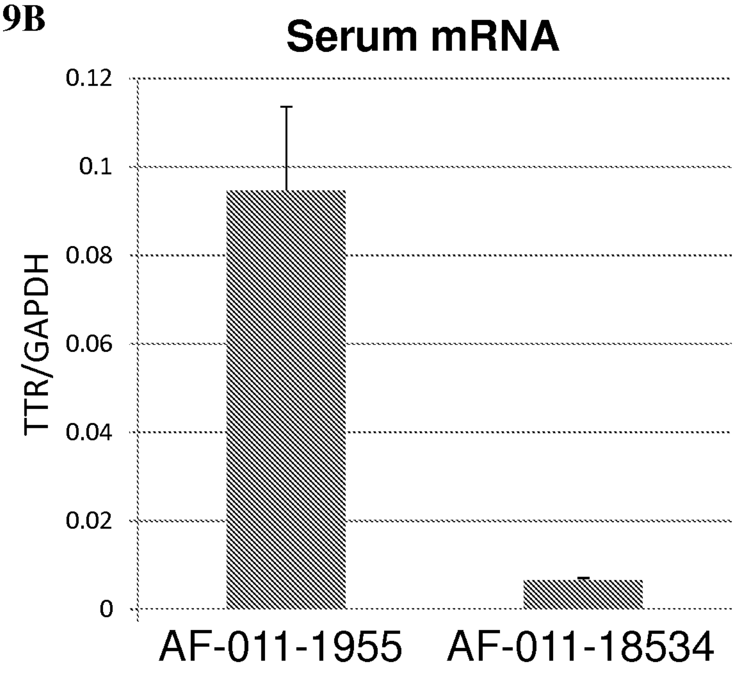
Serum mRNA
0.12
0.1
0.08
0.06
0.04
0.02
0
TTR/GAPDH
AF-011-1955
AF-011-18534

FIG. 10A

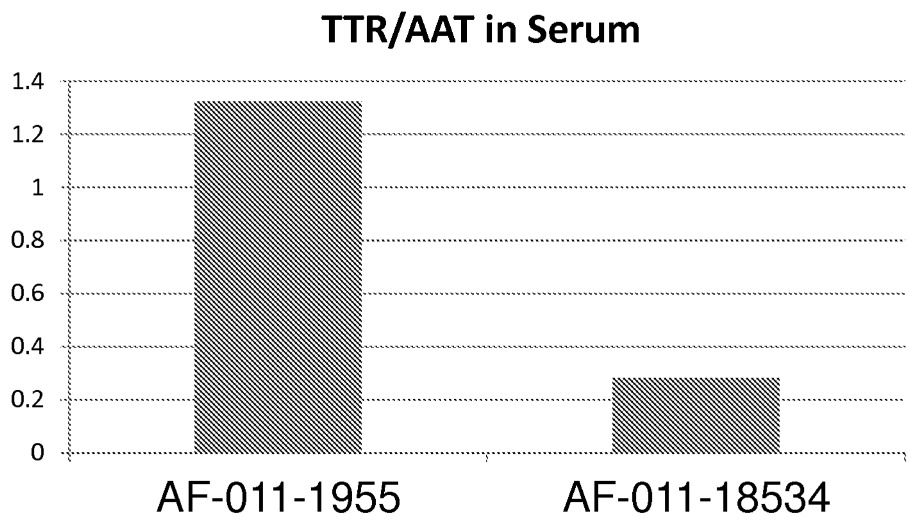


FIG. 10B

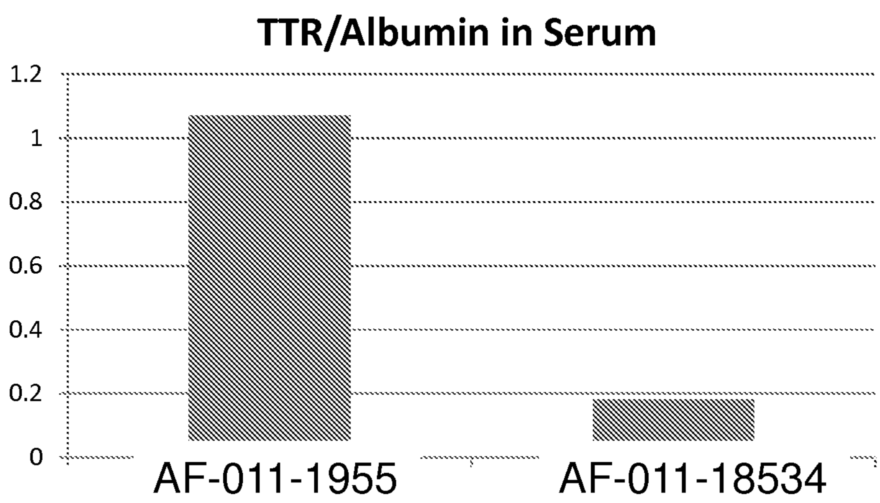

FIG. 10C

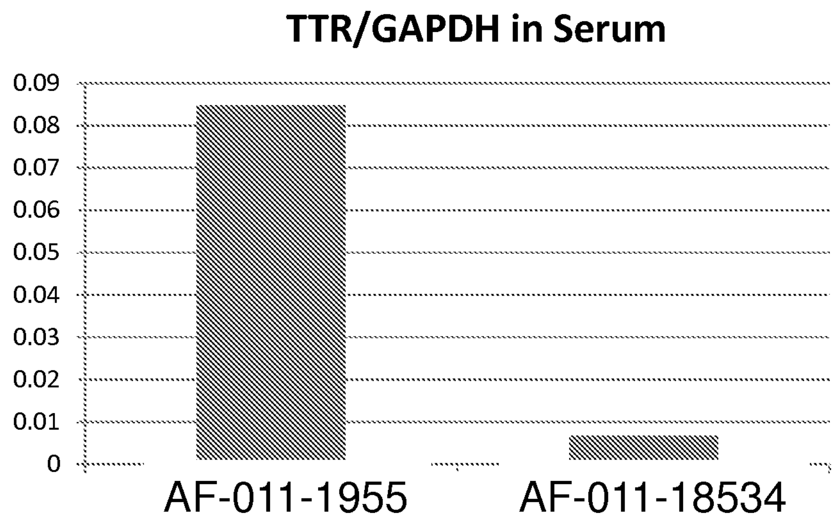


FIG. 10D

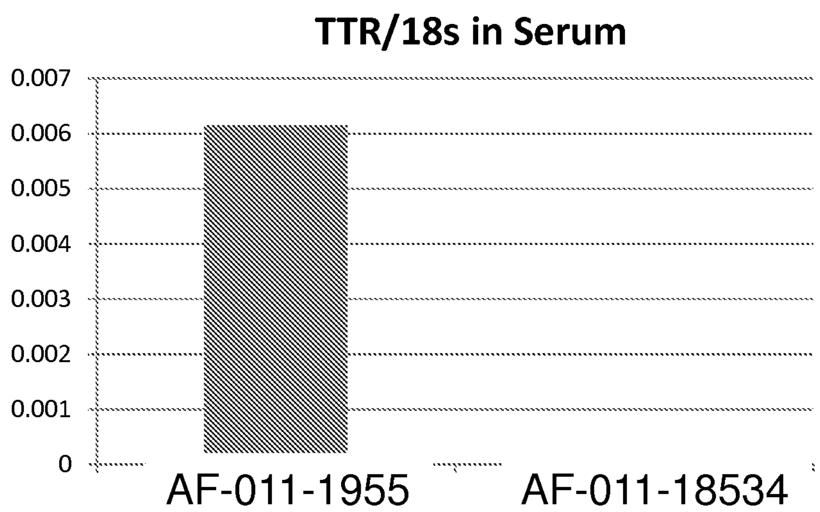

FIG. 11A

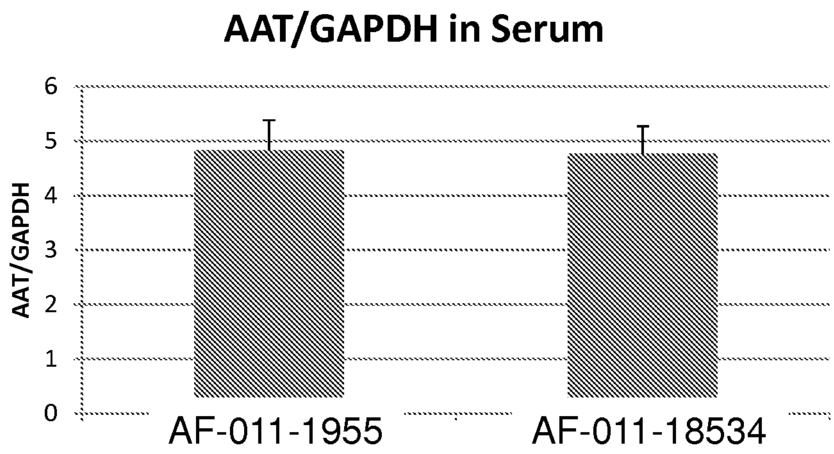


FIG. 11B

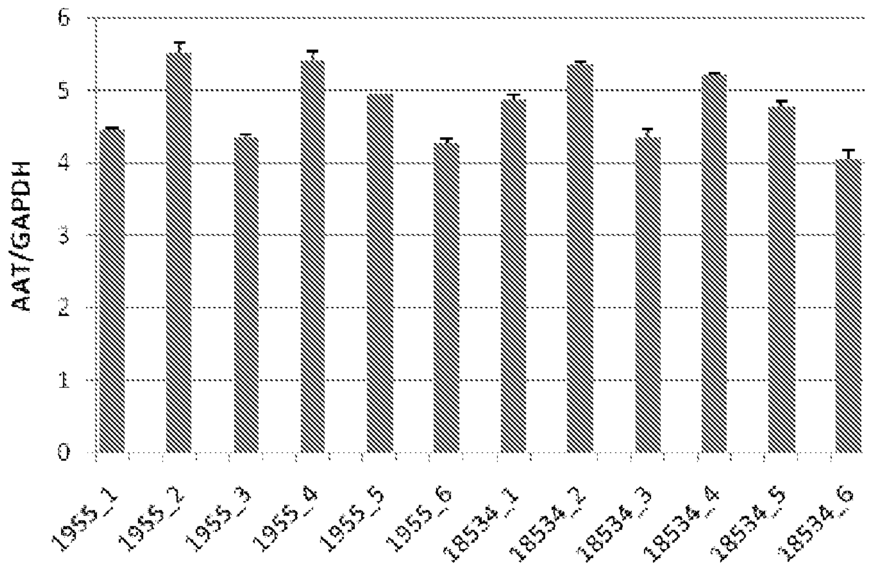

FIG. 11C

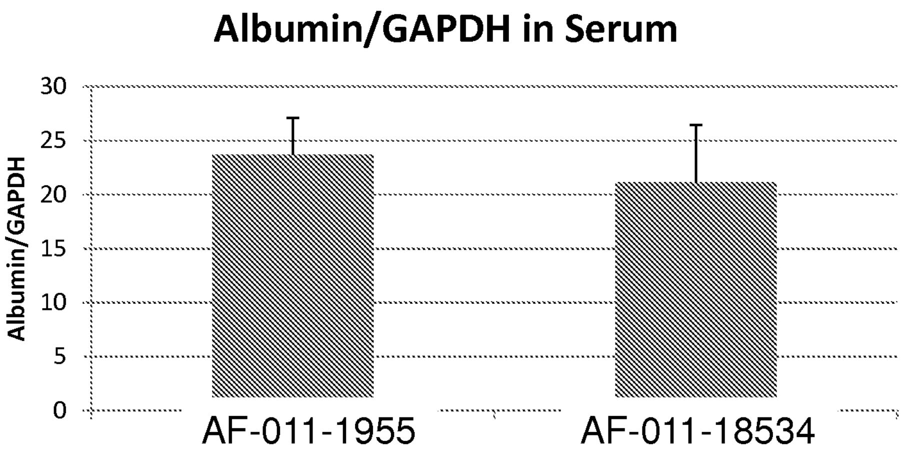


FIG. 11D

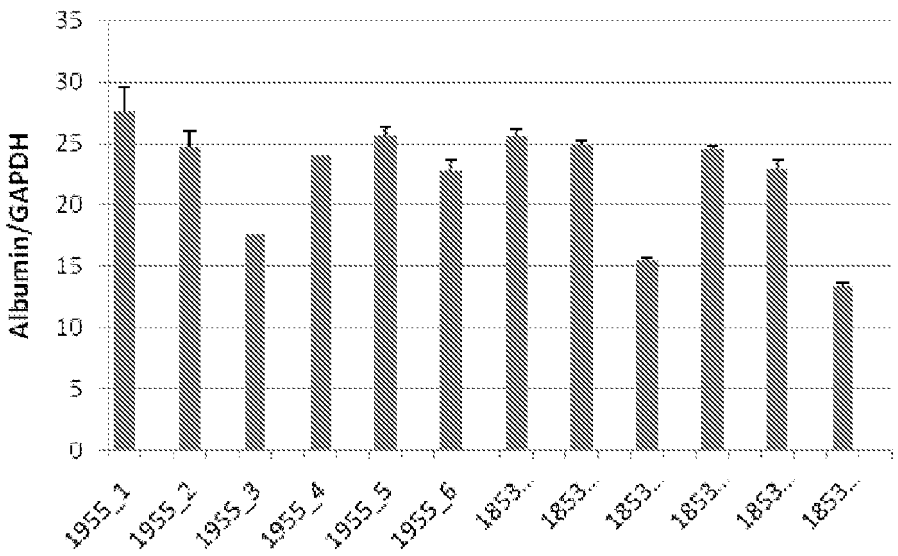

FIG. 12A

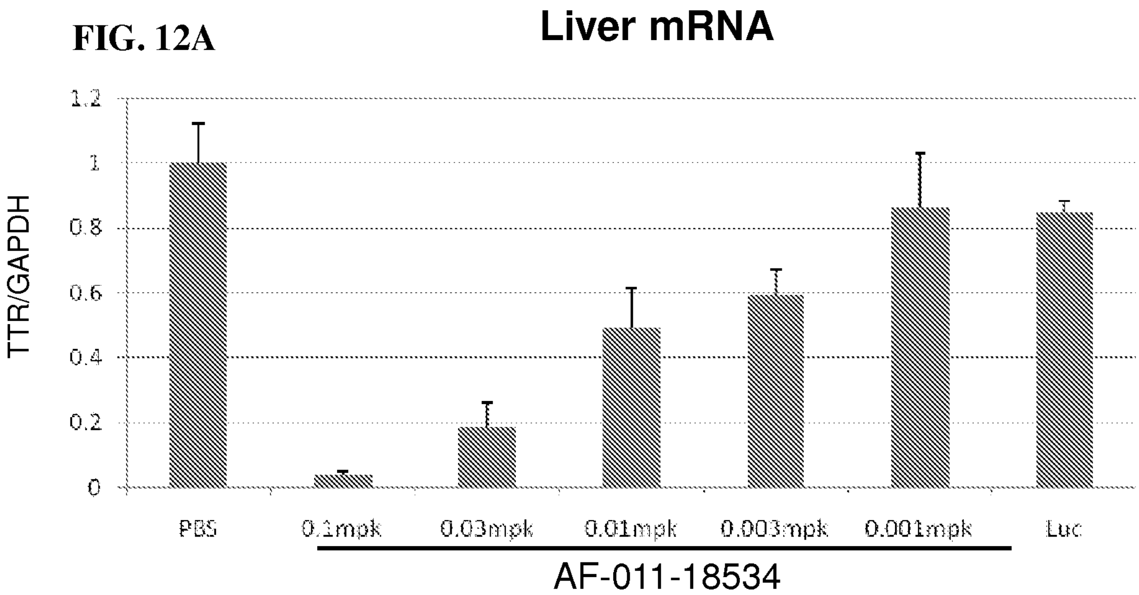


FIG. 12B

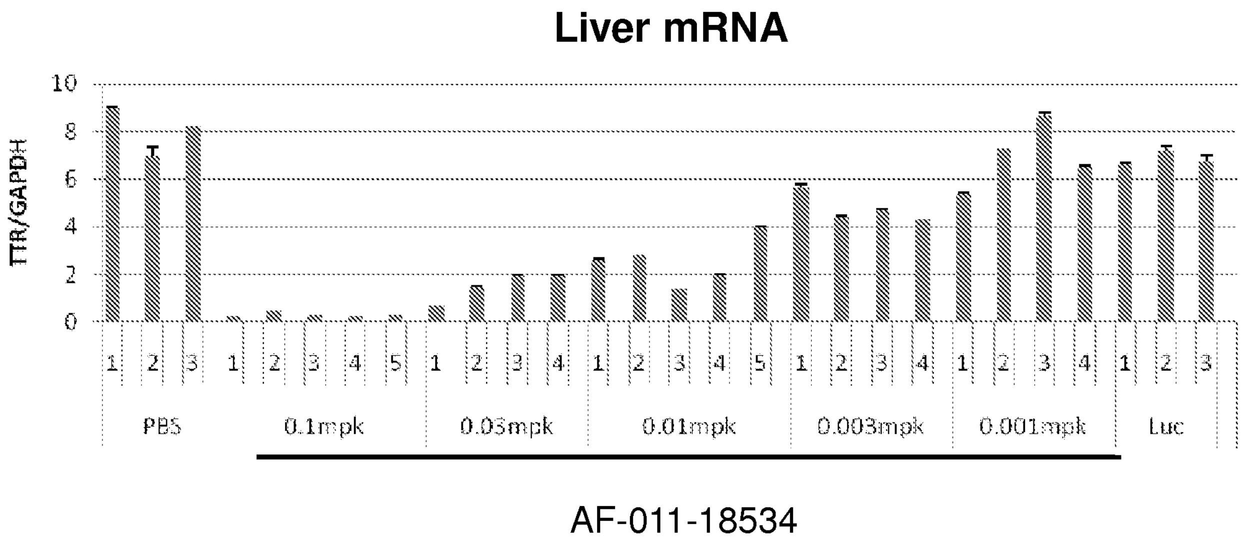

FIG. 13A

Serum mRNA

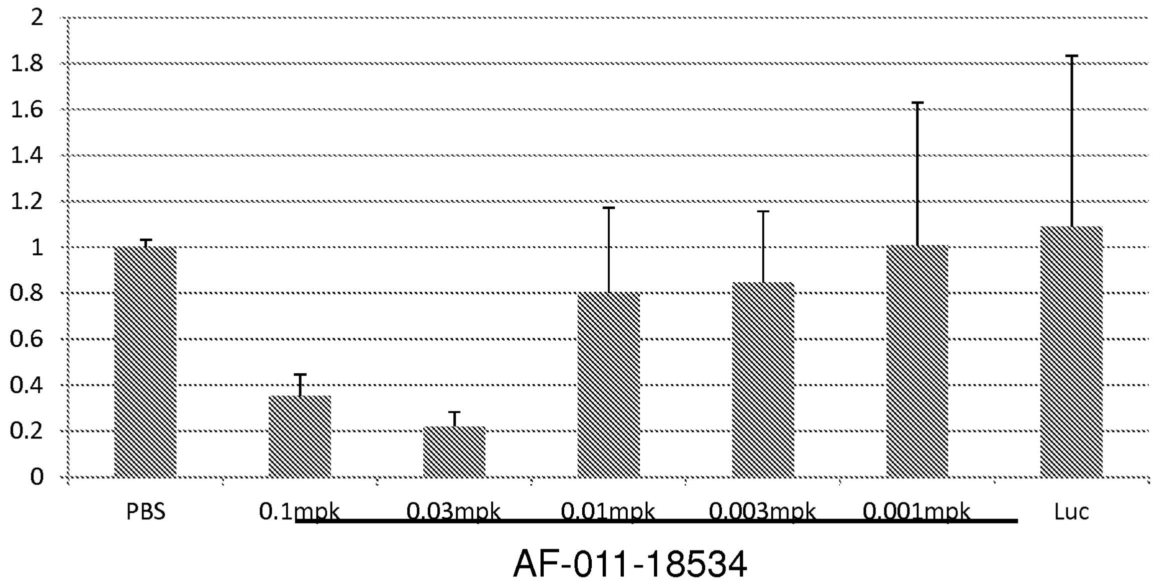


FIG. 13B

Serum mRNA

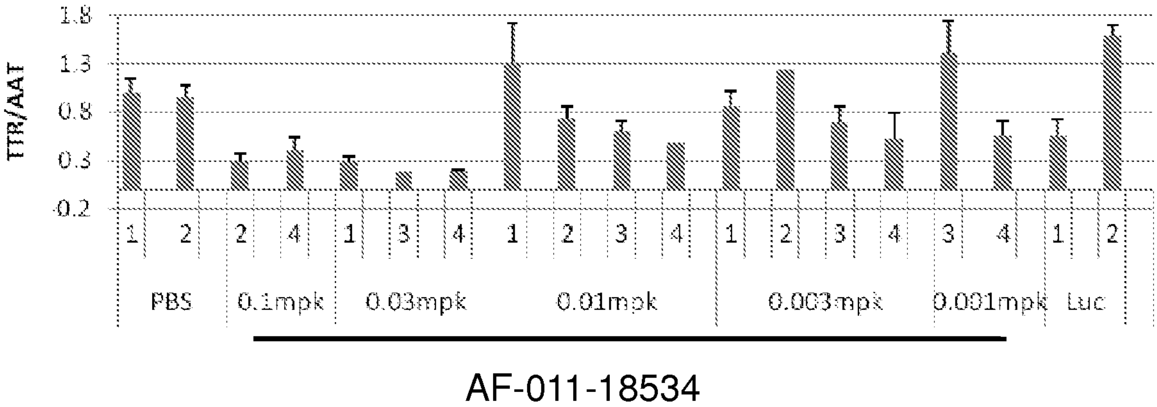

FIG. 14A

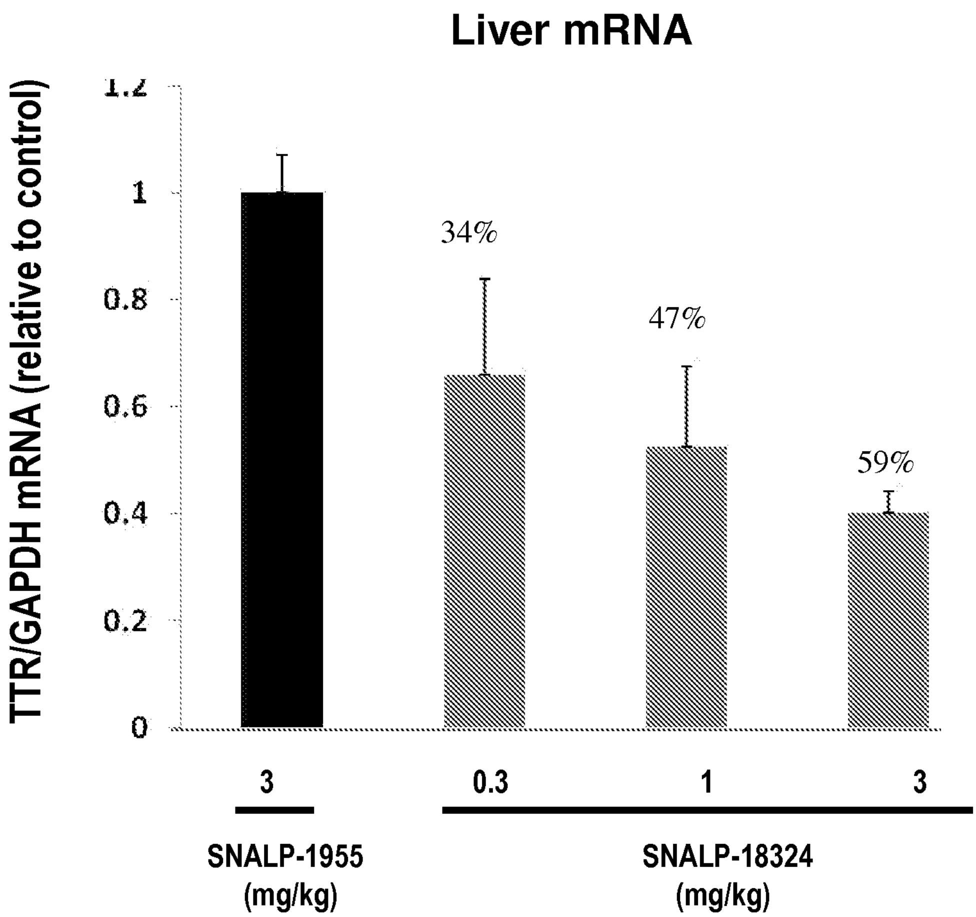
Liver mRNA
TTR/GAPDH mRNA (relative to control)
1.2
1
0.8
0.6
0.4
0.2
0
34%
47%
59%
3
0.3
1
3
SNALP-1955
(mg/kg)
SNALP-18324
(mg/kg)

FIG. 14B

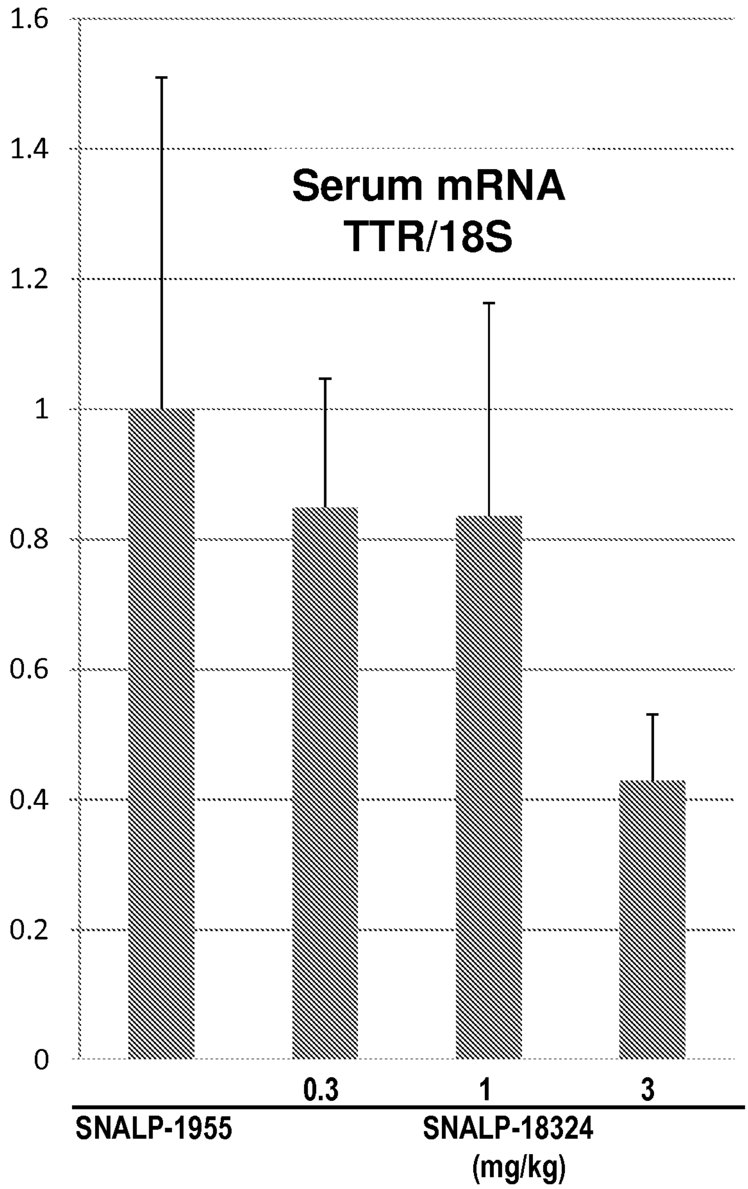
Serum mRNA
TTR/18S
1.6
1.4
1.2
1
0.8
0.6
0.4
0.2
0
0.3
1
3
SNALP-1955
SNALP-18324
(mg/kg)

FIG. 15

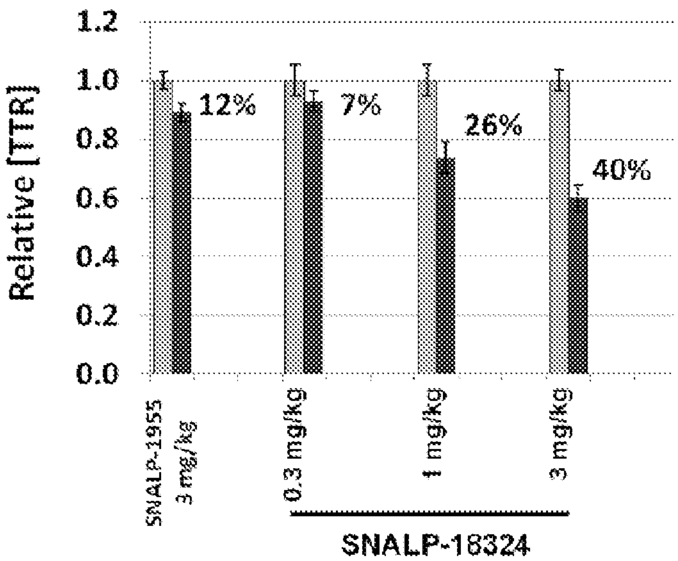
Relative [TTR]
1.2
1.0
0.8
0.6
0.4
0.2
0.0
12%
7%
26%
40%
SNALP-1955 3 mg/kg
0.3 mg/kg
1 mg/kg
3 mg/kg
SNALP-18324

FIG. 16A

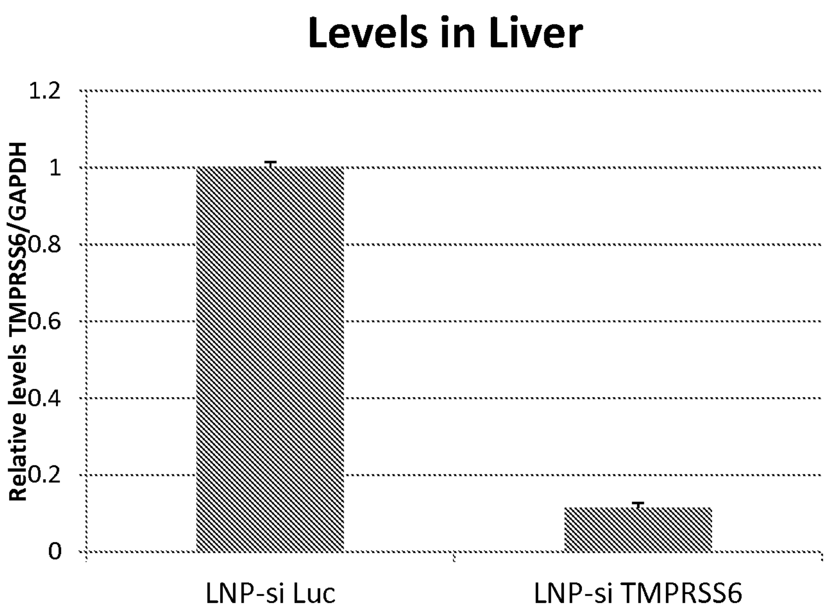


FIG. 16B

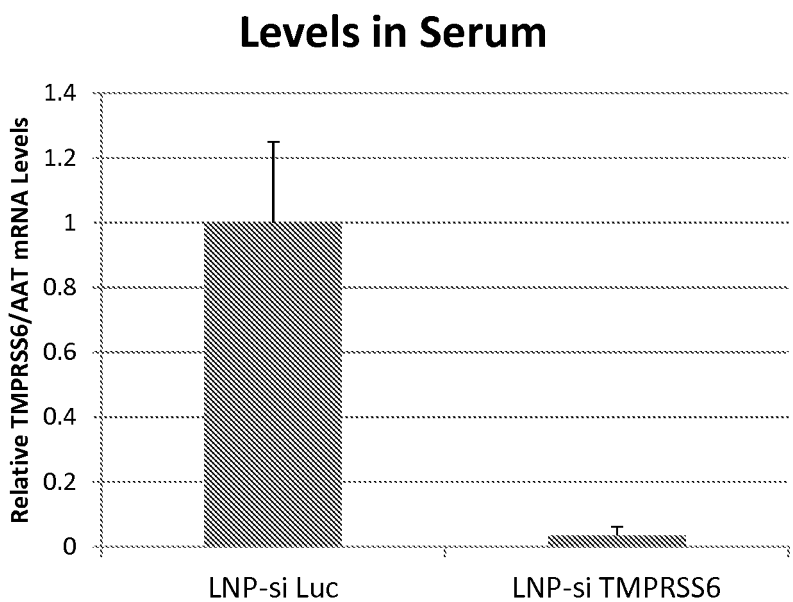

FIG. 17A

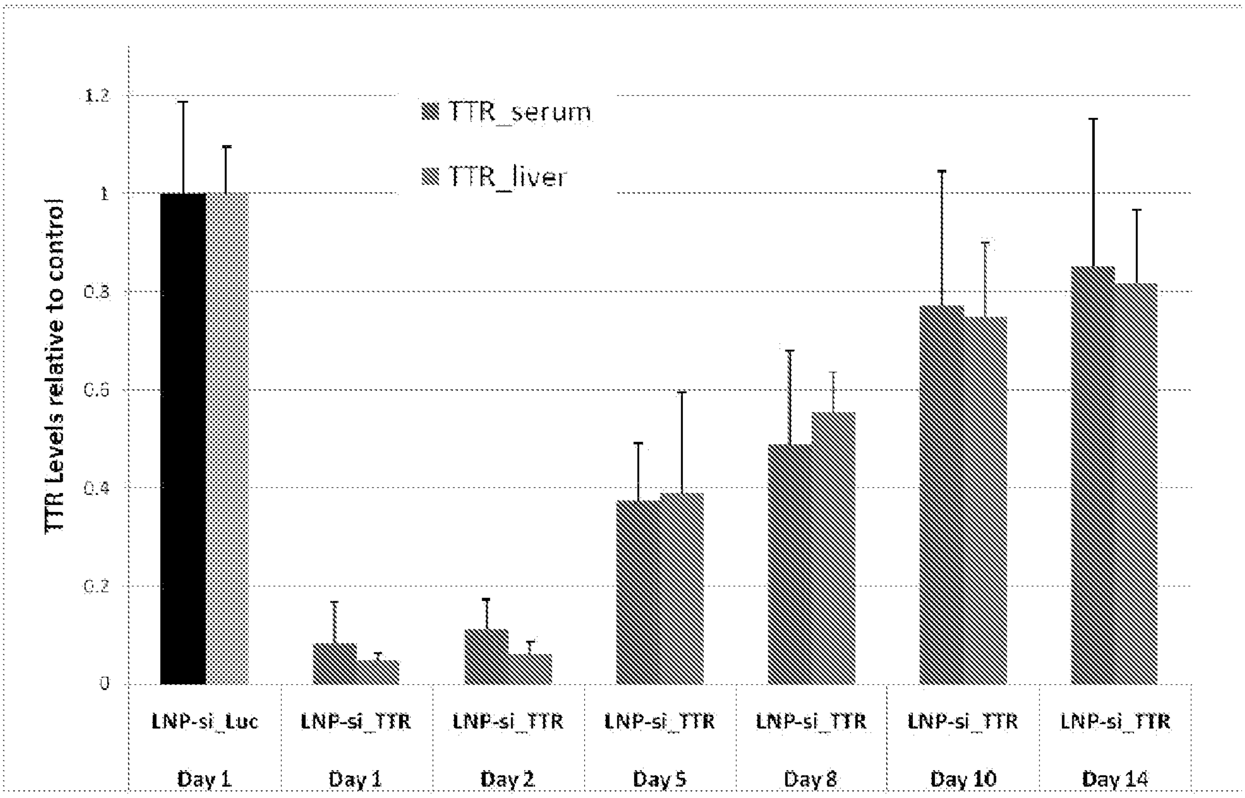


FIG. 17B

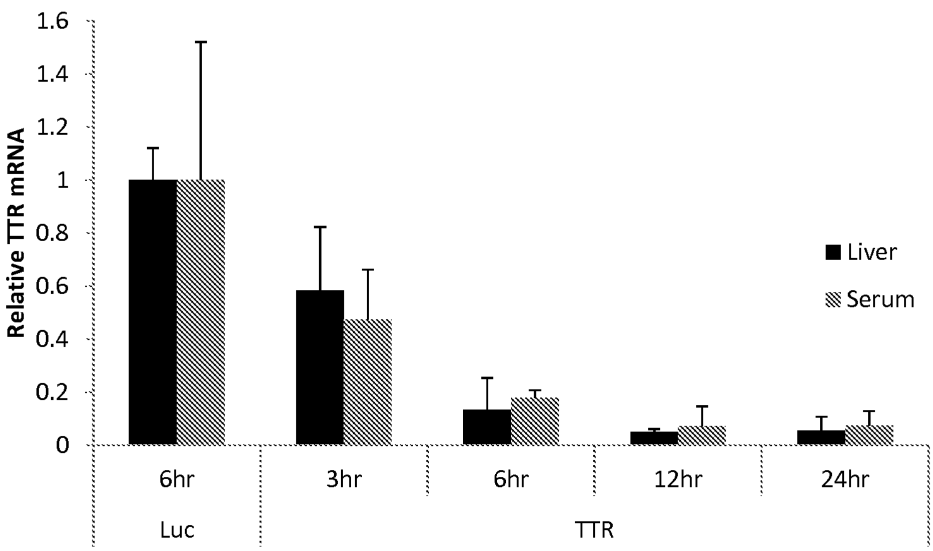

FIG. 18

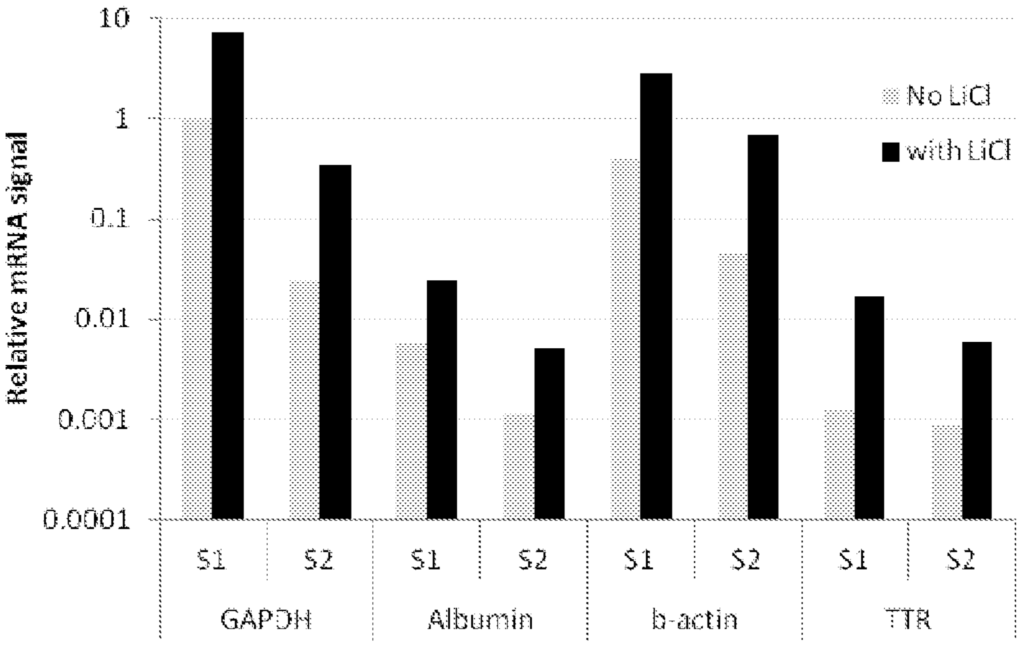

Relative mRNA signal
10
1
0.1
0.01
0.001
0.0001
No LiCl
with LiCl
S1
S2
S1
S2
S1
S2
S1
S2
GAPDH
Albumin
b-actin
TTR

**FIG. 19A**

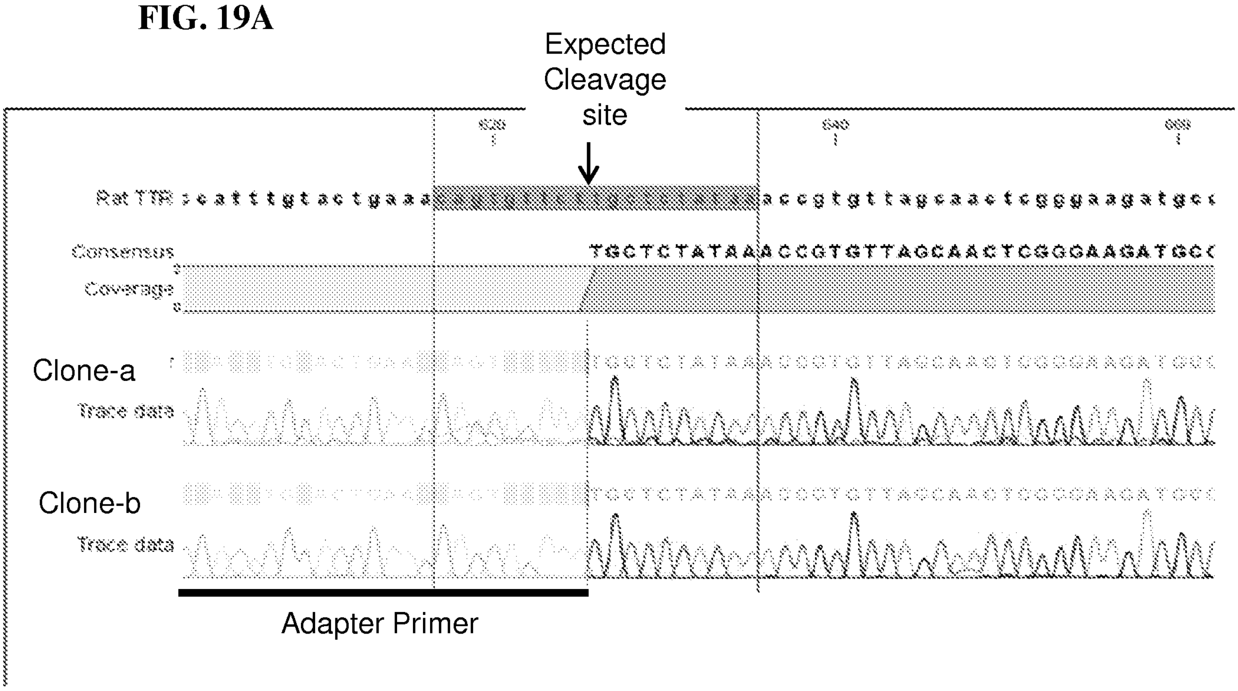


Highlighted area is siTTR

**FIG. 19B**

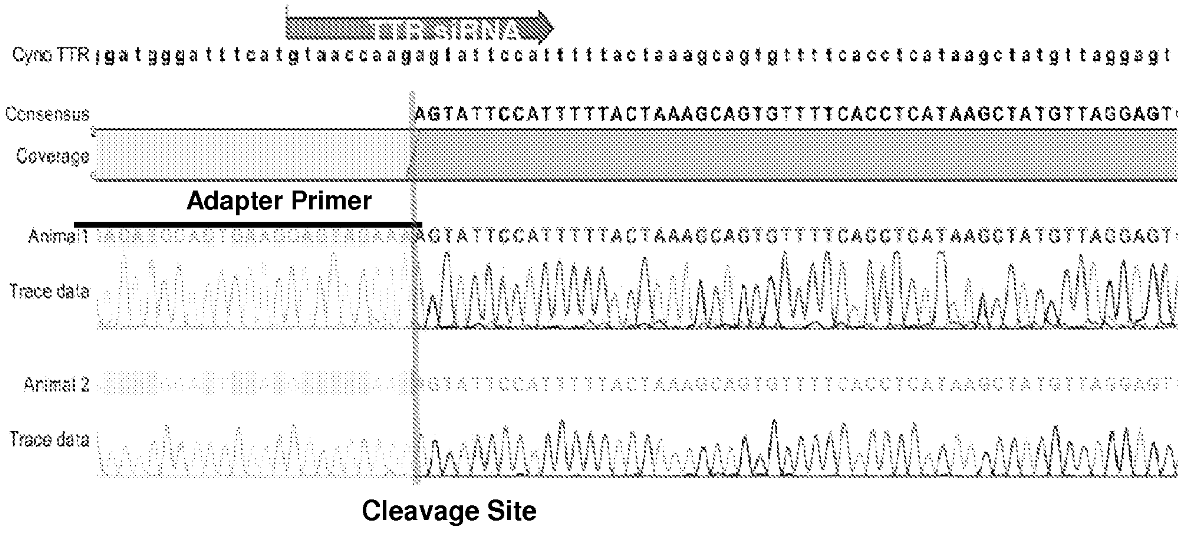

FIG. 20

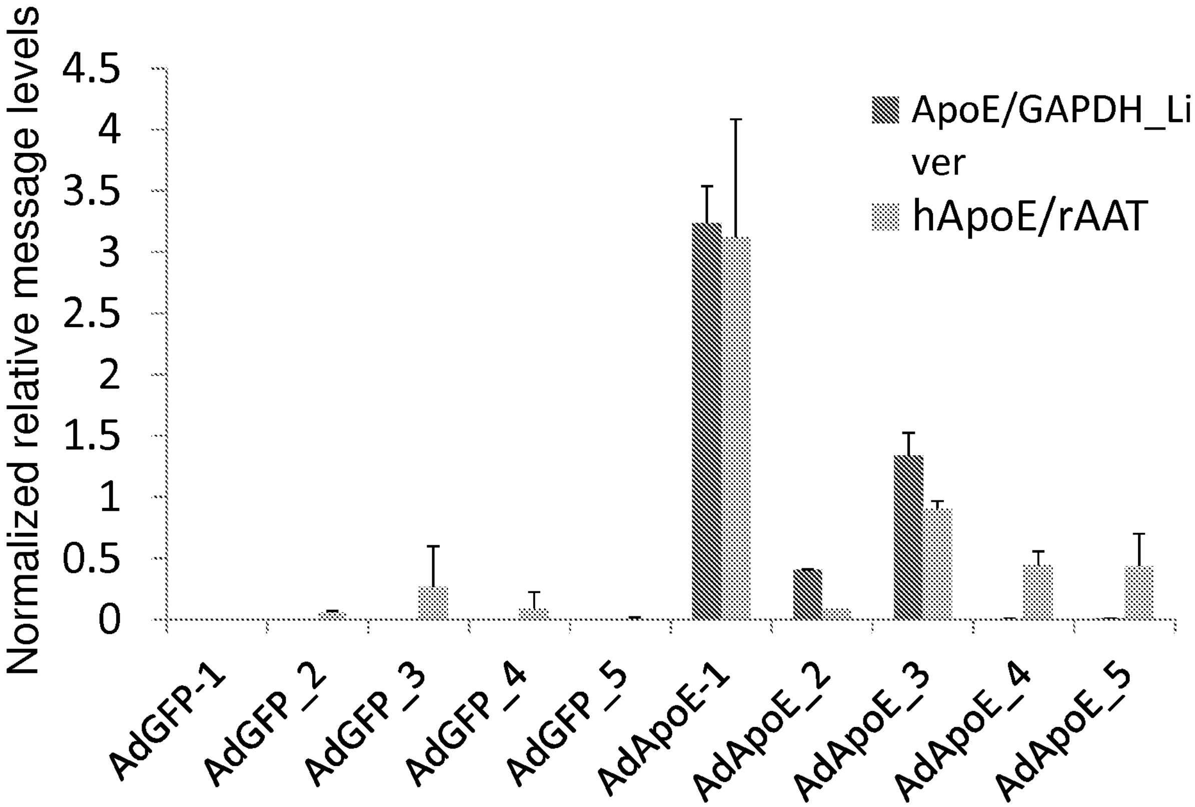
Normalized relative message levels
4.5
4
3.5
3
2.5
2
1.5
1
0.5
0
ApoE/GAPDH_Liver
hApoE/rAAT
AdGFP-1
AdGFP_2
AdGFP_3
AdGFP_4
AdGFP_5
AdApoE-1
AdApoE_2
AdApoE_3
AdApoE_4
AdApoE_5

FIG. 21

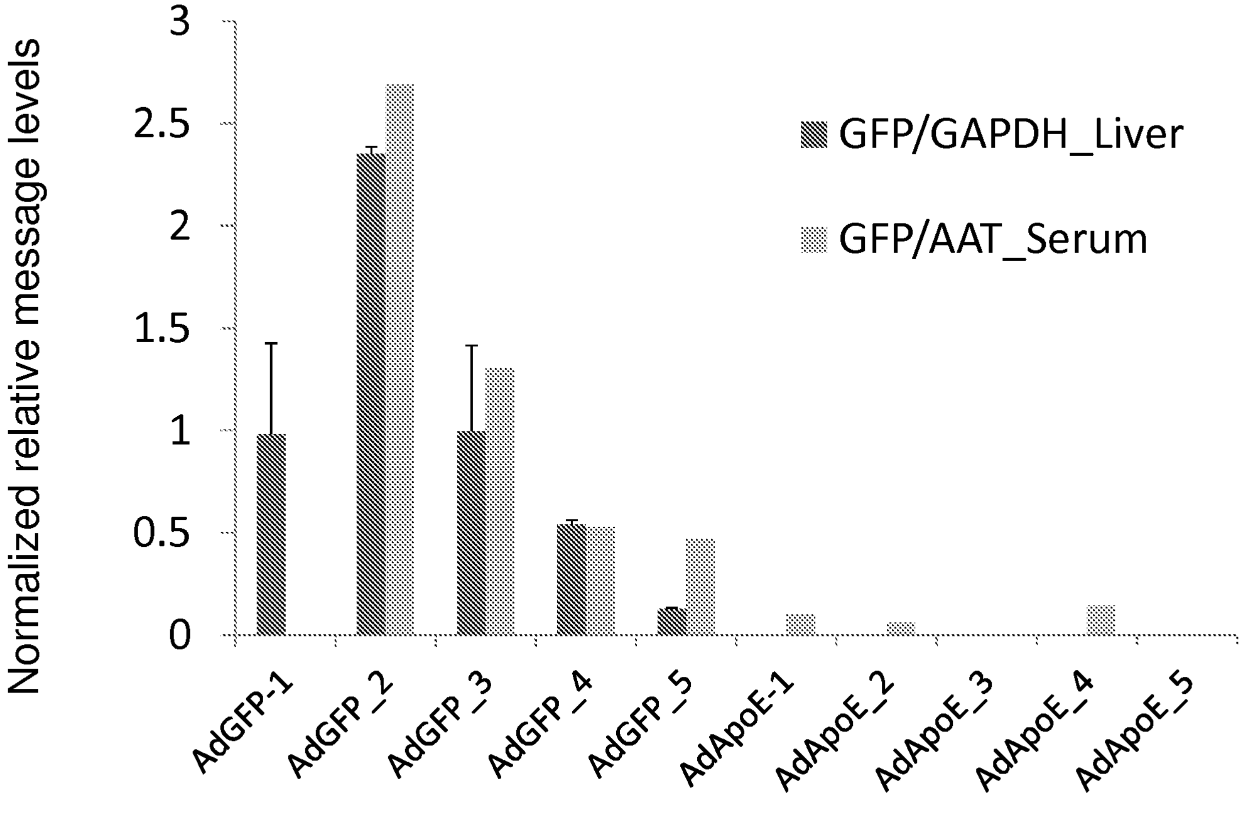
Normalized relative message levels
3
2.5
2
1.5
1
0.5
0
GFP/GAPDH_Liver
GFP/AAT_Serum
AdGFP-1
AdGFP_2
AdGFP_3
AdGFP_4
AdGFP_5
AdApoE-1
AdApoE_2
AdApoE_3
AdApoE_4
AdApoE_5

FIG. 22A

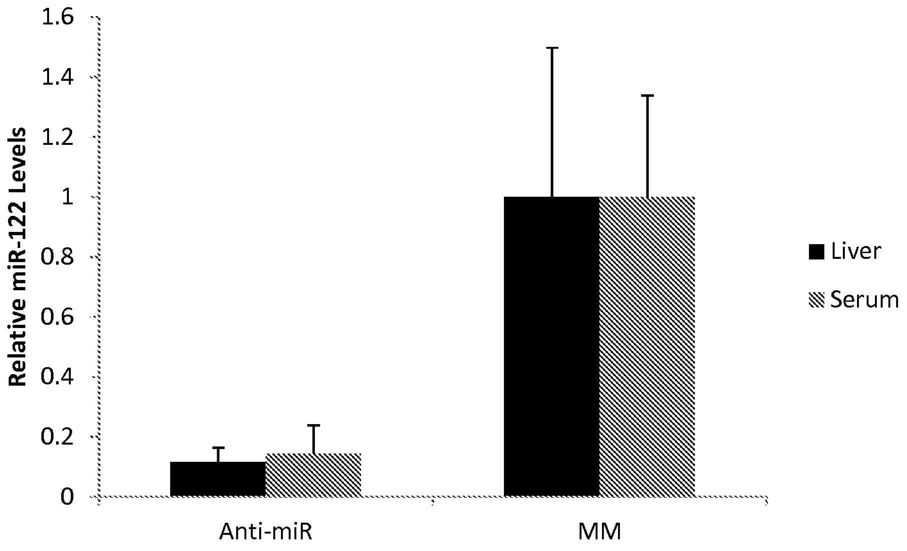


FIG. 22B

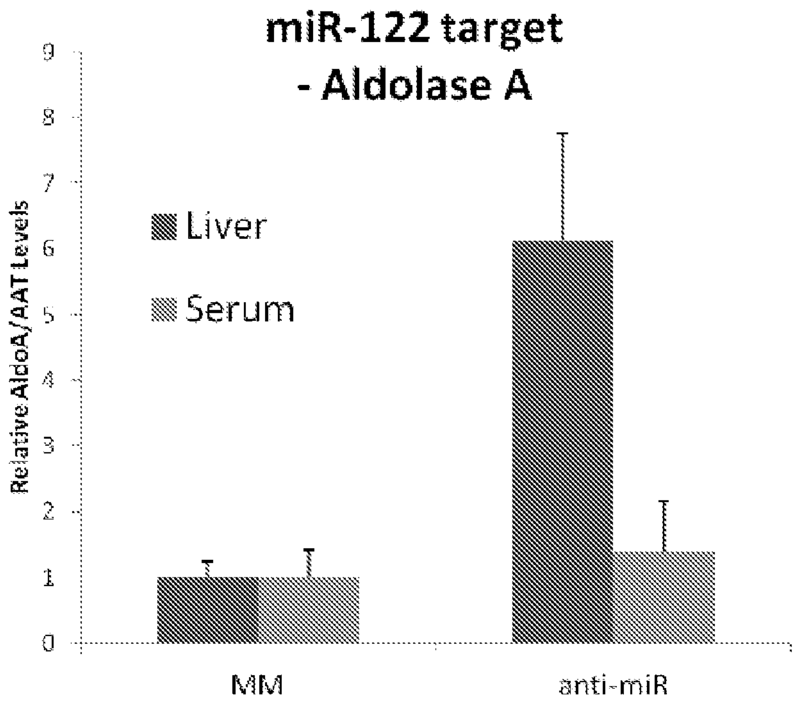

FIG. 23

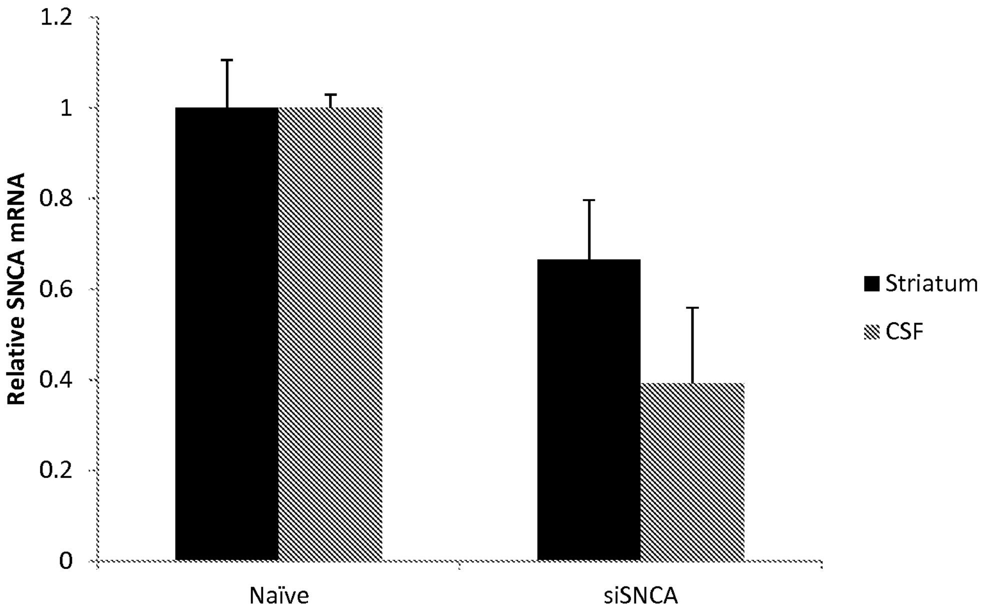
1.2
1
0.8
0.6
0.4
0.2
0
Relative SNCA mRNA
Naïve
siSNCA
Striatum
CSF

FIG. 24

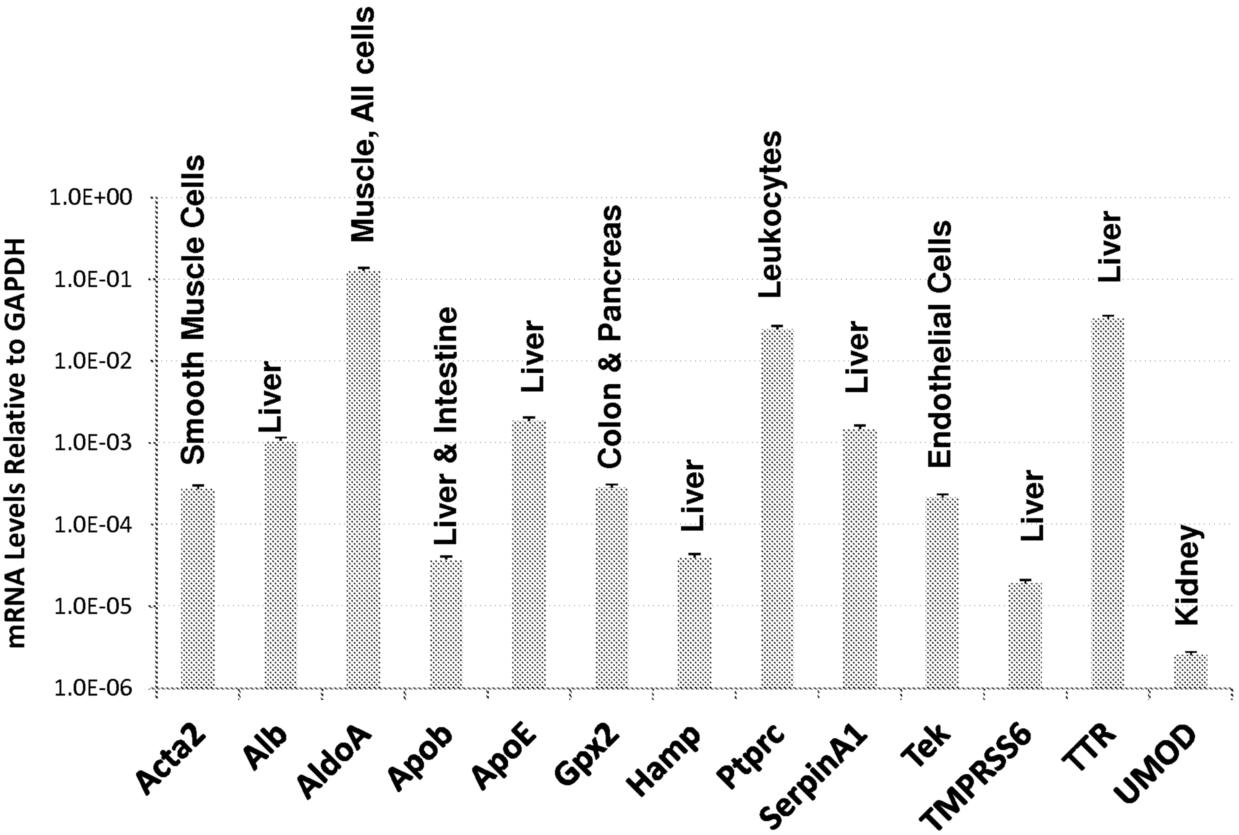
mRNA Levels Relative to GAPDH
1.0E+00
1.0E-01
1.0E-02
1.0E-03
1.0E-04
1.0E-05
1.0E-06
Smooth Muscle Cells
Liver
Muscle, All cells
Liver & Intestine
Liver
Colon & Pancreas
Liver
Leukocytes
Liver
Endothelial Cells
Liver
Liver
Kidney
Acta2
Alb
AldoA
Apob
ApoE
Gpx2
Hamp
Ptprc
SerpinA1
Tek
TMPRSS6
TTR
UMOD

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9932619 A, Fire
- WO 9953050 A, Waterhouse
- WO 9961631 A, Heifetz
- WO 0044895 A, Limmer
- DE 10100586, Kreutzer
- WO 2009015357 A
- US 20060240093 A
- US 20070135372 A
- WO 2009082817 A
- US 5032401 A
- US 5607677 A
- US 20050281781 A
- US 20070111963 A
- US 2005226848 A
- US 7198923 B
- US 6899863 B
- US 6812023 B
- US 20110177054 A, Gibbings
- US 5840867 A
- US 5582981 A
- WO 2003050290 A
- US 6525154 B
- US 7332553 B
- US 7384589 B
- US 4683202 A
- US 4683195 A
- US 4800159 A
- US 4965188 A
- US 5333675 A
- US 6300070 B
- US 513300 A
- WO 8810315 A
- WO 9006995 A
- US 6410276 B
- US 4437975 A
- US 5413909 A
- US 5861245 A
- US 5409818 A
- US 5554517 A
- US 6063603 A
- US 5242794 A
- US 5494810 A
- US 4988617 A
- US 6582938 B
- US 5322770 A
- US 5871928 A
- US 5874219 A
- US 6045996 A
- US 6386749 B
- US 6391623 B
- US 66186796 B
- US 6379897 B
- US 6664377 B
- US 6451536 B
- US 548257 A
- US 20030157485 A
- US 20030215858 A
- WO 2009046397 A
- WO 2006033756 A
- US 20050239728 A
- US 20060035344 A
- US 20090062228 A
- US 20090317907 A
- US 20090298174 A
- US 20090291907 A
- US 20090291906 A
- US 20090286969 A
- US 20090236225 A
- US 20090221685 A
- US 20090203893 A
- US 20070049547 A
- US 20050261218 A
- US 20090275729 A
- US 20090043082 A
- US 20070287179 A
- US 20060212950 A
- US 20060166910 A
- US 20050227934 A
- US 20050222067 A
- US 20050221490 A
- US 20050221293 A
- US 20050182005 A
- US 20050059005 A
- WO 9402595 A
- US 7427605 B
- WO 0022113 A, Skillern, A.
- WO 0022114 A
- US 6054299 A
- US 6143520 A
- US 5665557 A
- US 5981276 A
- US 5436146 A
- US 5252479 A
- US 5139941 A
- WO 9413788 A
- WO 9324641 A
- US 6747014 B
- US 4837028 A

- WO 8804924 A, Allen
- US 5543152 A, Webb
- WO 9713499 A, Lim
- US 4426330 A
- US 4534899 A
- EP 0445131 B1
- WO 9004384 A
- US 5013556 A, Woodle
- US 5356633 A
- US 5213804 A, Martin
- EP 0496813 B1
- WO 9105545 A
- US 5225212 A, Martin
- WO 9420073 A, Zalipsky
- WO 9610391 A, Choi
- US 5540935 A, Miyazaki
- US 5556948 A, Tagawa
- WO 9640062 A, Thierry
- US 5264221 A, Tagawa
- US 5665710 A, Rahman
- WO 9704787 A, Love
- WO 0003683 A
- US 5976567 A
- US 5981501 A
- US 6534484 B
- US 6586410 B
- US 6815432 B
- WO 9640964 A
- US 61107998 B
- US 05623008 A
- WO 2008042973 A
- WO 2009127060 A
- US 61239686 B
- US 61244834 B
- US 61185800 B
- US 1028224 W
- US 0963933 W
- US 61175770 B
- US 1033777 W
- US 6887906 B
- US 20030027780 A
- US 6191105 B
- US 7063860 B
- US 7070802 B
- US 7157099 B
- US 5705188 A, Junichi
- WO 9730731 A, Lollo
- US 502158 A
- US 11657341 B
- US 20050107325 A
- WO 2004080406 A
- WO 2007021896 A
- US 6875748 B
- US 5763270 A
- US 5580859 A
- US 6040295 A
- US 6034072 A

**Non-patent literature cited in the description**


- **YANG, D. et al.** *Curr. Biol.,* 2000, vol. 10, 1191-1200
- **DIAS, N. et al.** *Mol Cancer Ther,* 2002, vol. 1, 347-355
- **SHARP et al.** *Genes Dev.,* 2001, vol. 15, 485
- **BERNSTEIN et al.** *Nature,* 2001, vol. 409, 363
- **NYKANEN et al.** *Cell,* 2001, vol. 107, 309
- **ELBASHIR et al.** *Genes Dev.,* 2001, vol. 15, 188
- **LI et al.** *Proc. Natl. Acad. Sci. U.S.A.,* 2006, vol. 103, 17337-42
- **CONDE-VANCELLS et al.** *J. Proteome Res.,* 2008, vol. 7, 5157-5166
- **RAPOSO et al.** *J Exp Med,* 1996, vol. 183, 1161-1172
- **TAYLOR ; GERCEL-TAYLOR.** *Gynecology Oncology,* 2008, vol. 110 (1), 13-21
- **CHERUVANKY et al.** *Am J Physiol Renal Physiol,* 2007, vol. 292 (5), F1657-61
- **AL-NEDAWI et al.** *Nat Cell Biol,* 2008, vol. 10, 619-624
- **NAGRATH et al.** *Nature,* 2007, vol. 450, 1235-9
- **GEISS et al.** *Nat Biotech,* 2008, vol. 26, 317-325
- **SAMBROOK et al.** Molecular Biology: A laboratory Approach. Cold Spring Harbor, 1989
- **AUSUBEL et al.** Current protocols in Molecular Biology. Greene Publishing, 1995
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **WU ; WALLACE.** *Genomics,* 1989, vol. 4, 560
- **LANDEGREN et al.** *Science,* 1988, vol. 241, 1077
- **BARRINGER et al.** *Gene,* 1990, vol. 89, 117
- **KWOH et al.** *Proc. Natl. Acad. Sci. USA,* 1989, vol. 86, 1173
- **GUATELLI et al.** *Proc. Nat. Acad. Sci. USA,* 1990, vol. 87, 1874
- **R. L. MARSHALL et al.** *PCR Methods and Applications,* 1994, vol. 4, 80-84
- **MANIATIS et al.** Molecular Cloning: A Laboratory Manual. Cold Spring Harbor, 1989

- Guide to Molecular Cloning Techniques. **BERGER ; KIMMEL.** Methods in Enzymology. Academic Press, Inc, 1987, vol. 152
- **YOUNG ; DAVISM.** *P.N.A.S,* 1983, vol. 80, 1194
- **SCHENA et al.** *Science,* 1995, vol. 20, 467-470
- **GERHOLD et al.** *Trends in Biochem. Sci.,* 1999, vol. 24, 168-173
- **LENNON et al.** *Drug discovery Today,* 2000, vol. 5, 59-65
- **ELBASHIR et al.** *EMBO,* 2001, vol. 20, 6877-88
- **ELBASHIR et al.** *EMBO,* 2001, vol. 20, 6877-6888
- Current protocols in nucleic acid chemistry. John Wiley & Sons, Inc,
- **SUBBARAO et al.** *Biochemistry,* 1987, vol. 26, 2964-2972
- **VOGEL et al.** *J. Am. Chem. Soc.,* 1996, vol. 118, 1581-1586
- **TURK et al.** *Biochem. Biophys. Acta,* 2002, vol. 1559, 56-68
- **LAM et al.** *Nature,* 1991, vol. 354, 82-84
- **ZITZMANN et al.** *Cancer Res.,* 2002, vol. 62, 5139-43
- **AOKI et al.** *Cancer Gene Therapy,* 2001, vol. 8, 783-787
- **HAUBNER et al.** *Jour. Nucl. Med.,* 2001, vol. 42, 326-336
- **SIMEONI et al.** *Nucl. Acids Res.,* 2003, vol. 31, 2717-2724
- **KUBO, T. et al.** *Biochem. Biophys. Res. Comm.,* 2007, vol. 365 (1), 54-61
- **LETSINGER et al.** *Proc. Natl. Acad. Sci. USA,* 1989, vol. 86, 6553
- **MANOHARAN et al.** *Bioorg. Med. Chem. Lett.,* 1994, vol. 4, 1053
- **MANOHARAN et al.** *Ann. N.Y. Acad. Sci.,* 1992, vol. 660, 306
- **MANOHARAN et al.** *Bioorg. Med. Chem. Let.,* 1993, vol. 3, 2765
- **OBERHAUSER et al.** *Nucl. Acids Res.,* 1992, vol. 20, 533
- **SAISON-BEHMOARAS et al.** *EMBO J.,* 1991, vol. 10, 111
- **KABANOV et al.** *FEBS Lett.,* 1990, vol. 259, 327
- **SVINARCHUK et al.** *Biochimie,* 1993, vol. 75, 49
- **MANOHARAN et al.** *Tetrahedron Lett.,* 1995, vol. 36, 3651
- **SHEA et al.** *Nucl. Acids Res.,* 1990, vol. 18, 3777
- **MANOHARAN et al.** *Nucleosides & Nucleotides,* 1995, vol. 14, 969
- **MISHRA et al.** *Biochim. Biophys. Acta,* 1995, vol. 1264, 229
- **CROOKE et al.** *J. Pharmacol. Exp. Ther.,* 1996, vol. 277, 923
- **AKHTAR S. ; JULIAN RL.** *Trends Cell. Biol.,* 1992, vol. 2 (5), 139-144
- **KIM SH et al.** *Journal of Controlled Release,* 2008, vol. 129 (2), 107-116
- **SORENSEN, DR et al.** *J. Mol. Biol,* 2003, vol. 327, 761-766
- **VERMA, UN et al.** *Clin. Cancer Res.,* 2003, vol. 9, 1291-1300
- **ARNOLD, AS et al.** *J. Hypertens.,* 2007, vol. 25, 197-205
- **ZIMMERMANN, TS et al.** *Nature,* 2006, vol. 441, 111-114
- **CHIEN, PY et al.** *Cancer Gene Ther.,* 2005, vol. 12, 321-328
- **PAL, A. et al.** *Int J. Oncol.,* 2005, vol. 26, 1087-1091
- **BONNET ME et al.** *Pharm. Res. Aug,* 2008, 16
- **AIGNER, A.** *J. Biomed. Biotechnol.,* 2006, 71659
- **LIU, S.** *Mol. Pharm.,* 2006, vol. 3, 472-487
- **TOMALIA, DA et al.** *Biochem. Soc. Trans.,* 2007, vol. 35, 61-67
- **YOO, H et al.** *Pharm. Res.,* 1999, vol. 16, 1799-1804
- **COUTURE, A et al.** *TIG,* 1996, vol. 12, 5-10
- **GASSMANN et al.** *Proc. Natl. Acad. Sci. USA,* 1995, vol. 92, 1292
- **DOCHERTY et al.** *FASEB J.,* 1994, vol. 8, 20-24
- **MILLER et al.** *Meth. Enzymol.,* 1993, vol. 217, 581-599
- **BOESEN et al.** *Biotherapy,* 1994, vol. 6, 291-302
- **CLOWES et al.** *J. Clin. Invest.,* 1994, vol. 93, 644-651
- **KIEM et al.** *Blood,* 1994, vol. 83, 1467-1473
- **SALMONS ; GUNZBERG.** *Human Gene Therapy,* 1993, vol. 4, 129-141
- **GROSSMAN ; WILSON.** *Curr. Opin. in Genetics and Devel.,* 1993, vol. 3, 110-114
- **WALSH et al.** *Proc. Soc. Exp. Biol. Med.,* 1993, vol. 204, 289-300
- **SAMULSKI R et al.** *J. Virol.,* 1987, vol. 61, 3096-3101
- **FISHER K J et al.** *J. Virol,* 1996, vol. 70, 520-532
- **SAMULSKI R et al.** *J. Virol.,* 1989, vol. 63, 3822-3826
- **RABINOWITZ J E et al.** *J Virol,* 2002, vol. 76, 791-801
- **WANG et al.** *Biochem. Biophys. Res. Commun.,* 1987, vol. 147, 980-985
- **ZHOU et al.** *Journal of Controlled Release,* 1992, vol. 19, 269-274
- **HU et al.** *S.T.P.Pharma. Sci.,* 1994, vol. 4 (6), 466

- **ALLEN et al.** *FEBS Letters,* 1987, vol. 223, 42
- **WU et al.** *Cancer Research,* 1993, vol. 53, 3765
- **PAPAHADJOPOULOS et al.** *Ann. N.Y. Acad. Sci.,* 1987, vol. 507, 64
- **GABIZON et al.** *Proc. Natl. Acad. Sci. U.S.A.,* 1988, vol. 85, 6949
- **SUNAMOTO et al.** *Bull. Chem. Soc. Jpn.,* 1980, vol. 53, 2778
- **ILLUM et al.** *FEBS Lett.,* 1984, vol. 167, 79
- **KLIBANOV et al.** *FEBS Lett.,* 1990, vol. 268, 235
- **BLUME et al.** *Biochimica et Biophysica Acta,* 1990, vol. 1029, 91
- **RIEGER.** Pharmaceutical Dosage Forms. Marcel Dekker, Inc, 1988, 285
- **ALLEN, LV ; POPOVICH NG ; ANSEL HC.** Ansel's Pharmaceutical Dosage Forms and Drug Delivery Systems. Lippincott Williams & Wilkins, 2004
- **IDSON.** Pharmaceutical Dosage Forms. Marcel Dekker, Inc, 1988, vol. 1, 199
- **ROSOFF.** Pharmaceutical Dosage Forms. Marcel Dekker, Inc, 1988, vol. 1, 245
- Block in Pharmaceutical Dosage Forms. Marcel Dekker, Inc, 1988, vol. 2, 335
- **HIGUCHI et al.** Remington's Pharmaceutical Sciences. Mack Publishing Co, 1985, 301
- **IDSON.** Pharmaceutical Dosage Forms. Marcel Dekker, Inc, 1988, vol. 1, 199
- **RIEGER.** Pharmaceutical Dosage Forms. Marcel Dekker, Inc, 1988, vol. 1, 285
- **IDSON.** Pharmaceutical Dosage Forms, Lieberman. Marcel Dekker, Inc, 1988, vol. 1, 199
- **BLOCK.** Pharmaceutical Dosage Forms. Marcel Dekker, Inc, 1988, vol. 1, 335
- **LEUNG ; SHAH.** Controlled Release of Drugs: Polymers and Aggregate Systems. VCH Publishers, 1989, 185-215
- **SCHOTT.** Remington's Pharmaceutical Sciences. Mack Publishing Co, 1985, 271
- **CONSTANTINIDES et al.** *Pharmaceutical Research,* 1994, vol. 11, 1385-1390
- **RITSCHEL.** *Meth. Find. Exp. Clin. Pharmacol.,* 1993, vol. 13, 205
- **CONSTANTINIDES et al.** *Pharmaceutical Research,* 1994, vol. 11, 1385
- **HO et al.** *J. Pharm. Sci.,* 1996, vol. 85, 138-143
- **LEE et al.** *Critical Reviews in Therapeutic Drug Carrier Systems,* 1991, 92
- **MALMSTEN, M.** Surfactants and polymers in drug delivery. Informa Health Care, 2002
- **TAKAHASHI et al.** *J. Pharm. Pharmacol.,* 1988, vol. 40, 252
- **TOUITOU, E. et al.** Enhancement in Drug Delivery. CRC Press, 2006
- **MURANISHI.** *Critical Reviews in Therapeutic Drug Carrier Systems,* 1990, vol. 7, 1-33
- **EL HARIRI et al.** *J. Pharm. Pharmacol.,* 1992, vol. 44, 651-654
- **BRUNTON et al.** Goodman & Gilman's The Pharmacological Basis of Therapeutics. McGraw-Hill, 1996, 934-935
- **SWINYARD.** Remington's Pharmaceutical Sciences. Mack Publishing Co, 1990, 782-783
- **YAMAMOTO.** *J. Pharm. Exp. Ther.,* 1992, vol. 263, 25
- **YAMASHITA et al.** *J. Pharm. Sci.,* 1990, vol. 79, 579-583
- **JARRETT.** *J. Chromatogr.,* 1993, vol. 618, 315-339
- **KATDARE, A. et al.** Excipient development for pharmaceutical, biotechnology, and drug delivery. CRC Press, 2006
- **BUUR et al.** *J. Control Rel.,* 1990, vol. 14, 43-51
- **YAMASHITA et al.** *J. Pharm. Pharmacol.,* 1987, vol. 39, 621-626
- **MIYAO et al.** *DsRNA Res. Dev.,* 1995, vol. 5, 115-121
- **TAKAKURA et al.** *DsRNA & Nucl. Acid Drug Dev.,* 1996, vol. 6, 177-183
- **KRUTZFELDT et al.** *Nature,* 2005, vol. 438, 685-689
- **LUXEMBOURG A. et al.** *Expert Opinion Biol. Ther.,* 2007, vol. 7 (11), 1647-1664
- **KESARAJU P et al.** *Mol. Ther.,* 2006, vol. 14 (3), 416-422
- **LUXEMBOURG et al.** *Vaccine,* 2006, vol. 24 (21), 4490-4493
- **DZAU et al.** *Trends in Biotechnology,* 1993, vol. 11 (5), 205-10